(19) Europäisches Patentamt
European Patent Office
Office européen des brevets

(11) Veröffentlichungsnummer: **0 251 330 B1**

(12) **EUROPÄISCHE PATENTSCHRIFT**

(45) Veröffentlichungstag der Patentschrift: **01.12.93**

(51) Int. Cl.[5]: **C07D 501/34**, A61K 31/545

(21) Anmeldenummer: **87109613.7**

(22) Anmeldetag: **03.07.87**

Die Akte enthält technische Angaben, die nach dem Eingang der Anmeldung eingereicht wurden und die nicht in dieser Patentschrift enthalten sind.

# (54) Cephalosporinderivate, Verfahren zu ihrer Herstellung sowie entsprechende pharmazeutische Präparate.

(30) Priorität: **03.07.86 US 881555**

(43) Veröffentlichungstag der Anmeldung:
**07.01.88 Patentblatt 88/01**

(45) Bekanntmachung des Hinweises auf die Patenterteilung:
**01.12.93 Patentblatt 93/48**

(84) Benannte Vertragsstaaten:
**AT BE CH DE ES FR GB GR IT LI LU NL SE**

(56) Entgegenhaltungen:
**DE-A- 2 362 553**
**DE-A- 3 031 767**
**DE-A- 3 106 013**
**FR-A- 2 501 209**

(73) Patentinhaber: **F. HOFFMANN-LA ROCHE AG**
**Postfach 3255**
**CH-4002 Basel(CH)**

(72) Erfinder: **Albrecht, Harry Allen**
**139 Waughaw Road**
**Towaco, N.J.(US)**
Erfinder: **Chan, Ka-Kong**
**Hopatcong, P.O. Box 641**
**Landing, N.J.(US)**
Erfinder: **Keith, Dennis Dalton**
**8 Mendl Terrace**
**Montclair, N.J.(US)**
Erfinder: **Then, Rudolf Ludwig**
**Schutzackerstrasse39**
**Weil am Rhein(DE)**
Erfinder: **Weigele, Manfred**
**4 Andover Drive**
**North Caldwell, N.J.(US)**

(74) Vertreter: **Lederer, Franz, Dr. et al**
**Lederer, Keller & Riederer,**
**Patentanwälte,**
**Lucile-Grahn-Strasse 22**
**D-81675 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft Acylderivate der allgemeinen Formel

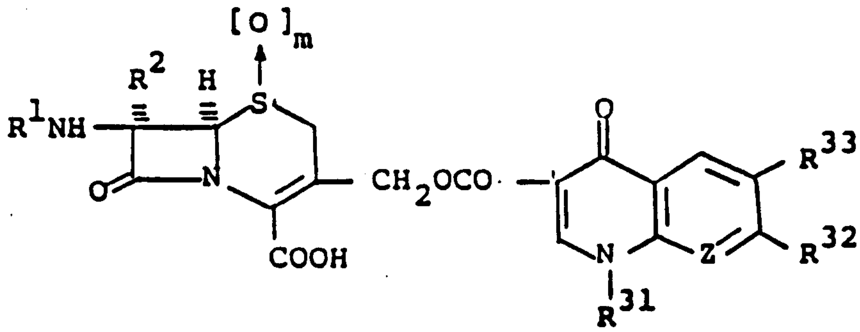

I

in der m 0, 1 oder 2, $R^1$ Wasserstoff oder eine Acylgruppe, $R^2$ Wasserstoff $C_1$-$C_8$- Alkoxy, Amino, $C_1$-$C_8$-Alkylthio oder $C_1$-$C_7$- Alkanoylamino, $R^{31}$ Wasserstoff, $C_1$-$C_8$- Alkyl, $C_2$-$C_6$- Alkenyl, $C_3$-$C_7$-Cycloalkyl, Halogen- $C_1$-$C_8$- Alkyl oder Halogenphenyl; Z $R^{30}$-C oder Stickstoff, $R^{30}$ Wasserstoff oder Halogen, oder $R^{30}$ und $R^{31}$ zusammen eine $C_3$-$C_5$-Alkylengruppe, eine $C_2$-$C_4$-Alkylenmonooxygruppe oder eine $C_1$-$C_2$-Alkylendioxygruppe, $R^{32}$ Wasserstoff, Halogen, $C_1$-$C_8$- Alkyl oder einen gegebenenfalls durch $C_1$-$C_8$- Alkyl, $C_1$-$C_8$- Alkoxy, Halogen, Halogen- $C_1$-$C_8$- Alkyl, Amino, Mercapto, Hydroxy, Carbamoyl oder Carboxy substituierten 5- oder 6-gliedrigen heterocyclischen Ring mit einem, zwei oder drei Sauerstoff-, Stickstoff- und/oder Schwefelatomen und $R^{33}$ Wasserstoff oder Halogen oder $R^{32}$ und $R^{33}$ zusammen eine $C_1$-$C_4$-Alkylendioxygruppe darstellen,
sowie leicht hydrolysierbare Ester und Salze dieser Verbindungen und Hydrate der Verbindungen der Formel I bzw. von deren Estern und Salzen.

Der Ausdruck "$C_1$-$C_8$-Alkyl" bezieht sich auf gerad- und verzweigtkettige Kohlenwasserstoffgupen mit 1-8, bevorzugt mit 1-4 Kohlenstoffatomen, z.B. Methyl, Aethyl, n-Propyl, Isopropyl, oder t-Butyl.

Der Ausdruck "$C_1$-$C_8$-Alkoxy" bezieht sich auf gerad- oder verzweigtkettige Kohlenwasserstoffoxygruppen, worin der Alkylrest die oben gegebene Bedeutung hat. Beispiele sind Methoxy, Aethoxy oder n-Propoxy.

Der Ausdruck "Halogen" bezieht sich auf Chlor, Brom, Jod oder Fluor, sofern nicht anderweitig definiert.

Der Ausdruck "$C_1$-$C_7$-Alkanoyl" bezeichnet einen Rest der allgemeinen Formel

$R^{25}$-CO-

in der $R^{25}$ Wasserstoff oder $C_1$-$C_6$ Alkyl darstellt, Beispiele dieser Gruppen sind Acetyl, Formyl, Propionyl oder n-Butyl.

Der Ausdruck "substituiertes Phenyl" bedeutet eine mono- oder disubstituierte Phenylgruppe, wobei als Substituenten Halogen (z.B. Chlor, Brom, Fluor), $C_1$-$C_8$-Alkyl, Amino, Nitro oder Trifluormethyl in Frage kommen.

Der Ausdruck "substituiertes Alkyl" bedeutet eine $C_1$-$C_8$-Alkylgruppe, die z.B. durch Halogen (z.B. Chlor, Fluor, Brom), Trifluormethyl, Amino oder Cyan substituiert sein kann.

Der Ausdruck "$C_2$-$C_6$-Alkenyl" bedeutet gerad- oder verzweigkettige Kohlenwasserstoffgruppen mit einer olefinischen Doppelbindung und 2-6 Kohlenstoffatomen, d.h. den Rest von Verbndungen der Formel $C_nH_{2n}$, worin n 2-6 ist, z.B. Allyl oder Vinyl.

Der Ausdruck "5-, 6 oder 7-gliedriger heterocyclischer Ring mit 1-4 Sauerstoff-, Stickstoff- und/oder Schwefelatomen" bedeutet z.B. die folgenden Gruppen: 6-gliedrige, Stickstoff-enthaltende heterocyclische Ringe, wie Pyridyl, Piperidyl, Piperidino, N-oxido-pyridyl, Pyrimidyl, Piperazinyl, Pyridazinyl, N-oxidopyridazinyl, etc.; 5-gliedrige Stickstoff-enthaltende heterocyclische Ringe, z.B. Pyrrolidinyl, Pyrazolyl, Imidazolyl, Thiazolyl, 1,2,3-Thiadiazolyl, 1,2,4-Thiadiazolyl, 1,3,4-Thiadiazolyl, 1,2,3-Oxadiazolyl, 1,2,4-Oxadiazolyl, 1,3,4-Oxadiazolyl, 1,2,5-Oxadiazolyl, 1,2,3-Triazolyl, 1,2,4-Triazolyl, 1H-Tetrazolyl oder 2H-Tetrazolyl. Diese heterocyclischen Ringe können weiter substituiert sein, wobei z.B. folgende Substituenten in Frage kommen: $C_1$-$C_8$-Alkyl wie Methyl, Aethyl oder n-Propyl, niederes Alkoxy, z.B. Methoxy oder Aethoxy, Halogen wie Chlor oder Brom, Halogen-$C_1$-$C_8$- Alkyl wie Trifluormethyl oder Trichloräthyl, Amino, Mercapto,

Hydroxy, Carbamoyl oder Carboxy.

Der Ausdruck "$C_3$-$C_7$-Cycloalkyl" bedeutet einen 3-7-gliedrigen gesättigten carboxyclischen Rest, z.B. Cyclopropyl, Cyclobutyl oder Cyclohexyl.

$R^{30}$ und $R^{31}$ können zusammen $C_3$-$C_5$-Alkylen bedeuten, z.B. $-(CH_2)_3-$, $-(CH_2)_4-$, $-(CH_2)_5-$ oder -CH-($CH_3$)-$(CH_2)_2$-, oder auch $C_2$-$C_4$-Alkylenmonooxy, z.B. $-(CH_2)_2-O-$, $-(CH_2)_3-O-$, $-(CH_2)_4-O-$ oder -CH($CH_3$)-$CH_2$-O-; oder auch $C_1$-$C_2$-Alkylendioxy, z.B. -O-$CH_2$-O-, -O-$(CH_2)_2$-O- oder -O-CH($CH_3$)-O-. Vorzugsweise wird ein 5- oder 6-gliedriger kondensierter Ring gebildet. $R^{32}$ und $R^{33}$ können zusammen $C_1$-$C_4$-Alkylendioxy bedeuten, z.B. -O-$CH_2$-O-, -O-$(CH_2)_2$-O-, -O-$(CH_2)_3$-O-, -O-$(CH_2)_4$-O-, -O-CH($CH_3$)-O- oder -O-CH($CH_3$)-CH($CH_3$)-O-. In diesem Falle wird vorzugsweise ein 5- oder 6-gliedriger kondensierter Ring gebildet.

Der Ausdruck "Acyl" (Substituent $R^1$) steht für alle organischen Reste, welche durch Entfernung der Hydroxygruppe aus einer Carbonsäure gebildet werden. Gewisse Acylreste sind bevorzugt: Beispiele solcher bevorzugten Acylgruppen sind diejenigen, welche bisher zur Acylierung von $\beta$-Lactamantibiotika, einschliesslich der 6-Aminopenicillansäure und deren Derivaten, sowie der 7-Aminocephalosporansäure und deren Derivaten, verwendet worden sind; siehe z.B. Cephalosporins and Penicillins, Verlag Flynn, Academic Press (1972), Belgische Patentschrift 866,038, veröffentlicht am 17, Oktober 1978, Belgische Patentschrift 867,994, veröffentlicht am 11. Dezember 1978, U.S. Patentschrift 4,152,432, veröffentlicht am 1. Mai 1979, U.S. Patentschrift 3,971,778, veröffentlicht am 27. Juli 1976 und U.S. Patentschrift 4,173,199, veröffentlicht am 23. Oktober 1979. In diesen Publikationen werden verschiedene Acylreste, die im vorliegenden Falle Verwendung finden, erwähnt. Die nachfolgend aufgeführten Acylreste veranschaulichen weiter den Begriff Acyl. Beispiele für Acylreste sind:

a) aliphatische Gruppen der Formel

$R^5$-CO-

in der $R^5$ $C_1$-$C_8$-Alkyl, $C_3$-$C_7$-Cycloalkyl, $C_1$-$C_8$-Alkoxy, $C_2$-$C_6$-Alkylen, $C_3$-$C_7$-Cycloalkenyl oder Cyclohexadienyl; oder durch einen oder mehrere der Reste Halogen, Cyan, Nitro, Amino, Mercapto, $C_1$-$C_8$-Alkylthio oder Cyanmethylthio substituiertes $C_1$-$C_8$-Alkyl oder $C_2$-$C_6$-Alkenyl darstellen.

(b) eine carbocyclische aromatische Gruppe der allgemeinen Formeln

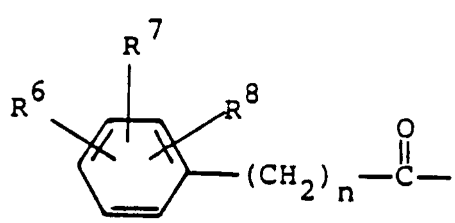

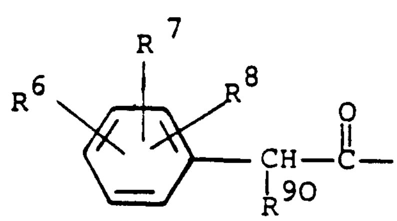

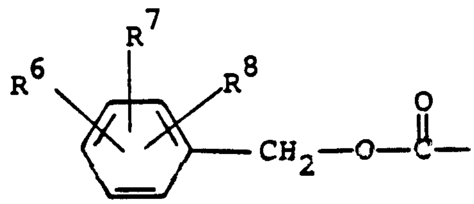

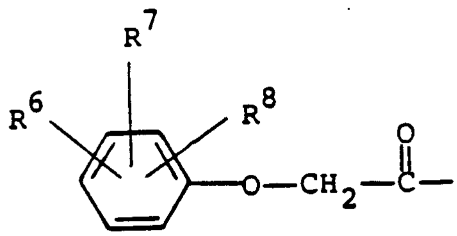

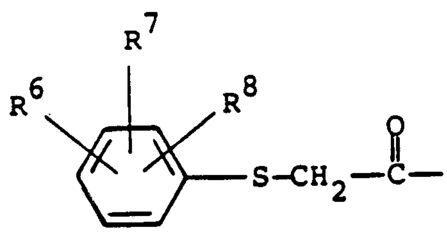

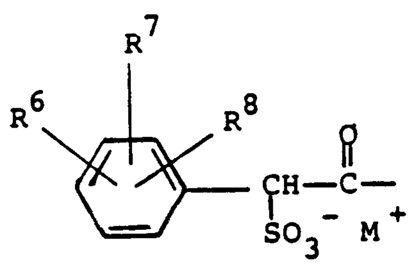

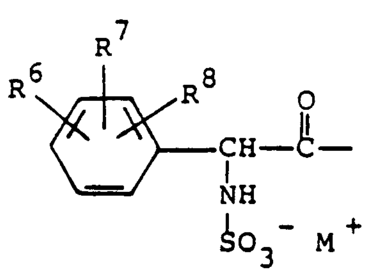

in der n 0, 1, 2 oder 3; $R^6$, $R^7$ und $R^8$ je Wasserstoff, Halogen, Hydroxy, Nitro, Amino, Cyano, Trifluormethyl, $C_1$-$C_4$-Alkyl, $C_1$-$C_4$-Alkoxy oder Aminomethyl, $R^{90}$ Amino, Hydroxy, ein Carboxysalz, geschütztes Carboxy, wie z.B. Benzyloxycarbonyl, Formyloxy oder Azido und M ein Kation darstellen.

Bevorzugte carbocyclische aromatische Acylgruppen sind diejenigen der allgemeinen Formel

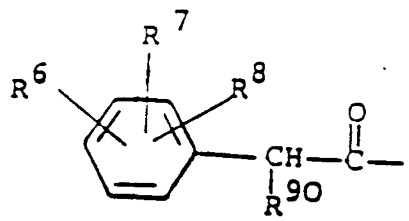

in der $R^{90}$ Amino, Hydroxy, ein Carboxysalz, Benzyloxycarbonyl, Formyloxy oder Azido und $R^6$, $R^7$ und $R^8$ Wasserstoff, Halogen, Hydroxy, Nitro, Amino, Cyan, Trifluormethyl, $C_1$-$C_4$-Alkyl, $C_1$-$C_4$-Alkoxy oder Aminomethyl darstellen, ebenfalls solche worin $R^6$, $R^7$ und $R^8$ Wasserstoff und $R^{90}$ Wasserstoff oder Hydroxy darstellen,
sowie diejenigen der Formeln

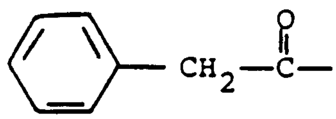

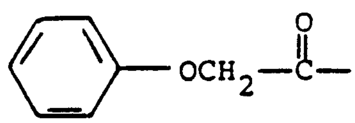

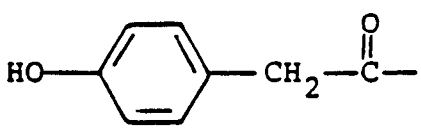

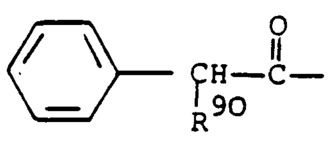

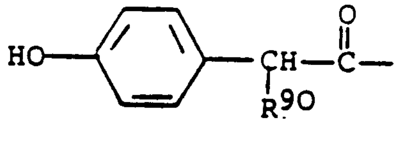

$R^{90}$ ist bevorzugt eine Aminogruppe, eine Hydroxygruppe oder ein Carboxy- oder Sulfosalz.

Beispiele anderer erfindungsgemäss einsetzbarer Acylgruppen sind:
Sulfophenylacetyl,
Hydroxysulfonyloxyphenylacetyl,
Sulfamoylphenylacetyl,
(Phenoxycarbonyl)phenylacetyl,
(p-Tolyloxycarbonyl)phenylacetyl,
Formyloxyphenylacetyl,
Carboxyphenylacetyl,
Formylaminophenylacetyl,
Benzyloxycarbonylphenylacetyl,
2-(N,N-Dimethylsulfamoyl)-2-phenylacetyl oder
2-Brom-2-thienylacetyl.

c) Heteroaromatische Gruppen der allgemeinen Formeln

$R^{101}-(CH_2)_n-CO-$

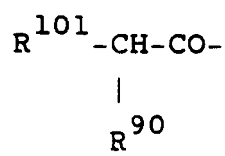

$R^{101}-O-CH_2-CO-$
$R^{101}-S-CH_2-CO-$

or

$R^{101}-CO-CO-$

in der $R^{90}$ die oben gegebene Bedeutung hat, n 0, 1, 2 oder 3 und $R^{101}$ einen gegebenenfalls durch $C_1$-$C_8$- Alkyl, $C_1$-$C_8$- Alkoxy, Halogen, Halogen- $C_1$-$C_8$- Alkyl, Amino, Mercapto, Hydroxy, Carbamoyl oder Carboxy substituierten 5-, 6- oder 7-gliedrigen heterocyclischen Ring mit 1, 2, 3 oder 4 (vorzugsweise 1 oder 2) Stickstoff-, Sauerstoff- und/oder Schwefelatomen darstellt.

Beispiele heterocyclischer Ringe sind Thienyl, Furyl, Pyrrolyl, Pyridinyl, Pyrazinyl, Thiazolyl, Pyrimidinyl und Tetrazolyl. Beispiele für Substituenten am heterocyclischen Ring sind Halogen, Hydroxy, Nitro, Amino, Cyan, Trifluormethyl, $C_1$-$C_4$-Alkyl sowie $C_1$-$C_4$-Alkoxy.

Bevorzugte heteroaromatische Acylgruppen sind diejenigen, worin $R^{101}$ 2-Amino-4-thiazolyl, 2-Amino-5-halogen-4-thiazolyl, 4-Aminopyridin-2-yl, 2-Amino-1,3,4-thiadiazol-5-yl, 2-Thienyl, 2-Furanyl, 4-Pyridinyl oder 2,6-Dichlor-4-pyridinyl bedeutet.

d) [[4-Substituierte-2,3-dioxo -1-piperazinyl)carbonyl]amino]arylacetylgruppen der Formel

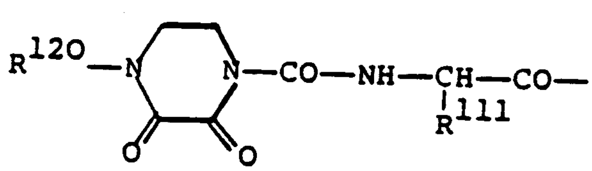

in der $R^{111}$ $C_1$-$C_8$-Alkyl, Hydroxy-$C_1$-$C_8$-Alkyl oder eine aromatische Gruppe der allgemeinen Formel

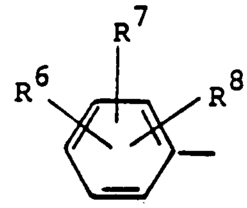

(in der $R^6$, $R^7$, und $R^8$ die obige Bedeutung haben) oder einen wie für $R^{101}$ definierten heterocyclischen Ring und $R^{120}$ gegebenenfalls durch einen oder mehreren der Reste Halogen, Cyan, Nitro, Amino und/oder Mercapto substituiertes $C_1$-$C_8$-Alkyl darstellen, z.B. 4-($C_1$-$C_8$-Alkyl) (vorzugsweise Aethyl oder Methyl)-2,3-dioxo-1-piperazincarbonyl-D-phenylglycyl.

e) (Substituierte Oxyimino)arylacetylgruppen der allgemeinen Formel

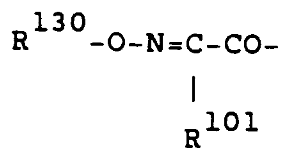

in der $R^{101}$ die obige Bedeutung hat und $R^{130}$ Wasserstoff, $C_1$-$C_8$-Alkyl, $C_3$-$C_7$-Cycloalkyl, Carboxy-$C_3$-$C_7$-cycloalkyl oder substituiertes $C_1$-$C_8$- Alkyl darstellt, wobei die $C_1$-$C_8$- Alkylgruppe substituiert ist durch einen oder mehrere der Reste Halogen, Cyan, Nitro, Amino, Mercapto, $C_1$-$C_8$- Alkylthio, eine durch $R^{111}$ oben definierte aromatische Gruppe, Carboxy (einschliesslich dessen Salze), $C_1$-$C_7$-Alkanoylamino, $C_2$-$C_9$- Alkoxycarbonyl, Phenylmethoxycarbonyl, Diphenylmethoxycarbonyl, Hydroxy( $C_1$-$C_8$-Alkoxy)phosphinyl, Dihydroxyphosphinyl, Hydroxy(phenylmethoxy)phosphinyl oder di($C_1$-$C_8$-Alkoxy)-phosphinyl.

Beispiele für Reste

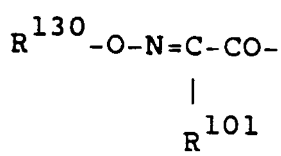

sind:
2-[(2-Chloracetamidothiazol-4-yl)]-2-[(p-nitrobenzyloxycarbonyl)methoxyimino]acetyl, 2-(2-Chloracetamidothiazol-4-yl)-2-methoxyiminoacetyl, 2-(2-Aminothiazol-4-yl)-2-isopropoxyiminoacetyl, 2-(2-Aminothiazol-4-yl)-2-methoxyiminoacetyl, 2-(2-Aminothiazol-4-yl)-2-hydroxyiminoacetyl, 2-Thienyl-2-methoxyiminoacetyl, 2-Furyl-2-methoxyiminoacetyl, 2-(4-Hydroxyphenyl)-2-methoxyiminoacetyl,2-Phenyl-2-methoxyiminoacetyl, 2-Phenyl-2-hydroxyiminoacetyl, 2-Thienyl-2-hydroxyiminoacetyl, 2-Thienyl-2-(dichloracetoxyimino)acetyl, 2-[4-($\gamma$-D-Glutamyloxy)phenyl]-2-hydroxyiminoacetyl, 2-[4-(3-Amino-3-carboxypropoxy)phenyl]-2-hydroxyiminoacetyl, 2-(5-Chlor-2-chloracetamidothiazol-4-yl) -2-methoxyiminoacetyl, 2-(5-Chlor-2-aminothiazol-4-yl)-2-methoxyiminoacetyl, 2-[2-(t-Butoxycarbonyl)isopropoxyimino]-2-(2-sulfoamino -thiazol-4-yl)acetyl, 2-[2-(t-Butoxycarbonyl)isopropoxyimino]-2 -(2-triphenyl-methylaminothiazol-4-yl)acetyl, 2-(2-Chloracetamidothiazol-4-yl)-2-isopropoxyiminoacetyl, 2-Methoxyimino-2-(2-sulfoaminothiazol-4-yl)acetyl, 2-(2-Aminothiazol-4-yl)-2-(carboxymethoxyimino)acetyl, 2-(2-Mesylaminothiazol-4-yl)-2-isopropoxyiminoacetyl, 2-(2-Imino-3-mesyl-4-thiazolin-4-yl)-2-isopropoxyiminoacetyl oder 2-(2-Aminothiazol-4-yl)-2-(carboxyisopropoxyimino)acetyl.

f) (Acylamino)arylacetylgruppen der allgemeinen Formel

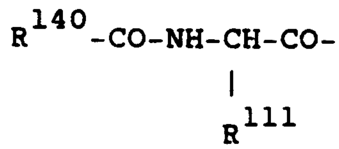

in der $R^{111}$ die obige Bedeutung hat und $R^{140}$ eine Gruppe der allgemeinen Formel

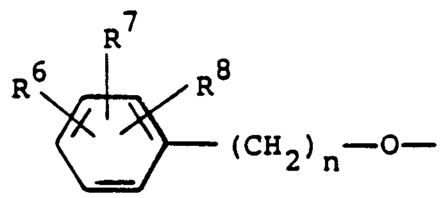

(worin $R^6$, $R^7$, $R^8$ und n die obige Bedeutung haben), Wasserstoff, $C_1$-$C_8$-Alkyl, substituiertes $C_1$-$C_8$-Alkyl, Amino, $C_1$-$C_8$-Alkylamino oder $C_1$-$C_8$-Cyanalkylamino darstellt.

Bevorzugte (Acylamino)arylacetylgruppen der obigen Formel sind diejenigen, worin $R^{140}$ Amino darstellt. Ebenfalls bevorzugt sind diejenigen Gruppen, worin $R^{111}$ Phenyl oder 2-Thienyl darstellt.

g) [[[3-Substituierte-2-oxo -1-imidazolidinyl]carbonyl]amino]arylacetylgruppen der allgemeinen Formel

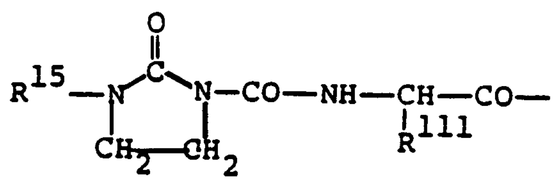

in der $R^{111}$ die oben gegebene Bedeutung hat, und $R^{15}$ Wasserstoff, $C_1$-$C_8$- Alkylsulfonyl, -N = CH-$R^{111}$ (worin $R^{111}$ die oben gegebene Bedeutung hat), $R^{16}$-CO- (worin $R^{16}$ Wasserstoff oder gegebenenfalls durch Halogen substituiertes $C_1$-$C_8$-Alkyl darstellt), eine wie unter $R^{111}$ oben definierte aromatische Gruppe oder gegebenenfalls durch einen oder mehrere der Reste Halogen, Cyan, Nitro, Amino und/oder Mercapto substituiertes $C_1$-$C_8$-Alkyl darstellt.

Bevorzugte [[[3-substituierte-2-oxo -1-imidazolidinyl]carbonyl]amino]arylacetylgruppen der obigen Formel sind diejenigen, worin $R^{111}$ Phenyl oder 2-Thienyl darstellt. Ebenfalls bevorzugt sind diejenigen Gruppen, worin $R^{15}$ Wasserstoff, Methylsulfonyl, Phenylmethylenamino oder 2-Furylmethylenamino darstellt.

In einer bevorzugten Ausführungsform des Chinolonyl- bzw. Azachinolonylsubstituenten in der 3-Stellung ist Z $R^{30}$-C, worin $R^{30}$ Wasserstoff, Chlor oder Fluor, vorzugsweise Wasserstoff oder Fluor, darstellt;

$R^{31}$ ist vorzugsweise $C_1$-$C_8$- Alkyl, bevorzugt Aethyl, oder Halogen- $C_1$-$C_8$- Alkyl, insbesondere Fluoräthyl, oder $C_3$-$C_7$-Cycloalkyl, vorzugsweise Cyclopropyl;

$R^{32}$ ist vorzugsweise $C_1$-$C_8$- Alkyl, insbesondere Methyl, oder Piperazinyl, welches am Stickstoffatom in 4-Stellung durch $C_1$-$C_8$- Alkyl, vorzugsweise durch Methyl substituiert sein kann;

$R^{33}$ ist vorzugsweise Wasserstoff, Chlor oder Fluor, insbesondere Wasserstoff oder Fluor, ganz besonders Fluor.

Der Chinolonyl- bzw. Azachinolonylsubstituent in 3-Stellung schliesst unter anderem Reste der folgenden Formeln ein:

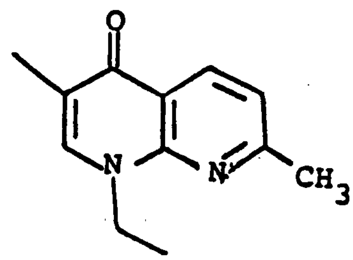

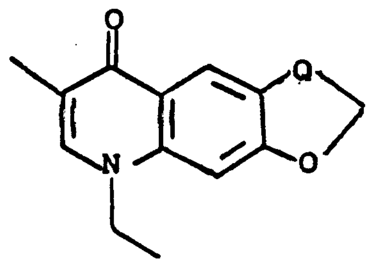

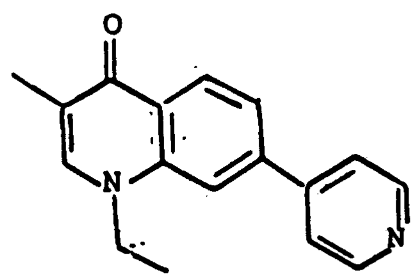

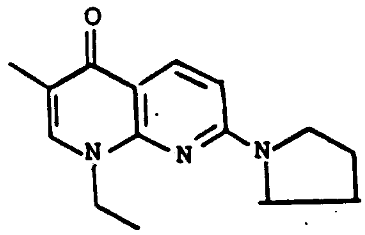

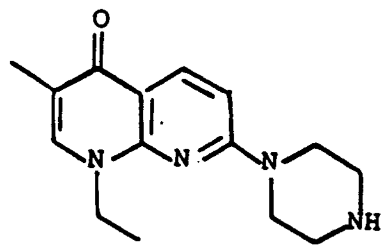

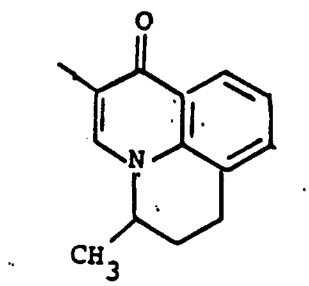

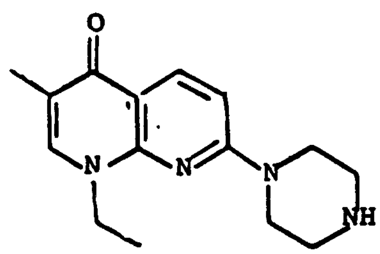

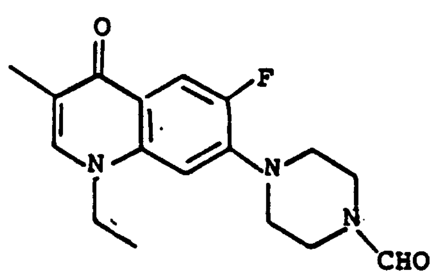

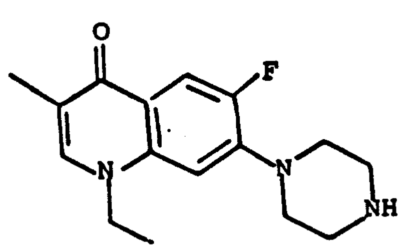

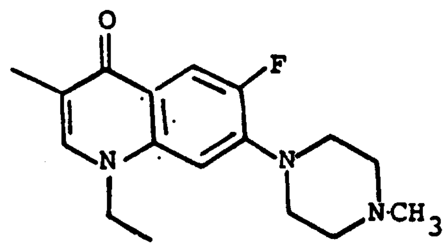

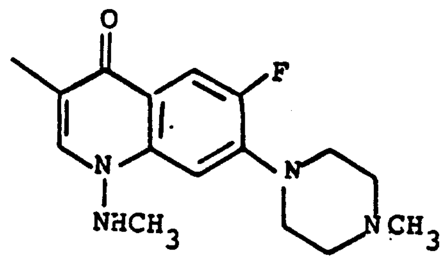

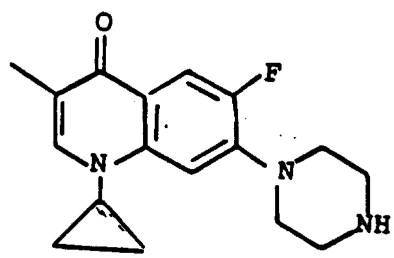

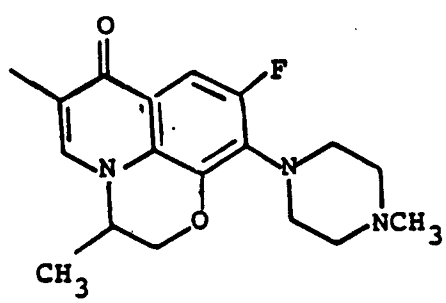

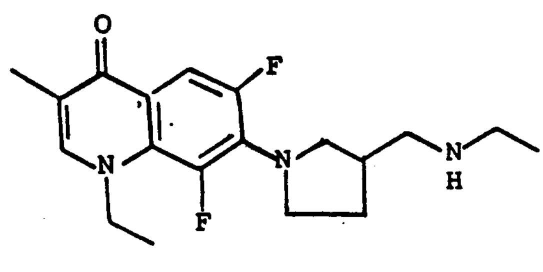

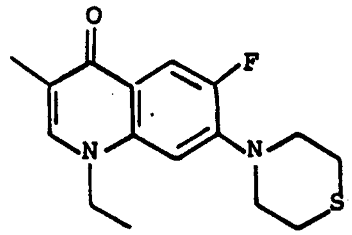

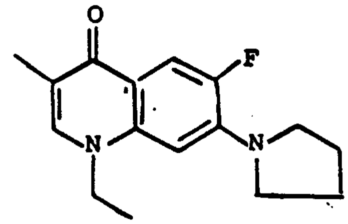

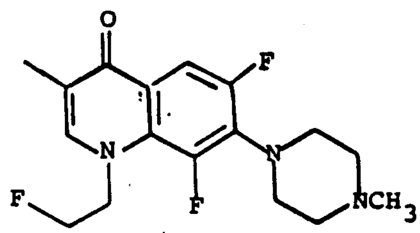

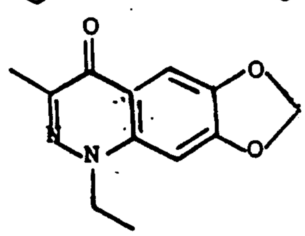

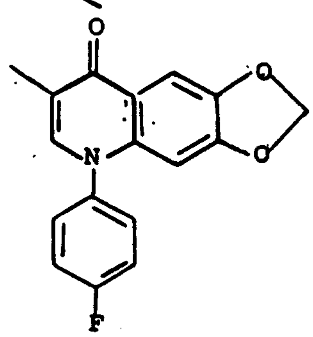

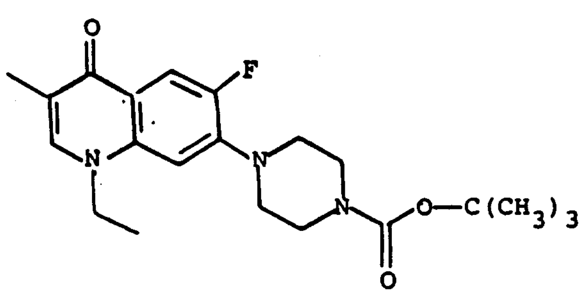

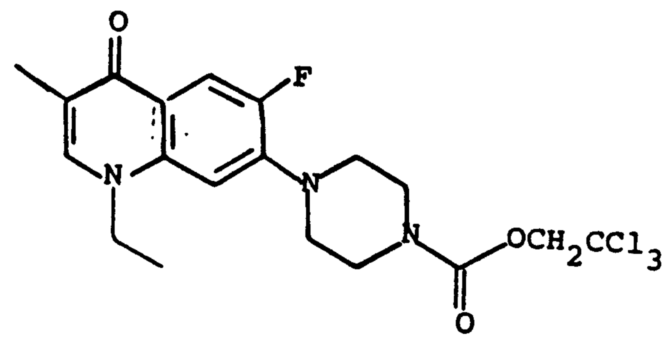

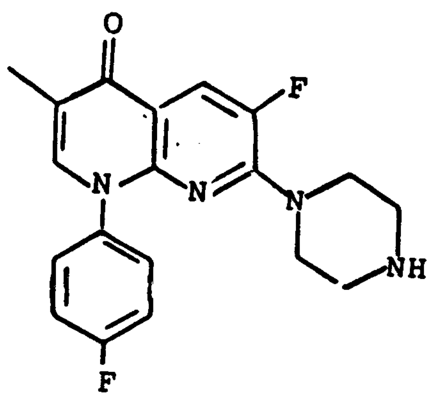

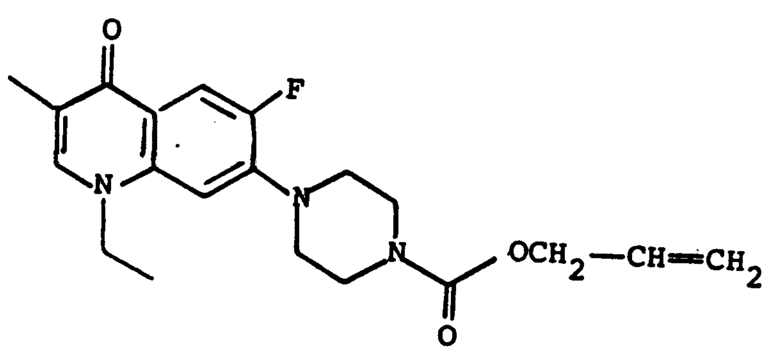

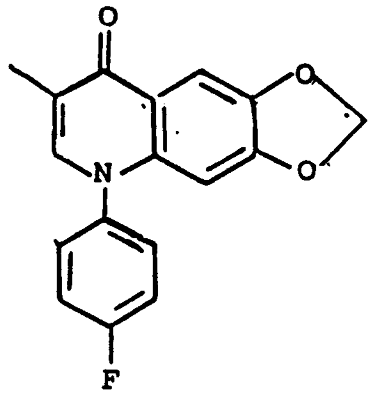

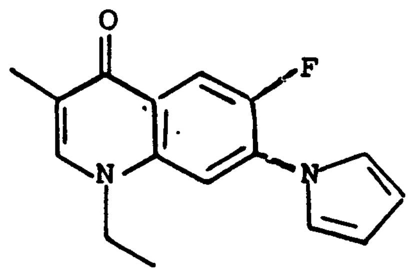

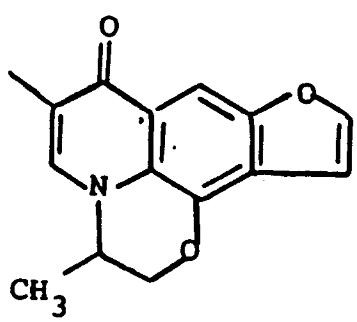

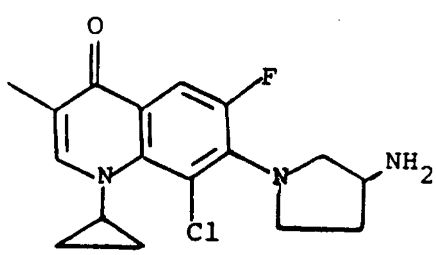

Bevorzugte Verbindungen der Formel I sind diejenigen worin m, O und $R^2$ Wasserstoff darstellt.Weitere, bevorzugte Verbindungen der Formel I sind diejenigen worin Z $R^{30}$-C darstellt, wobei $R^{30}$ Wasserstoff oder Halogen, $R^{31}$ $C_1$-$C_8$-Alkyl, Halogen-$C_1$-$C_8$-Alkyl oder $C_3$-$C_7$-Cycloalkyl, $R^{32}$ $C_1$-$C_8$-Alkyl, Piperazinyl oder ($C_1$-$C_8$-Alkyl)-Piperazinyl und $R^{33}$ Wasserstoff oder Halogen darstellen. Insbesondere sind $R^{30}$ Wasserstoff oder Fluor, $R^{31}$ Aethyl, Fluoräthyl oder Cyclopropyl, $R^{32}$ Piperazinyl oder 4-Methylpiperazinyl und $R^{33}$ Wasserstoff oder Fluor.

Eine bevorzugte Klasse der erfindungsgemässen Verbindungen ist diejenige der allgemeinen Formel

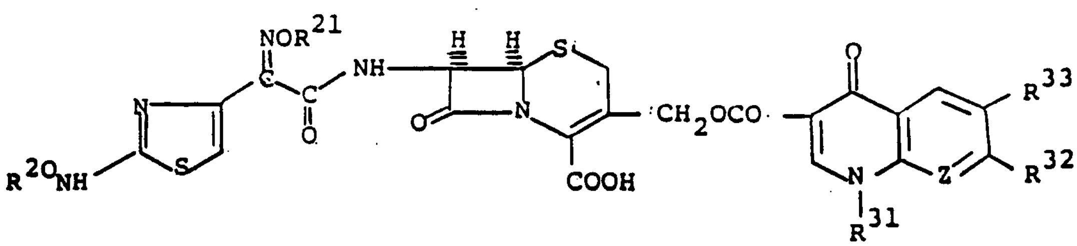

Ib

in der $R^{31}$, $R^{32}$, $R^{33}$ und Z die oben gegebene Bedeutung haben, $R^{20}$ eine Aminoschutzgruppe, z.B. Trityl oder Chloracetyl, oder, vorzugsweise, Wasserstoff und $R^{21}$ Wasserstoff, $C_1$-$C_8$-Alkyl oder eine Gruppe der allgemeinen Formel

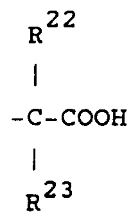

darstellt,

worin $R^{22}$ und $R^{23}$ Wasserstoff oder $C_1$-$C_8$- Alkyl oder $R^{22}$ und $R^{23}$ zusammen mit dem Kohlenstoffatom, an das sie gebunden sind, einen 3-7-gliedrigen carbocyclischen Ring, z.B. Cyclopropyl, Cyclobutyl oder Cyclopentyl, darstellen.

Besonders bevorzugt sind Verbindungen der Formel Ib, worin $R^{20}$ Wasserstoff, $R^{21}$ Methyl oder eine Gruppe der allgemeinen Formel

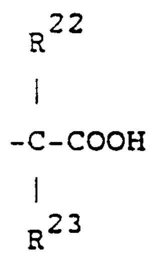

$R^{22}$ und $R^{23}$ Wasserstoff oder Methyl, Z die Gruppe $R^{30}$-C, $R^{30}$ Wasserstoff oder Halogen, $R^{31}$ $C_1$-$C_8$-Alkyl, Halogen- $C_1$-$C_8$- Alkyl oder $C_3$ -$C_7$-Cycloalkyl, $R^{32}$ $C_1$-$C_8$- Alkyl, Piperazinyl oder ($C_1$-$C_8$-Alkyl)-Piperazinyl und $R^{33}$ Wasserstoff oder Halogen darstellen. Insbesondere sind $R^{30}$ Wasserstoff oder Fluor, $R^{31}$ Aethyl, Fluoräthyl oder Cyclopropyl, $R^{32}$ Methyl, 4-Methyl-piperazinyl oder Piperazinyl und $R^{33}$ Wasserstoff oder Fluor.Weitere bevorzugte Ausführungsformen der Verbindungen der Formel I sind:

1)[6R-[6-alpha,7-beta(Z)]]-7-[[(2-amino -4-thiazolyl)($R^{21}$-oxyimino)acetyl]amino] -3-[[[[6-$R^{33}$,8-$R^{30}$, 1-$R^{31}$-1,4-dihydro -7-(4-$R^{34}$-1-piperazinyl) -4-oxo-3-chinolinyl]carbonyl]oxy]methyl] -8-oxo-5-thia-1-azabicyclo-[4.2.0]oct -2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, $C_1$-$C_8$- Alkyl oder 2-Halogen- $C_1$-$C_8$- Alkyl, $R^{34}$

Wasserstoff oder $C_1$-$C_8$-Alkyl und $R^{21}$ Wasserstoff, $C_1$-$C_8$- Alkyl oder eine Gruppe der allgemeinen Formel

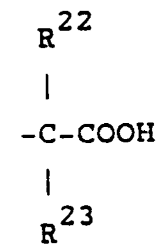

und $R^{22}$ und $R^{23}$ Wasserstoff oder $C_1$-$C_8$-Alkyl bedeuten. Insbesondere sind $R^{21}$ Methyl, $R^{33}$ und $R^{30}$ Wasserstoff oder Fluor, $R^{31}$ Aethyl oder 2-Fluoräthyl und $R^{34}$ Methyl, $R^{33}$ und $R^{30}$ Fluor und $R^{31}$ 2-Fluoräthyl, $R^{33}$ Fluor, $R^{30}$ Wasserstoff, $R^{31}$ 2-Fluoräthyl und $R^{34}$ Methyl, $R^{33}$ Fluor, $R^{30}$ Wasserstoff, $R^{31}$ Aethyl und $R^{34}$ Methyl, $R^{33}$ Fluor, $R^{31}$ 2-Fluoräthyl, $R^{30}$ Wasserstoff, und $R^{34}$ Methyl.

2)[6R-[6-alpha,7-beta]]-3-[[[[6-$R^{33}$,8-$R^{30}$, 1-$R^{31}$-1,4-dihydro -7-(4-$R^{34}$-1-piperazinyl)-4-oxo -3-chinolinyl]-carbonyl]oxy]methyl] -8-oxo-7-[(phenoxyacetyl)amino] -5-thia-1-azabicyclo[4.2.0]oct-2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, $C_1$-$C_8$-Alkyl oder 2-Halogen- $C_1$-$C_8$- Alkyl und $R^{34}$ Wasserstoff oder $C_1$-$C_8$-Alkyl darstellen. Insbesondere sind $R^{33}$ und $R^{30}$ Wasserstoff oder Fluor, $R^{31}$ Aethyl oder 2-Fluoräthyl und $R^{34}$ Methyl, $R^{33}$ und $R^{30}$ Fluor und $R^{31}$ 2-Fluoräthyl, $R^{33}$ Fluor, $R^{30}$ Wasserstoff, $R^{31}$ 2-Fluoräthyl und $R^{34}$ Methyl, $R^{33}$ Fluor, $R^{30}$ Wasserstoff, $R^{31}$ Aethyl und $R^{34}$ Methyl, $R^{33}$ Fluor, $R^{31}$ 2-Fluoräthyl, $R^{30}$ Wasserstoff, und $R^{34}$ Methyl.

3)[6R-[6-alpha,7-beta]] -7-(formylamino) -3-[[[[6-$R^{33}$,8-$R^{30}$, 1-$R^{31}$-1,4-dihydro -7-(4-$R^{34}$-1-piperazinyl)-4-oxo -3-chinolinyl]carbonyl]oxy]methyl] -8-oxo-5-thia-1-azabicyclo[4.2.0]oct-2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, $C_1$-$C_8$- Alkyl oder 2-Halogen- $C_1$-$C_8$- Alkyl und $R^{34}$ Wasserstoff oder $C_1$-$C_8$-Alkyl darstellen. Insbesondere sind $R^{33}$ und $R^{30}$ Wasserstoff oder Fluor, $R^{31}$ Aethyl oder 2-Fluoräthyl und $R^{34}$ Methyl, $R^{33}$ und $R^{30}$ Fluor und $R^{31}$ 2-Fluoräthyl, $R^{33}$ Fluor, $R^{30}$ Wasserstoff, $R^{31}$ 2-Fluoräthyl und $R^{34}$ Methyl, $R^{33}$ Fluor, $R^{30}$ Wasserstoff, $R^{31}$ Aethyl und $R^{34}$ Methyl, $R^{33}$ Fluor, $R^{31}$ 2-Fluoräthyl, $R^{30}$ Wasserstoff, und $R^{34}$ Methyl.

4)[6R-[6-alpha,7-beta(Z)]]-7-[[(2-amino -4-thiazolyl)($R^{21}$-oxyimino)acetyl]amino] -3-[[[[6-$R^{33}$,8-$R^{30}$, 1-$R^{31}$-1,4-dihydro -7-(4-$R^{34}$-1-piperazinyl)-4-oxo -3-chinolinyl]carbonyl]oxy]methyl] -8-oxo-5-thia-1-azabicyclo-[4.2.0]oct-2-en-2-carbonsäuren und deren Salze und Hydrate dieser Carbonsäuren und Salze. worin $R^{33}$ und $R^{30}$ Wasserstoff oder Halogen, $R^{31}$ Wasserstoff, $C_1$-$C_8$- Alkyl oder 2-Halogen- $C_1$-$C_8$- Alkyl, $R^{34}$ Wasserstoff oder $C_1$-$C_8$-Alkyl, $R^{21}$ die Gruppe

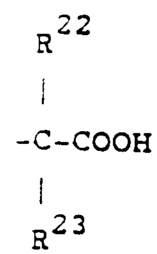

und $R^{22}$ und $R^{23}$ Wasserstoff oder $C_1$-$C_8$- Alkyl darstellen. Insbesondere sind $R^{22}$ und $R^{23}$ beide Methyl, $R^{33}$ und $R^{30}$ Wasserstoff oder Fluor, $R^{31}$ Aethyl oder 2-Fluoräthyl und $R^{34}$ Methyl, $R^{33}$ und $R^{30}$ Fluor und $R^{31}$ 2-Fluoräthyl, $R^{33}$ Fluor, $R^{30}$ Wasserstoff, $R^{31}$ 2-Fluoräthyl und $R^{34}$ Methyl, $R^{33}$ Fluor, $R^{30}$ Wasserstoff, $R^{31}$ Aethyl und $R^{34}$ Methyl, $R^{33}$ Fluor, $R^{31}$ 2-Fluoräthyl, $R^{30}$ Wasserstoff, und $R^{34}$ Methyl.

Bevorzugt liegen die Gruppen

$$\begin{array}{c} N{-}O{-}R^{130} \\ \| \\ {-}C{-} \end{array} \qquad \begin{array}{c} N{-}O{-}R^{21} \\ \| \\ {-}C{-} \end{array}$$

in der syn-Form, d.h. in der Z-Form, oder als Mischungen vor, in welchen die syn-Form überwiegt.

Als leicht hydrolysierbare Ester der Verbindungen der Formel I sind Verbindungen der Formel I zu verstehen, deren Carboxygruppe bzw. Carboxygruppen in Form einer leicht hydrolysierbaren Estergruppe vorliegt/vorliegen. Beispiele solcher Ester, die herkömmlicher Art sein können, sind die $C_1$-$C_7$-Alkanoyloxy-$C_1$-$C_8$-Alkylester, z.B. der Acetoxymethyl-, Pivaloyloxymethyl-, 1-Acetoxyäthyl- und der 1-Pivaloxyloxyäthylester; die $C_2$-$C_9$-Alkoxycarbonyloxy-$C_1$-$C_8$-alkylester, z.B. der Methoxycarbonyloxymethyl-, 1-Aethoxycarbonyloxyäthyl- und der 1-Isopropoxycarbonyloxyäthylester; die Lactonylester, z.B. der Phthalidyl und der Thiophthalidylester; die $C_2$-$C_9$-Alkoxymethylester, z.B. der Methoxymethylester und die ($C_1$-$C_7$-Alkanoyl)-aminomethylester, z.B. der Acetamidomethylester. Auch andere Ester, z.B. die Benzyl- und Cyanmethylester können brauchbar sein.

Beispiele von Salzen der Verbindungen der Formel I sind Alkalimetallsalze, wie das Natrium- und das Kaliumsalz, Ammoniumsalze; Erdalkalimetallsalze, wie das Calciumsalz; Salze mit organischen Basen, wie die Salze mit Aminen, z.B. Salze mit N-Aethylpiperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin.

Die Verbindungen der Formel I, sofern sie eine basische funktionelle Gruppe, wie z.B. eine Aminogruppe besitzen, bilden ebenfalls Additionssalze mit organischen oder anorganischen Säuren. Beispiele für solche Salze sind Hydrohalogenide, beispielsweise Hydrochloride, Hydrobromide, Hydrojodide, sowie andere Mineralsäuresalze, wie Sulfate, Nitrate, Phosphate und dergleichen, Alkyl- und Monoarylsulfonate, wie Aethansulfonate, Toluolsulfonate, Benzolsulfonate und dergleichen und auch andere organische Säuresalze wie Acetate, Tartrate, Maleate, Citrate, Benzoate, Salicylate oder Ascorbate.

Die Verbindungen der Formel I einschliesslich deren Salze und leicht hydrolysierbare Ester können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die obigen Acylderivate werden erfindungsgemäss dadurch hergestellt, dass man

(a) zur Herstellung eines leicht hydrolysierbaren Esters einer Carbonsäure der Formel I, eine Verbindung der allgemeinen Formel

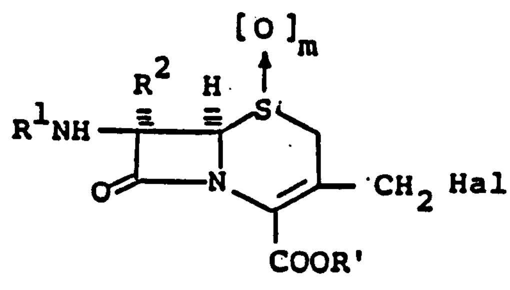

II

in der m, $R^1$ und $R^2$ obige Bedeutung haben, Hal Halogen und R' den Rest eines leicht hydrolysierbaren Esters darstellen,

mit einem Salz einer Carbonsäure der allgemeinen Formel

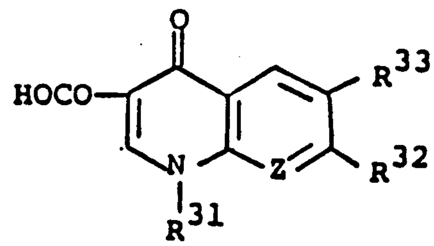

III

in der $R^{31}$, $R^{32}$ und $R^{33}$ obige Bedeutung haben, umsetzt, oder dass man
(b) zur Herstellung einer Carbonsäure der Formel I, einen Ester der allgemeinen Formel

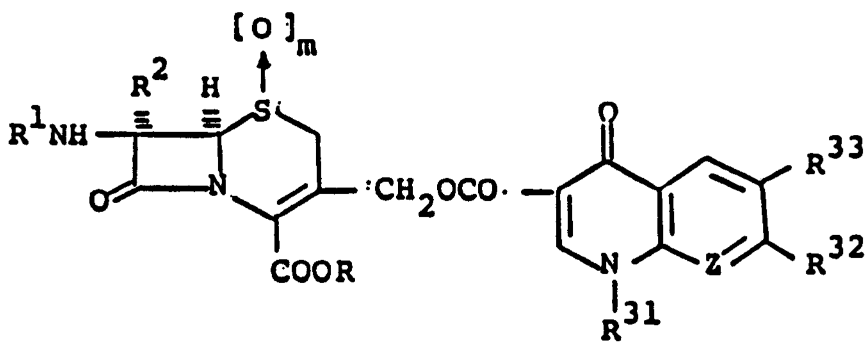

IV

in der m, $R^1$, $R^2$, $R^{31}$, $R^{32}$ und $R^{33}$ obige Bedeutung haben, und R eine Esterschutzgruppe darstellt, in die Carbonsäure der Formel I umwandelt, oder dass man
(c) zur Herstellung einer Verbindung der Formel I, worin m 0 darstellt, eine Verbindung der allgemeinen Formel

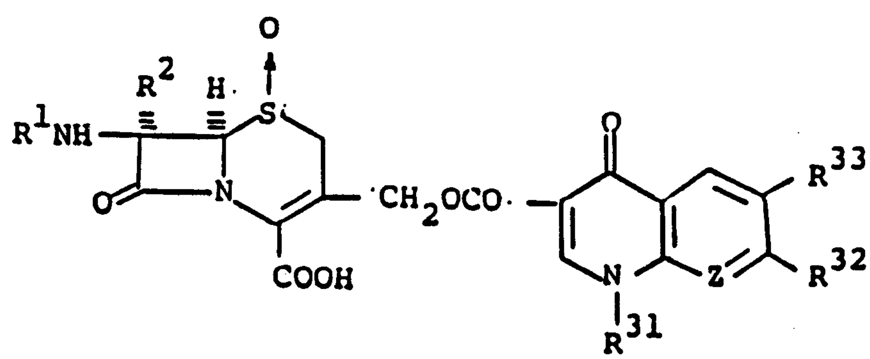

V

in der $R^1$, $R^2$, $R^{31}$, $R^{32}$ und $R^{33}$ obige Bedeutung haben,
reduziert, oder dass man
(d) zur Herstellung einer Verbindung der Formel I, worin m 1 oder 2 darstellt, oder eines ihrer Ester oder Salze, eine Verbindung der allgemeinen Formel

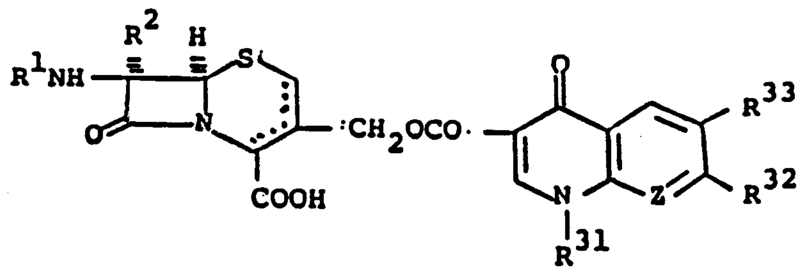

VI

in der $R^1$, $R^2$, $R^{31}$, $R^{32}$ und $R^{33}$ obige Bedeutung haben, die gestrichelten Linien die Anwesenheit eine Δ2- oder Δ3-Doppelbindung anzeigen,
oder eines ihrer Ester oder Salze oxidiert, oder dass man
(e) zur Herstellung einer Verbindung der Formel I, worin $R^1$ die Gruppe

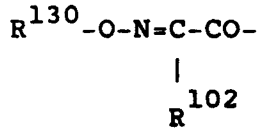

und $R^{102}$ einen aminosubstituierten 5-, 6- oder 7-gliedrigen heterocyclischen Ring mit 1, 2, 3 oder 4 Stickstoff-, Sauerstoff- und/oder Schwefelatomen bedeuten, und $R^{130}$ obige Bedeutung hat, oder eines ihrer Ester oder Salze, die Aminoschutzgruppe im Substituenten $R^{103}$ einer Verbindung der allgemeinen Formel

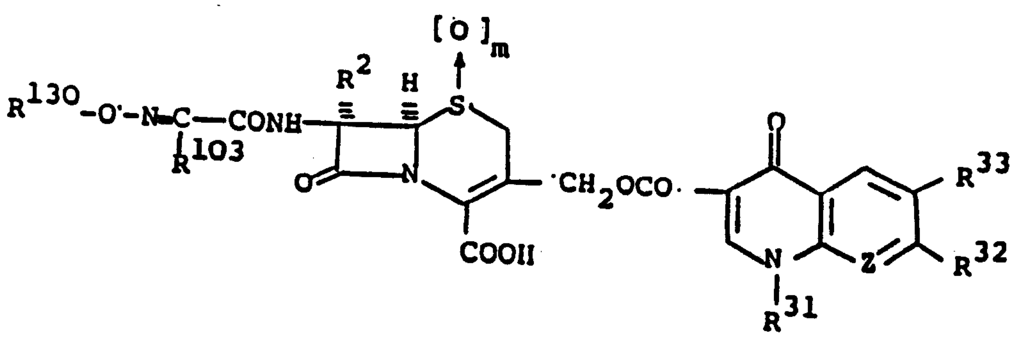

VII

in der m, $R^2$, $R^{31}$, $R^{32}$, $R^{33}$ und $R^{130}$ obige Bedeutung haben, und $R^{103}$ die für $R^{102}$ gegebene Bedeutung hat, wobei jedoch der Aminosubstituent geschützt ist,
oder eines ihrer Ester oder Salze abspaltet, oder dass man
(f) zur Herstellung eines leicht hydrolysierbaren Esters einer Verbindung der Formel I, eine Carbonsäure der Formel I einer entsprechenden Veresterung unterwirft, oder dass man
(g) zur Herstellung von Salzen oder Hydraten einer Verbindung der Formel I bzw. von Hydraten besagter Salze, eine Verbindung der Formel I in ein Salz oder Hydrat bzw. in ein Hydrat des besagten Salzes überführt.

Die erfindungsgemässe Umsetzung des Halogenids der Formel II mit einem Salz der Carbonsäure der Formel III gemäss Variante a) des erfindungsgemässen Verfahrens wird vorzugsweise in einem nichthydroxylierten Lösungsmittel durchgeführt, wie z.B. in Dimethylformamid, Methylenchlorid oder N,N′-Dimethylacetamid. Geeignete Salze der Carbonsäure der Formel III sind z.B. die Natrium-, Kalium-, Cäsium-, t-Butylammonium- oder Tetramethylammoniumsalze. Hal in Formel II bedeutet Halogen, insbesonder Brom oder Jod. Diese Umsetzung wird bevorzugt bei etwa 0-80 °C, insbesondere bei Zimmertemperatur, durchgeführt.

Esterschutzgruppen R der in Variante b) des erfindungsgemässen Verfahrens verwendeten Ester der Formel IV sind vorzugsweise solche, welche unter milden Bedingungen in die freie Carboxygruppe umgewandelt werden können, z.B. t-Butyl, p-Nitrobenzyl, Benzhydryl oder Allyl. Auch die oben erläuterten Reste eines leicht hydrolysierbaren Esters können verwendet werden. Die Esterschutzgruppen werden z.B.

wie folgt abgespalten: p-Nitrobenzyl durch Hydrolyse in Gegenwart von Natriumsulfid bei (oder unterhalb) 0°C bis Zimmertemperatur in einem Lösungsmittel, wie z.B. Dimethylformamid (vorzugsweise wässrig); t-Butyl durch Umsetzung mit Trifluoressigsäure in Gegenwart von Anisol bei etwa 0°C bis Zimmertemperatur, mit oder ohne zusätzliches Lösungsmittel, wie z.B. Methylenchlorid; Allyl durch Palladium-(O)-katalysierte Transallylierung in Gegenwart eines Natrium- oder Kaliumsalzes der 2-Aethylcapronsäure, siehe z.B. J. Org. Chem. 1982, 47, 587.

Die Reduktion der Sulfoxide der allgemeinen Formel V nach Variante c) des erfindungsgemässen Verfahrens kann nach einer Vielzahl von Reaktionen erfolgen, z.B. durch Behandlung mit Phosphortrichlorid in Dimethylformamid oder mit Trifluoressigsäureanhydrid in Gegenwart von Natriumjodid in Aceton/Methylenchlorid. Die Temperatur dieser Reaktionen liegt bevorzugt bei etwa 0 bis -20°C, insbesonder bei 0°C.

Durch die Oxidation der Verbindungen der Formel VI gemäss Variant d) des erfindungsgemässen Verfahrens wird ein allfälliges Δ2-Isomere der Formel VI in das entsprechende Δ3 Isomere der Formel I, worin m 1 oder 2 darstellt, übergeführt. Diese Oxidation wird durch Behandeln mit einem organischen oder anorganischen Oxidationsmittel durchgeführt. Als Oxidationsmittel dienen verschiedene, Sauerstoff leicht abgebende Verbindungen, wie z.B. organische Peroxide, z.B. monosubstituierte organische Peroxide wie $C_1$-$C_4$-Alkyl- oder Alkanoylhydroperoxide, wie t-Butylhydroperoxid, Perameisensäure oder Peressigsäure; sowie phenylsubstituierte Derivate dieser Hydroperoxide, wie Cumolhydroperoxid oder Perbenzoesäure. Der Phenylsubstituent kann gegebenenfalls eine weitere niedere Gruppe, z.B. $C_1$-$C_4$-Alkyl oder $C_1$-$C_4$-Alkoxy oder auch Halogen oder eine Carboxygruppe tragen, z.B. 4-Methylperbenzoesäure, 4-Methoxyperbenzoesäure, 3-Chlorperbenzoesäure oder Monoperphthalsäure. Als Oxidationsmittel können auch verschiedene anorganische Oxidationsmittel verwendet werden, z.B. Wasserstoffperoxid; Ozon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen, inerten Lösungsmittel, wie Tetrahydrofuran, Dioxan, Methylenchlorid, Chloroform, Aethylacetat oder Aceton; oder in einem protischen Lösungsmittel, wie Wasser, einem niederen Alkanol, z.B. Methanol oder Aethanol, oder einer niederen, gegebenenfalls halogenierten Alkancarbonsäure, z.B. Ameisensäure, Essigsäure oder Trifluoressigsäure. Die Reaktionstemperatur bewegt sich vornehmlich im Bereiche von -20°C bis +50°C.

Bei Verwendung von äquimolaren Mengen Oxidationsmittel bzw. leichtem Ueberschuss im Verhältnis zum Ausgangsmaterial erhält man vornehmlich das entsprechende Sulfoxid, d.h. eine Verbindung der Formel I, worin m die Zahl 1 darstellt. Erhöht man die Menge des Oxidationsmittels auf das Doppelte des stöchiometrischen Verhältnisses oder mehr, bildet sich das entsprechende Sulfon, d.h. eine Verbindung der Formel I, worin m die Zahl 2 darstellt. Es ist ebenfalls möglich, das Sulfon der Formel I aus dem entsprechenden Sulfoxid durch Behandlung mit dem Oxidationsmittel in äquimolarer oder grösserer Menge zu erhalten. Die Verfahrensbedingungen sind im wesentlichen die gleichen wie bei der Herstellung des Sulfoxids.

Durch Abspaltung der Aminoschutzgruppe im Substituenten $R^{103}$ einer Verbindung der Formel VII gemäss Variante e) des erfindungsgemässen Verfahrens erhält man entsprechende Verbindungen der Formel I mit einer freien Aminogruppe. Mögliche Aminoschutzgruppen sind diejenigen, welche in der Peptidchemie verwendet werden, z.B. Alkoxycarbonylgruppen, z.B. t-Butoxycarbonyl, substituierte Alkoxycarbonylgruppen, z.B. Trichloräthoxycarbonyl, substituierte Aralkoxycarbonylgruppen, z.B. p-Nitrobenzyloxycarbonyl, Aralkylgruppen, z.B. Trityl oder Benzhydryl, oder Halogenalkanoylgruppen, wie z.B. Chloracetyl, Bromacetyl, Jodacetyl oder Trifluoracetyl.

Bevorzugte Aminoschutzgruppen sind t-Butoxycarbonyl (t-BOC) und Trityl.

Die Aminoschutzgruppen sind beispielweise durch saure Hydrolyse abspaltbar (z.B. t-Butoxycarbonyl oder Trityl) oder auch durch basische Hydrolyse abspaltbar (z.B. Trifluoracetyl). Die Chlor-, Brom- und Jodacetylgruppen können durch Behandeln mit Thioharnstoff abgespalten werden.

Durch saure Hydrolyse abspaltbare Schutzgruppen werden vorzugsweise mit Hilfe einer niederen Alkancarbonsäure, die gegebenenfalls halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperaturen angewendet werden können, z.B. im Bereiche von etwa 0°C bis +40°C. Alkalisch abspaltbare Schutzgruppen werden im allgemeinen mit verdünnter wässriger Lauge bei 0°C bis +30°C hydrolysiert. Die Chlor-, Brom- und Jodacetylschutzgruppen können mittels Thioharnstoff in saurem, neutralem oder alkalischem Milieu bei etwa 0°C bis +30°C abgespalten werden.

Zur Herstellung der leicht hydrolysierbaren Ester der Carbonsäuren der Formel I gemäss Variante f) des erfindungsgemässen Verfahrens wird die Carbonsäure vorzugsweise mit dem entsprechenden, die Estergruppe enthaltenden Halogenid, bevorzugt mit dem Jodid, umgesetzt. Die Reaktion kann mit Hilfe

einer Base, z.B. einem Alkalimetallhydroxid oder -carbonat oder einem organischem Amin, wie Triäthylamin, beschleunigt werden. Die Veresterungsreaktion wird vorzugsweise in einem inerten organischen Lösungsmittel, wie Dimethylacetamid, Hexamethylphosphorsäuretriamid, Dimethylsulfoxid oder, vorzugsweise, Dimethylformamid durchgeführt. Die Temperatur liegt vorzugsweise im Bereiche von etwa 0-40 °C.

Die Herstellung der Salze und Hydrate der Verbindungen der Formel I bzw. der Hydrate dieser Salze gemäss Variante g) des erfindungsgemässen Verfahrens kann in an sich bekannter Weise erfolgen, z.B. durch Umsetzen der Carbonsäure der Formel I mit einer äquimolaren Menge der gewünschten Base, zweckmässig in einem Lösungsmittel, wie Wasser oder in einem organischen Lösungsmittel wie Aethanol, Methanol oder Aceton. Die Temperatur der Salzbildung ist nicht kritisch, sie liegt im allgemeinen bei Raumtemperatur kann aber auch leicht darüber oder darunter, etwas im Bereiche von 0 °C bis +50 °C, liegen.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I oder eines ihrer Salze) einer feuchten Atmosphäre, z.B. bei etwa +10 °C bis +40 °C, ausgesetzt werden.

Das folgende Reaktionsschema I veranschaulicht das Verfahren zur Herstellung der erfindungsgemässen Produkte:

Schema I

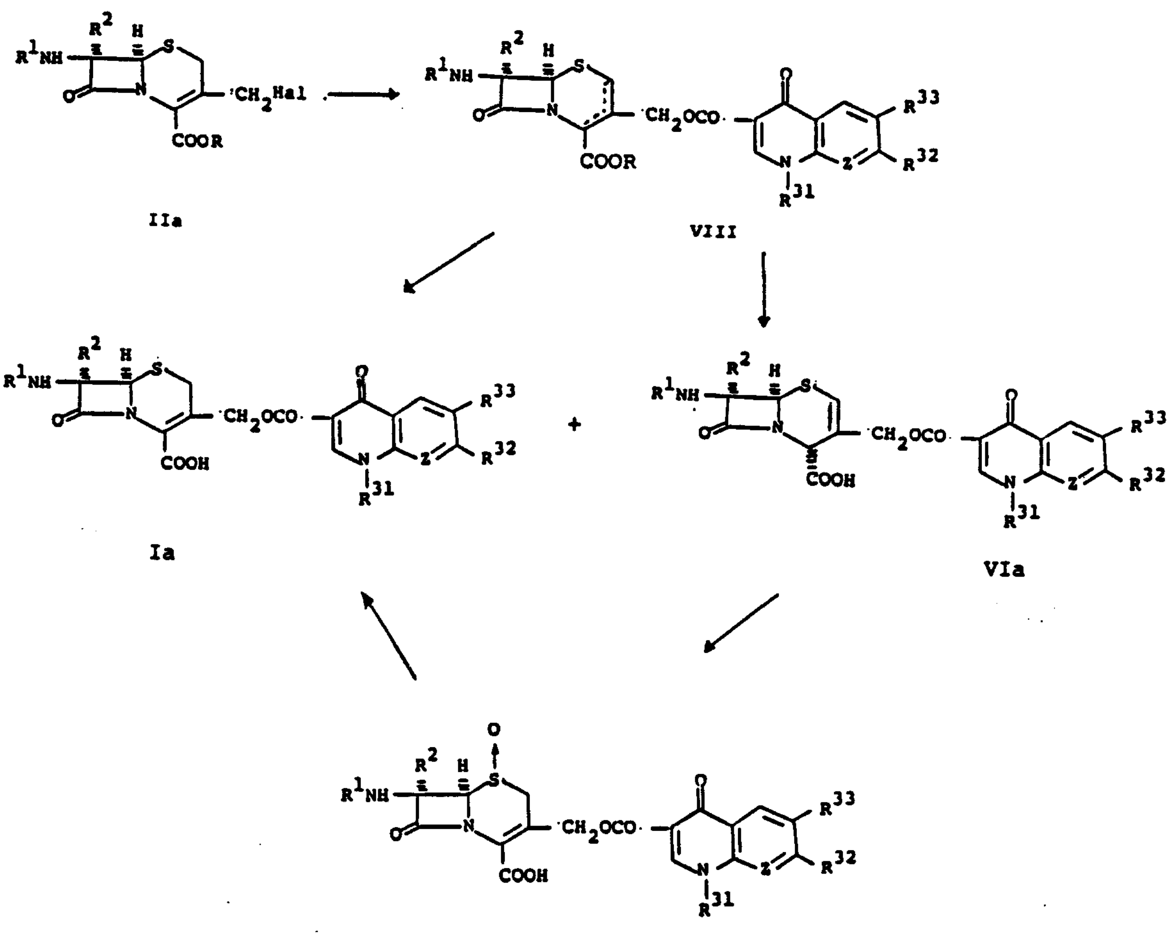
IIa
VIII
Ia
VIa

V

$R^1$, $R^2$, $R^{31}$, $R^{32}$, $R^{33}$, R und die gestrichelten Bindungen haben alle die oben gegebene Bedeutung.

Je nach Wahl der Esterschutzgruppe R und des Halogens Hal erhält man in Bezug auf die Doppelbindung im Cephemring ein Δ3- oder Δ2-Isomeres der Formel VIII. Ein erhaltenes Δ3-/Δ2-Gemisch kann notwendigenfalls durch Herstellung des Sulfoxids der Formel V und anschliessende Reduktion dieser Verbindung oder Trennung der beiden Komponenten zum gewünschten Δ3 Isomeren gereinigt werden.

Erläuterungen zum Schema I:

IIa → VIII

Die Verbindung der Formel IIa ist eine bekannte Verbindung oder ist in analoger Weise erhältlich (siehe z.B. die U.S. Patentschriften Nr. 4,406,899 und 4,266,049). Sie wird mit einem Salz des gewählten Chinolons der Formel III umgesetzt. Die Umsetzung erfolgt wie oben für die Verfahrensalternative a) beschrieben.

VIII → Ia oder VIa

Die Esterschutzgruppe R der Verbindung der Formel VIII wird anschliessend abgespalten wie oben für die Verfahrensalternative b) beschrieben, wobei eine Carbonsäure der Formel Ia oder eine Gemisch davon mit dem Δ3-Isomer der Formel VIa erhalten wird.

VIa → V

Falls die Doppelbindung unter Bildung des Δ3-Isomeren isomerisiert wird, wird die so erhaltene Verbindung der Formel VIa, wie oben dür Verfahrensalternative d) beschrieben, oxidiert, z.B. mit einer Persäure, wie z.B. 3-Chlorperbenzoesäure, in einem Lösungsmittel, wie z.B. Methylenchlorid, bei einer Reaktionstemperatur von etwa -20 °C bis 40 °C, vorzugsweise bei etwa 0 °C.

V → Ia

Die so erhaltene Verbindung der Formel V ist selbst ein unter Formel I fallendes Endprodukt. Sie kann jedoch zum Endprodukt Ia reduziert werden, wie oben für Verfahrensvariante c) beschrieben.

Verbindungen der Formel I mit den Gruppen

$$\begin{array}{l} \;\;N{-}O{-}R^{130} \\ \;\;\| \\ {-}C{-} \end{array}$$

oder

$$\begin{array}{l} \;\;N{-}O{-}R^{21} \\ \;\;\| \\ {-}C{-} \end{array}$$

(vgl. oben) liegen vorzugsweise als syn-Formen vor. Solche syn-Formen können erhalten werden durch Verwendung von Ausgangsmaterialien, worin diese syn-Form bereits vorliegt. Wahlweise kann ein erhaltenes syn/anti-Gemisch einer Verbindung der Formel I in üblicher Weise in die entsprechenden syn- und anti-Formen aufgetrennt werden, beispielsweise durch Umkristallisation oder durch chromatographische Methoden unter Verwendung eines geeigneten Lösungsmittel bzw. Lösungsmittelgemisches.

Erfindungsgemäss wurden erstmals die zwei antibiotischen Komponenten "Cephalosporin" und "Chinolon/Naphthyridinon" kombiniert (letztere bekannt z.B. aus DE-A-23 62 553, 30 31 767 und 31 06 013), wobei neue Produkte mit hervorragenden antibiotischen Eigenschaft erhalten werden.

Die Verbindungen der Formel I sowie die entsprechenden Salze bzw. die Hydrate dieser Produkte und Ester sind antibiotisch, insbesondere bakterizid wirksam. Sie können als Agenzien zur Bekämpfung von bakteriellen Infektionen (einschliesslich Harn- und Atemweginfektionen) bei Säugern, z.B. Hunden, Katzen, Pferden usw., sowie beim Menschen verwendet werden. Diese Cephalosporine sind wirksam gegen einen breiten Bereich sowohl gram-negativer als auch gram-positiver Bakterien.

Die in vitro Wirksamkeit der erfindungsgemässen Verbindungen gegen eine Vielzahl gram-positiver und gram-negativer Mikroorganismen, ausgedrückt als Mindesthemmkonzentration in Mikrogramm/ml und ermittelt unter Verwendung der Reihenverdünnungsmethode (in Flüssigmedium) ist wie folgt:

Verbindung A: [6R-(6α,7β)]-3-[[[(1-Aethyl-1,4-dihydro-7-methyl-4-oxo-1,8-naphthyridin-3-yl)carbonyl]-oxy]methyl]-8-oxo-7-[(phenoxyacetyl)amino]-5-thia-1-azabicyclo[4.2.0]oct-2-en-2-carbonsäurehydrat

Verbindung B: [6R-(6α,7β)]-3-[[[(5-Aethyl-5,8-dihydro-8-oxo-1,3-dioxolo[4,5-g]chinolin-7-yl)carbonyl]-oxy]methyl]-8-oxo-7-[(phenoxyacetyl)amino]-5-thia-1-azabicyclo[4.2.0]oct-2-en-2-carbonsäuremono-Natriumsalz

Verbindung C: [6R-(6α,7β)(Z)]-[[(2-Amino-4-thiazolyl)(methoxyimino)acetyl]amino]-3-[[[(1-äthyl-1,4-dihydro-7-methyl-4-oxo-1,8-naphthyridin-3-yl)carbonyl]oxy]methyl-8-oxo-5-thia-1-azabicyclo[4.2.0]oct-2-en-2-carbonsäure-Natriumsalz

Verbindung D: [6R-(6α,7β)(Z)]-[[(2-Amino-4-thiazolyl)(methoxyimino)acetyl]amino]-3-[[[(5-äthyl-5,8-dihydro-8-oxo-1,3-dioxolo[4,5-g]chinolin-7-yl)carbonyl]oxy]methyl]-8-oxo-5-thia-1-azabicyclo[4.2.0]oct-2-en-2-carbonsäure-Natriumsalz

Verbindung E: [6R-(6α,7β)]-3-[[[[1-Aethyl-6-fluor-1,4-dihydro-7-(4-formyl-1-piperazinyl)-4-oxochinolin-3-yl]carbonyl]oxy]methyl]-8-oxo-7-[(phenoxyacetyl)amino]-5-thia-1-azabicyclo[4.2.0]-oct-2-ene-2-carbonsäure-Natriumsalz

Verbindung F: [6R-(6α,7β)]-3-[[[5-Aethyl-5,8-dihydro-8-oxo-1,3-dioxolo[4,5-g]chinolin-7-yl)carbonyl]-oxy]methyl]-8-oxo-7-[(2-thienylacetyl)amino]-5-thia-1-azabicyclo[4.2.0]oct-2-en-2-carbonsäure-Natriumsalz

Verbindung G: [6R-(6α,7β)]-3-[[[[1-Aethyl-6-fluor-1,4-dihydro-7-(4-formyl-1-piperazinyl)-4-oxochinolin-3-yl]carbonyl]oxy]methyl]-7-[(2-thienylacetyl)amino]-8-oxo-5-thia-1-azabicyclo[4.2.0]-oct-2-ene-2-carbonsäure-Natriumsalz

Verbindung H: [6R-(6α,7β)]-3-[[[[1-Aethyl-6-fluor-1,4-dihydro-7-(1-pyrrolidinyl)-4-oxochinolin-3-yl]-carbonyl]oxy]methyl]-8-oxo-7-[(phenoxyacetyl)amino]-5-thia-1-azabicyclo[4.2.0]oct-2-en-2-carbonsäure-Natriumsalz

Verbindung I: [6R-(6α,7β)(Z)]-7-[[(2-Amino-4-thiazolyl)(methoxyimino)acetyl]amino]-3-[[[[(1-äthyl-6-fluor-1,4-dihydro-7-(1-pyrrolidinyl)-4-oxo-chinolin-3-yl]carbonyl]oxy]methyl]-8-oxo-5-thia-1-azabicylo-4.2.0]oct-2-en-2-carbonsäure-Natriumsalz

In vitro MHK (µg/ml)

| Erreger | Verbindungen A | B | C | D | E | F | G | H | I |
|---|---|---|---|---|---|---|---|---|---|
| E. coli 48 | 32 | 2 | 0.5 | 0.25 | 8 | 2 | 8 | 32 | 0.5 |
| K. pneumoniae A | 16 | 1 | 0.5 | 0.25 | 2 | 1 | 4 | 32 | 0.5 |
| E. cloacae 9570A | 64 | 2 | 2 | 1 | 8 | 2 | 8 | 32 | 2 |
| E. cloacae 948-1 | --- | 2 | 8 | 1 | 8 | 1 | 8 | 32 | 4 |
| E. cloacae 2367-2 | --- | 1 | 16 | 2 | 4 | 1 | 32 | 32 | 4 |
| E. cloacae 7099 | 16 | 0.5 | 8 | 0.5 | 2 | 0.5 | 2 | 2 | 2 |
| E. cloacae 214 | 128 | 2 | 8 | 0.5 | 8 | 2 | 64 | 32 | 4 |
| P. vulgaris ATCC 6380 | 8 | 0.5 | 0.25 | 0.063 | 4 | 0.5 | 4 | 32 | 0.5 |
| P. mirabilis 190 | 8 | 0.5 | 0.16 | 0.008 | 0.5 | 0.5 | 4 | 0.5 | 0.031 |
| S. marcescens SM | 16 | 1 | 1 | 0.5 | 4 | 1 | 4 | 32 | 1 |
| P. aeruginosa Stone 130 | 128 | 32 | 128 | 16 | 16 | 32 | 32 | 32 | 8 |
| P. aeruginosa 503-56 | 128 | 64 | 128 | 32 | 32 | 64 | 64 | 64 | 32 |
| S. aureus Smith | 0.25 | 0.25 | 2 | 2 | 0.063 | 0.25 | 0.25 | 0.25 | 0.25 |
| S. aureus 95 | --- | 4 | 8 | 4 | 2 | 4 | 4 | 0.5 | 0.5 |

Zur Bekämpfung einer bakteriellen Infektion in einem Säuger kann eine erfindungsgemässe Verbindung mit einer Tagesdosis von etwa 5 bis etwa 500 mg/kg, vorzugsweise etwa 10-100 mg/kg, insbesondere etwa 10-55 mg/kg, behandelt werden. Sämtliche Verabreichungsarten, welche bisher für die Penicillin- und Cephalosporintherapie in Frage kommen, sind ebenfalls für die erfindungsgemässen Cephalosporine verwendbar. Verabreichungsarten sind demnach z.B. intravenöse, intramuskuläre und enterale Administrierungen.

Die erfindungsgemässen Produkte können als Heilmittel, z.B. in Form pharmazeutischer Präparate Verwendung finden, welche die oder ihre Salze in Mischung mit einem für die enterale oder parenterale Applikation geeigneten pharmazeutischen, organischen oder anorganischen inerten Trägermaterial wie z.B. Wasser, Gelatine, Gummi arabicum, Milchzucker, Stärke, Magnesiumstearat, Talk, pflanzliche Oele, Polyalkylenglykole oder Vaseline enthalten. Die pharmazeutischen Präparate können in fester Form, z.B. als Tabletten, Dragées, Suppositorien, Kapseln oder in flüssiger Form, z.B. als Lösungen, Suspensionen oder Emulsionen vorliegen. Gegebenenfalls sind sie sterilisiert und bzw. oder enthalten Hilfsstoffe wie Konservierungs-, Stabilisierungs-, Netz- oder Emulgiermittel, Salze zur Veränderung des osmotischen Druckes, Anaesthetika oder Puffer. Sie können auch noch ander therapeutisch wertvolle Stoffe enthalten. Die Carbonsäuren der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien wie Wasser und isotonischer Kocksalzlösung, Lösungsmittelvermittler, wie Propylenglykol, zubereitet. Die leicht hydrolysierbaren Ester der Formel I kommen auch für die enterale Verabreichung in Betracht.

Beispiel 1

Gemisch von [6R-(6$\alpha$,7$\beta$)]-3-[[[(1-Aethyl-1,4-dihydro-7-methyl-4-oxo-1,8-naphthyridin-3-yl)carbonyl]oxy]-methyl]-8-oxo-7-[(phenoxyacetyl)amino]-5-thia-1-azabicyclo[4.2.0]oct-2-en-2-carbonsäure (4-nitrophenyl)-methylester und [2R-(2$\alpha$,6$\alpha$,7$\beta$)]-3-[[[(1-Aethyl-1,4-dihydro-7-methyl-4-oxo-1,8-naphthyridin-3-yl)carbonyl]-oxy]methyl]-8-oxo-7-[(phenoxyacetyl)amino]-5-thia-1-azabicyclo[4.2.0]oct-3-en-2-carbonsäure (4-nitrophenyl)methylester

Eine Lösung von 22,4 g (0,040 Mol) [6R-(6$\alpha$,7$\beta$)]-3-(bromomethyl)-8-oxo -7-[(phenoxyacetyl)amino]-5-thia-1-azabicyclo[4.2.0]oct -2-en-2-carbonsäure (4-nitrophenyl)methylester und 10,1 g (0,040 Mol) 1-Aethyl-1,4-dihydro-7-methyl-4-oxo-1,8-naphthyridin -3-carbonsäure-Natriumsalz in 200 ml trockenem Dimethylformamid wird in einer Argonatmosphäre 5 Stunden gerührt. Das Lösungsmittel wird unter vermindertem Druck abgedampft. Eine Lösung des Rückstandes in Aethylacetat wird mit wässriger Kochsalzlösung gewaschen, mit Aktivkohle entfärbt, über Natriumsulfat getrocknet und unter vermindertem Druck auf ein kleines Volumen eingedampft, wonach die obigen Endprodukte auskristallisieren. Die Mutterlauge wird an 800 g neutralem Kieselgel mit Aethylacetat als Eluierungsmittel säulenchromatographisch gereinigt (Korngrösse 0,063-0,200 mm). Die entsprechenden Fraktionen werden vereinigt und aus Aethylacetat kristallisiert wobei zusätzliches Endprodukt erhalten wird. Das erhalten Isomerengemisch wird für die nachstehende Umsetzung verwendet. Reiner Δ3-Ester wird durch fraktionierte Kristallisation aus Aethylacetat erhalten und der Δ2-Ester wird durch präparative Schichtchromatographie der Mutterlauge an Kieselgelplatten isoliert.

Kenndaten der beiden Isomeren:

Δ3-Isomers: IR (KBr) 3405, 1785, 1735, 1697, 1637, 1520, 1348 $cm^{-1}$; Messenspektrum m/z 714 ($M^+$ + H), 736 ($M^+$ + Na).

Δ2-Isomeres: IR (KBr) 3560, 3480, 3415, 1780, 1745, 1693, 1620, 1520 $cm^{-1}$; Messenspektrum m/z 713 ($M^+$).

Beispiel 2

[2R-(2$\alpha$,6$\alpha$,7$\beta$)]-3-[[[(1-Aethyl-1,4-dihydro-7-methyl-4-oxo-1,8-naphthyridin-3-yl)carbonyl]oxy]methyl]-8-oxo-7-[(phenoxyacetyl)amino]-5-thia-1-azabicyclo[4.2.0]oct-3-en-carbonsäure

Eine Lösung von 6,11 g (0,00856 Mol) eines Gemisches der beiden Isomeren aus Beispiel 1 in 45 ml Dimethylformamid wird bei -5 bis -10°C mit einer Lösung von 2,80 g (0,0116 Mol) Natriumsulfidhydrat in 20 ml Wasser tropfenweise versetzt. Nach 35 Minuten wird das Gemisch durch Zugabe von 1N wässriger Salzsäure auf pH 3,5 angesäuert, wobei ein Harz ausfällt. Das Harz verfestigt sich nach Zugabe von 50 ml Aethylacetat und 50 ml Aether. Nach Filtrieren, Waschen mit Wasser und Aether und Trocknen bei 50°C unter vermindertem Druck über Phosphorpentoxid erhält man das Endprodukt. Das Filtrat wird mit einer weiteren Aethermenge versetzt, wobei ein zusätzlicher Niederschlag anfällt, welcher filtriert und in wässriger Natriumbicarbonatlösung gelöst wird. Die wässrige Lösung wird mit Aethylacetat gewaschen und auf pH 3,5 angesäuert. Eine Lösung des ausgefällten Harzes in Methylenchlorid wird filtriert, mit Wasser gewaschen, über Natriumsulfat getrocknet und unter vermindertem Druck eingedampft. Man erhält eine weitere Menge des Endproduktes: IR (KBr) 3420, 3300, 1773, 1720 $cm^{-1}$; Massenspektrum m/z 579 ($M^+$ +H).

Beispiel 3

[(6R-(6α,7β)]-3-[[[(1-Aethyl-1,4-dihydro-7-methyl-4-oxo-1,8-naphthyridin-3-yl)carbonyl]oxy]methyl]-8-oxo-7-[-(phenoxyacetyl)amino-5-thia-1-azabicyclo[4.2.0]oct-2-en-2-carbonsäure-S-oxid

Eine Aufschlämmung von 2,55 g (0,00442 Mol) des Endproduktes von Beispiel 2 in 60 ml Methylenchlorid wird unter Rühren bei 0 °C tropfenweise mit einer Lösung von 0,986 g (85% rein, 0,00486 Mol) m-Chlorperbenzoesäure in 15 ml Methylenchlorid versetzt. Die Mischung wird während 4 Stunden bei 0 °C gerührt und dann filtriert. Der erhaltene Feststoff wird mit Methylenchlorid gewaschen und unter vermindertem Druck getrocknet. Das so erhaltene Endprodukt hat folgende Kenndaten:

IR (KBr) 3360, 1794, 1723, 1684, 1637, 1017 $cm^{-1}$; Massenspektrum m/z 595 ($M^+$ + H).

Beispiel 4

[(6R-(6α,7β)]-3-[[[(1-Aethyl-1,4-dihydro-7-methyl-4-oxo-1,8-naphthyridin-3-yl)carbonyl]oxy]methyl]-8-oxo-7-[-(phenoxyacetyl)amino-5-thia-1-azabicyclo[4.2.0]oct-2-en-2-carbonsäurehydrat

Eine Lösung von 1,64 g (0,0109 Mol) Natriumjodid in ml trockenem Aceton und 20 ml Methylenchlorid wird mit 1,30 g (0,00219 Mol) des Endprodukts von Beispiel 3 versetzt. Die erhaltene Aufschlämmung wird unter Rühren gekühlt und bei 0 °C mit 1,75 ml (0,0124 Mol) Trifluoressigsäureanhydrid versetzt. Nach 30 Minuten wird der pH mit wässriger Natriumbicarbonatlösung auf 6,0 eingestellt. Anschliessend wird der pH mit wässriger 1N Salzsäure auf 3,5 eingestellt und eine kleine unlösliche Portion durch Filtrieren entfernt. Die organische Phase wird mit wässriger Natriumsulfidlösung gewaschen, über Natriumsulfat getrocknet und unter vermindertem Druck eingedampft. Der Rückstand wird mit Aether gewaschen, in Methylenchlorid aufgenommen und durch Zugabe von Aether ausgefällt wobei das Endprodukt erhalten wird.

Wahlweise wird ein Gemisch von 0,435 g (0.73 mMol) des Endprodukts von Beispiel 3 und 7 ml trockenem Dimethylformamid unter Rühren auf -12 °C gekühlt und mit 0,128 ml (1.4 mMol) Phosphortrichlorid versetzt. Weitere 0,027 ml Phosphortrichlorid werden nach 7 Minuten hinzugefügt. Das Gemisch wird 6,5 Minuten gerührt und anschliessend mit einer kalten Lösung von 0,428 g (5,1 mMol) Natriumbicarbonat in 70 ml Wasser versetzt. Der erhaltene Niederschlag wird abfiltriert, mit Wasser gewaschen und unter vermindertem Druck über Phosphorpentoxid getrocknet, wobei das Endprodukt erhalten wird. Weiteres Endprodukt fällt aus dem Filtrat aus.

Kenndaten: IR (KBr) 3420, 1785, 1708, 1620 $cm^{-1}$; Massenspektrum m/z 579 ($M^+$ + H).

Beispiel 5

[6R-(6α,7β)]-3-[[[(5-Aethyl-5,8-dihydro-8-oxo-1,3-dioxolo-[4,5-g]chinolin-7-yl)carbonyl]oxy]methyl]-7-[-(phenoxyacetyl) amino]-5-thia-1-azabicyclo[4.2.0]oct-2-en-2-carbonsäure-1,1-dimethyläthylester

Eine Lösung von 5-Aethyl-5,8-dihydro-8-oxo-1,3-dioxolo[4,5-g]chinolin -7-carbonsäure-Natriumsalz (1 mMol) in 12 ml Dimethylformamid wird in einer Stickstoffatmosphäre 1 Stunde mit 1.5 g eines 4A Molekularsiebs gerührt, anschliessend mit einer Lösung von [6R-(6α,7β)]-3-(Jodmethyl)-8-oxo -7-[-(phenoxyacetyl)amino]-5-thia-1-azabicyclo[4.2.0]oct -2-en-2-carbonsäure-1,1-dimethyläthylester (1 mMol) in 6 ml trockenem Dimethylformamid vereinigt und 5 Stunden gerührt. Das Gemisch wird zur Trockene eingedampft. Der Rückstand wird in Aethylacetat oder Aethylacetat/Methylenchlorid aufgenommen, mit wässriger Natriumbicarbonatlösung und Kochsalzlösung gewaschen, über Natriumsulfat getrocknet und zur Trockene eingedampft. Der Rückstand wird gereinigt entweder durch präparative Schichtchromatographie oder durch Schnellchromatographie unter Verwendung eines 8:2 Lösungsmittelgemisch aus Aethylacetat und Methylenchlorid.

Beispiel 6

[6R-(6α,7β)(Z)]-3-[[[(1-Aethyl-1,4-dihydro-7-methyl-4-oxo-1,8-naphthyridin-3-yl)carbonyl]oxy]methyl]-7-[[-(methoxyimino)[2-(triphenylmethyl)amino-4-thiazolyl]acetyl]amino]-8-oxo-5-thia-1-azabicyclo[4.2.0]oct-2-en-2-carbonsäure-1,1-dimethyläthylester

Die Titelverbindung wird erhalten durch Wiederholung der Umsetzung von Beispiel 5 unter Verwendung von 1-Aethyl-1,4-dihydro-7-methyl-4-oxo-1,8-naphthylridin -3-carbonsäure-Natriumsalz und [6R-(6α,7β)(Z)-3-

Jodmethyl-7-[[(methoxyimino)[2-(triphenylmethyl)amino -4-thiazolyl]acetyl]amino] -8-oxo-5-thia-1-azabicyclo-[4.2.0)oct -2-en-2-carbonsäure-1,1-dimethyläthylester. Der Rückstand wird durch Schnellchromatographie mit einer 9:1 Mischung von Aethylacetat/Methylenchlorid als Lösungsmittel gereinigt.

Beispiel 7

[6R-(6α,7β)(Z)]-3-[[[(5-Aethyl-5,8-dihydro-8-oxo-1,3-dioxolo[4,5-g]chinolin-7-yl)carbonyl]oxy]methyl]-7-[[-(methoxyimino)[2-(triphenylmethyl)amino-4-thiazolyl]acetyl]amino]-8-oxo-5-thia-1-azabicyclo[4.2.0]oct-2-en-2-carbonsäure-1,1-dimethyläthylester

Die Titelverbindung wird erhalten durch Wiederholung der Umsetzung von Beispiel 5 mit 5-Aethyl-5,8-dihydro-8-oxo-1,3-dioxolo[4,5-g]chinolin-7-carbonsäure und [6R-(6α,7β)(Z)] -Jodmethyl -7-[[(methoxyimino)-[2-(triphenylmethyl)amino -4-thiazolyl]acetyl]amino] -8-oxo-5-thia-1-azabicyclo[4.2.0]oct -2-en-2-carbonsäure-1,1-dimethyläthylester als Reagenzien. Der Rückstand wird durch Schnellchromatographie mit einer Mischung von 9:1 Aethylacetat/Methylenchlorid als Lösungsmittel gereinigt.

Beispiel 8

[6R-(6α,7β)]-3-[[[[1-Aethyl-6-fluor-1,4-dihydro-7-(4-formyl-1-piperazinyl)-4-oxo-chinolin-3-yl]carbonyl]oxy]-methyl]-8-oxo-7-[(phenoxyacetyl)amino]-5-thia-1-azabicyclo[4.2.0]oct-2-en-2-carbonsäure-1,1-dimethyläthylester

Die Titelverbindung wird erhalten durch Wiederholung der Umsetzung von Beispiel 5 mit 1-Aethyl-6-fluor-1,4-dihydro-7-(4-formyl-1-piperazinyl)-4-oxo-3-chinolincarbonsäure und [6R-(6α,7β)]-3-(Jodmethyl)-8-oxo-7-[(phenoxyacetyl)amino]-5-thia-1-azabicylo[4.2.0]oct-2-en-2-carbonsäure-1,1-dimethyläthylester als Reagenzien. Der Rückstand wird durch Schnellchromatographie unter Verwendung eines 20:1 Gemisches von Methylenchlorid/Methanol als Lösungsmittel gereinigt.

Beispiel 9

[6R-(6α,7β)-3-[[[(5-Aethyl-5,8-dihydro-8-oxo-1,3-dioxolo-[4,5-g]chinolin-7-yl)carbonyl]oxy]methyl]-8-oxo-7-[-(phenoxyacetyl)amino]-5-thia-1-azabicyclo[4.2.0]oct-2-en-2-carbonsäure-Natriumsalz

Eine Lösung von 3.00 g (4,52 mMol) des Endprodukts von Beispiel 5 in 15 ml Anisol wird unter Rühren bei Zimmertemperatur mit 10,6 ml Trifluoressigsäure versetzt. Das Gemisch wird 4 Stunden gerührt und unter vermindertem Druck zur Trockene eingedampft. Man erhält als Rückstand ein Oel, das in Methylenchlorid gelöst und mit Wasser versetzt wird. Der pH-Wert wird mit Natriumbicarbonat auf 7,5 eingestellt. Das Gemisch wird 20 Minuten gerührt wonach man die wässrige Phase entfernt. Die Zugabe von Wasser und das Einstellen des pH-Wertes auf 7,5 mit Natriumbicarbonat unter Rühren werden zweimal wiederholt. Die drei erhaltenen wässrigen Extrakte werden vereinigt und gefriergetrocknet. Der Rückstand wird durch Chromatographie an einer unpolaren, stationären Phase ($C_{18}$ reverse phase) mit 50% wässrigem Methanol als Lösungsmittel gereinigt. Man erhält nach Eindampfen und Gefriertrocknung die Titelverbindung: IR (KBr) 3410, 1765, 1692, 1635, 1528 $cm^{-1}$; Massensprektrum m/z 630 ($M^+$ + H), 652 ($M^+$ + Na).

Beispiel 10

[6R-(6α,7β)(Z)]-7-[[(2-Amino-4-thiazolyl)(methoxyimino) acetyl]amino]-3-[[[(1-äthyl-1,4-dihydro-7-methyl-4-oxo-1,8-naphthyridin-3-yl)carbonyl]oxy]methyl]-8-oxo-5-thia-1-azabicyclo[4.2.0]oct-2-en-2-carbonsäure-Natriumsalz

Eine Aufschlämmung von 0.31 mMol des Endprodukts von Beispiel 6 in 0.66 ml Anisol wird in einer Stickstoffatmosphäre auf 0 °C abgekühlt und mit 3,3 ml Trifluoressigsäure versetzt. Die so erhaltene Lösung wird 18 Stunden bei 0 °C gehalten und anschliessend unter vermindertem Druck bei Zimmertemperatur eingedampft. Nach Zugabe von Methylenchlorid wird das Eindampfen unter vermindertem Druck widerholt. Der Rückstand wird mit Aethylacetat verrieben, damit nach Möglichkeit vor der Ueberführung in das Natriumsalz ein Feststoff erhalten wird. Sollte ein Feststoff nicht anfallen wird der Rückstand in das Natriumsalz direkt übergeführt. In beiden Fällen wird der Feststoff bzw. das erhaltene Oel in 9 ml Methylenchlorid gelöst, wonach man eine wässrige Natriumbicarbonatlösung, enthaltend genügend Natriumbicarbonat, um einen End-pH-Wert von 7,2 bis 7,4 zu erhalten, tropenweise bei 0 bis 3°C mit der so hergestellten Methylenchloridlösung versetzt. Die wässrige Phase wird gefriergetrocknet und anschliessend mit einem analytischen Hochdruckflüssigkeitschromatographen unter Verwendung einer reverse phase Kolonne und Eluieren mit einem Wasser-Methanolgradienten (0-100% Methanol, 20 Minuten) gereinigt. Nach dem Eindampfen und Gefriertrocknen erhält man das Endprodukt: IR (KBr) 3405, 3300, 3200, 1766, 1716, 1681, 1617, 1537 $cm^{-1}$.

Beispiel 11

[6R-(6α,7β)(Z)]-7-[[(2-Amino-4-thiazolyl)(methoxyimino) acetyl]amino]-3-[[[(5-äthyl-5,8-dihydro-8-oxo-1,3-dioxolo-[4,5-g]chinolin-7-yl)carbonyl]oxy]methyl]-8-oxo-5-thia-1-azabicyclo[4.2.0]oct-2-en-2-carbonsäure-Natriumsalz

Die Titelverbindung erhält man nach dem Verfahren von Beispiel 10, jedoch unter Verwendung des Endprodukts von Beispiel 7:

IR (KBr) 3400-3200, 1767, 1715, 1685, 1635, 1616, 1533 $cm^{-1}$; Massenspektrum m/z 679 ($M^+$ + H), 701 ($M^+$ + Na).

Beispiel 12

[6R-(6α,7β)]-3-[[[[1-Aethyl-6-fluor-1,4-dihydro-7-(4-formyl-1-piperazinyl)-4-oxo-chinolin-3-yl]carbonyl]oxy]-methyl]-7-[(phenoxyacetyl)amino]-8-oxo-5-thia-1-azabicyclo[4.2.0]oct-2-en-2-carbonsäure-Natriumsalz

Nach dem Verfahren von Beispeil 10 unter Verwendung des Endprodukts von Beispiel 8 erhält man die Titelverbindung:

IR (KBr) 3420, 1768, 1668, 1620 $cm^{-1}$; Massenspektrum m/z 716 ($M^+$ + H), 738 ($M^+$ + Na).

Beispiel 13

[6R-(6α,7β)]-3-[[[(5-Aethyl-5,8-dihydro-8-oxo-1.3-dioxolo-[4,5-g]chinolin-7-yl)carbonyl]oxy]methyl]-8-oxo-7-[-(2-thienylacetyl)amino]-5-thia-1-azabicyclo[4.2.0]oct-2-en-2-carbonsäure 1,1-dimethyläthylester

Nach dem Verfahren von Beispiel 5, jedoch unter Verwendung von [6R-(6α,7β)] -3-(Jodmethyl)-8-oxo-7-[(2-thienylacetyl)amino] -5-thia-1-azabicyclo[4.2.0]oct-2-en-2-carbonsäure -1,1-dimethyläthylester und 5-Aethyl-5,8-dihydro-7-oxo-1,3-dioxolo[4,5-g]chinolin -7-yl)carbonsäure als Reagenzien erhält man die Titelverbindung.

Beispiel 14

[6R-(6α,7β)]-3-[[[(5-Aethyl-5,8-dihydro-8-oxo-1,3-dioxolo[4,5-g]chinolin-7-yl)carbonyl]oxy]methyl]-8-oxo-7-[(2-thienylacetyl)amino]-5-thia-1-azabicyclo[4.2.0]oct-2-en-2-carbonsäure-Natriumsalz

Die Titelverbindung wird erhalten nach dem Verfahren von Beispiel 10, jedoch unter Verwendung des Endprodukts von Beispiel 13:

IR (KBr) 3410, 1763, 1682, 1632, 1608 $cm^{-1}$; Massenspektrum m/z 620 ($M^+$ + H), 642 ($M^+$ + Na).

Beispiel 15

[6R-(6α,7β)]-3-[[[[1-Aethyl-6-fluor-1,4-dihydro-7-(4-formyl-1-piperzinyl)-4-oxochinolin-3-yl]carbonyl]oxy]-methyl]-7-[(2-thienylacetyl)amino]-8-oxo-5-thia-1-azabicyclo[4.2.0]oct-2-en-2-carbonsäure-1,1-dimethyläthylester

Nach dem Verfahren von Beispiel 5, jedoch unter Verwendung von [6R-(6α,7β)]-3-(Jodmethyl) -8-oxo-7-[(2-thienylacetyl)amino]-5-thia -1-azabicyclo[4.2.0]oct-2-en-2-carbonsäure -1,1-dimethyläthylester und 1-Aethyl-6-fluor-1,4-dihydro -7-(4-formyl-1-piperazinyl) -4-oxo-3-chinolin-carbonsäure-Natriumsalz als Reagenzien, erhält man die Titelverbindung.

Beispiel 16

[6R-(6α,7β)]-3-[[[[1-Aethyl-6-fluor-1,4-dihydro-7-(4-formyl-1-piperzinyl)-4-oxochinolin-3-yl]carbonyl]oxy]-methyl]-7-[(2-thienylacetyl)amino]-8-oxo-5-thia-1-azabicyclo[4.2.0]oct-2-en-2-carbonsäure-Natriumsalz

Nach dem Verfahren von Beispiel 10, jedoch unter Verwendung des Endprodukts von Beispiel 15, erhält man die Titelverbindung:

IR (KBr) 3430, 1765, 1715, 1662, 1623 $cm^{-1}$; Massenspektrum m/z 706 ($M^+$ + H).

Beispiel 17

[6R-(6α,7β)(Z)]-3-[[[[1-Aethyl-6-fluor-1,4-dihydro-7-(4-formyl-1-piperazinyl)-4-oxochinolin-3-yl]carbonyl]oxy]-methyl]-7[[(methoxyimino)[2-(triphenylmethyl)amino-4-thiazolyl]acetyl]amino]-8-oxo-5-thia-1-azabicyclo-[4.2.0]oct-2-en-2-carbonsäure-1,1-dimethyläthylester

Nach dem Verfahren von Beispiel 5, jedoch unter Verwendung von [6R-(6α,7β)(Z)]-3-(Jodmethyl) -7-[-[methoxyimino)-[2-(triphenylmethyl)amino -4-thiazolyl]acetyl]amino]-8-oxo-5-thia-1-azabicyclo[4.2.0]oct-2-en -2-carbonsäure-1,1-dimethyläthylester und 1-Aethyl-6-fluor-1,4-dihydro-7-(4-formyl -1-piperazinyl)-4-oxo-3-chinolin-carbonsäure-Natriumsalz als Reagenzien, erhält man die Titelverbindung.

Beispiel 18

[6R-(6α,7β)(Z)]-7-[[(2-Amino-4-thiazolyl)(methoxyimino)acetyl]amino]-3-[[[[1-äthyl-6-fluor-1,4-dihydro-7-(4-formyl-1-piperazinyl)-4-oxochinolin-3-yl]carbonyl]oxy]methyl]-8-oxo-5-thia-1-azabicyclo[4.2.0]oct-2-en-2-carbonsäure-Natriumsalz

Die Titelverbindung wird erhalten nach dem Verfahren von Beispiel 10, jedoch unter Verwendung des Endprodukts von Beispiel 17:

IR (KBr) 3420, 1765, 1712, 1622 $cm^{-1}$; Massenspektrum m/z 764 ($M^+$ + H).

Beispiel 19

[6R-(6α,7β)]-3-[[[[1-Aethyl-6-fluor-1,4-dihydro-7-(1-pyrrolidinyl)-4-oxochinolin-3-yl]carbonyl]oxy]methyl]-7-[-(phenoxyacetyl)amino]-8-oxo-5-thia-1-azabicyclo[4.2.0]oct-2-en-2-carbonsäure-1,1-dimethyläthylester

Die Titelverbindung erhält man nach dem Verfahren von Beispiel 5, jedoch unter Verwendung von [6R-(6α,7β)]-3-(Jodmethyl) -8-oxo-7-[(phenoxyacetyl)amino] -5-thia-1-azabicyclo[4.2.0]oct-2-en-2-carbonsäure -1,1-dimethyläthylester und 1-Aethyl-6-fluor-1,4-dihydro -4-oxo-7-(1-pyrrolidinyl)-3-chinolincarbonsäure-Natriumsalz als Reagenzien.

Beispiel 20

[6R-(6α,7β)]-3-[[[[1-Aethyl-6-fluor-1,4-dihydro-7-(1-pyrrolidinyl)-4-oxochinolin-3-yl]carbonyl]oxy]methyl]-7-[-(phenoxyacetyl)amino]-8-oxo-5-thia-1-azabicyclo[4.2.0]oct-2-en-2-carbonsäure-Natriumsalz

Die Titelverbindung wird erhalten nach dem Verfahren von Beispiel 10, jedoch unter Verwendung des Endprodukts von Beispiel 19:

IR (KBr) 3410, 1770, 1695, 1628 $cm^{-1}$; Massenspektrum m/z 673 ($M^+$ + H), 695 ($M^+$ + Na).

Beispiel 21

[6R-(6α,7β)(Z)]-3-[[[[1-Aethyl-6-fluor-1,4-dihydro-7-(1-pyrrolidinyl)-4-oxochinolin-3-yl]carbonyl]oxy]methyl]-7-[[(methoxyimino)[2-(triphenylmethyl)amino-4-thiazolyl]acetyl]amino]-8-oxo-5-thia-1-azabicyclo[4.2.0]oct-2-en-2-carbonsäure-1,1-dimethyläthylester

Nach dem Verfahren von Beispiel 5, jedoch unter Verwendung von [6R-(6α,7β)]-3-(Jodmethyl) -7-[-[methoxyimino)[2-(triphenylmethyl)amino] -4-thiazolyl]acetyl]amino]-8-oxo-5-thia -1-azabicyclo[4.2.0]oct-2-en-2-carbonsäure-1,1-dimethyläthylesterund 1-Aethyl-6-fluor-1,4-dihydro-4-oxo-7-(1-pyrrolidinyl)-3-chinolincarbonsäure-Natriumsalz als Reagenzien, erhält man die Titelverbindung.

Beispiel 22

[6R-(6α,7β)(Z)]-7-[[(2-Amino-4-thiazolyl)(methoxyimino) acetyl]amino]-3-[[[[1-äthyl-6-fluor-1,4-dihydro-7-(1-pyrrolidinyl)-4-oxochinolin-3-yl]carbonyl]oxy]methyl]-8-oxo-5-thia-1-azabicyclo[4.2.0]oct-2-en-2-carbonsäure-Natriumsalz

Die Titelverbindung wird nach dem Verfahren von Beispiel 10 erhalten, jedoch unter Verwendung des Endprodukts von Beispiel 21:

IR (KBr) 3455, 3430, 1768, 1682, 1630 $cm^{-1}$; Massenspektrum m/z 722 ($M^{+}$ H).

Beispiel 23

[6R-(6α,7β)]-3-[[[[1-Aethyl-6-fluor-1,4-dihydro-7-[4-(1,1-dimethylethoxy)carbonyl-1-piperazinyl]-4-oxochinolin-3-yl]carbonyl]oxy]methyl]-8-oxo-7-[(phenoxyacetyl)amino]-5-thia-1-azabicyclo[4.2.0]oct-2-en-2-carbonsäure-1,1-dimethyläthylester

Nach dem Verfahren von Beispiel 5, jedoch unter Verwendung von [6R-(6α,7β)]-3-Jodmethyl-8-oxo -7-[-(phenoxyacetyl)amino]-5-thia -1-azabicyclo[4.2.0]oct-2-en-2-carbonsäure -1,1-dimethyläthylester und 1-Aethyl-6-fluor-1,4-dihydro -7-[4-(1,1-dimethyläthoxy)carbonyl-1-piperzinyl]-4-oxo-3-chinolin-carbonsäure-Kaliumsalz als Reagenzien, erhält man die Titelverbindung:

IR 3420, 1787, 1730, 1698, 1510 $cm^{-1}$; Massenspektrum m/z 822 ($M^{+}$ + H).

Beispiel 24

[6R-(6α,7β)]-3-[[[[1-Aethyl-6-fluor-1,4-dihydro-7-[4-(1,1-dimethylethoxy)carbonyl-1-piperazinyl]-4-oxochinolin-3-yl]-carbonyl]oxy]methyl]8-oxo-7-[(phenoxyacetyl)amino]5-thia-1-azabicyclo[4.2.0]oct-2-en-2-carbonsäure-2-propenylester

Nach dem Verfahren von Beispiel 5, jedoch unter Verwendung von [6R-(6α,7β)]-3-Jodmethyl-8-oxo -7-[-(phenoxyacetyl)amino]-5-thia -1-azabicyclo[4.2.0]oct-2-en-2-carbonsäure-2-propenylester und 1-Aethyl-6-fluor-1,4-dihydro-7-[4-(1,1-dimethyläthoxy)carbonyl-1-piperazinyl] -4-oxo-3-chinolin-carbonsäure-Kaliumsalz als Reagenzien, erhält man die Titelverbindung:

IR (KBr) 3415, 3300, 1789, 1729, 1694, 1622 $cm^{-1}$; Massenspektrum m/z 806 ($M^{+}$ + H).

Beispiel 25

[6R-(6α,7β)]-3-[[[[1-Aethyl-6-fluor-1,4-dihydro-7-[4-(1-propenoxy)carbonyl-1-piperazinyl]-4-oxochinolin-3-yl]-carbonyl]oxy]methyl]-8-oxo-7-[(phenoxyacetyl)amino]-5-thia-1-azabicyclo[4.2.0]oct-2-en-2-carbonsäure-2-propenylester

Nach dem Verfahren von Beispiel 5, jedoch unter Verwendung von [6R-(6α,7β)]-3-Jodmethyl-8-oxo -7-[-(phenoxyacetyl)amino]-5-thia-1-azabicyclo[4.2.0]oct -2-en-2-carbonsäure-2-propenylester und 1-Aethyl-6-fluor-1,4-dihydro-7-[4-(2-propenoxy)carbonyl -1-piperazinyl]-4-oxo-chinolin-3-carbonsäure-Kaliumsalz als Reagenzien, erhält man die Titelverbindung:

IR (KBr) 3410, 1789, 1725, 1699, 1622 $cm^{-1}$; Massenspektrum m/z 790 ($M^{+}$ + H).

Beispiel 26

[6R-(6α,7β)]-3-[[[(5-Aethyl-5,8-dihydro-8-oxo-1,3-dioxolo[4,5-g]chinolin-7-yl)carbonyl]oxy]methyl]-8-oxo-7-[-(phenoxyacetyl)amino]-8-oxo-5-thia-1-azabicyclo[4.2.0]oct-2-en-2-carbonsäure-2-propenylester

Nach dem Verfahren von Beispiel 5, jedoch unter Verwendung von [6R-(6α,7β)]-3-Jodmethyl-8-oxo -7-[-(phenoxyacetyl)amino]-5-thia-1-azabicyclo[4.2.0]oct -2-en-2-carbonsäure-2-propenylester und 5-Aethyl-5,8-dihydro-8-oxo-1,3-dioxolo[4,5-g]chinolin-7-carbonsäure-Kaliumsalz als Reagenzien, erhält man die Titelverbindung.

Beispiel 27

Alternative Synthese von [6R-(6α,7β)]-3-[[[(5-Aethyl-5,8-dihydro-8-oxo-1,3-dioxolo[4,5-g]chinolin-7-yl)-carbonyloxy]methyl]-8-oxo-7-[(phenoxyacetyl)amino]-5-thia-1-azabicyclo[4.2.0]oct-2-en-2-carbonsäure-Natriumsalz

Eine Lösung von 337 mg des Endprodukts von Beispiel 26, 0,03 ml Triäthylphosphit, 5,6 mg Palladium-(II)acetat, 3.9 ml Aethylacetat und 5,6 ml Methylenchlorid wird unter Rühren tropfenweise innerhalb 15 Minuten mit 1,68 ml einer 0,5 molar Lösung von Natrium-2-äthylhexanoat in Aethylacetat versetzt. Das Gemisch wird anschliessend 30 Minuten gerührt und mit 11 ml Aceton versetzt, wonach das Rühren 10 Minuten fortgeführt wird. Das Produkt wird abfiltriert, mit Aether gewaschen und luftgetrocknet. Nach Reinigung mittels $C_{18}$-reverse-phase-Hochdruckflüssigkeitschromatographie erhält man die Titelverbindung. Die Spektren dieses Produktes sind mit denjenigen des Produktes von Beispiel 9 identisch.

Beispiel 28

[6R-(6α,7β)]-3-[[[[1-Aethyl-6-fluor-1,4-dihydro-7-(4-thiomorpholinyl)-4-oxochinolin-3-yl]carbonyl]oxy]methyl]-8-oxo-7-[(phenoxyacetyl)amino]-5-thia-1-azabicyclo[4.2.0]oct-2-en-2-carbonsäure-Natriumsalz

Eine Lösung von 0,187 g 1-Aethyl-6-fluor-1,4-dihydro-4-oxo-7-(4-thiomorpholinyl) -3-chinolin-carbonsäure-Natriumsalz in 8 ml Dimethylformamid wird mit 4A Molekularsieb 1 Stunde gerührt. Nach Zugabe von 0.257 g [6R-(6α,7β)]-3-(Jodmethyl) -8-oxo-7-[(phenoxyacetyl)amino]-5-thia -1-azabicyclo[4.2.0]-oct-2-en-2-carbonsäure-2-propenylester in 8 ml Dimethylformamid wird das Gemisch 5 Stunden gerührt und anschliessend unter vermindertem Druck eingedampft. Eine Lösung des Rückstands in Aethylacetat wird nacheinander mit wässriger Natriumbicarbonatlösung und Kochsalzlösung gewaschen, über Natriumsulfat getrocknet und unter vermindertem Druck eingedampft. Der Rückstand wird durch präparative Schichtchromatographie gereinigt, wobei man als Zwischenprodukt den der Titelverbindung entsprechenden Allylester erhält.

Eine Lösung von 63,5 mg dieses Allylesters in 1,1 ml Methylenchlorid und 0,75 ml Aethylacetat wird mit 5,1 µl Triäthylphosphit, 1 mg Palladium(II)acetat und 0,28 ml 0,5M Natrium-2-äthylhexanoatlösung in Aethylacetat versetzt. Das Gemisch wird eine Stunde gerührt, mit 2 ml Aceton versetzt und anschliessend unter vermindertem Druck zur Trockene eingedampft. Der Rückstand wird mit Aether verrieben wobei ein Feststoff anfällt. Nach Reinigung mittels reverse-phase-Hochdruckflüssigkeitschromatographie erhält man die Titelverbindung:

IR (KBr) 3420, 1768, 1695, 1622 $cm^{-1}$; Massenspektrum m/z 705 ($M^+$ + H), 727 ($M^+$ + Na).

Beispiel 29

[6R-(6α,7β)]-3-[[[(1-Aethyl-6-fluor-1,4-dihydro-4-oxo-7-(1H-pyrrol-1-yl)chinolin-3-yl]carbonyl]oxy]methyl]-8-oxo-7-[(phenoxyacetyl)amino]-5-thia-1-azabicyclo[4.2.0]oct-2-en-2-carbonsäure-Natriumsalz

Eine Lösung von 0,845 g 1-Aethyl-6-fluro-1,4-dihydro-4-oxo -7-(1H-pyrrol-1-yl) -3-chinolincarbonsäure-Kaliumsalz in 30 ml Methylformamid wird 1 Stunde mit 4A Molekularsieb gerührt. Anschliessend wird mit 1,54 g [6R-(6α,7β)]-3-Jodmethyl -8-oxo-7-[(phenoxyacetyl)amino]-5-thia-1-azabicyclo[4.2.0]oct -2-en-2-carbonsäure-2-propenylester versetzt und dann 2,5 Stunden gerührt. Das Gemisch wird unter vermindertem Druck eingedampft, mit Aethylacetat versetzt und nacheinander mit wässriger Natriumbicarbonatlösung und Kochsalzlösung gewaschen. Nach dem Trocknen über Natriumsulfat wird das Aethylacetat unter vermindertem Druck eingedampft und der Rückstand durch Schnellchromatographie gereinigt, wobei als Zwischenprodukt der der obigen Titelverbindung entsprechende Allylester erhalten wird.

Eine Lösung von 69 mg dieses Allylesters in 1 ml Methylenchlorid wird mit 0,3 mg Palladium(II)acetat, 1 µl Triäthylphosphit, 0,2 ml 0,5M Natrium-2-äthylhexanoatlösung in Aethylacetat und 0,3 ml Aethylacetat versetzt. Nach 30-minütigem Rühren wird das Gemisch unter vermindertem Druck eingedampft. Der Rückstand wird mit Aether verrieben wobei ein Feststoff anfällt. Man erhält nach Reinigung durch reverse-phase-Hochdruckflüssigkeitschromatographie die Titelverbindung:

IR (KBr) 3420, 1765, 1695, 1620 $cm^{-1}$; Massenspektrum m/z 669 ($M^+$ + H), 691 ($M^+$ + Na).

Beispiel 30

[6R-(6α,7β)]-3-[[[[5-(4-fluorphenyl)-5,8-dihydro-8-oxo-1,3-dioxolo[4,5-g]chinolin-7-yl]carbonyl]oxy]methyl]-8-oxo-7-[(phenoxyacetyl)amino]-5-thia-1-azabicyclo[4.2.0]oct-2-en-2-carbonsäure-Natriumsalz

Eine Lösung von 0,183 g 5-(4-Fluorphenyl)-5,8-dihydro-8-oxo-1,3-dioxolo[4,5-g]chinolin-7-carbonsäure-Kaliumsalz in 8 ml Dimethylformamid wird 1 Stunde mit 4A Molekularsieb gerührt. Nach Zugabe von 0,308 g [6R-(6α,7β)]-3-Jodmethyl-8-oxo-7-[(phenoxyacetyl)amino] -5-thia-1-azabicyclo[4.2.0]oct-2-en-2-carbonsäure -2-propenylester wird das Gemisch 5 Stunden gerührt, anschliessend unter vermindertem Druck eingedampft und mit Aethylacetat versetzt. Das Gemisch wird nacheinander mit wässriger Natriumbicarbonatlösung und Kochsalzlösung gewaschen. Nach dem Trocknen über Natriumsulfat wird das Aethylacetat unter vermindertem Druck abgedampft und der Rückstand durch präparative Schichtchromatographie gereinigt, wobei man als Zwischenprodukt den der Titelverbindung entsprechenden Allylester erhält.

Eine Lösung von 0,122 g dieses Allylesters in 2,1 ml Methylenchlorid und 1,5 ml Aethylacetat wird mit 9,9 µl Triäthylphosphit, 1,94 mg Palladium(II)acetat und 0,51 ml einer 0,5M Natrium-2-äthylhexanoatlösung in Aethylacetat versetzt. Das erhaltene Gemisch wird 1 Stunde gerührt, mit Aceton verdünnt und unter vermindertem Druck eingedampft. Der Rückstand wird mit Aether verrieben wobei ein Feststoff anfällt. Nach Reinigung durch reverse-phase-Hochdruckflüssigkeitschromatographie erhält man die Titelsubstanz:

IR (KBr) 3420, 1765, 1695, 1632 $cm^{-1}$; Massenspektrum m/z 696 ($M^+$ + kH), 718 ($M^+$ + Na).

Beispiel 31

[6R-(6α,7β)(Z)]-7-[[(2-Amino-4-thiazolyl)(methoxyimino)acetyl]amino]-3-[[[[1-äthyl-6-fluor-1,4-dihydro-7-(4-thiomorpholinyl)-4-oxochinolin-3-yl]carbonyl]oxy]methyl]-8-oxo-5-thia-1-azabicyclo[4.2.0]oct-2-en-2-carbonsäure-Natriumsalz

Eine Lösung von 0,374 g 1-Aethyl-6-fluor-1,4-dihydro-4-oxo-7-(4-thiomorpholinyl)-3-chinolincarbonsäure-Kaliumsalz in 16 ml Dimethylformamid wird 1 Stunde mit 1,5 g 4A Molekularsieb gerührt. Nach Zugabe einer Lösung von 1,07 g [6R-(6α,7β)]-3-Jodmethyl -7-[[methoxyimino)-[(2-triphenylmethyl)amino -4-thiazolyl]acetyl]amino] -8-oxo-5-thia-1-azabicyclo[4.2.0]oct-2-en-2-carbonsäure -1,1-dimethyläthylester in 16 ml Dimethylformamid wird das Gemisch 5 Stunden gerührt. Das Gemisch wird unter vermindertem Druck eingedampft, der Rückstand in Aethylacetat aufgenommen, wonach die so erhaltene Mischung nacheinander mit wässriger Natriumbicarbonatlösung und Kochsalzlösung gewaschen wird. Nach dem Trocknen über Natriumsulfat wird die Aethylacetatlösung unter vermindertem Druck eingedampft. Der Rückstand wrid durch Schnellchromatographie gereinigt, wonach man als Zwischenprodukt den der obigen Titelverbindung entsprechenden t-Butylester erhält.

0,103 g dieses t-Butylesters werden in einem Gemisch von 0,21 ml Anisol und 1,06 ml Trifluoressigsäure gelöst und 18 Stunden bei 0°C gehalten. Das Gemisch wird unter vermindertem Druck eingedampft und der Rückstand in Methylenchlorid gelöst. Durch Zugabe von Wasser und Natriumbicarbonat wird der pH-Wert auf 7,5 eingestellt. Die wässrige Phase wird gefriergetrocknet. Nach Reinigung des Rückstandes durch reverse-phase-Hochdruckflüssigkeitschromatographie erhält man die Titelverbindung:

IR (KBr) 3410, 1768, 1715, 1682, 1622 $cm^{-1}$; Massenspektrum m/z 754 ($M^+$ + H).

Beispiel 32

[6R-(6α,7β(Z))]-7-[[(2-Amino-4-thiazolyl)(methoxyimino) acetyl]amino]-3-[[[[6,8-difluor-1-(2-fluoräthyl)-1,4-dihydro-7-(4-methyl-1-piperazinyl)-4-oxo-3-chinolinyl]carbonyl]oxy]methyl]-8-oxo-5-thia-1-azabicyclo[4.2.0]oct-2-en-2-carbonsäure-Mononatriumsalzdihydrat

Eine Aufschlämmung von 5 g 6,8-Difluor-1-(2-fluoräthyl)-1,4-dihydro -7-(4-methyl-1-piperazinyl)-4-oxo-3-chinolincarbonsäure-Kaliumsalzin 40 ml Dimethylformamid wird innerhalb 5 Minuten mit einer Lösung von 10 g [6R-(6α,7β)]-3-(Jodmethyl) -7-[[methoxyimino)[2-(triphenylmethyl)amino] -4-thiazolyl-acetyl]amino] -8-oxo-5-thia-1-azabicyclo[4.2.0]oct-2-en-2-carbonsäure-1,1-dimethyläthylester in 40 ml Dimethylformamid versetzt. Das Gemisch wird 2 Stunden gerührt und anschliessend unter vermindertem Druck eingedampft. Der Rückstand wird zwischen Aethylacetat und wässriger Natriumbicarbonatlösung verteilt. Die organische Phase wird nacheinander mit wässriger Natriumbicarbonatlösung und Kochsalzlösung getrocknet, über Magnesiumsulfat getrocknet und eingedampft wobei ein brauner Schaum anfällt. Reinigung durch Schnellchromatographie an 500 g Kieselgel (0,04-0,08 mm) mit Methanol-Chloroform (Gradient von 0-8% Methanol)

erhält man als Zwischenprodukt den der obigen Titelverbindung entsprechenden 1,1-Dimethyläthylester.

Eine Lösung von 2.30 g dieses 1,1-Dimethyläthylesters in 25 ml Methylenchlorid wird bei 0 °C mit 2 ml Anisol, 0,2 ml 1,2-Aethandithiol und 25 ml Trifluoressigsäure versetzt. Das Gemisch wird 3,5 Stunden bei 0 °C gerührt und anschliessend bei 0 °C unter vermindertem Druck eingedampft. Der Rückstand wird mit Methylenchlorid versetzt und das Eindampfen wiederholt. Der Rückstand wird mit 5 ml kaltem Aethylacetat und anschliessend mit 25 ml Aether versetzt, wobei ein Feststoff ausfällt. Nach Filtrieren, Waschen mit Aether und Trocknen an der Luft erhält man einen bräunlichen Feststoff. Dieser wird in 100 ml 1:4 Methanol-Chloroform gelöst und unter Eiskühlung mit 100 ml Wasser und 35 ml 5% wässriger Natriumbicarbonatlösung bis zum pH 7,8 versetzt. Das dabei ausgefällte Harz wird in 10 ml Dimethylformamid gelöst und mit 20 ml Chloroform und anschliessend Wasser und wässriger Natriumbicarbonatlösung bis zum pH 7,8 versetzt. Die vereinigten, das Endprodukt enthaltenden wässrigen Auszüge werden mit Chloroform gewaschen und durch $C_{18}$-reverse-phase-Chromatographie mit Hilfe eines Wasser/Acetonitril-gradienten als Lösungsmittel gereinigt. Die das Endprodukt enthaltenden Fraktionen werden unter vermindertem Druck eingedampft und gefriergetrocknet, wobei man die Titelverbindung erhält:

Massenspektrum m/z 787 ($M^+$ + H).

Die folgenden Verbindungen können durch die in den Beispielen veranschaulichten Methoden hergestellt werden:

[6R-[6α,7β(Z)]]-7-[[(2-Amino-4-thiazolyl)(methoxyimino)acetyl]amino]-3-[[[[1-äthyl-6-fluor-1,4-dihydro-7-(1-piperazinyl)-4-oxochinolin-3-yl]carbonyl]oxy]methyl]-8-oxo-5-thia-1-azabicyclo[4.2.0]oct-2-en-2-carbonsäure

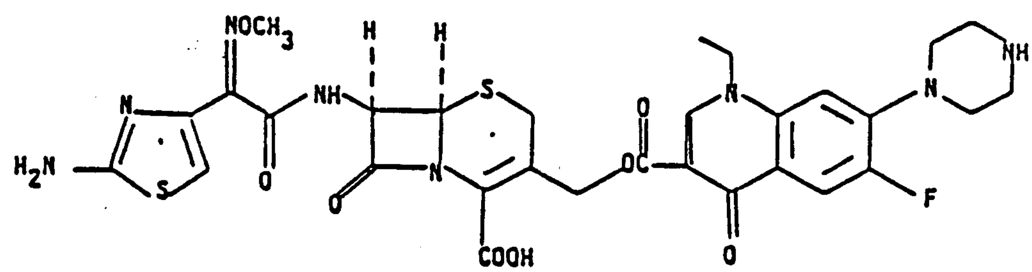

[6R-(6α,7β)]-3-[[[[1-Aethyl-6-fluor-1,4-dihydro-4-oxo-7-(1-piperazinyl-chinolin-3-yl]carbonyl]oxy]methyl]-8-oxo-7-[(phenoxyacetyl)amino]-5-thia-1-azabicyclo[4.2.0]oct-2-en-2-carbonsäure

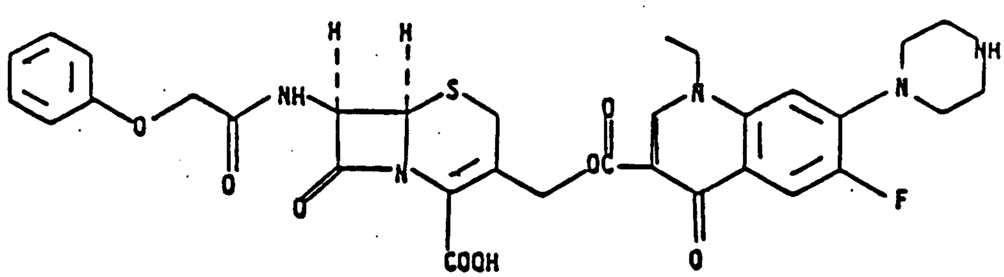

[6R-(6α,7β)]-3-[[[[6,8-Difluor-1-(2-fluoräthyl)-1,4-dihydro-7-(4-methyl-1-piperazinyl)-4-oxochinolin-3-yl]-carbonyl]oxy]methyl]-8-oxo-7-[(phenoxyacetyl)amino]-5-thia-1-azabicyclo[4.2.0]oct-2-en-2-carbonsäure

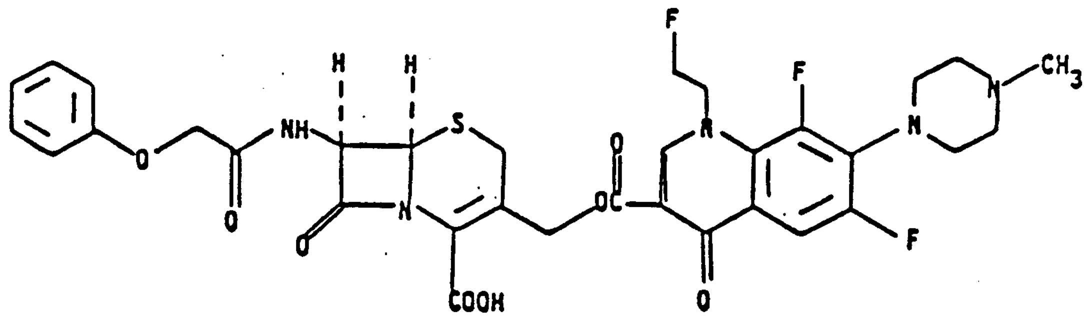

[6R-6α,7β(Z)]]-7-[[(2-Amino-4-thiazolyl)(methoxyimino)acetyl]amino]-3-[[[[-äthyl-6-fluor-1,4-dihydro-7-(4-methyl-1-piperazinyl)-4-oxochinolin-3-yl]carbonyl]oxy]methyl]-8-oxo-5-thia-1-azabicyclo[4.2.0]oct-2-en-2-carbonsäure

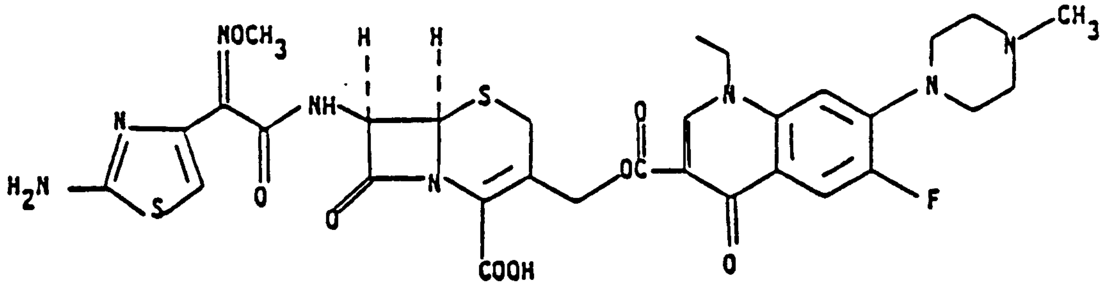

[6R-(6α,7β)]-3-[[[[1-Aethyl-6-fluor-1,4-dihydro-7-(4-methyl-1-piperazinyl)-4-oxochinolin-3-yl]carbonyl]oxy]-methyl]-3-oxo-7-[(phenoxyacetyl)amino]-5-thia-1-azabicyclo[4.2.0]oct-2-en-2-carbonsäure

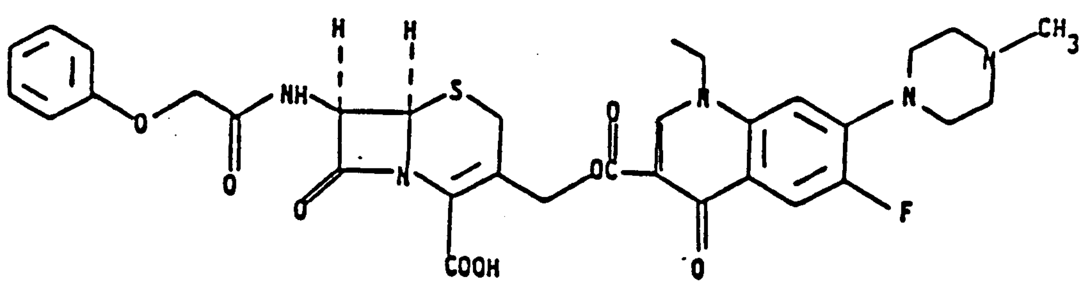

[6R-(6α,7β)]-3-[[[[1-Cyclopropyl-6-fluor-1,4-dihydro-7-(1-piperazinyl)-4-oxochinolin-3-yl]carbonyl]oxy]methyl]-8-oxo-7-[(phenoxyacetyl)amino]-5-thia-1-azabicyclo[4.2.0]oct-2-en-2-carbonsäure

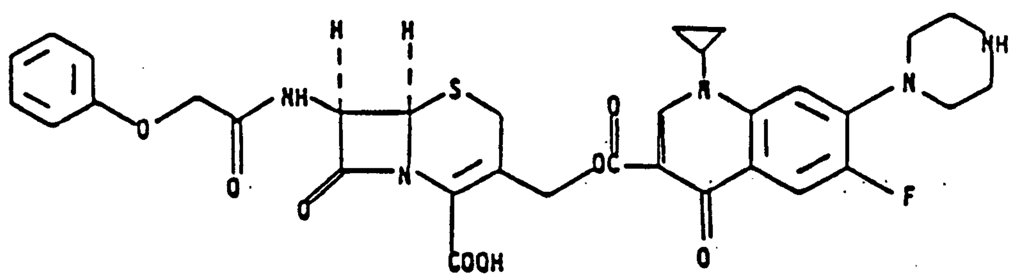

[6R-[6α,7β(Z)]]-7-[[(2-Amino-4-thiazolyl)(methoxyimino)acetyl]amino]-3-[[[[1-cyclopropyl-6-fluor-1,4-dihydro-4-oxo-7-(1-piperazinyl)-chinolin-3-yl]carbonyl]oxy]methyl]-8-oxo-5-thia-1-azabicyclo[4.2.0]oct-2-en-3-carbonsäure

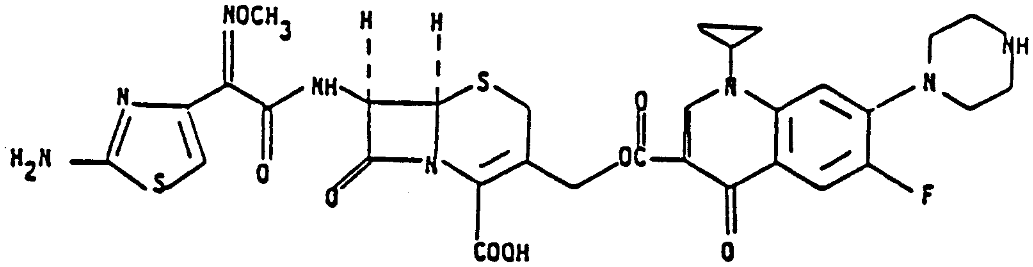

[6R-(6α,7β)]-7-[(Cyanacetyl)amino]-3-[[[[6,8-difluor-1-(2-fluoräthyl)-1,4-dihydro-7-(4-methyl-1-piperazinyl)-4-oxochinolin-3-yl]carbonyl]oxy]methyl]-8-oxo-5-thia-1-azabicyclo[4.2.0]oct-2-en-2-carbonsäure

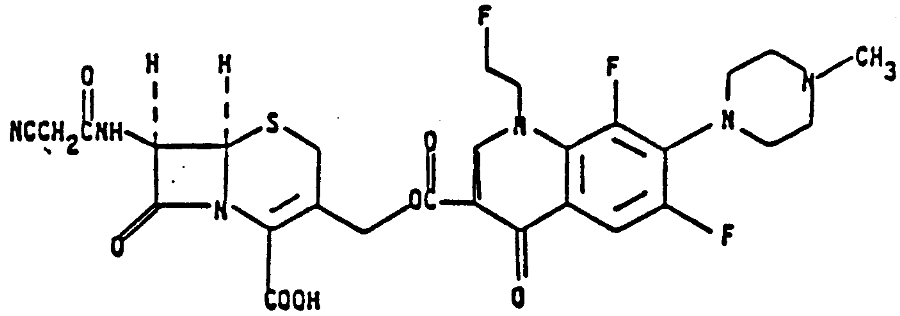

[6R,(6α,7β)]-7-(Formylamino)-3-[[[[6,8-difluor-1-(2-fluoräthyl)-1,4-dihydro-7-(4-methyl-1-piperazinyl)-4-oxochinolin-3-yl]carbonyl]oxy]methyl]-8-oxo-5-thia-1-azabicyclo[4.2.0]oct-2-en-2-carbonsäure

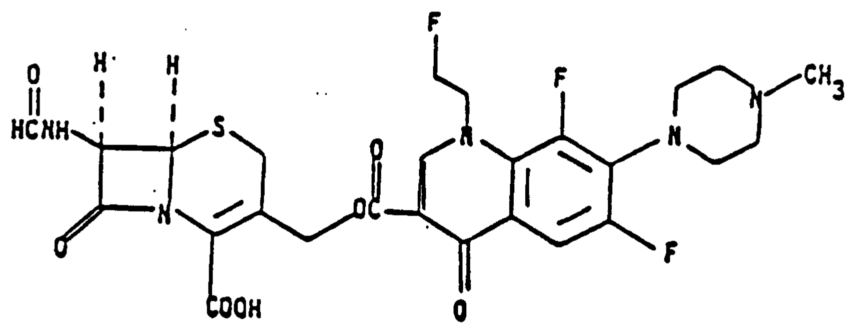

[6R-(6α,7β]-7-[[[[4-Aethyl-2,3-dioxo-1-piperazinyl)carbonyl]amino]phenylacetyl]amino]-3-[[[[6,8-difluor-1-(2-fluoräthyl]-1,4-dihydro-4-oxochinolin-3-yl]carbonyl]oxy]methyl]-8-oxo-5-thia-1-azabicyclo[4.2.0]oct-2-en-2-carbonsäure

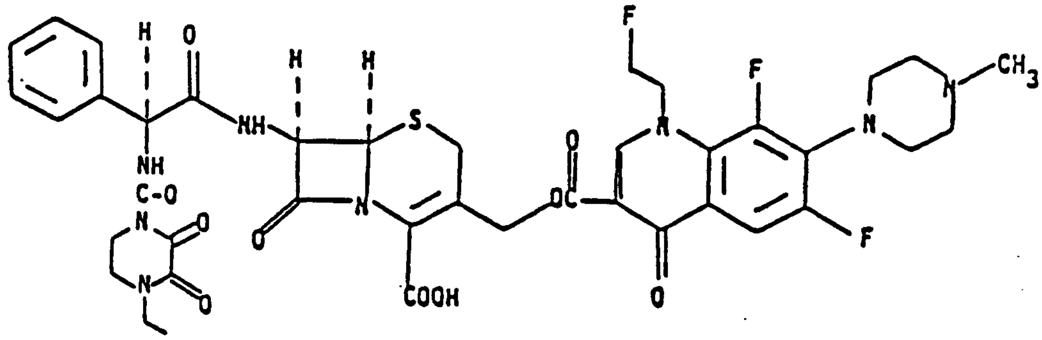

[6R-[6α,7β(Z)]]-7-[[2-Amino-4-thiazolyl)[[(1-carboxy-1-methyl)äthoxy]imino]acetyl]amino]-3-[[[[6,8-difluor-1-(2-fluoräthyl)-1,4-dihydro-7-(4-methyl-1-piperazinyl)-4-oxochinolin-3-yl]carbonyl]oxy]methyl]-8-oxo-5-thia-1-azabicyclo[4.2.0]oct-2-en-2-carbonsäure

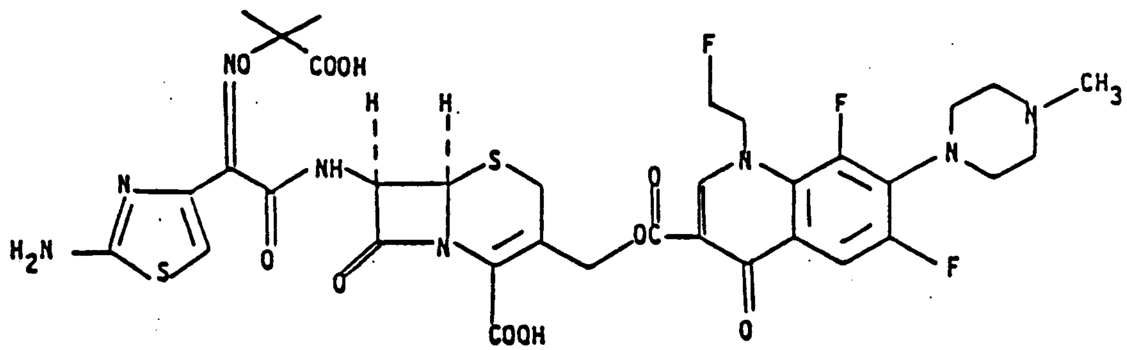

[6R-[6α,7β(Z)]]-7-[[[(2-Amino-4-thiazolyl)(carboxymethoxy)imino]acetyl]amino]-3-[[[[6,8-difluor-1-(2-fluoräthyl)-1,4-dihydro-7-(4-methyl-1-piperazinyl)-4-oxochinolin-3-yl]carbonyl]oxy]methyl]-8-oxo-5-thia-1-azabicyclo[4.2.0]oct-2-en-2-carbonsäure

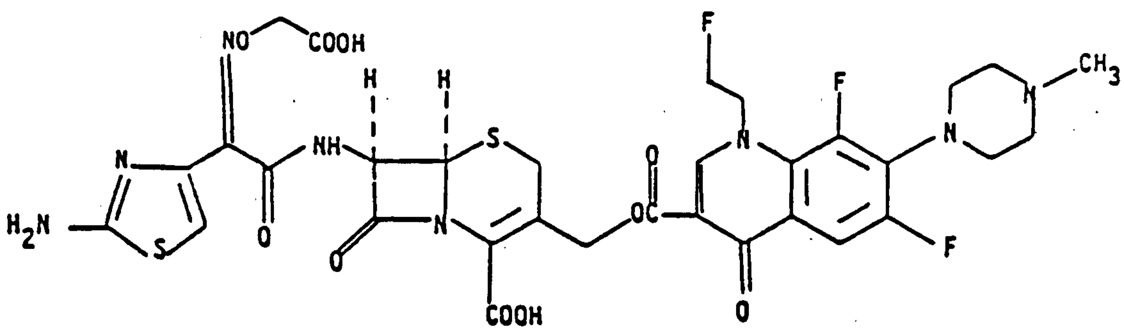

[6R-(6α,7β)]-3-[[[[6,8-Difluor-1-(2-fluoräthyl)-1,4-dihydro-7-(4-methyl-1-piperazinyl)-4-oxochinolin-3-yl]-carbonyl]oxy]methyl]-8-oxo-7-[(phenylacetyl)amino]-5-thia-1-azabicyclo[4.2.0]oct-2-en-2-carbonsäure

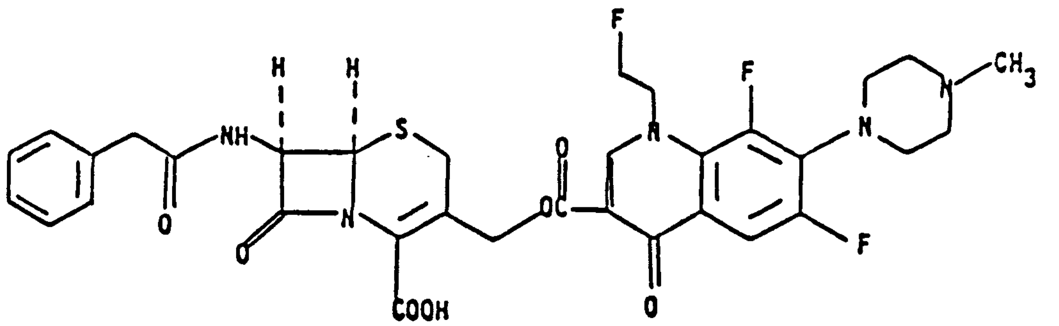

[6R-(6α,7β)]-3-[[[[6,8-Difluor-1-(2-fluoräthyl-1,4-dihydro-7-(4-methyl-1-piperazinyl)-4-oxochinolin-3-yl]-carbonyl]oxy]methyl]-8-oxo-7-[2-thienylacetyl)amino]-5-thia-1-azabicyclo[4.2.0]oct-2-en-2-carbonsäure

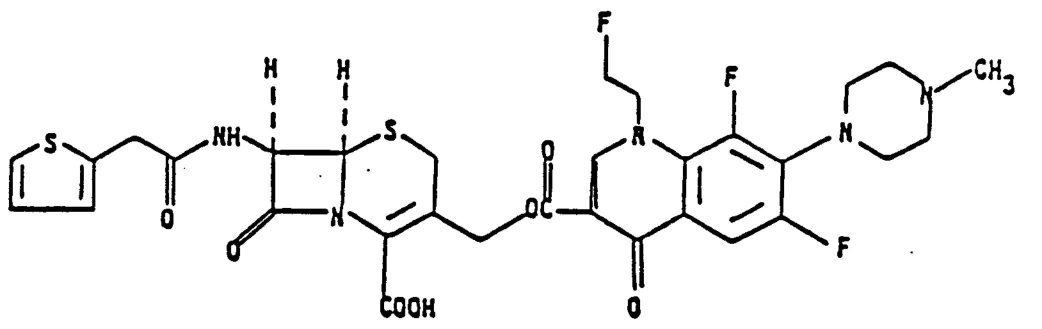

[6R-[6α,7β(Z)]]-7-[[(2-Amino-4-thiazolyl)(methoxyimino)acetyl]amino]-3-[[[[6,8-difluor-1-(4-fluorphenyl)-1,4-dihydro-7-(4-methyl-1-piperazinyl)-4-oxochinolin-3-yl]carbonyl]oxy]methyl]-8-oxo-5-thia-1-azabicyclo[4.2.0]-oct-2-en-2-carbonsäure

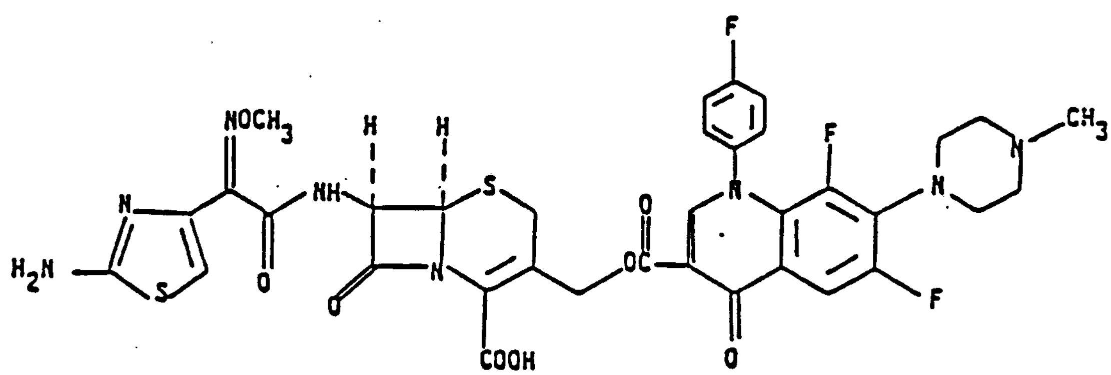

[6R-(6α,7β)]-3-[[[[6-Fluor-1-(4-fluorphenyl)-1,4-dihydro-7-(4-methyl-1-piperazinyl)-4-oxochinolin-3-yl]-carbonyl]oxy]methyl]-8-oxo-7-[(phenoxyacetyl)amino]-5-thia-1-azabicyclo[4.2.0]oct-2-en-2-carbonsäure

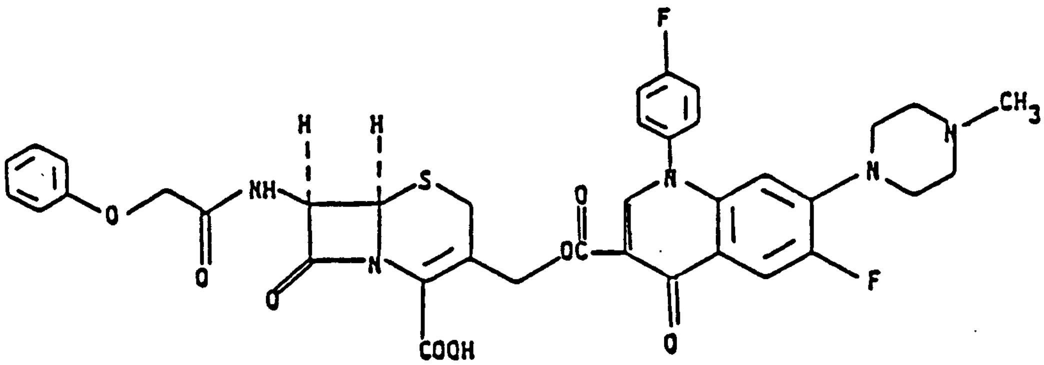

[6R-(6α,7β)]-3-[[[[9-Fluor-3,7-dihydro-3-methyl-10-(4-methyl-1-piperazinyl)-7-oxo-2H-pyrido[1,2,3-de]-1,4-benzoxazin-6-yl]carbonyl]oxy]methyl]-3-oxo-7-[(phenoxyacetyl)amino]-5-thia-1-azabicyclo[4.2.0]oct-2-en-2-carbonsäure

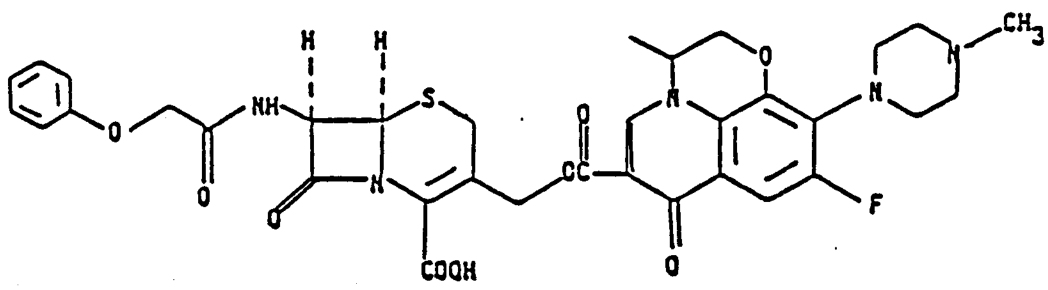

[6R-[6α,7β(Z)]]-7-[[2-Amino-4-thiazolyl)(methoxyimino)acetyl]amino]-3-[[[[9-fluor-3,7-dihydro-3-methyl-10-(4-methyl-1-piperazinyl)-7-oxo-2H-pyrido[1,2,3-de]-1,4-benzoxazin-6-yl]carbonyl]oxy]methyl]-8-oxo-5-thia-1-azabicyclo[4.2.0]oct-2-en-2-carbonsäure

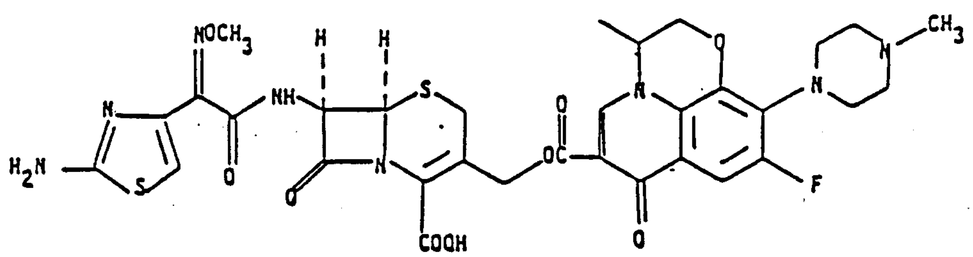

[6R-(6α,7β)]-α-[[[2-Carboxy-3-[[[[6,8-difluor-1-(2-fluoräthyl)-1,4-dihydro-7-(4-methyl-1-piperazinyl)-4-oxochinolin-3-yl]carbonyl]oxy]methyl]-8-oxo-5-thia-1-azabicyclo[4.2.0]oct-2-en-7-yl]amino]carbonyl]-benzoesäure

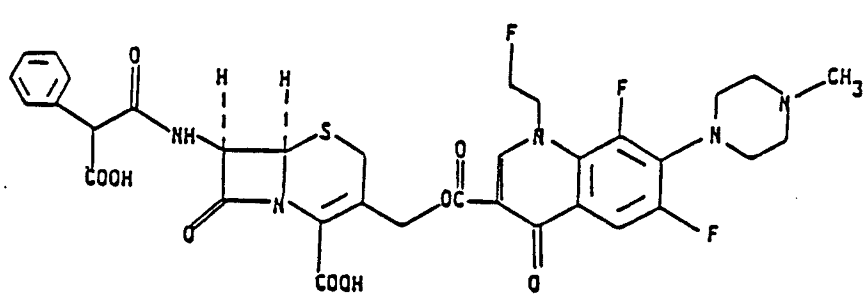

[6R-[6α,7β(Z)]]-3-[[[[7-(3-Amino-1-pyrrolidinyl)-8-chlor-1-cyclopropyl-6-fluor-1,4-dihydro-4-oxochinolin-3-yl]-carbonyl]oxy]methyll]-7-[[(2-amino-4-thiazolyl)[(carboxymethoxy)imino]acetyl]amino]-8-oxo-5-thia-1-azabicyclo[4.2.0]oct-2-en-2-carbonsäure

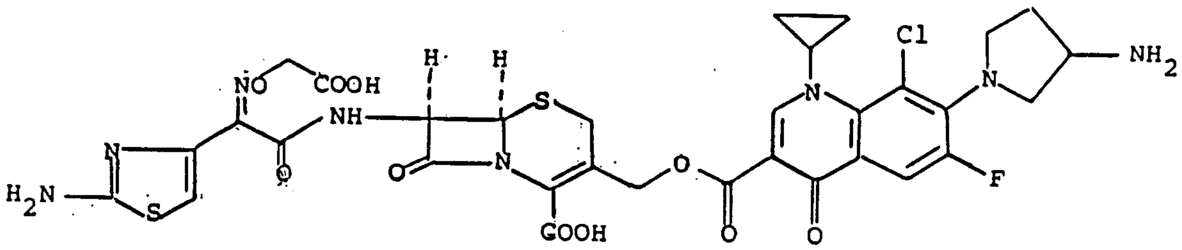

[6R-[6α,7β(Z)]]-3-[[[[7-(3-Amino-1-pyrrolidinyl)-8-chlor-1-cyclopropyl-6-fluor-1,4-dihydro-4-oxochinolin-3-yl]-carbonyl]oxy]methyl]-7-[[(2-amino-4-thiazolyl)[(1-carboxy-1-methyläthoxy)imino]acetyl]amino]-8-oxo-5-thia-1-azabicyclo[4.2.0]oct-2-en-2-carbonsäure

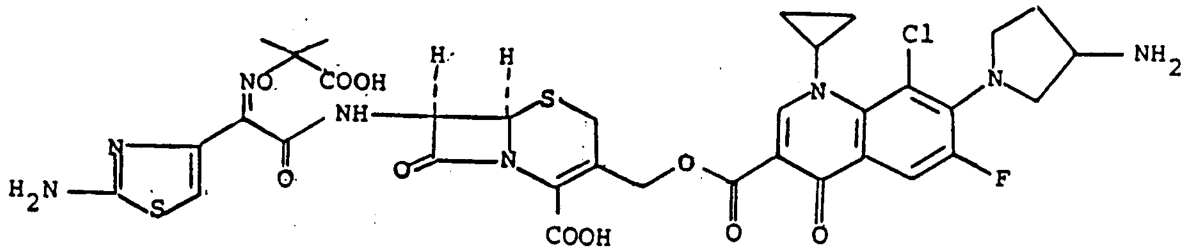

[6R-[6α,7β(Z)]]-3-[[[[7-(3-Amino-1-pyrrolidinyl)-8-chlor-1-cyclopropyl-6-fluor-1,4-dihydro-4-oxochinolin-3-yl]-carbonyl]oxy]methyl]-7-[[(2-amino-4-thiazolyl)(methoxyimino)acetyl]amino]-8-oxo-5-thia-1-azabicyclo[4.2.0]-oct-2-en-2-carbonsäure

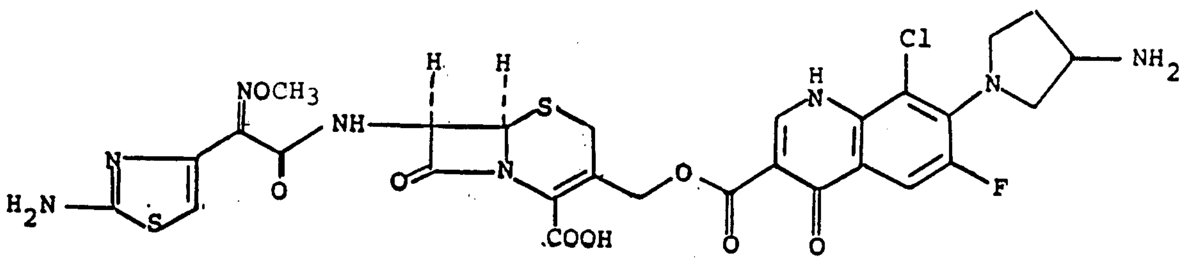

Folgendes Beispiel veranschaulicht pharmazeutische Zubereitungsformen enthaltend die erfindungsgemässen Cephalosporinderivate:

Beispiel A

Herstellung von Trockenampullen für die intramuskuläre Verabreichung:

Es wird in üblicher Weise ein Lyophilisat von 1 g des [6R-(6α,7β(Z))]-7-[[(2-Amino -4-thiazolyl-(methoxyimino)acetyl]amino]-3-[[[[6,8-difluor -1-(2-fluorethyl)-1,4-dihydro-7-(4-methyl-1-piperazinyl) -4-oxochinolin-3-yl]carbonyl]oxy]methyl]-8-oxo-5-thia -1-azabicyclo[4.2.0]oct-2-en -2-carbonsäure-Mononatriumsalzdihydrats hergestellt und in eine Ampulle abgefüllt. Die sterile Wasserampulle enthält 10% Propylenglykol. Vor der Verabreichung wird das Lyophilisat mit 2,5 ml einer 2%-igen wässrigen Lidocainhydrochloridlösung versetzt.

**Patentansprüche**

**Patentansprüche für folgende Vertragsstaaten : BE, CH, DE, FR, GB, IT, LI, LU, NL, SE**

**1.** Acylderivate der allgemeinen Formel

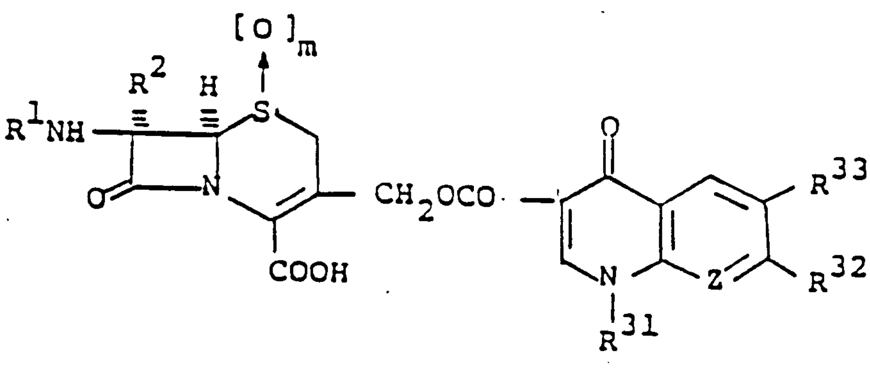

I

in der m 0, 1 oder 2, $R^1$ Wasserstoff oder eine Acylgruppe, $R^2$ Wasserstoff $C_1$-$C_8$- Alkoxy, Amino, $C_1$-$C_8$-Alkylthio oder $C_1$-$C_7$- Alkanoylamino, $R^{31}$ Wasserstoff, $C_1$-$C_8$- Alkyl, $C_2$-$C_6$- Alkenyl, $C_3$-$C_7$-Cycloalkyl, Halogen- $C_1$-$C_8$- Alkyl oder Halogenphenyl; Z $R^{30}$-C oder Stickstoff, $R^{30}$ Wasserstoff oder Halogen, oder $R^{30}$ und $R^{31}$ zusammen eine $C_3$-$C_5$-Alkylengruppe, eine $C_2$-$C_4$ -Alkylenmonooxygruppe oder eine $C_1$-$C_2$-Alkylendioxygruppe, $R^{32}$ Wasserstoff, Halogen, $C_1$-$C_8$- Alkyl oder einen gegebenenfalls durch $C_1$-$C_8$- Alkyl, $C_1$-$C_8$- Alkoxy, Halogen, Halogen- $C_1$-$C_8$- Alkyl, Amino, Mercapto, Hydroxy, Carbamoyl oder Carboxy substituierten 5- oder 6-gliedrigen heterocyclischen Ring mit einem, zwei oder drei Sauerstoff-, Stickstoff- und/oder Schwefelatomen und $R^{33}$ Wasserstoff oder Halogen oder $R^{32}$ und $R^{33}$ zusammen eine $C_1$-$C_4$-Alkylendioxygruppe darstellen,
sowie leicht hydrolysierbare Ester und Salze dieser Verbindungen und Hydrate der Verbindungen der Formel I bzw. von deren Estern und Salzen.

**2.** Verbindungen nach Anspruch 1, dadurch gekennzeichnet, dass m 0 und $R^2$ Wasserstoff darstellt.

**3.** Verbindungen nach Anspruch 1 oder 2, dadurch gekennzeichnet, dass Z $R^{30}$-C darstellt, wobei $R^{30}$ Wasserstoff oder Halogen, $R^{31}$ $C_1$-$C_8$- Alkyl, Halogen-$C_1$-$C_8$- Alkyl oder $C_3$-$C_7$-Cycloalkyl, $R^{32}$ $C_1$-$C_8$-Alkyl, Piperazinyl oder ($C_1$-$C_8$-Alkyl)-Piperazinyl und $R^{33}$ Wasserstoff oder Halogen darstellen.

**4.** Verbindungen nach Anspruch 3, dadurch gekennzeichnet, dass $R^{30}$ Wasserstoff oder Fluor, $R^{31}$ Aethyl, Fluoräthyl oder Cyclopropyl, $R^{32}$ Piperazinyl oder 4-Methylpiperazinyl und $R^{33}$ Wasserstoff oder Fluor darstellen.

**5.** Verbindungen nach einem der Ansprüche 1-4, dadurch gekennzeichnet, dass $R^1$ eine der folgenden Acylgruppen darstellt:

(a) eine aliphatische Gruppe der Formel

$R^5$-CO-

in der $R^5$ $C_1$-$C_8$- Alkyl, $C_3$-$C_7$-Cycloalkyl, $C_1$-$C_8$- Alkoxy, $C_2$-$C_6$- Alkenyl, $C_3$-$C_7$-Cycloalkenyl oder Cyclohexadienyl oder durch einen oder mehrere der Reste Halogen, Cyan, Nitro, Amino, Mercapto, $C_1$-$C_8$- Alkylthio oder Cyanmethylthio substituiertes $C_1$-$C_8$- Alkyl oder $C_2$-$C_6$- Alkenyl darstellt;

(b) eine carbocyclische aromatische Gruppe der allgemeinen Formel

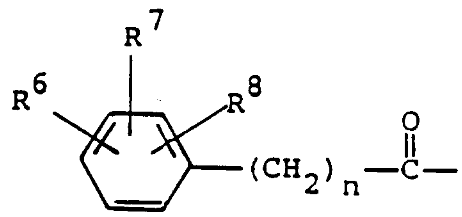

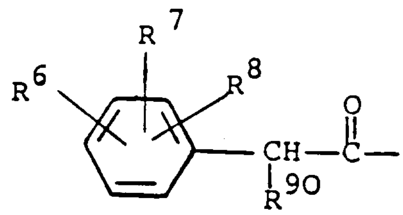

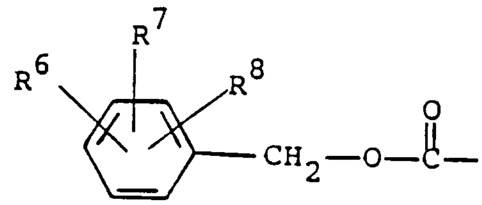

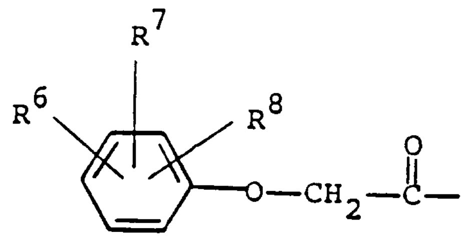

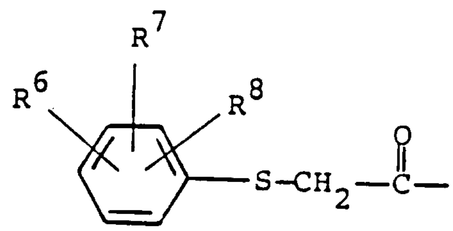

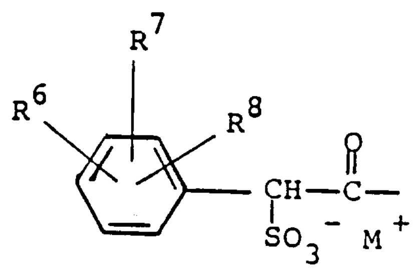

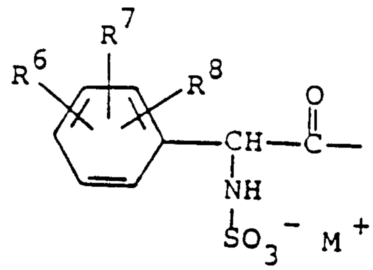

in der n 0, 1, 2 oder 3; $R^6$, $R^7$ und $R^8$ Wasserstoff, Halogen, Hydroxy, Nitro, Amino, Cyano, Trifluormethyl, $C_1$-$C_4$-Alkyl, $C_1$-$C_4$-Alkoxy oder Aminomethyl, $R^{90}$ Amino, Hydroxy, ein Carboxysalz, geschütztes Carboxy, Formyloxy oder Azido und M ein Kation darstellen;
(c) eine heteroaromatische Gruppe der allgemeinen Formeln

$R^{101}$-$(CH_2)_n$-CO-

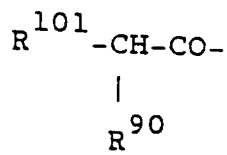

$R^{101}$-O-$CH_2$-CO-
$R^{101}$-S-$CH_2$-CO-

or

$R^{101}$-CO-CO-

in denen $R^{90}$ die oben gegebene Bedeutung hat, n 0, 1, 2 oder 3 und $R^{101}$ einen gegebenenfalls durch $C_1$-$C_8$- Alkyl, $C_1$-$C_8$- Alkoxy, Halogen, Halogen- $C_1$-$C_8$- Alkyl, Amino, Mercapto, Hydroxy, Carbamoyl oder Carboxy substituierten 5-, 6- oder 7-gliedrigen heterocyclischen Ring mit 1, 2, 3 oder 4 Stickstoff-, Sauerstoff- und/oder Schwefelatomen darstellt;
(d) eine [[4-Substituierte-2,3-dioxo -1-piperazinyl)carbonyl]amino]arylacetylgruppe der Formel

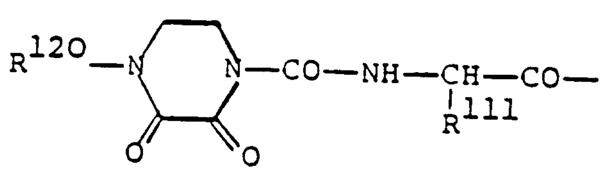

in der $R^{111}$ $C_1$-$C_8$- Alkyl, Hydroxy- $C_1$-$C_8$- Alkyl oder eine aromatische Gruppe der allgemeinen Formel

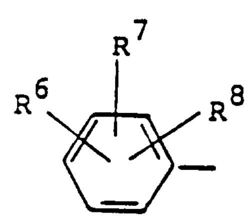

(in der $R^6$, $R^7$ und $R^8$ die oben gegebenen Bedeutung haben) oder einen wie für $R^{101}$ definierten heterocyclischen Ring und $R^{120}$ gegebenenfalls durch einen oder mehrere der Reste Halogen, Cyan, Nitro, Amino und/oder Mercapto substituiertes $C_1$-$C_8$- Alkyl darstellen;
(e) eine (substituierte Oxiimino)arylacetylgruppe der allgemeinen Formel

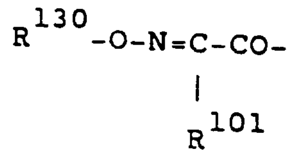

in der $R^{101}$ die oben gegebene Bedeutung hat und $R^{130}$ Wasserstoff, $C_1$-$C_8$- Alkyl, $C_3$-$C_7$-Cycloalkyl, Carboxy-$C_3$-$C_7$-cycloalkyl oder substituiertes $C_1$-$C_8$- Alkyl darstellt, wobei die $C_1$-$C_8$-Alkylgruppe substituiert ist durch einen oder mehrere der Reste Halogen, Cyan, Nitro, Amino, Mercapto, $C_1$-$C_8$- Alkylthio, eine durch $R^{111}$ oben definierte aromatische Gruppe, Carboxy (einschliesslich dessen Salze), $C_1$-$C_7$-Alkanoylamino, $C_2$-$C_9$- Alkoxycarbonyl, Phenylmethoxycarbonyl, Diphenylmethoxycarbonyl, Hydroxy($C_1$-$C_8$-Alkoxy)phosphinyl, Dihydroxyphosphinyl, Hydroxy-(phenylmethoxy)phosphinyl und/oder di($C_1$-$C_8$- Alkoxy)phosphinyl;
(f) eine (Acylamino)arylacetylgruppe der allgemeinen Formel

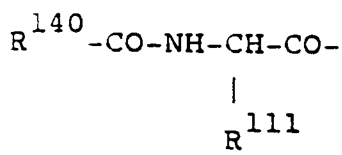

in der $R^{111}$ die oben gegebene Bedeutung hat und $R^{140}$ eine Gruppe der allgemeinen Formel

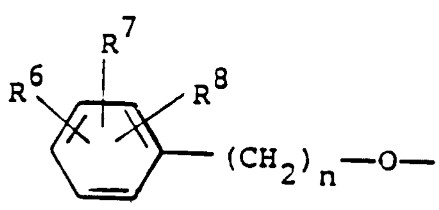

(worin $R^6$, $R^7$, $R^8$ und n die obige Bedeutung haben), Wasserstoff, $C_1$-$C_8$-Alkyl, substituiertes $C_1$-$C_8$-Alkyl, Amino, $C_1$-$C_8$-Alkylamino oder Cyan-$C_1$-$C_8$-alkylamino darstellt;
(g) eine [[[3-Substituierte-2-oxo -1-imidazolidinyl]carbonyl]amino]arylacetylgruppe der allgemeinen Formel

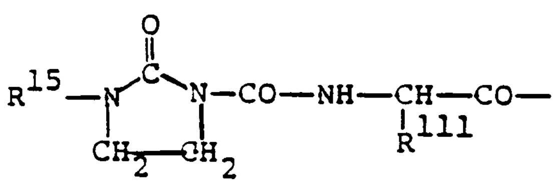

in der $R^{111}$ die oben gegebene Bedeutung hat, und $R^{15}$ Wasserstoff, $C_1$-$C_8$-Alkysulfonyl, -N=CH-$R^{111}$ (worin $R^{111}$ die oben gegebene Bedeutung hat), $R^{16}$-CO(worin $R^{16}$ Wasserstoff oder gegebenenfalls durch Halogen substituiertes $C_1$-$C_8$-Alkyl darstellt), eine wie unter $R^{111}$ oben definierte aromatische Gruppe oder gegebenenfalls durch einen oder mehrere der Reste Halogen, Cyan, Nitro, Amino und/oder Mercapto substituiertes $C_1$-$C_8$-Alkyl darstellt.

6. Verbindungen nach einem der Ansprüche 1-5, dadurch gekennzeichnet, dass $R^1$ eine carbocyclische aromatische Gruppe der allgemeinen Formel

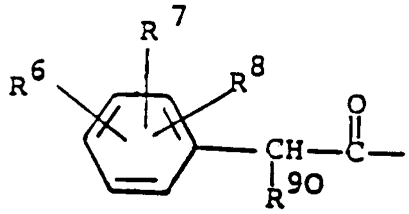

darstellt, in der $R^{90}$ Amino, Hydroxy,
ein Carboxysalz, Benzyloxycarbonyl, Formyloxy oder Azido und $R^6$, $R^7$ und $R^8$ Wasserstoff, Halogen, Hydroxy, Nitro, Amino, Cyan, Trifluormethyl, $C_1$-$C_4$-Alkyl, $C_1$-$C_4$-Alkoxy oder Aminomethyl darstellen.

7. Verbindungen nach Anspruch 6, dadurch gekennzeichnet, dass $R^6$, $R^7$ und $R^8$ Wasserstoff und $R^{90}$ Wasserstoff oder Hydroxy darstellen.

8. Verbindungen nach einem der Ansprüche 1-5, dadurch gekennzeichnet, dass $R^1$ eine Acylgruppe der allgemeinen Formel

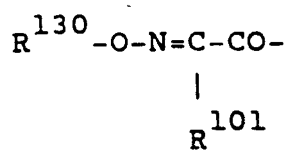

darstellt, in der $R^{101}$ eine gegebenenfalls durch Halogen, Hydroxy, Nitro, Amino, Cyan, Trifluormethyl, $C_1$-$C_4$-Alkyl oder $C_1$-$C_4$-Alkoxy substituierten 5-, 6- oder 7-gliedrigen heterocyclischen Ring mit 1, 2, 3 oder 4 Stickstoff-, Sauerstoff- und/oder Schwefelatomen und $R^{130}$ Wasserstoff, $C_1$-$C_8$- Alkyl, $C_3$-$C_7$-Cycloalkyl, Carboxy-$C_3$-$C_7$-Cycloalkyl oder durch einen oder mehrere der Reste Halogen, Cyan, Nitro, Amino, Mercapto, $C_1$-$C_8$- Alkylthio, eine durch $R^{111}$ gemäss Anspruch 5 definierte aromatische Gruppe, Carboxy (einschliesslich dessen Salze), $C_1$-$C_7$- Alkanoylamino, $C_2$-$C_9$- Alkoxycarbonyl, Phenylmethoxycarbonyl, Diphenylmethoxycarbonyl, Hydroxy $C_1$-$C_8$- Alkoxy)phosphinyl, Dihydroxyphosphinyl, Hydroxy(phenylmethoxy)phosphinyl und/oder Di( $C_1$-$C_8$- Alkoxy)phosphinyl substituiertes $C_1$-$C_8$- Alkyl bedeuten.

9. Verbindungen nach einem der Ansprüche 1-5 und 8, dadurch gekennzeichnet, dass $R^1$ eine Acylgruppe der allgemeinen Formel

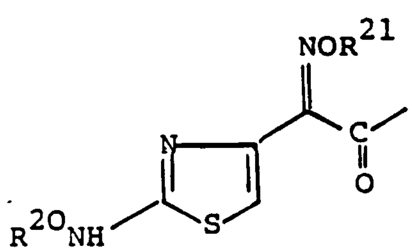

darstellt, in der $R^{20}$ Wasserstoff oder eine Aminoschutzgruppe und $R^{21}$ Wasserstoff, $C_1$-$C_8$- Alkyl oder eine Gruppe der allgemeinen Formel

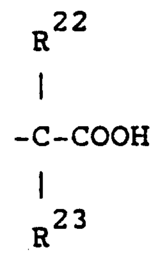

darstellen, worin $R^{22}$ und $R^{23}$ Wasserstoff, $C_1$-$C_8$-Alkyl oder zusammen mit dem verbindenden Kohlenstoffatom einen 3-7-gliedrigen carbocyclischen Ring darstellen.

**10.** Verbindungen nach Anspruch 9, dadurch gekennzeichnet, dass $R^{20}$ Wasserstoff und $R^{21}$ Methyl oder

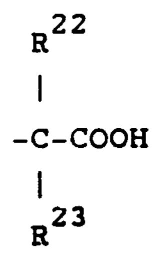

darstellen, worin $R^{22}$ und $R^{23}$ Wasserstoff oder Methyl bedeuten.

**11.** Verbindungen nach Anspruch 1 der allgemeinen Formel

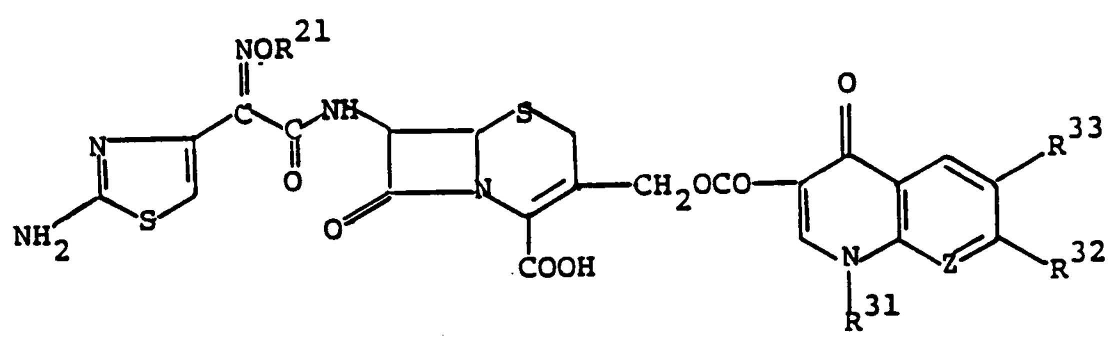

in der $R^{21}$ die in Anspruch 9 und Z, $R^{31}$, $R^{32}$ und $R^{33}$ die in Anspruch 1 gegebene Bedeutung haben.

**12.** Verbindungen nach einem der Ansprüche 1-11, dadurch gekennzeichnet, dass $R^{21}$ Methyl oder eine Gruppe der allgemeinen Formel

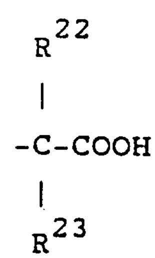

$R^{22}$ und $R^{23}$ Wasserstoff oder Methyl, Z die Gruppe $R^{30}$-C, $R^{30}$ Wasserstoff oder Halogen, $R^{31}$ $C_1$-$C_8$-

Alkyl, Halogen- $C_1$-$C_8$- Alkyl oder $C_3$-$C_7$-Cycloalkyl, $R^{32}$ $C_1$-$C_8$- Alkyl, Piperazinyl oder ($C_1$-$C_8$-Alkyl)-Piperazinyl und $R^{33}$ Wasserstoff oder Halogen darstellen.

**13.** Verbindungen nach Anspruch 12, dadurch gekennzeichnet, dass $R^{30}$ Wasserstoff oder Fluor, $R^{31}$ Aethyl, Fluoräthyl oder Cyclopropyl, $R^{32}$ Methyl, 4-Methyl-piperazinyl oder Piperazinyl und $R^{33}$ Wasserstoff oder Fluor darstellen.

**14.** Verbindungen nach Anspruch 1, gekennzeichnet als [6R-[6-alpha,7-beta(Z)]]-7-[[(2-amino -4-thiazolyl)-($R^{21}$-oxyimino)acetyl]amino] -3-[[[[6-$R^{33}$,8-$R^{30}$, 1-$R^{31}$-1,4-dihydro -7-(4-$R^{34}$-1-piperazinyl) -4-oxo-3-chinolinyl]carbonyl]oxy]methyl] -8-oxo-5-thia-1-azabicyclo[4.2.0]oct -2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, $C_1$-$C_8$- Alkyl oder 2-Halogen- $C_1$-$C_8$- Alkyl, $R^{34}$ Wasserstoff oder $C_1$-$C_8$-Alkyl und $R^{21}$ Wasserstoff, $C_1$-$C_8$- Alkyl oder eine Gruppe der allgemeinen Formel

$$\begin{array}{c} R^{22} \\ | \\ -C-COOH \\ | \\ R^{23} \end{array}$$

und $R^{22}$ und $R^{23}$ Wasserstoff oder $C_1$-$C_8$- Alkyl bedeuten.

**15.** Verbindungen nach Anspruch 14, dadurch gekennzeichnet, dass $R^{21}$ Methyl darstellt.

**16.** Verbindungen nach Anspruch 15, dadurch gekennzeichnet, dass $R^{33}$ und $R^{30}$ Wasserstoff oder Fluor, $R^{31}$ Aethyl oder 2-Fluoräthyl und $R^{34}$ Methyl darstellen.

**17.** Verbindungen nach Anspruch 16, dadurch gekennzeichnet, dass $R^{33}$ und $R^{30}$ Fluor und $R^{31}$ 2-Fluoräthyl darstellen.

**18.** Verbindungen nach Anspruch 15, dadurch gekennzeichnet, dass $R^{33}$ Fluor, $R^{30}$ Wasserstoff, $R^{31}$ 2-Fluoräthyl und $R^{34}$ Methyl darstellen.

**19.** Verbindungen nach Anspruch 15, dadurch gekennzeichnet, dass $R^{33}$ Fluor, $R^{30}$ Wasserstoff, $R^{31}$ Aethyl und $R^{34}$ Methyl darstellen.

**20.** Verbindungen nach Anspruch 15, dadurch gekennzeichnet, dass $R^{33}$ Fluor, $R^{31}$ 2-Fluoräthyl, $R^{30}$ Wasserstoff und $R^{34}$ Methyl darstellen.

**21.** Verbindungen nach einem der Ansprüche 8-20, dadurch gekennzeichnet, dass die Gruppen der allgemeinen Formeln

$$\begin{array}{c} N-O-R^{130} \\ \| \\ -C- \end{array}$$

und

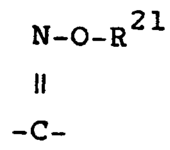

in der synisomeren Form oder als Gemische vorliegen, in denen die synisomere Form überwiegt.

**22.** Verbindungen nach Anspruch 1, gekennzeichnet als [6R-[6-alpha,7-beta]]-3-[[[[6-$R^{33}$,8-$R^{30}$, 1-$R^{31}$-1,4-dihydro -7-(4-$R^{34}$-1-piperazinyl)-4-oxo -3-chinolinyl]carbonyl]oxy]methyl] -8-oxo-7-[(phenoxyacetyl)-amino] -5-thia-1-azabicyclo[4.2.0]oct-2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, $C_1$-$C_8$-Alkyl oder 2-Halogen- $C_1$-$C_8$- Alkyl und $R^{34}$ Wasserstoff oder $C_1$-$C_8$- Alkyl darstellen.

**23.** Verbindungen nach Anspruch 22, dadurch gekennzeichnet, dass $R^{33}$ und $R^{30}$ Wasserstoff oder Fluor, $R^{31}$ Aethyl oder 2-Fluoräthyl und $R^{34}$ Methyl darstellen.

**24.** Verbindungen nach Anspruch 23, dadurch gekennzeichnet, dass $R^{33}$ und $R^{30}$ Fluor und $R^{31}$ 2-Fluoräthyl darstellen.

**25.** Verbindungen nach Anspruch 22, dadurch gekennzeichnet, dass $R^{33}$ Fluor, $R^{30}$ Wasserstoff, $R^{31}$ 2-Fluoräthyl und $R^{34}$ Methyl darstellen.

**26.** Verbindungen nach Anspruch 22, dadurch gekennzeichnet, dass $R^{33}$ Fluor, $R^{30}$ Wasserstoff, $R^{31}$ Aethyl und $R^{34}$ Methyl darstellen.

**27.** Verbindungen nach Anspruch 22, dadurch gekennzeichnet, dass $R^{33}$ Fluor, $R^{31}$ 2-Fluoräthyl, $R^{30}$ Wasserstoff und $R^{34}$ Methyl darstellen.

**28.** Verbindungen nach Anspruch 1, gekennzeichnet als [6R-[6-alpha,7-beta]] -7-(formylamino) -3-[[[[6-$R^{33}$,8--$R^{30}$, 1-$R^{31}$-1,4-dihydro -7-(4-$R^{34}$-1-piperazinyl)-4-oxo -3-chinolinyl]carbonyl]oxy]methyl] -8-oxo-5-thia-1-azabicyclo[4.2.0]oct-2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, $C_1$-$C_8$- Alkyl oder 2-Halogen-$C_1$-$C_8$- Alkyl und $R^{34}$ Wasserstoff oder $C_1$-$C_8$- Alkyl darstellen.

**29.** Verbindungen nach Anspruch 28, dadurch gekennzeichnet, dass $R^{33}$ und $R^{30}$ Wasserstoff oder Fluor, $R^{31}$ Aethyl oder 2-Fluoräthyl und $R^{34}$ Methyl darstellen.

**30.** Verbindungen nach Anspruch 29, dadurch gekennzeichnet, dass $R^{33}$ und $R^{30}$ Fluor und $R^{31}$ 2-Fluoräthyl darstellen.

**31.** Verbindungen nach Anspruch 28, dadurch gekennzeichnet, dass $R^{33}$ Fluor, $R^{30}$ Wasserstoff, $R^{31}$ 2-Fluoräthyl und $R^{34}$ Methyl darstellen.

**32.** Verbindungen nach Anspruch 28, dadurch gekennzeichnet, dass $R^{33}$ Fluor, $R^{30}$ Wasserstoff, $R^{31}$ Aethyl und $R^{34}$ Methyl darstellen.

**33.** Verbindungen nach Anspruch 28, dadurch gekennzeichnet, dass $R^{33}$ Fluor, $R^{31}$ 2-Fluoräthyl, $R^{30}$ Wasserstoff und $R^{34}$ Methyl darstellen.

**34.** Verbindungen nach Anspruch 1, gekennzeichnet als [6R-[6-alpha,7-beta(Z)]]-7-[[(2-amino -4-thiazolyl)-($R^{21}$-oxyimino)acetyl]amino] -3-[[[[6-$R^{33}$,8-$R^{30}$, 1-$R^{31}$-1,4-dihydro -7-(4-$R^{34}$-1-piperazinyl)-4-oxo -3-chinolinyl]carbonyl]oxy]methyl] -8-oxo-5-thia-1-azabicyclo[4.2.0]oct-2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, $C_1$-$C_8$- Alkyl oder 2-Halogen- $C_1$-$C_8$- Alkyl, $R^{34}$ Wasserstoff oder $C_1$-$C_8$-Alkyl, $R^{21}$ die Gruppe

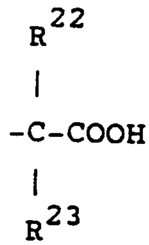

und $R^{22}$ und $R^{23}$ Wasserstoff oder $C_1$-$C_8$- Alkyl darstellen.

**35.** Verbindungen nach Anspruch 34, dadurch gekennzeichnet, dass $R^{22}$ und $R^{23}$ beide Methyl darstellen.

**36.** Verbindungen nach Anspruch 35, dadurch gekennzeichnet, dass $R^{33}$ und $R^{30}$ Wasserstoff oder Fluor, $R^{31}$ Aethyl oder 2-Fluoräthyl und $R^{34}$ Methyl darstellen.

**37.** Verbindungen nach Anspruch 36, dadurch gekennzeichnet, dass $R^{33}$ und $R^{30}$ Fluor und $R^{31}$ 2-Fluoräthyl darstellen.

**38.** Verbindungen nach Anspruch 35, dadurch gekennzeichnet, dass $R^{33}$ Fluor, $R^{30}$ Wasserstoff, $R^{31}$ 2-Fluoräthyl und $R^{34}$ Methyl darstellen.

**39.** Verbindungen nach Anspruch 35, dadurch gekennzeichnet, dass $R^{33}$ Fluor, $R^{30}$ Wasserstoff, $R^{31}$ Aethyl und $R^{34}$ Methyl darstellen.

**40.** Verbindungen nach Anspruch 35, dadurch gekennzeichnet, dass $R^{33}$ Fluor, $R^{31}$ 2-Fluoräthyl, $R^{30}$ Wasserstoff und $R^{34}$ Methyl darstellen.

**41.** Verbindungen nach Anspruch 1,
[6R-(6α,7β)]-3-[[[(1-Aethyl-1,4-dihydro-7-methyl-4-oxo-1,8-naphthyridin -3-yl)carbonyl]oxy]methyl]-8-oxo-7-[(phenoxyacetyl)amino] -5-thia-1-azabicyclo[4.2.0]oct-2-en-2-carbonsäure, sowie Salze dieser Verbindung und Hydrate dieser Verbindung bzw. Salze.

**42.** Verbindungen nach Anspruch 1,
[6R-(6α,7β)]-3-[[[(5-Aethyl -5,8-dihydro-8-oxo-1,3-dioxolo[4,5-g]chinolin -7-yl)carbonyl]oxy]methyl]-8-oxo-7-[(phenoxyacetyl]amino] -5-thia-1-azabicyclo[4.2.0]oct-en-2-carbonsäure, sowie Salze dieser Verbindung und Hydrate dieser Verbindung bzw. Salze.

**43.** Verbindungen nach Anspruch 1,
[6R-(6α,7β)(Z)]-7-[((2-Amino -4-thiazolyl)(methoxyimino)acetyl]amino] -3-[[[(1-äthyl-1,4-dihydro-7-methyl-4-oxo-1,8-naphthyridin-3-yl)carbonyl]oxy]methyl-8-oxo-5-thia -1-azabicyclo[4.2.0]oct-2-en-2-carbonsäure, sowie Salze dieser Verbindung und Hydrate dieser Verbindung bzw. Salze.

**44.** Verbindungen nach Anspruch 1,
[6R-(6α,7β)(Z)]-7-[[(2-Amino -4-thiazolyl)(methoxyimino)acetyl]amino] -3-[[[(5-äthyl-5,8-dihydro-8-oxo-1,3-dioxolo[4,5-g]chinolin -7-yl)carbonyl]oxy]methyl] -8-oxo-5-thia-1-azabicyclo[4.2.0]oct-2-en-2-carbonsäure, sowie Salze dieser Verbindung und Hydrate dieser Verbindung bzw. Salze.

**45.** Verbindungen nach Anspruch 1,
[6R-(6α,7β)]-3-[[[[1-Aethyl-6-fluor-1,4-dihydro -7-(4-formyl-1-piperazinyl) -4-oxochinolin-3-yl]-carbonyl]oxy]methyl -8-oxo-7-[(phenoxyacetyl)amino] -5-thia-1-azabicyclo[4.2.0]oct-2-en-2-carbonsäure, sowie Salze dieser Verbindung und Hydrate dieser Verbindung bzw. Salze.

**46.** Verbindungen nach Anspruch 1,
[6R-(6α,7β)]-3-[[[(5-Aethyl-5,8-dihydro -8-oxo-1,3-dioxolo[4,5-g]chinolin -7-yl)carbonyl)oxy]methyl]-7-[(2-thienylacetyl)amino]-8-oxo-5-thia -1-azabicyclo[4.2.0]oct-2-en-2-carbonsäure, sowie Salze dieser Verbindung und Hydrate dieser Verbindung bzw. Salze.

**47.** Verbindungen nach Anspruch 1,
[6R-(6α,7β)]-3-[[[[1-Aethyl -6-fluor-1,4-dihydro-7-(4-formyl-1-piperazinyl)-4-oxochinolin -3-yl]-carbonyl]oxy]methyl] -7-[(2-thienylacetyl)amino]-8-oxo-5-thia-1-azabicyclo[4.2.0]oct-2-en-2-carbonsäure, sowie Salze dieser Verbindung und Hydrate dieser Verbindung bzw. Salze.

**48.** Verbindungen nach Anspruch 1,
[6R-(6α,7β)]-3-[[[[1-Aethyl-6-fluor-1,4-dihydro-7-(1-pyrrolidinyl) -4-oxochinolin-3-yl)carbonyl]oxy]-methyl]-8-oxo -7-[(phenoxyacetyl)amino] -5-thia-1-azabicyclo[4.2.0]oct-2-en-2-carbonsäure, sowie Salze dieser Verbindung und Hydrate dieser Verbindung bzw. Salze.

**49.** Verbindungen nach Anspruch 1,
[6R-(6α,7β)(Z)]-7-[[(2-Amino -4-thiazolyl)(methoxyimino)acetyl]amino] -3-[[[[1-äthyl -6-fluor-1,4-dihydro-7-(1-pyrrolidinyl) -4-oxochinolin-3-yl]carbonyl]oxy]methyl] -8-oxo-5-thia-1-azabicyclo[4.2.0]oct-2-en-2-carbonsäure, sowie Salze dieser Verbindung und Hydrate dieser Verbindung bzw. Salze.

**50.** Verbindungen nach Anspruch 1,
[6R-(6α,7β)(Z)]-7-[[(2-Amino -4-thiazolyl)(methoxyimino)acetyl]amino] -3-[[[[1-äthyl-6-fluor-1,4-dihydro -7-(4-formyl-1-piperazinyl)-4-oxochinolin -3-yl]carbonyl]oxy]methyl]-8-oxo-5-thia -1-azabicyclo-[4.2.0]oct-2-en-2-carbonsäure, sowie Salze dieser Verbindung und Hydrate dieser Verbindung bzw. Salze.

**51.** Verbindungen nach Anspruch 1,
[6R-(6α,7β)]-3-[[[[1-Aethyl-6-fluor-1,4-dihydro-7-(4-thiomorpholinyl) -4-oxochinolin-3-yl]carbonyl]-oxy]methyl]-8-oxo -7-[(phenoxyacetyl)amino]-5-thia -1-azabicyclo[4.2.01oct-2-en-2-carbonsäure, sowie Salze dieser Verbindung und Hydrate dieser Verbindung bzw. Salze.

**52.** Verbindungen nach Anspruch 1,
[6R-(6α,7β)]-3-[[[(1-Aethyl-6-fluor-1,4-dihydro-4-oxo-7-(1H-pyrrol-1-yl)chinolin -3-yl]carbonyl]oxy]-methyl]-8-oxo -7-[(phenoxyacetyl)amino]-5-thia -1-azabicyclo[4.2.0]oct-2-en-2-carbonsäure, sowie Salze dieser Verbindung und Hydrate dieser Verbindung bzw. Salze.

**53.** Verbindungen nach Anspruch 1,
[6R-(6α,7β)]-3-[[[[5-(4-Fluorphenyl)-5,8-dihydro-8-oxo-1,3-dioxolo[4,5-g]chinolin -7-yl]carbonyl]oxy]-methyl]-8-oxo -7-[(phenoxyacetyl)amino]-5-thia -1-azabicyclo[4.2.0]oct-2-en-2-carbonsäure, sowie Salze dieser Verbindung und Hydrate dieser Verbindung bzw. Salze.

**54.** Verbindungen nach Anspruch 1,
[6R-(6α,7β)(Z)]-7-[[(2-Amino -4-thiazolyl)(methoxyimino)acetyl]amino] -3-[[[[1-äthyl-6-fluor-1,4-dihydro -7-(4-thiomorpholinyl)-4-oxo-chinolin-3-yl]carbonyl]oxy]methyl]-8-oxo-5-thia -1-azabicyclo[4.2.0]oct-2-en-2-carbonsäure, sowie Salze dieser Verbindung und Hydrate dieser Verbindung bzw. Salze.

**55.** Verbindungen nach Anspruch 1,
[6R-(6α,7β(Z)]]-7-[[(2-Amino -4-thiazolyl(methoxyimino)acetyl]amino] -3-[[[[6,8-difluor-1-(2-fluoräthyl)-1,4-dihydro -7-(4-methyl-1-piperazinyl)-4-oxo-3-chinolinyl]carbonyl]oxy]methyl]-8-oxo-5-thia -1-azabicyclo[4.2.0loct-2-en -2-carbonsäure, sowie Salze dieser Verbindung und Hydrate dieser Verbindung bzw. Salze.

**56.** Verbindungen nach Anspruch 1,
[6R-[6α,7β(Z)]]-3-[[[[7-(3-Amino-1-pyrrolidinyl)-8-chlor-1-cyclopropyl-6-fluor-1,4-dihydro -4-oxochinolin-3-yl]carbonyl]oxy]methyl] -7-[[(2-amino-4-thiazolyl)[(carboxymethoxy)-imino]acetyl]amino]-8-oxo-5-thia-1-azabicyclo[4.2.0]oct -2-en-2-carbonsäure sowie Salze dieser Verbindung und Hydrate dieser Verbindung bzw. Salze.

**57.** Verbindungen nach Anspruch 1,
[6R-[6α,7β(Z)]]-3-[[[[7-(3-Amino-1-pyrrolidinyl)-8-chlor-1-cyclopropyl-6-fluor-1,4-dihydro-4-oxochinolin-3-yl]carbonyl]oxy]methyl]-7-[[(2-amino -4-thiazolyl)[(1-carboxy-1-methyläthoxy)imino]acetyl]amino]-8-oxo-5-thia-1-azabicyclo-[4.2.0]oct -2-en-2-carbonsäure sowie Salze dieser Verbindung und Hydrate dieser Verbindung bzw. Salze.

**58.** Verbindungen nach Anspruch 1,
[6R-[6α,7β(Z)]]-3-[[[[7-(3-Amino-1-pyrrolidinyl) -8-chlor-1-cyclopropyl-6-fluor-1,4-dihydro-4-oxochinolin-3-yl]carbonyl]oxy]methyl]-7-[[(2-amino -4-thiazolyl)(methoxyimino)-acetyl]amino]-8-oxo -5-thia-1-azabicyclo[4.2.0]oct-2-en-2-carbonsäure sowie Salze dieser Verbindung und Hydrate dieser Verbindung bzw. Salze.

**59.** Verbindungen gemäss einem der Ansprüche 1-58 als pharmazeutische Wirkstoffe.

**60.** Verbindungen gemäss einem der Ansprüche 1-58 als pharmazeutische Wirkstoffe zur Behandlung und Prophylaxe von Infektionskrankheiten.

**61.** Pharmazeutische Präparate, gekennzeichnet durch einen Gehalt an einer Verbindung gemäss einem der Ansprüche 1-58.

**62.** Pharmazeutische Präparate zur Behandlung und Prophylaxe von Infektionskrankheiten, gekennzeichnet durch einen Gehalt an einer Verbindung gemäss einem der Ansprüche 1-58.

**63.** Verfahren zur Herstellung der Verbindungen gemäss einem der Ansprüche 1-58, dadurch gekennzeichnet, dass man
(a) zur Herstellung eines leicht hydrolysierbaren Esters einer Carbonsäure der Formel I, eine Verbindung der allgemeinen Formel

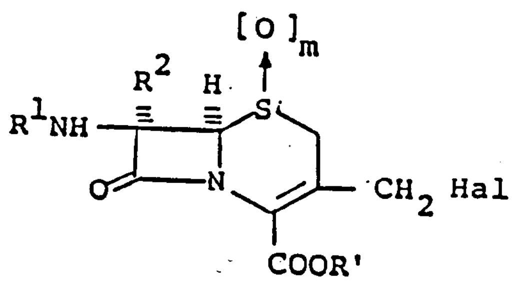

II

in der m, $R^1$ und $R^2$ die in Anspruch 1 gegebene Bedeutung haben, Hal Halogen und R' den Rest eines leicht hydrolysierbaren Esters darstellen,
mit einem Salz einer Carbonsäure der allgemeinen Formel

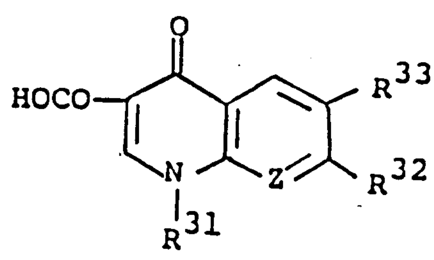

III

in der $R^{31}$, $R^{32}$ und $R^{33}$ die in Anspruch 1 gegebene Bedeutung haben,
umsetzt, oder dass man

(b) zur Herstellung einer Carbonsäure der Formel I, einen Ester der allgemeinen Formel

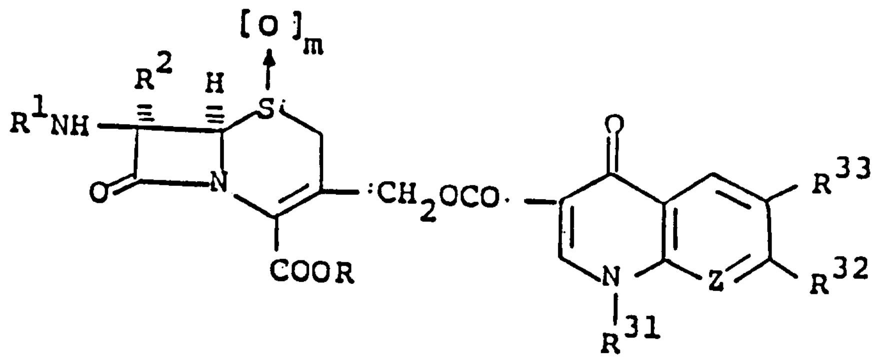

IV

in der m, $R^1$, $R^2$, $R^{31}$, $R^{32}$ und $R^{33}$ die in Anspruch 1 gegebene Bedeutung haben, und R eine Esterschutzgruppe darstellt,
in die Carbonsäure der Formel I umwandelt, oder dass man
(c) zur Herstellung einer Verbindung der Formel I, worin m 0 darstellt, eine Verbindung der allgemeinen Formel

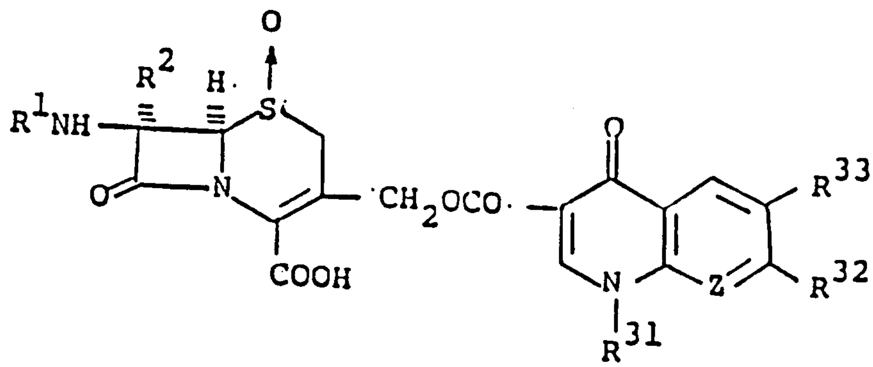

V

in der $R^1$, $R^2$, $R^{31}$, $R^{32}$ und $R^{33}$ die in Anspruch 1 gegebene Bedeutung haben,
reduziert, oder dass man
(d) zur Herstellung einer Verbindung der Formel I, worin m 1 oder 2 darstellt, oder eines ihrer Ester oder Salze, eine Verbindung der allgemeinen Formel

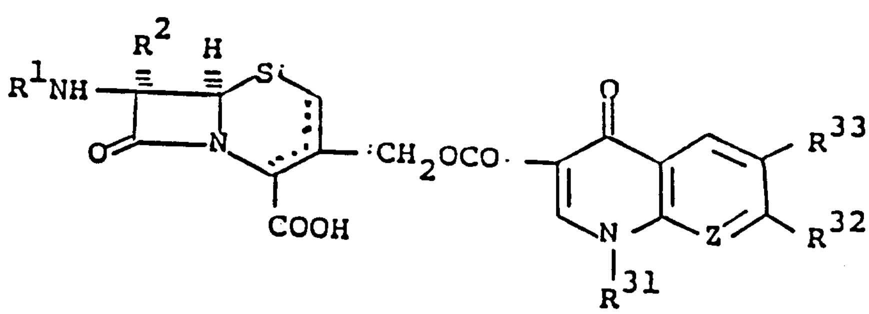

VI

in der $R^1$, $R^2$, $R^{31}$, $R^{32}$ und $R^{33}$ die in Anspruch 1 gegebene Bedeutung haben, und die gestrichelten Linien die Anwesenheit einer Δ2- oder Δ3-Doppelbindung anzeigen,
oder eines ihrer Ester oder Salze oxidiert, oder dass man

(e) zur Herstellung einer Verbindung der Formel I, worin $R^1$ die Gruppe

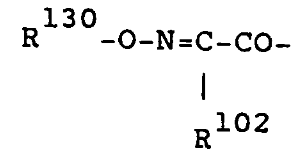

und $R^{102}$ einen aminosubstituierten 5-, 6- oder 7-gliedrigen heterocyclischen Ring mit 1, 2, 3 oder 4 Stickstoff-, Sauerstoff- und/oder Schwefelatomen bedeuten, und $R^{130}$ die in Anspruch 8 gegebene Bedeutung hat, oder eines ihrer Ester oder Salze, die Aminoschutzgruppe im Substituenten $R^{103}$ einer Verbindung der allgemeinen Formel

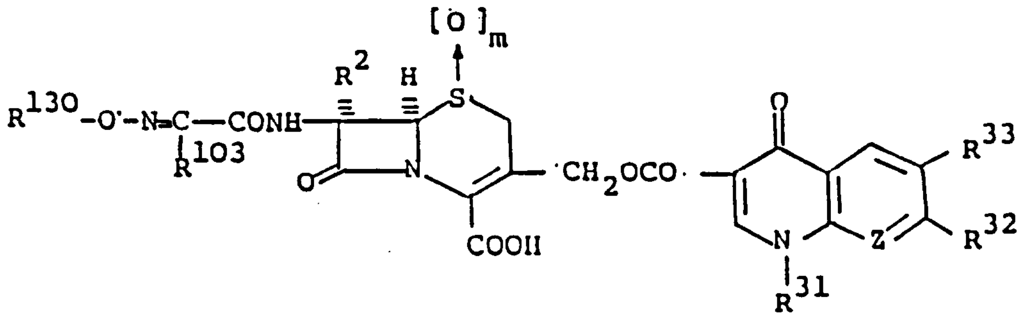

VII

in der m, $R^2$, $R^{31}$, $R^{32}$ und $R^{33}$ die in Anspruch 1 und $R^{130}$ die in Anspruch 8 gegebene Bedeutung haben, und $R^{103}$ die für $R^{102}$ gegebene Bedeutung hat, wobei jedoch der Aminosubstituent geschützt ist,
oder eines ihrer Ester oder Salze abspaltet, oder dass man
(f) zur Herstellung eines leicht hydrolysierbaren Esters einer Verbindung der Formel I, eine Carbonsäure der Formel I einer entsprechenden Veresterung unterwirft, oder dass man
(g) zur Herstellung von Salzen oder Hydraten einer Verbindung der Formel I bzw. von Hydraten besagter Salze, eine Verbindung der Formel I in ein Salz oder Hydrat bzw. in ein Hydrat des besagten Salzes überführt.

**64.** Verwendung von Verbindungen gemäss einem der Ansprüche 1-58 bei der Herstellung von Arzneimitteln für die Behandlung bzw. Prophylaxe von Infektionskrankheiten.

**Patentansprüche für folgende Vertragsstaaten : AT, ES, GR**

**1.** Verfahren zur Herstellung von Acylderivaten der allgemeinen Formel

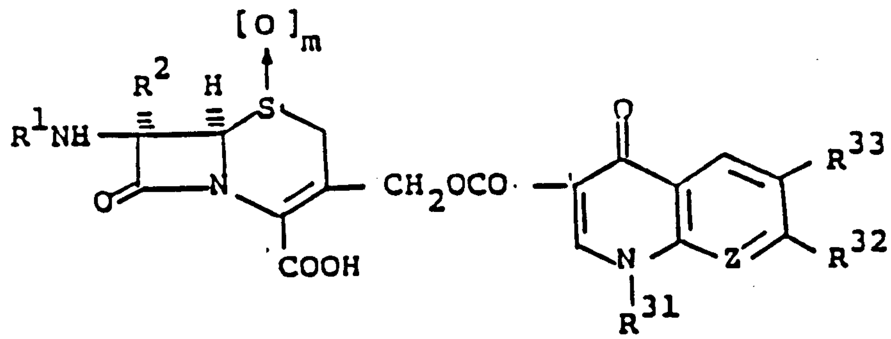

in der m 0, 1 oder 2, $R^1$ Wasserstoff oder eine Acylgruppe, $R^2$ Wasserstoff $C_1$-$C_8$- Alkoxy, Amino, $C_1$-$C_8$-Alkylthlo oder $C_1$-$C_7$- Alkanoylamino, $R^{31}$ Wasserstoff, $C_1$-$C_8$- Alkyl, $C_2$-$C_6$- Alkenyl, $C_3$-$C_7$-

Cycloalkyl, Halogen- $C_1$-$C_8$- Alkyl oder Halogenphenyl; Z $R^{30}$-C oder Stickstoff, $R^{30}$ Wasserstoff oder Halogen, oder $R^{30}$ und $R^{31}$ zusammen eine $C_3$-$C_5$-Alkylengruppe, eine $C_2$-$C_4$-Alkylenmonooxygruppe oder eine $C_1$-$C_2$-Alkylendioxygruppe, $R^{32}$ Wasserstoff, Halogen, $C_1$-$C_8$- Alkyl oder einen gegebenenfalls durch $C_1$-$C_8$- Alkyl, $C_1$-$C_8$- Alkoxy, Halogen, Halogen- $C_1$-$C_8$- Alkyl, Amino, Mercapto, Hydroxy, Carbamoyl oder Carboxy substituierten 5- oder 6-gliedrigen heterocyclischen Ring mit einem, zwei oder drei Sauerstoff-, Stickstoff- und/oder Schwefelatomen und $R^{33}$ Wasserstoff oder Halogen oder $R^{32}$ und $R^{33}$ zusammen eine $C_1$-$C_4$-Alkylendioxygruppe darstellen,
sowie von leicht hydrolysierbaren Estern und Salzen dieser Verbindungen und von Hydraten der Verbindungen der Formel bzw. von deren Estern und Salzen, dadurch gekennzeichnet, dass man

(a) zur Herstellung eines leicht hydrolysierbaren Esters einer Carbonsäure der Formel I, eine Verbindung der allgemeinen Formel

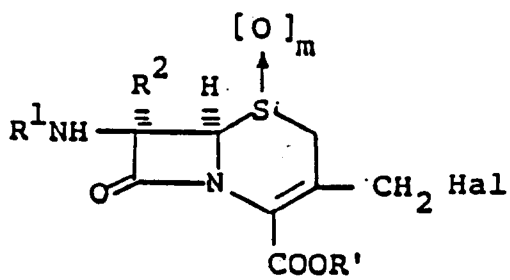

II

in der m, $R^1$ und $R^2$ die oben gegebene Bedeutung haben, Hal Halogen und R' den Rest eines leicht hydrolysierbaren Esters darstellen,
mit einem Salz einer Carbonsäure der allgemeinen Formel

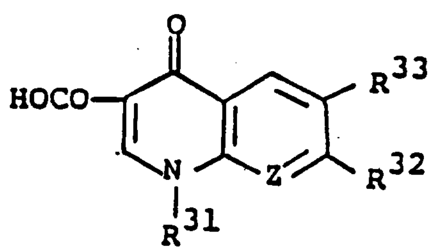

III

in der $R^{31}$, $R^{32}$ und $R^{33}$ die oben gegebene Bedeutung haben,
umsetzt, oder dass man
(b) zur Herstellung einer Carbonsäure der Formel I, einen Ester der allgemeinen Formel

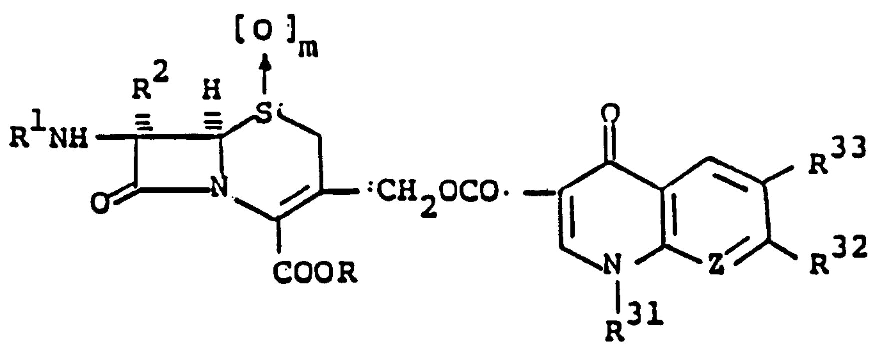

IV

in der m, $R^1$, $R^2$, $R^{31}$, $R^{32}$ und $R^{33}$ die oben gegebene Bedeutung haben, und R eine Esterschutzgruppe darstellt,

in die Carbonsäure der Formel I umwandelt, oder dass man
(c) zur Herstellung einer Verbindung der Formel I, worin m 0 darstellt, eine Verbindung der allgemeinen Formel

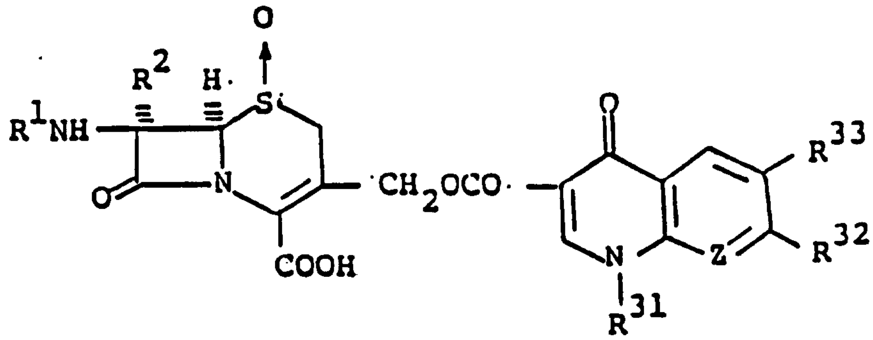

V

in der $R^1$, $R^2$, $R^{31}$, $R^{32}$ und $R^{33}$ die oben gegebene Bedeutung haben,
reduziert, oder dass man
(d) zur Herstellung einer Verbindung der Formel I, worin m 1 oder 2 darstellt, oder eines ihrer Ester oder Salze, eine Verbindung der allgemeinen Formel

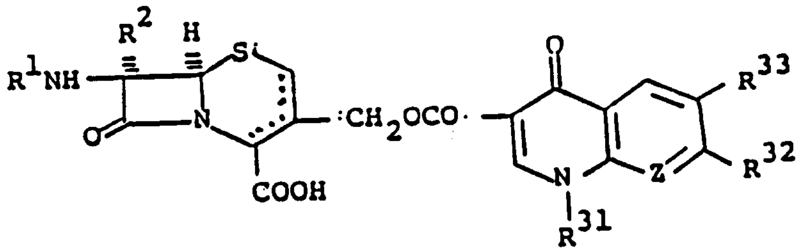

VI

in der $R^1$, $R^2$, $R^{31}$, $R^{32}$ und $R^{33}$ die oben gegebene Bedeutung haben, und die gestrichelten Linien die Anwesenheit einer Δ2- oder Δ3-Doppelbindung anzeigen,
oder eines ihrer Ester oder Salze oxidiert, oder dass man
(e) zur Herstellung einer Verbindung der Formel I, worin $R^1$ die Gruppe

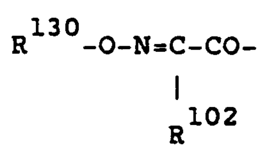

und $R^{102}$ einen aminosubstituierten 5-, 6- oder 7-gliedrigen heterocyclischen Ring mit 1, 2, 3 oder 4 Stickstoff-, Sauerstoff- und/oder Schwefelatomen bedeuten, und $R^{130}$ die in Anspruch 8 gegebene Bedeutung hat, oder eines ihrer Ester oder Salze, die Aminoschutzgruppe im Substituenten $R^{103}$ einer Verbindung der allgemeinen Formel

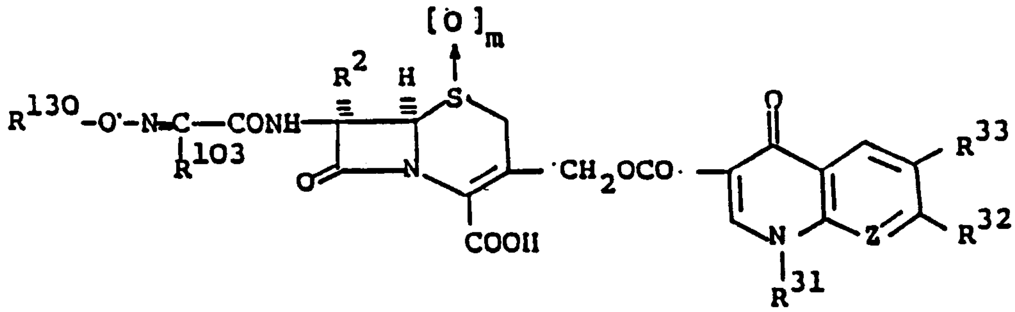

VII

in der m, $R^2$, $R^{31}$, $R^{32}$ und $R^{33}$ die oben und $R^{130}$ die in Anspruch 8 gegebene Bedeutung haben, und $R^{103}$ die für $R^{102}$ gegebene Bedeutung hat, wobei jedoch der Aminosubstituent geschützt ist, oder eines ihrer Ester oder Salze abspaltet, oder dass man

(f) zur Herstellung eines leicht hydrolysierbaren Esters einer Verbindung der Formel I, eine Carbonsäure der Formel I einer entsprechenden Veresterung unterwirft, oder dass man

(g) zur Herstellung von Salzen oder Hydraten einer Verbindung der Formel I bzw. von Hydraten besagter Salze, eine Verbindung der Formel I in ein Salz oder Hydrat bzw. in ein Hydrat des besagten Salzes überführt.

**2.** Verfahren zur Herstellung von Verbindungen nach Anspruch 1, worin m 0 und $R^2$ Wasserstoff darstellt, dadurch gekennzeichnet, dass man entsprechend substituierte Ausgangsverbindungen einsetzt.

**3.** Verfahren zur Herstellung von Verbindungen nach Anspruch 1 oder 2, worin Z die Gruppe Z $R^{30}$-C, $R^{30}$ Wasserstoff oder Halogen, $R^{31}$ $C_1$-$C_8$- Alkyl, Halogen-$C_1$-$C_8$- Alkyl oder $C_3$-$C_7$-Cycloalkyl, $R^{32}$ $C_1$-$C_8$-Alkyl, Piperazinyl oder ($C_1$-$C_8$-Alkyl)-Piperazinyl- und $R^{33}$ Wasserstoff oder Halogen darstellen, dadurch gekennzeichnet, dass man entsprechend substituierte Ausgangsverbindungen einsetzt.

**4.** Verfahren zur Herstellung von Verbindungen nach Anspruch 3, worin $R^{30}$ Wasserstoff oder Fluor, $R^{31}$ Aethyl, Fluoräthyl oder Cyclopropyl, $R^{32}$ Piperazinyl oder 4-Methylpiperazinyl und $R^{33}$ Wasserstoff oder Fluor darstellen, dadurch gekennzeichnet, dass man entsprechend substituierte Ausgangsverbindungen einsetzt.

**5.** Verfahren zur Herstellung von Verbindungen nach einem der Ansprüche 1-4, worin $R^1$ eine der folgenden Acylgruppen darstellt:

(a) eine aliphatische Gruppe der Formel

$R^5$-CO-

in der $R^5$ $C_1$-$C_8$- Alkyl, $C_3$-$C_7$-Cycloalkyl, $C_1$-$C_8$- Alkoxy, $C_2$-$C_6$- Alkenyl, $C_3$-$C_7$-Cycloalkenyl oder Cyclohexadienyl oder durch einen oder mehrere der Reste Halogen, Cyan, Nitro, Amino, Mercapto, $C_1$-$C_8$- Alkylthio oder Cyanmethylthio substituiertes $C_1$-$C_8$- Alkyl oder $C_2$-$C_6$- Alkenyl darstellt;

(b) eine carbocyclische aromatische Gruppe der allgemeinen Formel

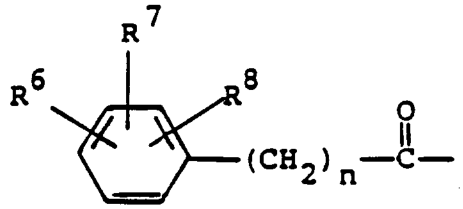

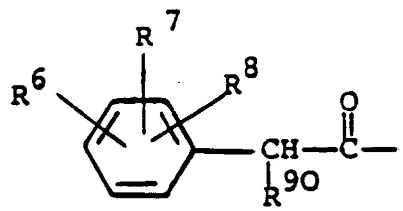

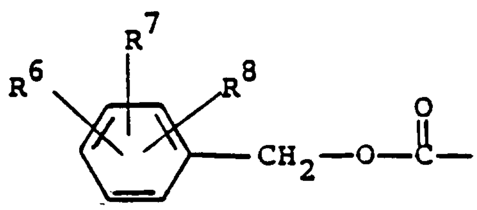

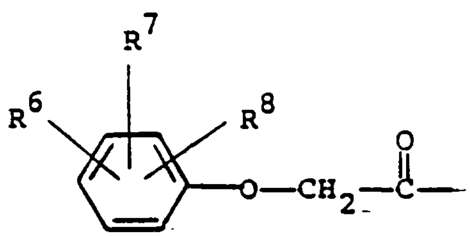

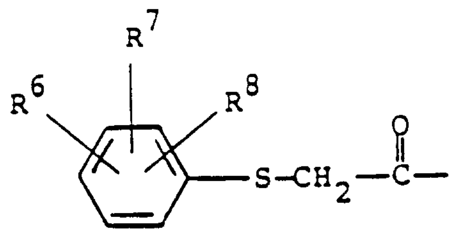

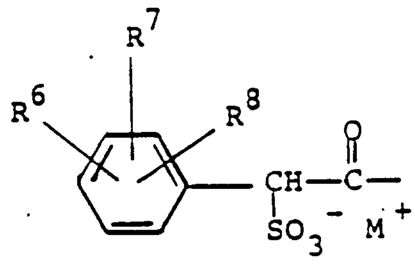

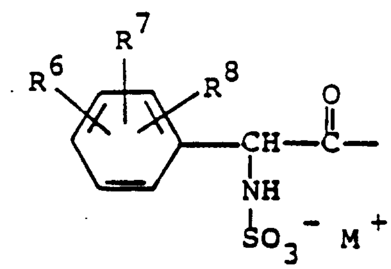

in der n 0, 1, 2 oder 3; $R^6$, $R^7$ und $R^8$ Wasserstoff, Halogen, Hydroxy, Nitro, Amino, Cyano, Trifluormethyl, $C_1$-$C_4$-Alkyl, $C_1$-$C_4$-Alkoxy oder Aminomethyl, $R^{90}$ Amino, Hydroxy, ein Carboxysalz, geschütztes Carboxy, Formyloxy oder Azido und M ein Kation darstellen;

(c) eine heteroaromatische Gruppe der allgemeinen Formeln

$R^{101}$-$(CH_2)_n$-CO-

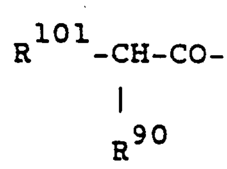

$R^{101}$-O-$CH_2$-CO-
$R^{101}$-S-$CH_2$-CO-
$R^{101}$-CO-CO-

in denen $R^{90}$ die oben gegebene Bedeutung hat, n 0, 1, 2 oder 3 und $R^{101}$ einen gegebenenfalls durch $C_1$-$C_8$- Alkyl, $C_1$-$C_8$- Alkoxy, Halogen, Halogen- $C_1$-$C_8$- Alkyl, Amino, Mercapto, Hydroxy, Carbamoyl oder Carboxy substituierten 5-, 6- oder 7-gliedrigen heterocyclischen Ring mit 1, 2, 3 oder 4 Stickstoff-, Sauerstoff und/oder Schwefelatomen darstellt;

(d) eine [[4-Substituierte-2,3-dioxo -1-piperazinyl)carbonyl]amino]arylacetylgruppe der Formel

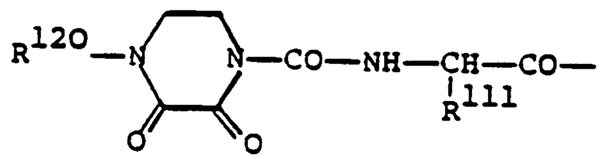

in der $R^{111}$ $C_1$-$C_8$- Alkyl, Hydroxy- $C_1$-$C_8$- Alkyl oder eine aromatische Gruppe der allgemeinen Formel

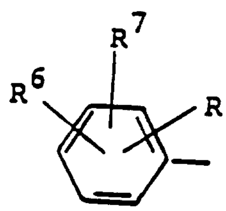

(in der $R^6$, $R^7$ und $R^8$ die oben gegebenen Bedeutung haben) oder einen wie für $R^{101}$ definierten heterocyclischen Ring und $R^{120}$ gegebenenfalls durch einen oder mehrere der Reste Halogen, Cyan, Nitro, Amino und/oder Mercapto substituiertes $C_1$-$C_8$- Alkyl darstellen;

(e) eine (substituierte Oxiimino)arylacetylgruppe der allgemeinen Formel

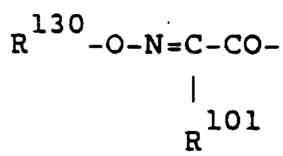

in der $R^{101}$ die oben gegebene Bedeutung hat und $R^{130}$ Wasserstoff, $C_1$-$C_8$- Alkyl, $C_3$-$C_7$-Cycloalkyl, Carboxy-$C_3$-$C_7$-Cycloalkyl oder substituiertes $C_1$-$C_8$- Alkyl darstellt, wobei die $C_1$-$C_8$-Alkylgruppe substituiert ist durch einen oder mehrere der Reste Halogen, Cyan, Nitro, Amino, Mercapto, $C_1$-$C_8$- Alkylthio, eine durch $R^{111}$ oben definierte aromatische Gruppe, Carboxy (einschliesslich dessen Salze), $C_1$-$C_7$-Alkanoylamino, $C_2$-$C_9$- Alkoxycarbonyl, Phenylmethoxycarbonyl, Diphenylmethoxycarbonyl, Hydroxy( $C_1$-$C_8$-Alkoxy)phosphinyl, Dihydroxyphosphinyl, Hydroxy-(phenylmethoxy)phosphinyl und/oder di($C_1$-$C_8$- Alkoxy)phosphinyl;

(f) eine (Acylamino)arylacetylgruppe der allgemeinen Formel

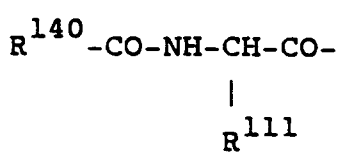

in der $R^{111}$ die oben gegebene Bedeutung hat und $R^{140}$ eine Gruppe der allgemeinen Formel

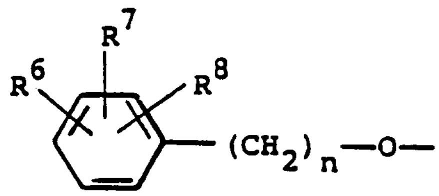

(worin $R^6$, $R^7$, $R^8$ und n die obige Bedeutung haben), Wasserstoff, $C_1$-$C_8$-Alkyl, substituiertes $C_1$-$C_8$-Alkyl, Amino, $C_1$-$C_8$-Alkylamino oder Cyan-$C_1$-$C_8$-alkylamino darstellt;

(g) eine [[[3-Substituierte-2-oxo -1-imidazolidinyl]carbonyl]amino]arylacetylgruppe der allgemeinen Formel

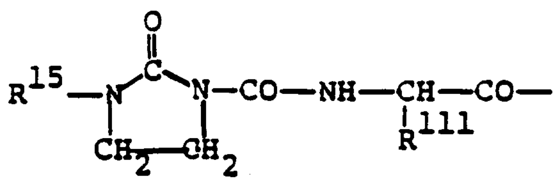

in der $R^{111}$ die oben gegebene Bedeutung hat, und $R^{15}$ Wasserstoff, $C_1$-$C_8$- Alkylsulfonyl, -N = CH-$R^{111}$ (worin $R^{111}$ die oben gegebene Bedeutung hat), $R^{16}$-CO(worin $R^{16}$ Wasserstoff oder gegebenenfalls durch Halogen substituiertes $C_1$-$C_8$- Alkyl darstellt), eine wie unter $R^{111}$ oben definierte aromatische Gruppe oder gegebenenfalls durch einen oder mehrere der Reste Halogen, Cyan, Nitro, Amino und/oder Mercapto substituiertes $C_1$-$C_8$- Alkyl darstellt,

dadurch gekennzeichnet, dass man entsprechend substituierte Ausgangsverbindungen einsetzt.

**6.** Verfahren zur Herstellung von Verbindungen nach einem der Ansprüche 1-5, worin $R^1$ eine carbocyclische aromatische Gruppe der allgemeinen Formel

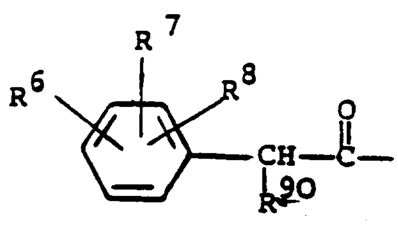

bedeutet, in der $R^{90}$ Amino, Hydroxy,

ein Carboxysalz, Benzyloxycarbonyl, Formyloxy oder Azido und $R^6$, $R^7$ und $R^8$ Wasserstoff, Halogen, Hydroxy, Nitro, Amino, Cyan, Trifluormethyl, $C_1$-$C_4$-Alkyl, $C_1$-$C_4$-Alkoxy oder Aminomethyl darstellen,

dadurch gekennzeichnet, dass man entsprechend substituierte Ausgangsverbindungen einsetzt.

**7.** Verfahren zur Herstellung von Verbindungen nach Anspruch 6, worin $R^6$, $R^7$ und $R^8$ Wasserstoff und $R^{90}$ Wasserstoff oder Hydroxy darstellen, dadurch gekennzeichnet, dass man entsprechend substituierte Ausgangsverbindungen einsetzt.

**8.** Verfahren zur Herstellung von Verbindungen nach einem der Ansprüche 1-5, worin $R^1$ eine Acylgruppe der allgemeinen Formel

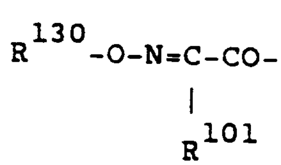

darstellt, in der $R^{101}$ eine gegebenenfalls durch Halogen, Hydroxy, Nitro, Amino, Cyan, Trifluormethyl, $C_1$-$C_4$-Alkyl oder $C_1$-$C_4$-Alkoxy substituierten 5-, 6- oder 7-gliedrigen heterocyclischen Ring mit 1, 2, 3 oder 4 Stickstoff-, Sauerstoff- und/oder Schwefelatomen und $R^{130}$ Wasserstoff, $C_1$-$C_8$- Alkyl, $C_3$-$C_7$-Cycloalkyl, Carboxy-$C_3$-$C_7$-Cycloalkyl oder durch einen oder mehrere der Reste Halogen, Cyan, Nitro, Amino, Mercapto, $C_1$-$C_8$- Alkylthio, eine durch $R^{111}$ gemäss Anspruch 5 definierte aromatische Gruppe, Carboxy (einschliesslich dessen Salze), $C_1$-$C_7$- Alkanoylamino, $C_2$-$C_9$- Alkoxycarbonyl, Phenylmethoxycarbonyl, Diphenylmethoxycarbonyl, Hydroxy( $C_1$-$C_8$- Alkoxy)phosphinyl, Dihydroxyphosphinyl, Hydroxy(phenylmethoxy)phosphinyl und/oder Di( $C_1$-$C_8$- Alkoxy)phosphinyl substituiertes $C_1$-$C_8$- Alkyl bedeuten,
dadurch gekennzeichnet, dass man entsprechend substituierte Ausgangsverbindungen einsetzt.

**9.** Verfahren zur Herstellung von Verbindungen nach einem der Ansprüche 1-5 und 8, worin $R^1$ eine Acylgruppe der allgemeinen Formel

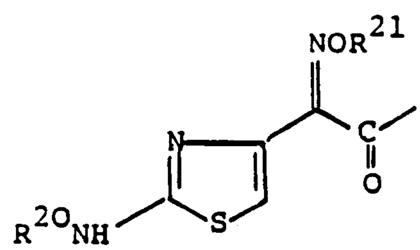

darstellt, in der $R^{20}$ Wasserstoff oder eine Aminoschutzgruppe und $R^{21}$ Wasserstoff, $C_1$-$C_8$- Alkyl oder eine Gruppe der allgemeinen Formel

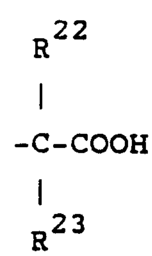

bedeuten, worin $R^{22}$ und $R^{23}$ Wasserstoff, $C_1$-$C_8$-Alkyl oder zusammen mit dem verbindenden Kohlenstoffatom einen 3-7-gliedrigen carbocyclischen Ring darstellen,
dadurch gekennzeichnet, dass man entsprechend substituierte Ausgangsverbindungen einsetzt.

**10.** Verfahren zur Herstellung von Verbindungen nach Anspruch 9, worin $R^{20}$ Wasserstoff und $R^{21}$ Methyl oder

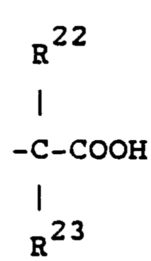

darstellen, worin $R^{22}$ und $R^{23}$ Wasserstoff oder Methyl bedeuten,
dadurch gekennzeichnet, dass man entsprechend substituierte Ausgangsverbindungen einsetzt.

**11.** Verfahren zur Herstellung von Verbindungen nach Anspruch 1 der allgemeinen Formel

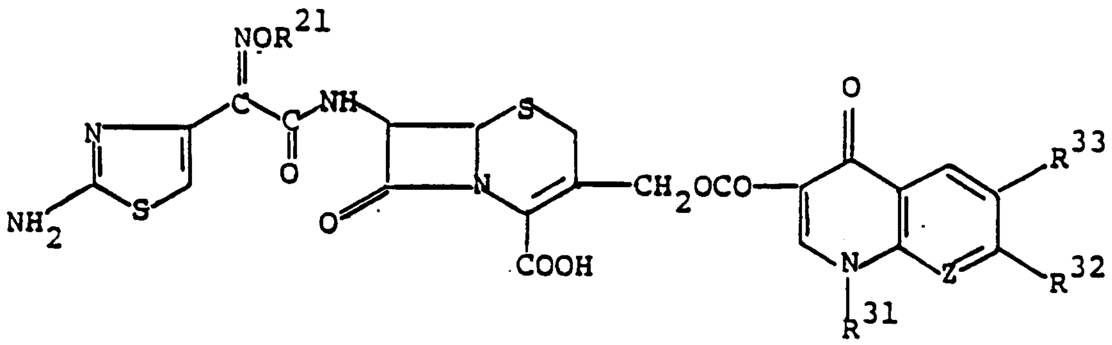

in der $R^{21}$ die in Anspruch 9 und Z, $R^{31}$, $R^{32}$ und $R^{33}$ die in Anspruch 1 gegebene Bedeutung haben,
dadurch gekennzeichnet, dass man entsprechend substituierte Ausgangsverbindungen einsetzt.

**12.** Verfahren zur Herstellung von Verbindungen nach einem der Ansprüche 1-11, worin $R^{21}$ Methyl oder eine Gruppe der allgemeinen Formel

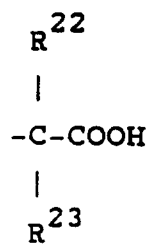

$R^{22}$ und $R^{23}$ Wasserstoff oder Methyl, Z die Gruppe $R^{30}$-C, $R^{30}$ Wasserstoff oder Halogen, $R^{31}$ $C_1$-$C_8$-Alkyl, Halogen $C_1$-$C_8$- Alkyl oder $C_3$-$C_7$-Cycloalkyl, $R^{32}$ $C_1$-$C_8$- Alkyl, Piperazinyl oder ($C_1$-$C_8$-Alkyl)-Piperazinyl und $R^{33}$ Wasserstoff oder Halogen darstellen, dadurch gekennzeichnet, dass man entsprechend substituierte Ausgangsverbindungen einsetzt.

**13.** Verfahren zur Herstellung von Verbindungen nach Anspruch 12, worin $R^{30}$ Wasserstoff oder Fluor, $R^{31}$ Aethyl, Fluoräthyl oder Cyclopropyl, $R^{32}$ Methyl, 4-Methyl-piperazinyl oder Piperazinyl und $R^{33}$ Wasserstoff oder Fluor darstellen, dadurch gekennzeichnet, dass man entsprechend substituierte Ausgangsverbindungen einsetzt.

**14.** Verfahren zur Herstellung von Verbindungen nach Anspruch 1, gekennzeichnet als [6R-[6-alpha,7-beta-(Z)]]-7-[[(2-amino -4-thiazolyl)($R^{21}$-oxyimino)acetyl]amino] -3-[[[[6-$R^{33}$,8-$R^{30}$, 1-$R^{31}$-1,4-dihydro-7-(4-$R^{34}$-1-piperazinyl)-4-oxo-3-chinolinyl]carbonyl]oxy]methyl] -8-oxo-5-thia-1-azabicyclo[4.2.0]oct-2-en-2-carbonsäuren und von deren Salzen und Hydraten dieser Carbonsäuren und Salze, worin $R^{33}$ und $R^{30}$ Wasserstoff oder Halogen, $R^{31}$ Wasserstoff, $C_1$-$C_8$- Alkyl oder 2-Halogen- $C_1$-$C_8$- Alkyl, $R^{34}$ Wasserstoff oder $C_1$-$C_8$- Alkyl und $R^{21}$ Wasserstoff, $C_1$-$C_8$- Alkyl oder eine Gruppe der allgemeinen Formel

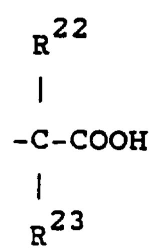

und $R^{22}$ und $R^{23}$ Wasserstoff oder $C_1$-$C_8$- Alkyl bedeuten, dadurch gekennzeichnet, dass man entsprechend substituierte Ausgangsverbindungen einsetzt.

**15.** Verfahren zur Herstellung von Verbindungen nach Anspruch 14, worin $R^{21}$ Methyl darstellt, dadurch gekennzeichnet, dass man entsprechend substituierte Ausgangsverbindungen einsetzt.

**16.** Verfahren zur Herstellung von Verbindungen nach Anspruch 15, worin $R^{33}$ und $R^{30}$ Wasserstoff oder Fluor, $R^{31}$ Aethyl oder 2-Fluoräthyl und $R^{34}$ Methyl darstellen, dadurch gekennzeichnet, dass man entsprechend substituierte Ausgangsverbindungen einsetzt.

**17.** Verfahren zur Herstellung von Verbindungen nach Anspruch 16, worin $R^{33}$ und $R^{30}$ Fluor und $R^{31}$ 2-Fluoräthyl darstellen, dadurch gekennzeichnet, dass man entsprechend substituierte Ausgangsverbindungen einsetzt.

**18.** Verfahren zur Herstellung von Verbindungen nach Anspruch 15, worin $R^{33}$ Fluor, $R^{30}$ Wasserstoff, $R^{31}$ 2-Fluoräthyl und $R^{34}$ Methyl darstellen, dadurch gekennzeichnet, dass man entsprechend substituierte Ausgangsverbindungen einsetzt.

**19.** Verfahren zur Herstellung von Verbindungen nach Anspruch 15, worin $R^{33}$ Fluor, $R^{30}$ Wasserstoff, $R^{31}$ Aethyl und $R^{34}$ Methyl darstellen, dadurch gekennzeichnet, dass man entsprechend substituierte Ausgangsverbindungen einsetzt.

**20.** Verfahren zur Herstellung von Verbindungen nach Anspruch 15, worin $R^{33}$ Fluor, $R^{31}$ 2-Fluoräthyl, $R^{30}$ Wasserstoff und $R^{34}$ Methyl darstellen, dadurch gekennzeichnet, dass man entsprechend substituierte Ausgangsverbindungen einsetzt.

**21.** Verfahren zur Herstellung von Verbindungen nach einem der Ansprüche 8-20, worin die Gruppen der allgemeinen Formeln

$$\begin{array}{c} N\text{-}O\text{-}R^{130} \\ \| \\ \text{-}C\text{-} \end{array}$$

und

$$\begin{array}{c} N\text{-}O\text{-}R^{21} \\ \| \\ \text{-}C\text{-} \end{array}$$

in der synisomeren Form oder als Gemische vorliegen, in denen die synisomere Form überwiegt, dadurch gekennzeichnet, dass man entsprechend substituierte Ausgangsverbindungen einsetzt.

**22.** Verfahren zur Herstellung von Verbindungen nach Anspruch 1, gekennzeichnet als [6R-[6-alpha,7-beta]-]-3-[[[[6-$R^{33}$,8-$R^{30}$, 1-$R^{31}$-1,4-dihydro -7-(4-$R^{34}$-1-piperazinyl)-4-oxo -3-chinolinyl]carbonyl]oxy]methyl] -8-oxo-7-[(phenoxyacetyl)amino] -5-thia-1-azabicyclo[4.2.0]oct-2-en-2-carbonsäuren und von deren Salzen und Hydraten dieser Carbonsäuren und Salze, worin $R^{33}$ und $R^{30}$ Wasserstoff oder Halogen, $R^{31}$ Wasserstoff, $C_1$-$C_8$-Alkyl oder 2-Halogen- $C_1$-$C_8$- Alkyl und $R^{34}$ Wasserstoff oder $C_1$-$C_8$- Alkyl darstellen, dadurch gekennzeichnet, dass man entsprechend substituierte Ausgangsverbindungen einsetzt.

**23.** Verfahren zur Herstellung von Verbindungen nach Anspruch 22, worin $R^{33}$ und $R^{30}$ Wasserstoff oder Fluor, $R^{31}$ Aethyl oder 2-Fluoräthyl und $R^{34}$ Methyl darstellen, dadurch gekennzeichnet, dass man entsprechend substituierte Ausgangsverbindungen einsetzt.

**24.** Verfahren zur Herstellung von Verbindungen nach Anspruch 23, worin $R^{33}$ und $R^{30}$ Fluor und $R^{31}$ 2-Fluoräthyl darstellen, dadurch gekennzeichnet, dass man entsprechend substituierte Ausgangsverbindungen einsetzt.

**25.** Verfahren zur Herstellung von Verbindungen nach Anspruch 22, worin $R^{33}$ Flour, $R^{30}$ Wasserstoff, $R^{31}$ 2-Fluoräthyl und $R^{34}$ Methyl darstellen, dadurch gekennzeichnet, dass man entsprechend substituierte Ausgangsverbindungen einsetzt.

**26.** Verfahren zur Herstellung von Verbindungen nach Anspruch 22, worin $R^{33}$ Fluor, $R^{30}$ Wasserstoff, $R^{31}$ Aethyl und $R^{34}$ Methyl darstellen, dadurch gekennzeichnet, dass man entsprechend substituierte Ausgangsverbindungen einsetzt.

**27.** Verfahren zur Herstellung von Verbindungen nach Anspruch 22, worin $R^{33}$ Fluor, $R^{31}$ 2-Fluoräthyl, $R^{30}$ Wasserstoff und $R^{34}$ Methyl darstellen, dadurch gekennzeichnet, dass man entsprechend substituierte Ausgangsverbindungen einsetzt.

**28.** Verfahren zur Herstellung von Verbindungen nach Anspruch 1, gekennzeichnet als [6R-[6-alpha,7-beta]-]-7-(formylamino) -3-[[[[6-$R^{33}$,8-$R^{30}$, 1-$R^{31}$-1,4-dihydro-7-(4-$R^{34}$-1-piperazinyl)-4-oxo -3-chinolinyl]-carbonyl]oxy]methyl] -8-oxo-5-thia-1-azabicyclo[4.2.0]oct-2-en-2-carbonsäuren und von deren Salzen und Hydraten dieser Carbonsäuren und Salze, worin $R^{33}$ und $R^{30}$ Wasserstoff oder Halogen, $R^{31}$ Wasserstoff, $C_1$-$C_8$- Alkyl oder 2-Halogen- $C_1$-$C_8$- Alkyl und $R^{34}$ Wasserstoff oder $C_1$-$C_8$- Alkyl darstellen, dadurch gekennzeichnet, dass man entsprechend substituierte Ausgangsverbindungen einsetzt.

**29.** Verfahren zur Herstellung von Verbindungen nach Anspruch 28, worin $R^{33}$ und $R^{30}$ Wasserstoff oder Fluor, $R^{31}$ Aethyl oder 2-Fluoräthyl und $R^{34}$ Methyl darstellen, dadurch gekennzeichnet, dass man entsprechend substituierte Ausgangsverbindungen einsetzt.

**30.** Verfahren zur Herstellung von Verbindungen nach Anspruch 29, worin $R^{33}$ und $R^{30}$ Fluor und $R^{31}$ 2-Fluoräthyl darstellen, dadurch gekennzeichnet, dass man entsprechend substituierte Ausgangsverbindungen einsetzt.

**31.** Verfahren zur Herstellung von Verbindungen nach Anspruch 28, worin $R^{33}$ Fluor, $R^{30}$ Wasserstoff, $R^{31}$ 2-Fluoräthyl und $R^{34}$ Methyl darstellen, dadurch gekennzeichnet, dass man entsprechend substituierte Ausgangsverbindungen einsetzt.

**32.** Verfahren zur Herstellung von Verbindungen nach Anspruch 28, worin $R^{33}$ Fluor, $R^{30}$ Wasserstoff, $R^{31}$ Aethyl und $R^{34}$ Methyl darstellen, dadurch gekennzeichnet, dass man entsprechend substituierte Ausgangsverbindungen einsetzt.

**33.** Verfahren zur Herstellung von Verbindungen nach Anspruch 28, worin $R^{33}$ Fluor, $R^{31}$ 2-Fluoräthyl, $R^{30}$ Wasserstoff und $R^{34}$ Methyl darstellen, dadurch gekennzeichnet, dass man entsprechend substituierte Ausgangsverbindungen einsetzt.

**34.** Verfahren zur Herstellung von Verbindungen nach Anspruch 1, gekennzeichnet als [6R-[6-alpha,7-beta-(Z)]]-7-[[(2-amino -4-thiazolyl)($R^{21}$-oxyimino)acetyl]amino] -3-[[[[6-$R^{33}$,8-$R^{30}$, 1-$R^{31}$-1,4-dihydro -7-(4-$R^{34}$-1-piperazinyl)-4-oxo -3-chinolinyl]carbonyl]oxy]methyl] -8-oxo-5-thia-1-azabicyclo- [4.2.0]oct-2-en-2-carbonsäuren und von deren Salzen und Hydraten dieser Carbonsäuren und Salze, worin $R^{33}$ und $R^{30}$ Wasserstoff oder Halogen $R^{31}$ Wasserstoff, $C_1$-$C_8$- Alkyl oder 2-Halogen- $C_1$-$C_8$- Alkyl, $R^{34}$ Wasserstoff oder $C_1$-$C_8$- Alkyl, $R^{21}$ die Gruppe

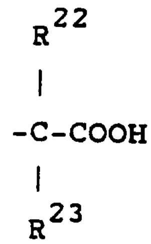

und $R^{22}$ und $R^{23}$ Wasserstoff oder $C_1$-$C_8$- Alkyl darstellen, dadurch gekennzeichnet, dass man entsprechend substituierte Ausgangsverbindungen einsetzt.

**35.** Verfahren zur Herstellung von Verbindungen nach Anspruch 34, worin $R^{22}$ und $R^{23}$ beide Methyl darstellen, dadurch gekennzeichnet, dass man entsprechend substituierte Ausgangsverbindungen einsetzt.

**36.** Verfahren zur Herstellung von Verbindungen nach Anspruch 35, worin $R^{33}$ und $R^{30}$ Wasserstoff oder Fluor, $R^{31}$ Aethyl oder 2-Fluoräthyl und $R^{34}$ Methyl darstellen, dadurch gekennzeichnet, dass man entsprechend substituierte Ausgangsverbindungen einsetzt.

**37.** Verfahren zur Herstellung von Verbindungen nach Anspruch 36, worin $R^{33}$ und $R^{30}$ Fluor und $R^{31}$ 2-Fluoräthyl darstellen, dadurch gekennzeichnet, dass man entsprechend substituierte Ausgangsverbindungen einsetzt.

**38.** Verfahren zur Herstellung von Verbindungen nach Anspruch 35, worin $R^{33}$ Fluor, $R^{30}$ Wasserstoff, $R^{31}$ 2-Fluoräthyl und $R^{34}$ Methyl darstellen, dadurch gekennzeichnet, dass man entsprechend substituierte Ausgangsverbindungen einsetzt.

**39.** Verfahren zur Herstellung von Verbindungen nach Anspruch 35, worin $R^{33}$ Fluor, $R^{30}$ Wasserstoff, $R^{31}$ Aethyl und $R^{34}$ Methyl darstellen, dadurch gekennzeichnet, dass man entsprechend substituierte Ausgangsverbindungen einsetzt.

**40.** Verfahren zur Herstellung von Verbindungen nach Anspruch 35, worin $R^{33}$ Fluor, $R^{31}$ 2-Fluoräthyl, $R^{30}$ Wasserstoff und $R^{34}$ Methyl darstellen, dadurch gekennzeichnet, dass man entsprechend substituierte Ausgangsverbindungen einsetzt.

**41.** Verfahren zur Herstellung von Verbindungen nach Anspruch 1, [6R-(6α,7β)]-3-[[[(1-Aethyl-1,4-dihydro-7-methyl-4-oxo-1,8-naphthyridin -3-yl)carbonyl]oxy]methyl]-8-oxo-7-[(phenoxyacetyl)amino] -5-thia-1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**42.** Verfahren zur Herstellung von Verbindungen nach Anspruch 1, [6R-(6α,7β)]-3-[[[(5-Aethyl -5,8-dihydro-8-oxo-1,3-dioxolo[4,5-g]chinolin -7-yl)carbonyl]oxy]methyl]-8-oxo-7-[(phenoxyacetyl]amino] -5-thia-1-azabicyclo[4.2.0]oct-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**43.** Verfahren zur Herstellung von Verbindungen nach Anspruch 1, [6R-(6α,7β)(Z)]-7-[[(2-Amino -4-thiazolyl)(methoxyimino)acetyl]amino] -3-[[[(1-äthyl-1,4-dihydro-7-methyl-4-oxo-1,8-naphthyridin-3-yl)-carbonyl]oxy]methyl-8-oxo-5-thia -1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**44.** Verfahren zur Herstellung von Verbindungen nach Anspruch 1, [6R-(6α,7β)(Z)]-7-[[(2-Amino -4-thiazolyl)(methoxyimino)acetyl]amino] -3-[[[(5-äthyl-5,8-dihydro-8-oxo-1,3-dioxolo[4,5-g]chinolin -7-yl)-carbonyl]oxy]methyl]-8-oxo-5-thia-1-azabicyclo[4.2.0]oct-2-en-2-carbonsäure, sowie von Salzen dieser

Verbindung und von Hydraten dieser Verbindung bzw. Salze, dadurch gekennzeichnet, dass man entsprechend substituierte Ausgangsverbindungen einsetzt.

**45.** Verfahren zur Herstellung von Verbindungen nach Anspruch 1, [6R-(6α,7β)]-3-[[[[1-Aethyl-6-fluor-1,4-dihydro-7-(4-formyl-1-piperazinyl) -4-oxochinolin-3-yl]-carbonyl]oxy]methyl -8-oxo-7-[(phenoxyacetyl)-amino] -5-thia-1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**46.** Verfahren zur Herstellung von Verbindungen nach Anspruch 1, [6R-(6α,7β)]-3-[[[(5-Aethyl-5,8-dihydro -8-oxo-1,3-dioxolo[4,5-g]chinolin -7-yl)carbonyl]oxy]methyl]-7-[(2-thienylacetyl)amino]-8-oxo-5-thia -1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**47.** Verfahren zur Herstellung von Verbindungen nach Anspruch 1, [6R-(6α,7β)]-3-[[[[1-Aethyl -6-fluor-1,4-dihydro-7-(4-formyl-1-piperazinyl)-4-oxochinolin -3-yl]carbonyl]oxy]methyl] -7-[(2-thienylacetyl)amino]-8-oxo-5-thia-1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**48.** Verfahren zur Herstellung von Verbindungen nach Anspruch 1, [6R-(6α,7β)]-3-[[[[1-Aethyl-6-fluor-1,4-dihydro -7-(1-pyrrolidinyl) -4-oxochinolin-3-yl]carbonyl]oxy]methyl]-8-oxo -7-[(phenoxyacetyl)amino] -5-thia-1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**49.** Verfahren zur Herstellung von Verbindungen nach Anspruch 1, [6R-(6α,7β)(Z)]-7-[[(2-Amino -4-thiazolyl)(methoxyimino)acetyl]amino] -3-[[[[1-äthyl -6-fluor-1,4-dihydro-7-(1-pyrrolidinyl) -4-oxochinolin-3-yl]-carbonyl]oxy]methyl] -8-oxo-5-thia-1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**50.** Verfahren zur Herstellung von Verbindungen nach Anspruch 1, [6R-(6α,7β)(Z)]-7-[[(2-Amino -4-thiazolyl)(methoxyimino)acetyl]amino] -3-[[[[1-äthyl-6-fluor-1,4-dihydro -7-(4-formyl-1-piperazinyl)-4-oxochinolin -3-yl]carbonyl]oxy]methyl]-8-oxo-5-thia -1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**51.** Verfahren zur Herstellung von Verbindungen nach Anspruch 1, [6R-(6α,7β)]-3-[[[[1-Aethyl-6-fluor-1,4-dihydro -7-(4-thiomorpholinyl) -4-oxochinolin-3-yl]carbonyl]oxy]methyl]-8-oxo -7-[(phenoxyacetyl)amino]-5-thia -1-azabicyclo[4.2.0loct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**52.** Verfahren zur Herstellung von Verbindungen nach Anspruch 1, [6R-(6α,7β)]-3-[[[(1-Aethyl-6-fluor-1,4-dihydro -4-oxo-7-(1H-pyrrol-1-yl)chinolin -3-yl]carbonyl]oxy]methyl]-8-oxo -7-[(phenoxyacetyl)amino]-5-thia -1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**53.** Verfahren zur Herstellung von Verbindungen nach Anspruch 1, [6R-(6α,7β)]-3-[[[[5-(4-Fluorphenyl)-5,8-dihydro -8-oxo-1,3-dioxolo[4,5-g]chinolin -7-yl]carbonyl]oxy]methyl]-8-oxo -7-[(phenoxyacetyl)amino]-5-thia -1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**54.** Verfahren zur Herstellung von Verbindungen nach Anspruch 1, [6R-(6α,7β)(Z)]-7-[[(2-Amino -4-thiazolyl)(methoxyimino)acetyl]amino] -3-[[[[1-äthyl-6-fluor-1,4-dihydro -7-(4-thiomorpholinyl)-4-oxo-chinolin-3-yl] carbonyl]oxy]methyl]-8-oxo-5-thia -1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**55.** Verfahren zur Herstellung von Verbindungen nach Anspruch 1, [6R-(6α,7β(Z)]]-7-[[(2-Amino -4-thiazolyl-(methoxyimino)acetyl]amino] -3-[[[[6,8-difluor-1-(2-fluoräthyl)-1,4-dihydro -7-(4-methyl-1-piperazinyl)-4-oxo -3-chinolinyl]carbonyl]oxy]methyl]-8-oxo-5-thia -1-azabicyclo[4.2.0loct-2-en -2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**56.** Verfahren zur Herstellung von Verbindungen nach Anspruch 1, [6R-[6α,7β(Z)]]-3-[[[[7-(3-Amino-1-pyrrolidinyl)-8-chlor-1-cyclopropyl -6-fluor-1,4-dihydro-4-oxochinolin-3-yl]carbonyl] oxy]methyll]-7-[[(2-amino -4-thiazolyl)[(carboxymethoxy)-imino]acetyl]amino] -8-oxo-5-thia-1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**57.** Verfahren zur Herstellung von Verbindungen nach Anspruch 1, [6R-[6α,7β(Z)]]-3-[[[[7-(3-Amino-1-pyrrolidinyl) -8-chlor-1-cyclopropyl-6-fluor-1,4-dihydro-4-oxochinolin -3-yl]carbonyl]oxy]methyl]-7-[[(2-amino -4-thiazolyl)[(1-carboxy-1-methyläthoxy)imino]acetyl]amino]-8-oxo-5-thia-1-azabicyclo-[4.2.0]oct-2-en -2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**58.** Verfahren zur Herstellung von Verbindungen nach Anspruch 1, [6R-[6α,7β(Z)]]-3-[[[[7-(3-Amino-1-pyrrolidinyl)-8-chlor-1-cyclopropyl-6-fluor-1,4-dihydro-4-oxochinolin-3-yl]carbonyl]oxy]methyl]-7-[[(2-amino-4-thiazolyl)(methoxyimino)acetyl]amino]-8-oxo-5-thia-1-azabicyclo[4.2.0]oct-2-en-2-carbonsäure, sowie von Salzen dieser Verbindung und von Hydraten dieser Verbindung bzw. Salze, dadurch gekennzeichnet, dass man entsprechend substituierte Ausgangsverbindungen einsetzt.

**59.** Verfahren zur Herstellung von pharmazeutischen Präparaten, dadurch gekennzeichnet, dass man ein in Anspruch 1 definiertes Acylderivat der Formel I oder einen leicht hydrolysierbaren Ester oder ein Salz dieser Verbindung oder ein Hydrat einer Verbindung der Formel I bzw. eines Esters oder Salzes davon als wirksamen Bestandteil mit zur therapeutischen Verabreichung geeigneten, nicht-toxischen, inerten, an sich in solchen Präparaten üblichen festen und flüssigen Trägern und/oder Excipientien vermischt.

**60.** Verwendung von Verbindungen gemäss einem der Ansprüche 1-58 bei der Herstellung von Arzneimitteln für die Behandlung bzw. Prophylaxe von Infektionskrankheiten.

## Claims

### Claims for the following Contracting States : BE, CH, DE, FR, GB, IT, LI, LU, NL, SE

**1.** Acyl derivatives of the general formula

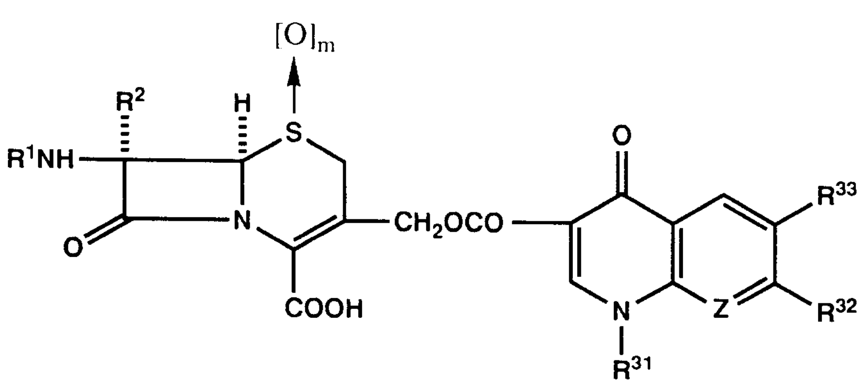

I

wherein m represents 0, 1 or 2, $R^1$ represents hydrogen or an acyl group, $R^2$ represents hydrogen, $C_1$-$C_8$-alkoxy, amino, $C_1$-$C_8$-alkylthio or $C_1$-$C_8$-alkanoylamino, $R^{31}$ represents hydrogen, $C_1$-$C_8$-alkyl, $C_2$-$C_6$-alkenyl, $C_3$-$C_7$-cycloalkyl, halo$_1$-$C_8$-alkyl or halo-phenyl; Z represents $R^{30}$-C or nitrogen; $R^{30}$ represents hydrogen or halogen, or $R^{30}$ and $R^{31}$ together represent a $C_3$-$C_5$-alkylene group, a $C_2$-$C_4$ alkylenemonooxy group or a $C_1$-$C_2$-alkylenedioxy group; $R^{32}$ represents hydrogen, halogen, $C_1$-$C_8$-alkyl or a 5- or 6-membered heterocyclic ring which contains one, two or three oxygen, nitrogen and/or sulphur atoms and which is optionally substituted by $C_1$-$C_8$-alkyl, $C_1$-$C_8$-alkoxy, halogen, halo-$C_1$-$C_8$-alkyl, amino, mercapto, hydroxy, carbamoyl or carboxy and $R^{33}$ represents hydrogen or halogen or $R^{32}$ and $R^{33}$ together represent a $C_1$-$C_4$-alkylenedioxy group,
and readily hydrolyzable esters and salts of these compounds and hydrates of the compounds of formula I or of their esters and salts.

**2.** Compounds according to claim 1, characterized in that m represents 0 and $R^2$ represents hydrogen.

**3.** Compounds according to claim 1 or 2, characterized in that Z represents $R^{30}$-C in which $R^{30}$ represents hydrogen or halogen, $R^{31}$ represents $C_1$-$C_8$-alkyl, halo-$C_1$-$C_8$-alkyl or $C_3$-$C_7$-cycloalkyl, $R^{32}$ represents $C_1$-$C_8$-alkyl, piperazinyl or ($C_1$-$C_8$-alkyl)-piperazinyl and $R^{33}$ represents hydrogen or halogen.

**4.** Compounds according to claim 3, characterized in that $R^{30}$ represents hydrogen or fluorine, $R^{31}$ represents ethyl, fluoroethyl or cyclopropyl, $R^{32}$ represents piperazinyl or 4-methylpiperazinyl and $R^{33}$ represents hydrogen or fluorine.

**5.** Compounds according to any one of claims 1-4, characterized in that $R^1$ represents one of the following acyl groups:
(a) an aliphatic group of the formula

$R^5$-CO-

in which $R^5$ represents $C_1$-$C_8$-alkyl, $C_3$-$C_7$-cycloalkyl, $C_1$-$C_8$-alkoxy, $C_2$-$C_6$-alkenyl, $C_3$-$C_7$-cycloalkenyl or cyclohexadienyl or $C_1$-$C_8$-alkyl or $C_2$-$C_6$-alkenyl substituted with one or more halogen, cyano, nitro, amino, mercapto, $C_1$-$C_8$-alkylthio or cyanomethylthio residues;
(b) a carbocyclic aromatic group of the general formula

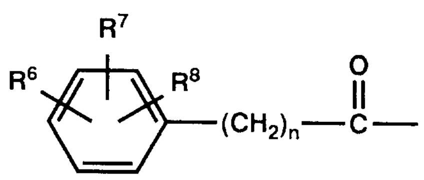

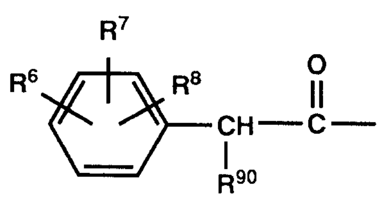

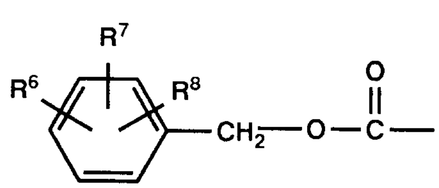

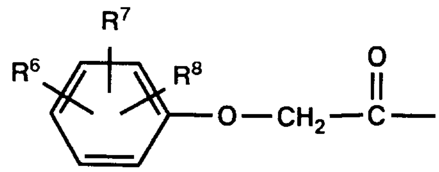

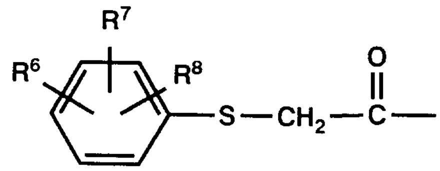

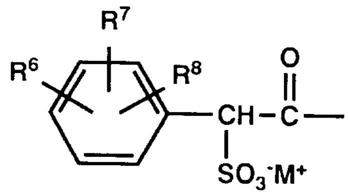

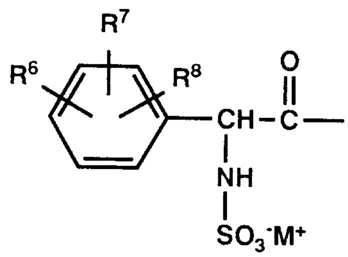

in which n represents 0, 1, 2 or 3; $R^6$ and $R^7$ and $R^8$ represent hydrogen, halogen, hydroxy, nitro, amino, cyano, trifluoromethyl, $C_1$-$C_4$-alkyl, $C_1$-$C_4$-alkoxy or aminomethyl, $R^{90}$ represents amino, hydroxy, a carboxy salt, protected carboxy, formyloxy or azido and M represents a cation;
(c) a heteroaromatic group of the general formulae

$R^{101}$-$(CH_2)_n$-CO-

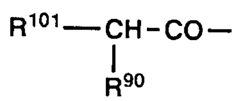

$R^{101}$-O-$CH_2$-CO-
$R^{101}$-S-$CH_2$-CO-

or

$R^{101}$-CO-CO-

in which $R^{90}$ has the significances given above, n represents 0, 1, 2 or 3 and $R^{101}$ represents a 5-, 6- or 7-membered heterocyclic ring which contains 1, 2, 3 or 4 nitrogen, oxygen and/or sulphur atoms and which is optionally substituted by $C_1$-$C_8$-alkyl, $C_1$-$C_8$-alkoxy, halogen, halo-$C_1$-$C_8$-alkyl, amino, mercapto, hydroxy, carbamoyl or carboxy;

(d) a [[(4-substituted-2,3-dioxo-1-piperazinyl)carbonyl]amino]arylacetyl group of the formula

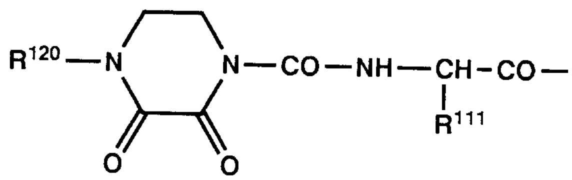

in which $R^{111}$ represents $C_1$-$C_8$-alkyl, hydroxy-$C_1$-$C_8$-alkyl or an aromatic group of the general formula

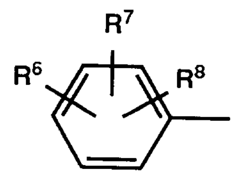

(in which $R^6$, $R^7$ and $R^8$ have the significance give above) or a heterocyclic ring as defined for $R^{101}$ and $R^{120}$ represents $C_1$-$C_8$-alkyl optionally substituted with one or more halogen, cyano, nitro, amino and/or mercapto residues;

(e) a (substituted oxyimino)arylacetyl group of the general formula

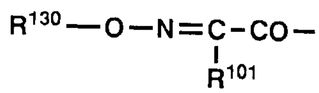

in which $R^{101}$ has the significance given above and $R^{130}$ represents hydrogen, $C_1$-$C_8$-alkyl, $C_3$-$C_7$-cycloalkyl, carboxy-$C_3$-$C_7$-cycloalkyl or substituted $C_1$-$C_8$-alkyl wherein the $C_1$-$C_8$-alkyl group is substituted with one or more halogen, cyano, nitro, amino, mercapto, $C_1$-$C_8$-alkylthio, aromatic group defined above by $R^{111}$, carboxy (including salts thereof), $C_1$-$C_7$-alkanoylamino, $C_2$-$C_9$-alkoxycarbonyl, phenylmethoxycarbonyl, diphenylmethoxycarbonyl, hydroxy ($C_1$-$C_8$-alkoxy)phosphinyl, dihydroxyphosphinyl, hydroxy(phenylmethoxy)phosphinyl and/or di($C_1$-$C_8$-alkoxy)phosphinyl residues;

(f) an (acylamino)arylacetyl group of the general formula

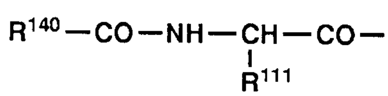

in which $R^{111}$ has the significances given above and $R^{140}$ represents a group of the general formula

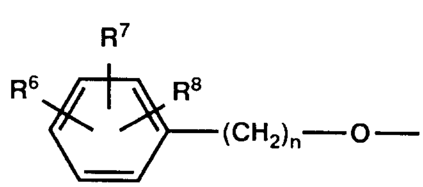

(wherein $R^6$, $R^7$, $R^8$ and n have the above significance), hydrogen, $C_1$-$C_8$-alkyl, substituted $C_1$-$C_8$-alkyl, amino, $C_1$-$C_8$-alkylamino or cyano-$C_1$-$C_8$-alkylamino;

(g) a [[[3-substituted-2-oxo-1-imidazolidinyl]carbonyl]amino]arylacetyl group of the general formula

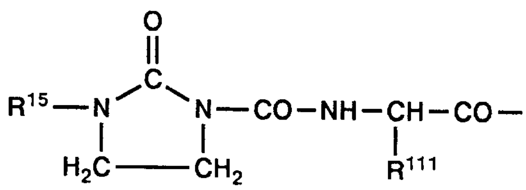

in which $R^{111}$ has the significance given above and $R^{15}$ represents hydrogen, $C_1$-$C_8$-alkylsulphonyl, -N=CHR$^{111}$ (wherein $R^{111}$ has the significance given above), $R^{16}$CO-(wherein $R^{16}$ represents hydrogen or $C_1$-$C_8$-alkyl optionally substituted with halogen), an aromatic group as defined under $R^{111}$ above or $C_1$-$C_8$-alkyl optionally substituted with one or more halogen, cyano, nitro, amino and/or mercapto residues.

6. Compounds according to any one of claims 1-5, characterized in that $R^1$ represents a carbocyclic aromatic group of the general formula

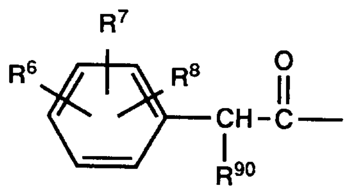

in which $R^{90}$ represents amino, hydroxy, a carboxy salt, benzyloxycarbonyl, formyloxy or azido and $R^6$, $R^7$ and $R^8$ represent hydrogen, halogen, hydroxy, nitro, amino, cyano, trifluoromethyl, $C_1$-$C_4$-alkyl, $C_1$-$C_4$-alkoxy or aminomethyl.

7. Compounds according to claim 6, characterized in that $R^6$, $R^7$ and $R^8$ represent hydrogen and $R^{90}$ represents hydrogen or hydroxy.

8. Compounds according to any one of claims 1-5, characterized in that $R^1$ represents an acyl group of the general formula

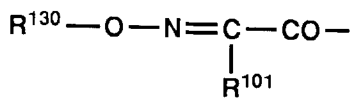

in which $R^{101}$ represents a 5-, 6- or 7-membered heterocyclic ring which contains 1, 2, 3 or 4 nitrogen, oxygen and/or sulphur atoms and which is optionally substituted by halogen, hydroxy, nitro, amino, cyano, trifluoromethyl, $C_1$-$C_4$-alkyl or $C_1$-$C_4$-alkoxy and $R^{130}$ represents hydrogen $C_1$-$C_8$-alkyl, $C_3$-$C_7$-cycloalkyl, carboxy-$C_3$-$C_7$-cycloalkyl or $C_1$-$C_8$-alkyl substituted with one or more halogen, cyano, nitro, amino, mercapto, $C_1$-$C_8$-alkylthio, aromatic group defined by $R^{111}$ in claim 5, carboxy (including salts thereof), $C_1$-$C_7$-alkanoylamino, $C_2$-$C_9$-alkoxycarbonyl, phenylmethoxycarbonyl, diphenylmethoxycarbonyl, hydroxy($C_1$-$C_8$-alkoxy)phosphinyl, dihydroxyphosphinyl, hydroxy(phenylmethoxy)phosphinyl and/or di($C_1$-$C_8$-alkoxy)phosphinyl residues.

9. Compounds according to any one of claims 1-5 and 8, characterized in that $R^1$ represents an acyl group of the general formula

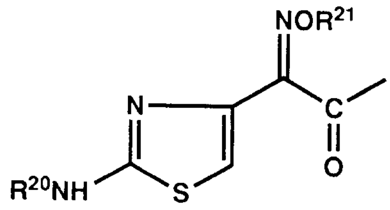

in which $R^{20}$ represents hydrogen or an amino protecting group and $R^{21}$ represents hydrogen, $C_1$-$C_8$-alkyl or a group of the general formula

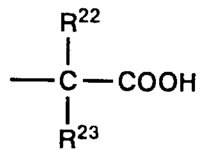

wherein $R^{22}$ and $R^{23}$ represent hydrogen, $C_1$-$C_8$-alkyl or together with the linking carbon atoms represent a 3-7-membered carbocyclic ring.

**10.** Compounds according to claim 9, characterized in that $R^{20}$ represents hydrogen and $R^{21}$ represents methyl or

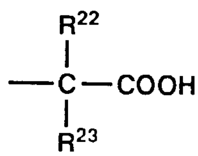

wherein $R^{22}$ and $R^{23}$ signify hydrogen or methyl.

**11.** Compounds according to claim 1 of the general formula

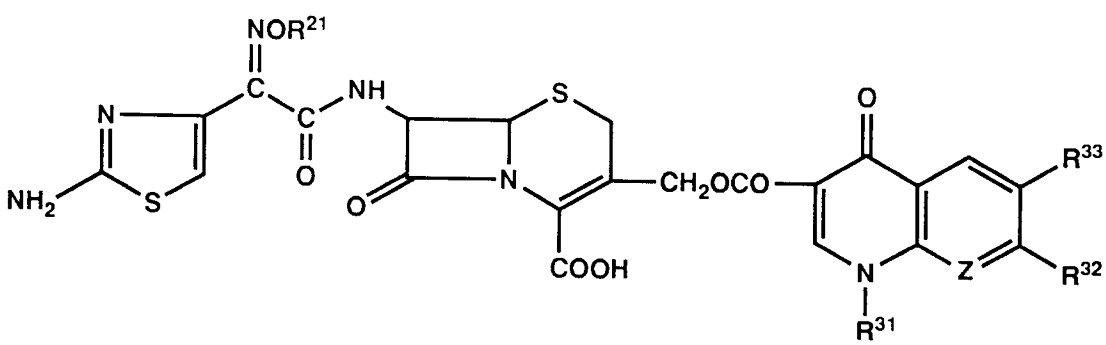

in which $R^{21}$ has the significance given in claim 9 and Z, $R^{31}$, $R^{32}$ and $R^{33}$ have the significance given in claim 1.

**12.** Compounds according to any one of claims 1-11, characterized in that $R^{21}$ represents methyl or a group of the general formula

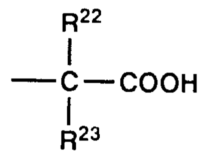

$R^{22}$ and $R^{23}$ represent hydrogen or methyl, Z represents the group $R^{30}$-C, $R^{30}$ represents hydrogen or halogen, $R^{31}$ represents $C_1$-$C_8$-alkyl, halo-$C_1$-$C_8$-alkyl or $C_3$-$C_7$-cycloalkyl, $R^{32}$ represents $C_1$-$C_8$-alkyl, piperazinyl or ($C_1$-$C_8$-alkyl)piperazinyl and $R^{33}$ represents hydrogen or halogen.

**13.** Compounds according to claim 12, characterized in that $R^{30}$ represents hydrogen or fluorine, $R^{312}$ represents ethyl, fluoroethyl or cyclopropyl, $R^{32}$ represents methyl, 4-methylpiperazinyl or piperazinyl and $R^{33}$ represents hydrogen or fluorine.

**14.** Compounds according to claim 1, characterized as [6R-[6-alpha,7-beta(Z)]]-7-[[(2-amino-4-thiazolyl)($R^{21}$-oxyimino)acetyl]amino]-3-[[[[6-$R^{33}$,3-$R^{30}$, 1-$R^{31}$-1,4-dihydro-7-(4-$R^{34}$-1-piperazinyl)-4-oxo-3-quinolinyl]-carbonyl]oxy]methyl]-8-oxo-5-thia-1-azabicycl[4.2.0]oct-2-ene-2-carboxylic acids and salts thereof and hydrates of these carboxylic acids and salts, wherein $R^{33}$ and $R^{30}$ signify hydrogen or halogen, $R^{31}$ signifies hydrogen, $C_1$-$C_8$-alkyl or 2-halo-$C_1$-$C_8$-alkyl, $R^{34}$ signifies hydrogen or $C_1$-$C_8$-alkyl and $R^{21}$ signifies hydrogen, $C_1$-$C_8$-alkyl or a group of the general formula

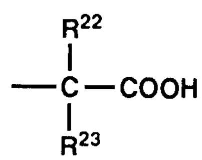

and $R^{22}$ and $R^{23}$ signify hydrogen or $C_1$-$C_8$-alkyl.

**15.** Compounds according to claim 14, characterized in that $R^{21}$ represents methyl.

**16.** Compounds according to claim 15, characterized in that $R^{33}$ and $R^{30}$ represent hydrogen or fluorine, $R^{31}$ represents ethyl or 2-fluoroethyl and $R^{34}$ represents methyl.

**17.** Compounds according to claim 16, characterized in that $R^{33}$ and $R^{30}$ represent fluorine and $R^{31}$ represents 2-fluoroethyl.

**18.** Compounds according to claim 15, characterized in that $R^{33}$ represents fluorine, $R^{30}$ represents hydrogen, $R^{31}$ represents 2-fluoroethyl and $R^{34}$ represents methyl.

**19.** Compounds according to claim 15, characterized in that $R^{33}$ represents fluorine, $R^{30}$ represents hydrogen, $R^{31}$ represents ethyl and $R^{34}$ represents methyl.

**20.** Compounds according to claim 15, characterized in that $R^{33}$ represents fluorine, $R^{31}$ represents 2-fluoroethyl, $R^{30}$ represents hydrogen and $R^{34}$ represents methyl.

**21.** Compounds according to any one of claims 8-20, characterized in that the groups of the general formulae

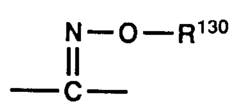

and

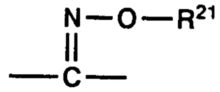

are present in the syn-isomeric form or as mixtures in which the syn-isomeric form predominates.

**22.** Compounds according to claim , characterized as [6R-[6-alpha-7-beta]]-3-[[[[6-$R^{33}$,8-$R^{30}$,1-$R^{31}$-1,4-dihydro-7-(4-$R^{34}$-1-piperazinyl)-4-oxo-3-quinolinyl]carbonyl]oxy]methyl]-8-oxo-7-[(phenoxyacetyl)amino]-5-thia-1-azabicyclo[4.2.0]oct-2-ene-carboxylic acids and salts thereof and hydrates of these carboxylic acids and salts, wherein $R^{33}$ and $R^{30}$ represent hydrogen or halogen, $R^{31}$ represents hydrogen, $C_1$-$C_8$-alkyl or 2-halo-$C_1$-$C_8$-alkyl and $R^{34}$ represents hydrogen or $C_1$-$C_8$-alkyl.

**23.** Compounds according to claim 22, characterized in that $R^{33}$ and $R^{30}$ represent hydrogen or fluorine, $R^{31}$ represents ethyl or 2-fluoroethyl and $R^{34}$ represents methyl.

**24.** Compounds according to claim 23, characterized in that $R^{33}$ and $R^{30}$ represent fluorine and $R^{31}$ represents 2-fluoroethyl.

**25.** Compounds according to claim 22, characterized in that $R^{33}$ represents fluorine, $R^{30}$ represents hydrogen, $R^{31}$ represents 2-fluoroethyl and $R^{34}$ represents methyl.

**26.** Compounds according to claim 22, characterized in that $R^{33}$ represents fluorine, $R^{30}$ represents hydrogen, $R^{31}$ represents ethyl and $R^{34}$ represents methyl.

**27.** Compounds according to claim 22, characterized in that $R^{33}$ represents fluorine, $R^{31}$ represents 2-fluoroethyl, $R^{30}$ represents hydrogen and $R^{34}$ represents methyl.

**28.** Compounds according to claim 1, characterized as [6R-[6-alpha,7-beta]]-7-(formylamino)-3-[[[[6-$R^{33}$,8-$R^{30}$, 1-$R^{31}$-1,4-dihydro-7-(4-$R^{34}$-1-piperazinyl)-4-oxo-3-quinolinyl]carbonyl]oxy]methyl]-8-oxo-5-thia-1-azabicyclo[4.2.0]oct-2-ene-2-carboxylic acids and salts thereof and hydrates of these carboxylic acids and salts, wherein $R^{33}$ and $R^{30}$ represent hydrogen or halogen, $R^{31}$ represents hydrogen, $C_1$-$C_8$-alkyl or 2-halo-$C_1$-$C_8$-alkyl and $R^{34}$ represents hydrogen or $C_1$-$C_8$-alkyl.

**29.** Compounds according to claim 28, characterized in that $R^{33}$ and $R^{30}$ represent hydrogen or fluorine, $R^{31}$ represents ethyl or 2-fluoroethyl and $R^{34}$ represents methyl.

**30.** Compounds according to claim 29, characterized in that $R^{33}$ and $R^{30}$ represent fluorine, and $R^{31}$ represents 2-fluoroethyl.

**31.** Compounds according to claim 28, characterized in that $R^{33}$ represents fluorine, $R^{30}$ represent hydrogen, $R^{31}$ represents 2-fluoroethyl and $R^{34}$ represents methyl.

**32.** Compounds according to claim 28, characterized in that $R^{33}$ represents fluorine, $R^{30}$ represent hydrogen, $R^{31}$ represents ethyl and $R^{34}$ represents methyl.

**33.** Compounds according to claim 28, characterized in that $R^{33}$ represents fluorine, $R^{30}$ represent 2-fluoroethyl, $R^{30}$ represents hydrogen and $R^{34}$ represents methyl.

**34.** Compounds according to claim 28, characterized as [6R-[6-alpha,7-beta(Z)]]-7-[[(2-amino-4-thiazolyl)-($R^{21}$-oxyimino)acetyl]amino]-3-[[[[6-$R^{33}$,8-$R^{30}$,1-$R^{31}$-1,4-dihydro-7-(4-$R^{34}$-1-piperazinyl)-4-oxo-3-quinolinyl]carbonyl]oxy]methyl]-8-oxo-5-thia-1-azabicyclo[4.2.0]oct-2-ene-2-carboxylic acids and salts thereof and hydrates of these carboxylic acids and salts, wherein $R^{33}$ and $R^{30}$ represent hydrogen or halogen, $R^{31}$ represents hydrogen, $C_1$-$C_8$-alkyl or 2-halo-$C_1$-$C_8$-alkyl, $R^{34}$ represents hydrogen or $C_1$-$C_8$-alkyl, $R^{21}$ represents the group

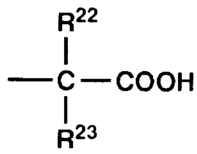

and $R^{22}$ and $R^{23}$ represent hydrogen or $C_1$-$C_8$-alkyl.

**35.** Compounds according to claim 34, characterized in that $R^{22}$ and $R^{23}$ both represent methyl.

**36.** Compounds according to claim 35, characterized in that $R^{33}$ and $R^{30}$ represent hydrogen or fluorine, $R^{31}$ represents ethyl or 2-fluoroethyl and $R^{34}$ represents methyl.

**37.** Compounds according to claim 36, characterized in that $R^{33}$ and $R^{30}$ represent fluorine and $R^{31}$ represents 2-fluoroethyl.

**38.** Compounds according to claim 35, characterized in that $R^{33}$ represents fluorine, $R^{30}$ represent hydrogen, $R^{31}$ represents 2-fluoroethyl and $R^{34}$ represents methyl.

**39.** Compounds according to claim 35, characterized in that $R^{33}$ represents fluorine, $R^{30}$ represent hydrogen, $R^{31}$ represents ethyl and $R^{34}$ represents methyl.

**40.** Compounds according to claim 35, characterized in that $R^{33}$ represents fluorine, $R^{31}$ represent 2-fluoroethyl, $R^{30}$ represents hydrogen and $R^{34}$ represents methyl.

**41.** Compounds according to claim 1,
[6R-(6α,7β)]-3-[[[(1-ethyl-1,4-dihydro-7-methyl-4-oxo-1,8-naphthyridin-3-yl)carbonyl]oxy]methyl]-8-oxo-7-[(phenoxyacetyl)amino]-5-thia-1-azabicyclo[4.2.0]oct-2-ene-2-carboxylic acid as well as salts of this compound and hydrates of this compound and salts.

**42.** Compounds according to claim 1,
[6R-(6α,7β)]-3-[[[(5-ethyl-5,8-dihydro-8-oxo-1,3-dioxoxo[4,5-g]quinolin-7-yl)carbonyl]oxy]methy]-8-oxo-7-[(phenoxyacetyl]amino]-5-thia-1-azabicyclo[4.2.0]oct-2-ene-2-carboxylic acid as well as salts of this compound and hydrates of this compound and salts.

**43.** Compounds according to claim 1,
[6R-(6α,7β)(Z)]-7-[[(2-amino-4-thiazolyl)(methoxyimino)acetyl]amino]-3-[[[(1-ethyl-1,4-dihdyro-7-methyl-4-oxo-1,8-naphthyridin-3-yl)carbonyl]oxy]methyl]-8-oxo-5-thia-1-azabicyclo[4.2.0]oct-2-ene-2-carboxylic acid as well as salts of this compound and hydrates of this compound and salts.

**44.** Compounds according to claim 1,
[6R-(6α,7β)(Z)-7-[[(2-amino-4-thiazolyl)(methoxyimino)acetyl]amino]-3-[[[(5-ethyl-5,8-dihydro-8-oxo-1,3-dioxolo[4.5-g]quinolin-7-yl)carbonyl]oxy]methyl]-8-oxo-5-thia-1-azabicyclo[4.2.0]oct-2-ene-2-carboxylic acid as well as salts of this compound and hydrates of this compound and salts.

**45.** Compounds according to claim 1,
[6R-(6α,7β)]-3-[[[(1-ethyl-6-fluoro-1,4-dihydro-7-(4-formyl-1-piperazinyl)-4-oxoquinolin-3-yl]carbonyl]-oxy]methyl]-8-oxo-7-[(phenoxyacetyl)amino]-5-thia-1-azabicyclo[4.2.0]oct-2-ene-2-carboxylic acid as well as salts of this compound and hydrates of this compound and salts.

**46.** Compounds according to claim 1,
[6R-(6α,7β)]-3-[[[(5-ethyl-5,8-dihdyro-8-oxo-1,3-dioxolo[4,5-g]quinolin-7-yl)carbonyl]oxy]methyl]-7-[-(2-thienylacetyl)amino]-8-oxo-5-thia-1-azabicyclo[4.2.0]oct-2-ene-2-carboxylic acid as well as salts of this compound and hydrates of this compound and salts.

**47.** Compounds according to claim 1,
[6R-(6α,7β)]-3-[[[(1-ethyl-6-fluoro-1,4-dihydro-7-(4-formyl-1-piperazinyl)-4-oxoquinolin-3-yl]carbonyl]-

oxy]methyl]-7-[(2-thienylacetyl)amino]-8-oxo-5-thia-1-azabicyclo[4.2.0]-oct-1-ene-2-carboxylic acid as well as salts of this compound and hydrates of this compound and salts.

**48.** Compounds according to claim 1,
[6R-(6α,7β)]-3-[[[(1-ethyl-6-fluoro-1,4-dihydro-7-(1-pyrrolidinyl)-4-oxoquinolin-3-yl]carbonyl]oxy]-methyl]-8-oxo-7-[(phenoxyacetyl)amino]-5-thia-1-azabicyclo[4.2.0]oct-2-ene-2-carboxylic acid as well as salts of this compound and hydrates of this compound and salts.

**49.** Compounds according to claim 1,
[6R-(6α,7β)(Z)-7-[[(2-amino-4-thiazolyl)(methoxyimino)acetyl]amino]-3-[[[[1-ethyl-6-fluoro-1,4-dihydro-7-(1-pyrrolidinyl)-4-oxoquinolin-3-yl]carbonyl]oxy]methyl]-8-oxo-5-thia-1-azabicyclo[4.2.0]oct-2-ene-2-carboxylicacid as well as salts of this compound and hydrates of this compound and salts.

**50.** Compounds according to claim 1,
[6R-(6α,7β)(Z)]-7-[[(2-amino-4-thiazolyl)(methoxyimino)acetyl]amino]-3-[[[[1-ethyl-6-fluoro-1,4-dihydro-7-(4-formyl-1-piperazinyl)-4-oxoquinolin-3-yl]carbonyl]oxy]methy]-8-oxo-5-thia-1-azabicyclo-[4.2.0]oct-2-ene-2-carboxylic acid as well as salts of this compound and hydrates of this compound and salts.

**51.** Compounds according to claim 1,
[6R-(6α,7β)]-3-[[[(1-ethyl-6-fluoro-1,4-dihydro-7-(4-thiomorpholinyl)-4-oxoquinolin-3-yl]carbonyl]oxy]-methyl]-8-oxo-7-[(phenoxyacetyl)amino]-5-thia-1-azabicyclo[4.2.0]oct-2-ene-2-carboxylic acid as well as salts of this compound and hydrates of this compound and salts.

**52.** Compounds according to claim 1,
[6R-(6α,7β)]-3-[[[(1-ethyl-6-fluoro-1,4-dihydro-4-oxo-7-(1H-pyrrol-1-yl)quinolin-3-yl]carbonyl]oxy]-methyl]-8-oxo-7-[(phenoxyacetyl)amino]-5-thia-1-azabicyclo[4.3.0]oct-2-ene-2-carboxylic acid as well as salts of this compound and hydrates of this compound and salts.

**53.** Compounds according to claim 1,
[6R-(6α,7β)]-3-[[[(1-(4-fluorophenyl)-5,8-dihydro-8-oxo-1,3-dioxolo[4,5-g]quinolin-7-yl]carbonyl]oxy]-methyl]-8-oxo-7-[(phenoxyacetyl)amino]-5-thia-1-azabicyclo[4.2.0]oct-2-ene-2-carbonxylic acid as well as salts of this compound and hydrates of this compound and salts.

**54.** Compounds according to claim 1,
[6R-(6α,7β)(Z)-7-[[(2-amino-4-thiazolyl)(methoxyimino)acetyl]amino]-3-[[[[1-ethyl-6-fluoro-1,4-dihydro-7-(4-thiomorpholinyl)-4-oxo-quinolin-3-yl]carbonyl]oxy]methyl]-8-oxo-5-thia-1-azabicyclo[4.2.0]-oct-2-ene-2-carboxylic acid as well as salts of this compound and hydrates of this compound and salts.

**55.** Compounds according to claim 1,
[6R-(6α,7β)(Z)]]-7-[[(2-amino-4-thiazoyl)(methoxyimino)acetyl]amino]-3-[[[[6,8-difluoro-1-(2-fluoroethyl)-1,4-dihydro-7-(4-methyl-1-piperazinyl)-4-oxo-quinolin-3-yl]-carbonyl]oxy]methyl]-8-oxo-5-thia-1-azabicyclo[4.2.0]oct-2-ene-2-carboxylic acid as well as salts of this compound and hydrates of this compound and salts.

**56.** Compounds according to claim 1,
[6R-(6α,7β)(Z)]]-3-[[[[7-(3-amino-1-pyrrolidinyl)-8-chloro-1-cyclopropyl-6-fluoro-1,4-dihydro-4-oxo-3-quinolinyl]carbonyl]oxy]methyl]-7-[[(2-amino-4-thiazolyl)[(carboxymethoxy)-imino]acetyl]amino]-8-oxo-5-thia-1-azabicyclo[4.2.0]oct-2-ene-2-carboxylic acid as well as salts of this compound and hydrates of this compound and salts.

**57.** Compounds according to claim 1,
[6R-(6α,7β)(Z)]]-3-[[[[7-(3-amino-1-pyrrolidinyl)-8-chloro-1-cyclopropyl-6-fluoro-1,4-dihydro-4-oxo-3-quinolinyl]carbonyl]oxy]methyl]-7-[[(2-amino-4-thiazolyl)[(1-carboxy-1-methylethoxy)imino]acetyl]amino]-8-oxo-5-thia-1-azabicyclo[4.2.0]oct-2-ene-2-carboxylic acid as well as salts of this compound and hydrates of this compound and salts.

**58.** Compounds according to claim 1,
[6R-(6α,7β)(Z)]]-3-[[[[7-(3-amino-1-pyrrolidinyl)-8-chloro-1-cyclopropyl-6-fluoro-1,4-dihydro-4-oxo-3-quinolinyl]carbonyl]oxy]methyl]-7-[[(2-amino-4-thiazolyl)(methoxyimino)acetyl]amino]-8-oxo-5-thia-1-azabicyclo[4.2.0]oct-2-ene-2-carboxylic acid as well as salts of this compound and hydrates of this compound and salts.

**59.** Compounds according to any one of claims 1-58 as pharmaceutically active substances.

**60.** Compounds according to any one of claims 1-58 as pharmaceutically active substances for the treatment and prophylaxis of infectious diseases.

**61.** A pharmaceutical preparation, characterized in that it contains a compound according to any one of claims 1-58.

**62.** A pharmaceutical preparation for the treatment and prophylaxis of infectious diseases, characterized in that it contains a compound according to any one of claims 1-58.

**63.** A process for the manufacture of the compounds according to any one of claims 1-58, characterized by
(a) for the manufacture of a readily hydrolyzable ester of a carboxylic acid of formula I, reacting a compound of the general formula

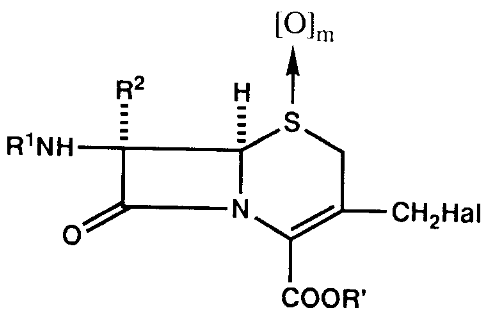

II

in which m, $R^1$ and $R^2$ have the significance given in claim 1, Hal represents halogen and R' represents the residue of a readily hydrolyzable ester,
with a salt of a carboxylic acid of the formula

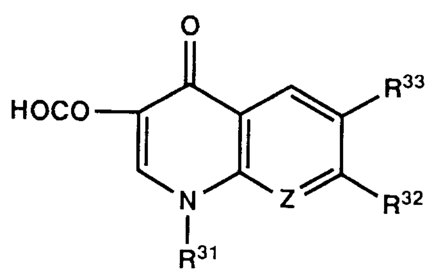

III

in which $R^{31}$, $R^{32}$ and $R^{33}$ have the significance give in claim 1,
or

(b) for the manufacture of a carboxylic acid of formula I, converting an ester of the general formula

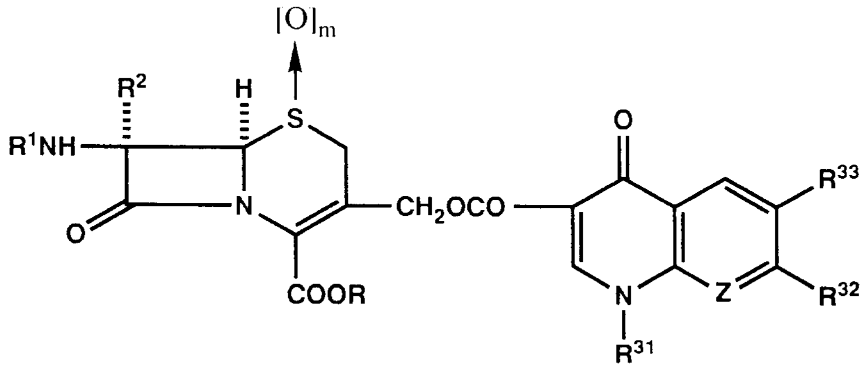

IV

in which m, $R^1$, $R^2$, $R^{31}$, $R^{32}$ and $R^{33}$ have the significance given in claim 1 and R represents an ester protecting group,
into the carboxylic acid of formula I, or
(c) for the manufacture of a compound of formula I in which m represents 0, reducing a compound of the general formula

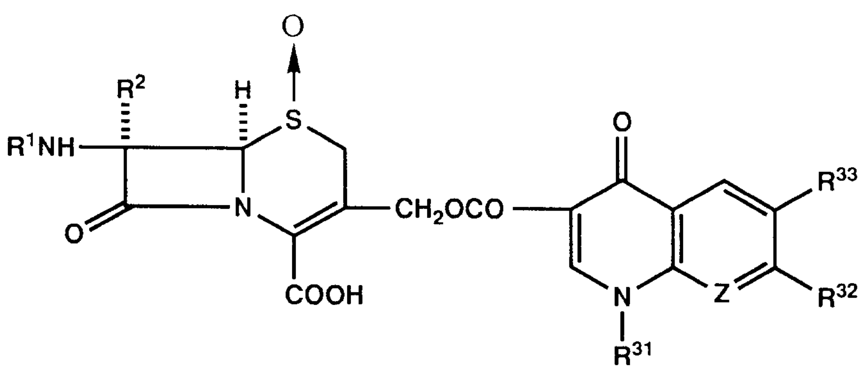

V

in which $R^1$, $R^2$, $R^{31}$, $R^{32}$ and $R^{33}$ have the significance given in claim 1,
(d) for the manufacture of a compound of formula I in which m represents 1 or 2, or an ester of salt thereof, oxidizing a compound of the general formula

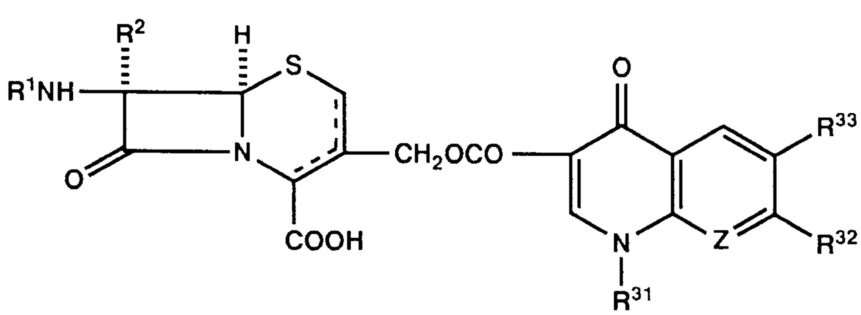

VI

wherein $R^1$, $R^2$, $R^{31}$, $R^{32}$ and $R^{33}$ have the significance given in claim 1 and the dotted lines indicate the presence of a Δ2- or Δ3-double bond,
or an ester or salt thereof, or

(e) for the manufacture of a compound of formula I in which $R^1$ signifies the group

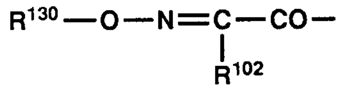

and $R^{102}$ signifies an amino-substituted 5-, 6- or 7-membered heterocyclic ring having 1, 2, 3 or 4 nitrogen, oxygen and/or sulphur atoms and $R^{130}$ has the significance given in claim 8, or an ester as salt thereof, cleaving off the amino-protecting group in the substituent $R^{103}$ of a compound of the general formula

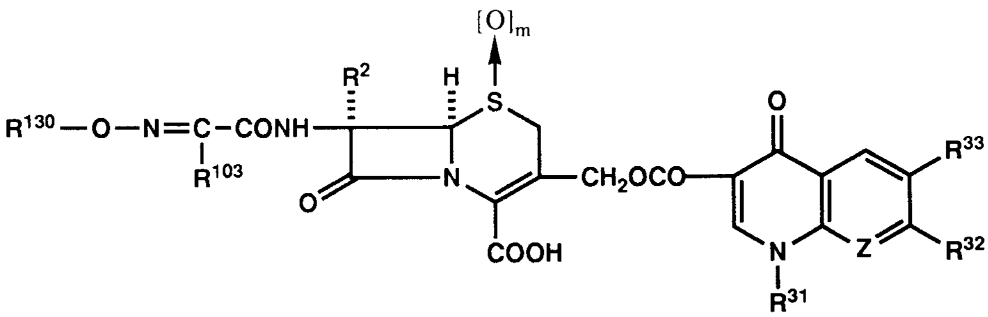

VII

in which m, $R^2$, $R^{31}$, $R^{32}$, $R^{33}$ have the significance given in claim 1 and $R^{130}$ has the significance given in claim 8 and $R^{103}$ has the significance given for $R^{102}$ except that the amino substitutent is protected,
or of an ester or salt thereof, or
(f) for the manufacture of a readily hydrolyzable ester of a compound of formula I, subjecting a carboxylic acid of formula I to a corresponding esterification, or
(g) for the manufacture of salts or hydrates of a compound of formula I or hydrates of said salts, converting a compound of formula I into a salt or hydrate or into a hydrate of said salt.

**64.** The use of the compounds according to any one of claims 1-58 for the manufacture of medicaments for the treatment or prophylaxis of infectious diseases.

**Claims for the following Contracting States : AT, ES, GR**

**1.** A process for the manufacture of acyl derivatives of the general formula

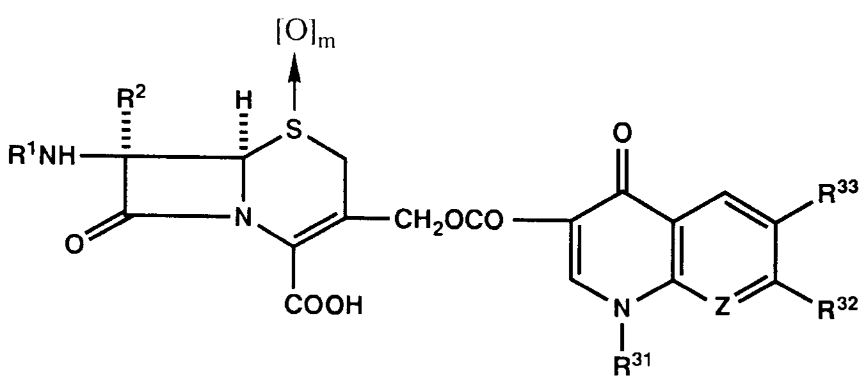

wherein m represents 0, 1 or 2, $R^1$ represents hydrogen or an acyl group, $R^2$ represents hydrogen, $C_1$-$C_8$-alkoxy, amino, $C_1$-$C_8$-alkylthio or $C_1$-$C_8$-alkanoylamino, $R^{31}$ represents hydrogen, $C_1$-$C_8$-alkyl, $C_2$-$C_6$-alkenyl, $C_3$-$C_7$-cycloalkyl, halo$_1$-$C_8$-alkyl or halo-phenyl; Z represents $R^{30}$-C or nitrogen; $R^{30}$ represents hydrogen or halogen, or $R^{30}$ and $R^{31}$ together represent a $C_3$-$C_5$-alkylene group, a $C_2$-$C_4$

alkylenemonooxy group or a $C_1$-$C_2$-alkylenedioxy group; $R^{32}$ represents hydrogen, halogen, $C_1$-$C_8$-alkyl or a 5- or 6-membered heterocyclic ring which contains one, two or three oxygen, nitrogen and/or sulphur atoms and which is optionally substituted by $C_1$-$C_8$-alkyl, $C_1$-$C_8$-alkoxy, halogen, halo-$C_1$-$C_8$-alkyl, amino, mercapto, hydroxy, carbamoyl or carboxy and $R^{33}$ represents hydrogen or halogen or $R^{32}$ and $R^{33}$ together represent a $C_1$-$C_4$-alkylenedioxy group,
and of readily hydrolyzable esters and salts of these compounds and of hydrates of the compounds of formula I or of their esters and salts, characterized by

(a) for the manufacture of a readily hydrolyzable ester of a carboxylic acid of formula I, reacting a compound of the general formula

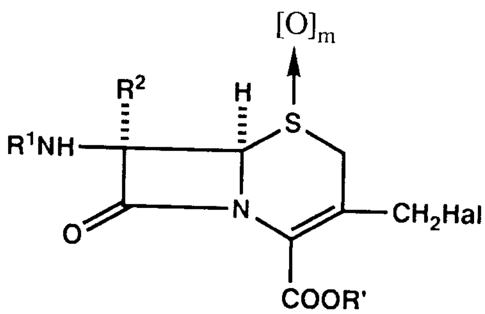

II

in which m, $R^1$ and $R^2$ have the significance given above, Hal represents halogen and R' represents the residue of a readily hydrolyzable ester,
with a salt of a carboxylic acid of the formula

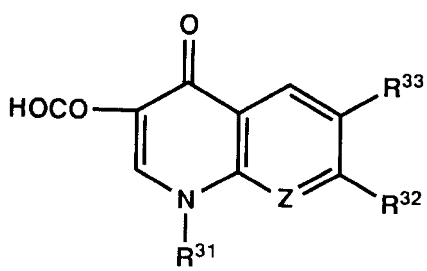

III

in which $R^{31}$, $R^{32}$ and $R^{33}$ have the significance give above,
or
(b) for the manufacture of a carboxylic acid of formula I, converting an ester of the general formula

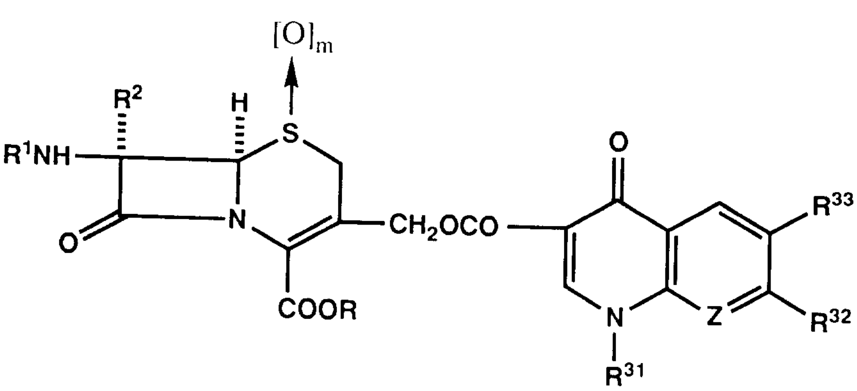

IV

in which m, $R^1$, $R^2$, $R^{31}$, $R^{32}$ and $R^{33}$ have the significance given above and R represents an ester protecting group,
into the carboxylic acid of formula I, or

(c) for the manufacture of a compound of formula I in which m represents 0, reducing a compound of the general formula

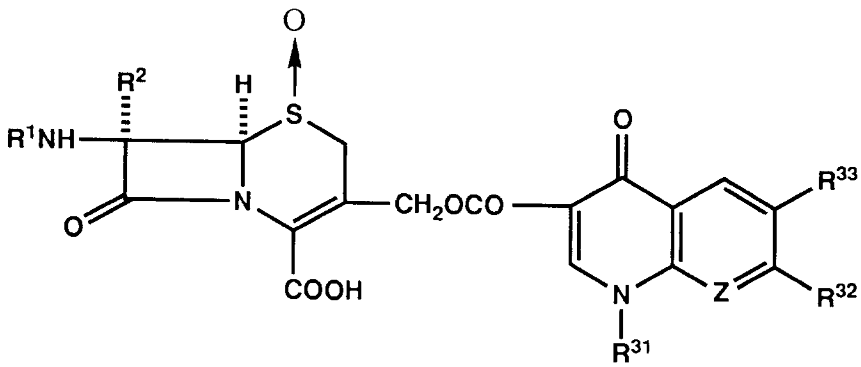

V

in which $R^1$, $R^2$, $R^{31}$, $R^{32}$ and $R^{33}$ have the significance given above,

(d) for the manufacture of a compound of formula I in which m represents 1 or 2, or an ester of salt thereof, oxidizing a compound of the general formula

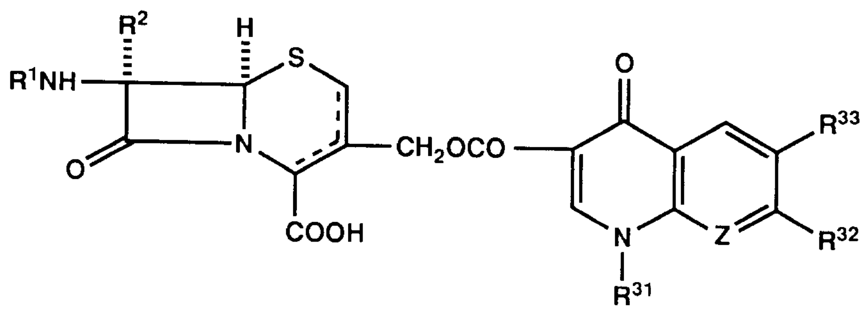

VI

wherein $R^1$, $R^2$, $R^{31}$, $R^{32}$ and $R^{33}$ have the significance given above and the dotted lines indicate the presence of a Δ2- or Δ3-double bond,

or an ester or salt thereof, or

(e) for the manufacture of a compound of formula I in which $R^1$ signifies the group

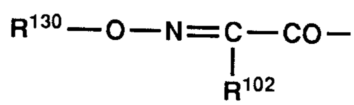

and $R^{102}$ signifies an amino-substituted 5-, 6- or 7-membered heterocyclic ring having 1, 2, 3 or 4 nitrogen, oxygen and/or sulphur atoms and $R^{130}$ has the significance given in claim 8, or an ester as salt thereof, cleaving off the amino-protecting group in the substituent $R^{103}$ of a compound of the general formula

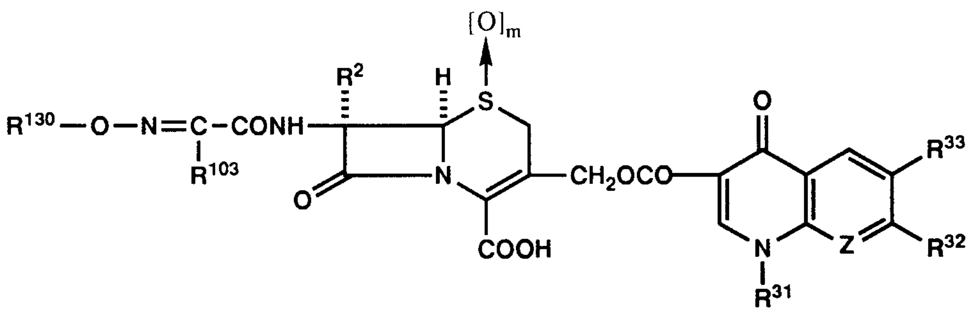

VII

in which m, $R^2$, $R^{31}$, $R^{32}$, $R^{33}$ have the significance given above and $R^{130}$ has the significance given in claim 8 and $R^{103}$ has the significance given for $R^{102}$ except that the amino substitutent is protected,

or of an ester or salt thereof, or

(f) for the manufacture of a readily hydrolyzable ester of a compound of formula I, subjecting a carboxylic acid of formula I to a corresponding esterification, or

(g) for the manufacture of salts or hydrates of a compound of formula I or hydrates of said salts, converting a compound of formula I into a salt or hydrate or into a hydrate of said salts.

**2.** A process for the manufacture of compounds according to claim 1 in which m represents 0 and $R^2$ represents hydrogen, characterized in that correspondingly substituted starting compounds are used.

**3.** A process for the manufacture of compounds according to claim 1 or 2 in which Z represents the group Z $R^{30}$-C, $R^{30}$ represents hydrogen or halogen, $R^{31}$ represents $C_1$-$C_8$-alkyl, halo-$C_1$-$C_8$-alkyl or $C_3$-$C_7$-cycloalkyl, $R^{32}$ represents $C_1$-$C_8$-alkyl, piperazinyl or ($C_1$-$C_8$-alkyl)-piperazinyl and $R^{33}$ represents hydrogen or halogen, characterized in that correspondingly substituted starting compounds are used.

**4.** A process for the manufacture of compounds according to claim 3 in which $R^{30}$ represents hydrogen or fluorine, $R^{31}$ represents ethyl, fluoroethyl or cyclopropyl, $R^{32}$ represents piperazinyl or 4-methyl-piperazinyl and $R^{33}$ represents hydrogen or fluorine, characterized in that correspondingly substituted in claims 4 to 40 starting compounds are used.

**5.** A process for the manufacture of compounds according to any one of claims 1-4 in which $R^1$ represents one of the following acyl groups:

(a) an aliphatic group of the formula

$R^5$-CO-

in which $R^5$ represents $C_1$-$C_8$-alkyl, $C_3$-$C_7$-cycloalkyl, $C_1$-$C_8$-alkoxy, $C_2$-$C_6$-alkenyl, $C_3$-$C_7$-cycloalkenyl or cyclohexadienyl or $C_1$-$C_8$-alkyl or $C_2$-$C_6$-alkenyl substituted with one or more halogen, cyano, nitro, amino, mercapto, $C_1$-$C_8$-alkylthio or cyanomethylthio residues;

(b) a carbocyclic aromatic group of the general formula

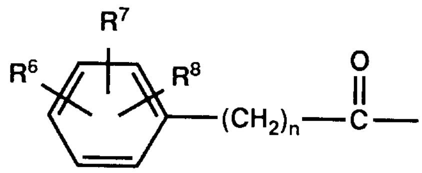

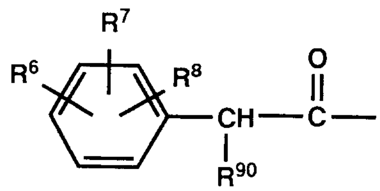

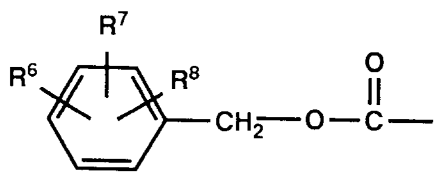

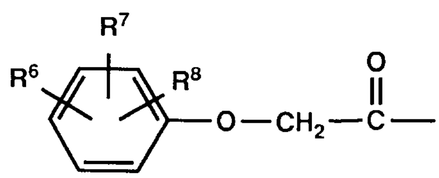

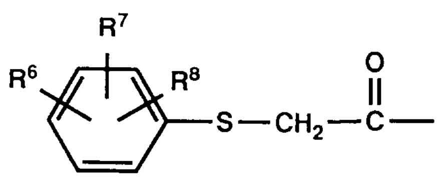

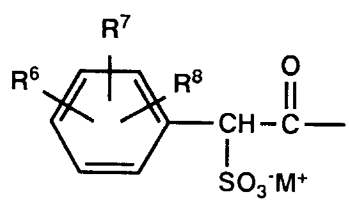

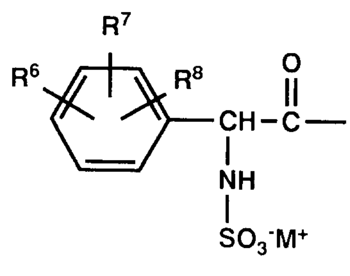

in which n represents 0, 1, 2 or 3; $R^6$ and $R^7$ and $R^8$ represent hydrogen, halogen, hydroxy, nitro, amino, cyano, trifluoromethyl, $C_1$-$C_4$-alkyl, $C_1$-$C_4$-alkoxy or aminomethyl, $R^{90}$ represents

amino, hydroxy, a carboxy salt, protected carboxy, formyloxy or azido and M represents a cation;
(c) a heteroaromatic group of the general formulae

$R^{101}$-$(CH_2)_n$-CO-

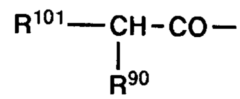

$R^{101}$-O-$CH_2$-CO-
$R^{101}$-S-$CH_2$-CO-

or

$R^{101}$-CO-CO-

in which $R^{90}$ has the significances given above, n represents 0,1,2 or 3 and $R^{101}$ represents a 5-, 6- or 7-membered heterocyclic ring which contains 1, 2, 3 or 4 nitrogen, oxygen and/or sulphur atoms and which is optionally substituted by $C_1$-$C_8$-alkyl, $C_1$-$C_8$-alkoxy, halogen, halo-$C_1$-$C_8$-alkyl, amino, mercapto, hydroxy, carbamoyl or carboxy;
(d) a [[(4-substituted-2,3-dioxo-1-piperazinyl)carbonyl]amino]arylacetyl group of the formula

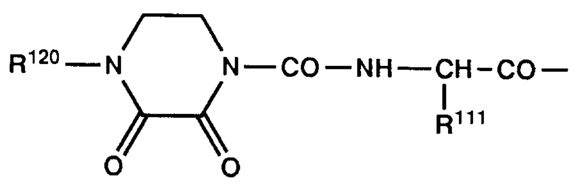

in which $R^{111}$ represents $C_1$-$C_8$-alkyl, hydroxy-$C_1$-$C_8$-alkyl or an aromatic group of the general formula

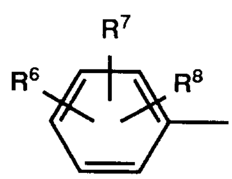

in which $R^6$, $R^7$ and $R^8$ have the significance give above) or a heterocyclic ring as defined for $R^{101}$ and $R^{120}$ represents$C_1$-$C_8$-alkyl optionally substituted with one or more halogen, cyano, nitro, amino and/or mercapto residues;
(e) a (substituted oxyimino)arylacetyl group of the general formula

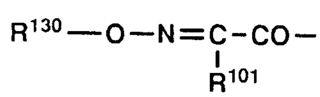

in which $R^{101}$ has the significances given above and $R^{130}$ represents hydrogen, $C_1$-$C_8$-alkyl, $C_3$-$C_7$-cycloalkyl, carboxy-$C_3$-$C_7$-cycloalkyl or substituted $C_1$-$C_8$-alkyl wherein the $C_1$-$C_8$-alkyl group is substituted with one or more halogen, cyano, nitro, amino, mercapto, $C_1$-$C_8$-alkylthio, aromatic group defined above by $R^{111}$, carboxy (including salts thereof), $C_1$-$C_7$-alkanoylamino, $C_2$-$C_9$-alkoxycarbonyl, phenylmethoxycarbonyl, diphenylmethoxycarbonyl, hydroxy ($C_1$-$C_8$-alkoxy)phosphinyl, dihydrox-

yphosphinyl, hydroxy(phenylmethoxy)phosphinyl and/or di($C_1$-$C_8$-alkoxy)phosphinyl residues;
(f) an (acylamino)arylacetyl group of the general formula

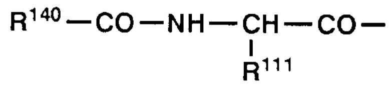

in which $R^{111}$ has the significances given above and $R^{140}$ represents a group of the general formula

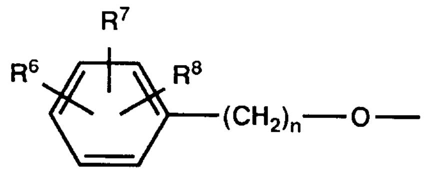

(wherein $R^6$, $R^7$, $R^8$ and n have the above significances), hydrogen, $C_1$-$C_8$-alkyl, substituted $C_1$-$C_8$-alkyl, amino $C_1$-$C_8$-alkylamino or cyano-$C_1$-$C_8$-alkylamino;
(g) a [[[3-substituted-2-oxo-1-imidazolidinyl]carbonyl]amino]arylacetyl group of the general formula

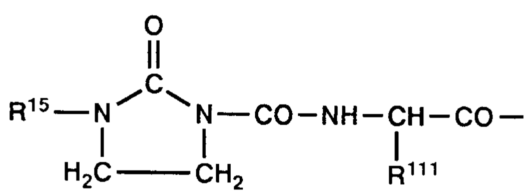

in which $R^{111}$ has the significance given above and $R^{15}$ represents hydrogen, $C_1$-$C_8$-alkylsulphonyl, -N = $CHR^{111}$ (wherein $R^{111}$ has the significance given above), $R^{16}$CO-(wherein $R^{16}$ represents hydrogen or $C_1$-$C_8$-alkyl optionally substituted with halogen), an aromatic group as defined under $R^{111}$ above or $C_1$-$C_8$-alkyl optionally substituted with one or more halogen, cyano, nitro, amino and/or mercapto residues,

characterized in that corresponding starting compounds are used.

6. A process for the manufacture of compounds according to any one of claims 1-5, in which $R^1$ represents a carbocyclic aromatic group of the general formula

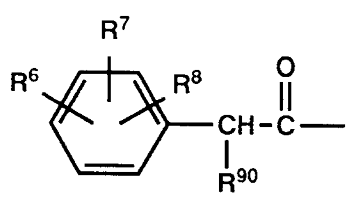

in which $R^{90}$ represents amino, hydroxy, a carboxy salt, benzyloxycarbonyl, formyloxy or azido and $R^6$, $R^7$ and $R^8$ represent hydrogen, halogen, hydroxy, nitro, amino, cyano, trifluoromethyl, $C_1$-$C_4$-alkyl, $C_1$-$C_4$-alkoxy or aminomethyl,

characterized in that corresponding starting compounds are used.

7. A process for the manufacture of compounds according to claim 6 in which $R^6$, $R^7$ and $R^8$ represent hydrogen and $R^{90}$ represents hydrogen or hydroxy, characterized in that corresponding starting

compounds are used.

**8.** A process for the manufacture of compounds according to any one of claims 1-5 in which $R^1$ represents an acyl group of the general formula

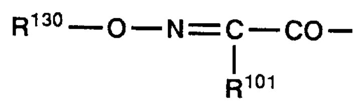

in which $R^{101}$ represents a 5-, 6- or 7-membered heterocyclic ring which contains 1, 2, 3 or 4 nitrogen, oxygen and/or sulphur atoms and which is optionally substituted by halogen, hydroxy, nitro, amino, cyano, trifluoromethyl, $C_1$-$C_4$-alkyl or $C_1$-$C_4$-alkoxy and $R^{130}$ represents hydrogen $C_1$-$C_8$-alkyl, $C_3$-$C_7$-cycloalkyl, carboxy-$C_3$-$C_7$-cycloalkyl or $C_1$-$C_8$-alkyl substituted with one or more halogen, cyano, nitro, amino, mercapto, $C_1$-$C_8$-alkylthio, aromatic group defined by $R^{111}$ in claim 5, carboxy (including salts thereof), $C_1$-$C_7$-alkanoylamino, $C_2$-$C_9$-alkoxycarbonyl, phenylmethoxycarbonyl, diphenylmethoxycarbonyl, hydroxy($C_1$-$C_8$-alkoxy)phosphinyl, dihydroxyphosphinyl, hydroxy(phenylmethoxy)phosphinyl and/or di($C_1$-$C_8$-alkoxy)phosphinyl residues,
characterized in that corresponding starting compounds are used.

**9.** A process for the manufacture of compounds according to any one of claims 1-5 and 8 in which $R^1$ represents an acyl group of the general formula

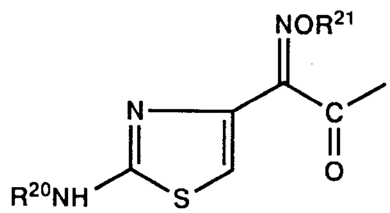

wherein $R^{20}$ represents hydrogen or an amino protecting group and $R^{21}$ represents hydrogen, $C_1$-$C_8$-alkyl or a group of the general formula

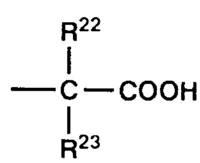

wherein $R^{22}$ and $R^{23}$ represent hydrogen, $C_1$-$C_8$-alkyl or together with the linking carbon atoms represent a 3-7-membered carbocyclic ring,
characterized in that corresponding starting compounds are used.

**10.** A process for the manufacture of compounds according to claim 9 in which $R^{20}$ represents hydrogen and $R^{21}$ represents methyl or

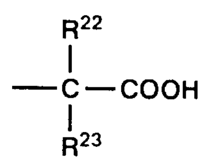

wherein $R^{22}$ and $R^{23}$ signify hydrogen or methyl, characterized in that corresponding starting compounds are used.

**11.** A process for the manufacture of compounds according to claim 1 of the general formula

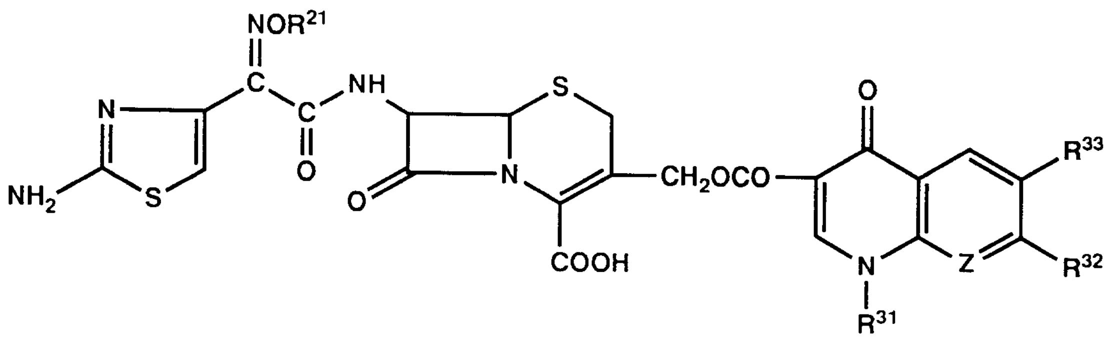

in which $R^{21}$ has the significance given in claim 9 and Z, $R^{31}$, $R^{32}$ and $R^{33}$ have the significance given in claim 1, characterized in that corresponding starting compounds are used.

**12.** A process for the manufacture of compounds according to any one of claims 1-11 in which $R^{21}$ represents methyl or the group of the general formula

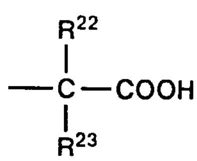

$R^{22}$ and $R^{23}$ represent hydrogen or methyl, Z represents the group $R^{30}$-C, $R^{30}$ represents hydrogen or halogen, $R^{31}$ represents $C_1$-$C_8$-alkyl, halo-$C_1$-$C_8$-alkyl or $C_3$-$C_7$-cycloalkyl, $R^{32}$ represents $C_1$-$C_8$-alkyl, piperazinyl or ($C_1$-$C_8$-alkyl)piperazinyl and $R^{33}$ represents hydrogen or halogen, characterized in that corresponding starting compounds are used.

**13.** A process for the manufacture of compounds according to claim 12 in which $R^{30}$ represents hydrogen or fluorine, $R^{312}$ represents ethyl, fluoroethyl or cyclopropyl, $R^{32}$ represents methyl, 4-methyl-piperazinyl or piperazinyl and $R^{33}$ represents hydrogen or fluorine, characterized in that corresponding starting compounds are used.

**14.** A process for the manufacture of compounds according to claim 1, characterized as [6R-[6-alpha,7-beta(Z)]]-7-[[(2-amino-4-thiazolyl)($R^{21}$-oxyimino)acetyl]amino]-3-[[[[6-$R^{33}$,3-$R^{30}$, 1-$R^{31}$-1,4-dihydro-7-(4-$R^{34}$-1-piperazinyl)-4-oxo-3-quinolinyl]carbonyl]oxy]methyl]-8-oxo-5-thia-1-azabicyclo[4.2.0]oct-2-ene-2-carboxylic acids and salts thereof and hydrates of these carboxylic acids and salts, wherein $R^{33}$ and $R^{30}$ signify hydrogen or halogen, $R^{31}$ signifies hydrogen, $C_1$-$C_8$-alkyl or 2-halo-$C_1$-$C_8$-alkyl, $R^{34}$ signifies hydrogen or $C_1$-$C_8$-alkyl and $R^{21}$ signifies hydrogen, $C_1$-$C_8$-alkyl or a group of the general formula

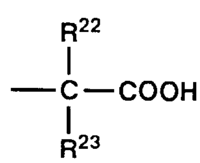

and $R^{22}$ and $R^{23}$ signify hydrogen or $C_1$-$C_8$-alkyl, characterized in that corresponding starting compounds are used.

**15.** A process for the manufacture of compounds according to claim 14 in which $R^{21}$ represents methyl, characterized in that corresponding starting compounds are used.

**16.** A process for the manufacture of compounds according to claim 15 in which $R^{33}$ and $R^{30}$ represent hydrogen or fluorine, $R^{31}$ represents ethyl or 2-fluoroethyl and $R^{34}$ represents methyl, characterized in that corresponding starting compounds are used.

**17.** A process for the manufacture of compounds according to claim 16 in which $R^{33}$ and $R^{30}$ represent fluorine and $R^{31}$ represents 2-fluoroethyl, characterized in that corresponding starting compounds are used.

**18.** A process for the manufacture of compounds according to claim 15 in which $R^{33}$ represents fluorine, $R^{30}$ represents hydrogen, $R^{31}$ represents 2-fluoroethyl and $R^{34}$ represents methyl, characterized in that corresponding starting compounds are used.

**19.** A process for the manufacture of compounds according to claim 15 in which $R^{33}$ represents fluorine, $R^{30}$ represents hydrogen, $R^{31}$ represents ethyl and $R^{34}$ represents methyl, characterized in that corresponding starting compounds are used.

**20.** A process for the manufacture of compounds according to claim 15 in which $R^{33}$ represents fluorine, $R^{31}$ represents 2-fluoroethyl, $R^{30}$ represents hydrogen and $R^{34}$ represents methyl, characterized in that corresponding starting compounds are used.

**21.** A process for the manufacture of compounds according to any one of claims 8-20 in which the groups of the general formulae

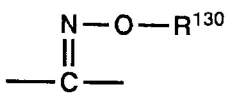

and

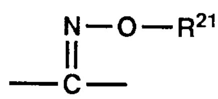

are present in the syn-isomeric form or as mixtures in which the syn-isomeric form predominates, characterized in that corresponding starting compounds are used.

**22.** A process for the manufacture of compounds according to, characterized as [6R-[6-alpha-7-beta]]-3-[[[-[6-$R^{33}$,8-$R^{30}$,1-$R^{31}$-1,4-dihydro-7-(4-$R^{34}$-1-piperazinyl)-4-oxo-3-quinolinyl]carbonyl]oxy]methyl]-8-oxo-7-[-(phenoxyacetyl)amino]-5-thia-1-azabicyclo[4.2.0]oct-2-ene-carboxylic acids and salts thereof and hydrates of these carboxylic acids and salts, wherein $R^{33}$ and $R^{30}$ represent hydrogen or halogen, $R^{31}$ represents hydrogen, $C_1$-$C_8$-alkyl or 2-halo-$C_1$-$C_8$-alkyl and $R^{34}$ represents hydrogen or $C_1$-$C_8$-alkyl, characterized in that corresponding starting compounds are used.

**23.** A process for the manufacture of compounds according to claim 22 in which $R^{33}$ and $R^{30}$ represent hydrogen or fluorine, $R^{31}$ represents ethyl or 2-fluoroethyl and $R^{34}$ represents methyl, characterized in that corresponding starting compounds are used.

**24.** A process for the manufacture of compounds according to claim 23 in which $R^{33}$ and $R^{30}$ represent fluorine and $R^{31}$ represents 2-fluoroethyl, characterized in that corresponding starting compounds are

used.

**25.** A process for the manufacture of compounds according to claim 22 in which $R^{33}$ represents fluorine, $R^{30}$ represents hydrogen, $R^{31}$ represents 2-fluoroethyl and $R^{34}$ represents methyl, characterized in that corresponding starting compounds are used.

**26.** A process for the manufacture of compounds according to claim 22 in which $R^{33}$ represents fluorine, $R^{30}$ represents hydrogen, $R^{31}$ represents ethyl and $R^{34}$ represents methyl, characterized in that corresponding starting compounds are used.

**27.** A process for the manufacture of compounds according to claim 22 in which $R^{33}$ represents fluorine, $R^{31}$ represents 2-fluoroethyl, $R^{30}$ represents hydrogen and $R^{34}$ represents methyl, characterized in that corresponding starting compounds are used.

**28.** A process for the manufacture of compounds according to claim 1, characterized as [6R-[6-alpha,7-beta]]-7-(formylamino)-3-[[[[6-$R^{33}$,8-$R^{30}$, 1-$R^{31}$-1,4-dihydro-7-(4-$R^{34}$-1-piperazinyl)-4-oxo-3-quinolinyl]-carbonyl]oxy]methyl]-8-oxo-5-thia-1-azabicyclo[4.2.0]oct-2-ene-carboxylic acids and salts thereof and hydrates of these carboxylic acids and salts, wherein $R^{33}$ and $R^{30}$ represent hydrogen or halogen, $R^{31}$ represents hydrogen, $C_1$-$C_8$-alkyl or 2-halo-$C_1$-$C_8$-alkyl and $R^{34}$ represents hydrogen or $C_1$-$C_8$-alkyl, characterized in that corresponding starting compounds are used.

**29.** A process for the manufacture of compounds according to claim 28 in which $R^{33}$ and $R^{30}$ represent hydrogen or fluorine, $R^{31}$ represents ethyl or 2-fluoroethyl and $R^{34}$ represents methyl, characterized in that corresponding starting compounds are used.

**30.** A process for the manufacture of compounds according to claim 29 in which $R^{33}$ and $R^{30}$ represent fluorine, and $R^{31}$ represents 2-fluoroethyl, characterized in that corresponding starting compounds are used.

**31.** A process for the manufacture of compounds according to claim 28 in which $R^{33}$ represents fluorine, $R^{30}$ represent hydrogen, $R^{31}$ represents 2-fluoroethyl and $R^{34}$ represents methyl, characterized in that corresponding starting compounds are used.

**32.** A process for the manufacture of compounds according to claim 28 in which $R^{33}$ represents fluorine, $R^{30}$ represent hydrogen, $R^{31}$ represents ethyl and $R^{34}$ represents methyl, characterized in that corresponding starting compounds are used.

**33.** A process for the manufacture of compounds according to claim 28 in which $R^{33}$ represents fluorine, $R^{30}$ represent 2-fluoroethyl, $R^{30}$ represents hydrogen and $R^{34}$ represents methyl, characterized in that corresponding starting compounds are used.

**34.** A process for the manufacture of compounds according to claim 28, characterized as [6R-[6-alpha,7-beta(Z)]]-7-[[(2-amino-4-thiazolyl)($R^{21}$-oxyimino)acetyl]amino]-3-[[[[6-$R^{33}$,8-$R^{30}$,1-$R^{31}$-1,4-dihydro-7-(4-$R^{34}$-1-piperazinyl)-4-oxo-3-quinolinyl]carbonyl]oxy]methyl]-8-oxo-5-thia-1-azabicyclo[4.2.0]oct-2-ene-2-carboxylic acids and salts thereof and hydrates of these carboxylic acids and salts, wherein $R^{33}$ and $R^{30}$ represent hydrogen or halogen, $R^{31}$ represents hydrogen, $C_1$-$C_8$-alkyl or 2-halo-$C_1$-$C_8$-alkyl, $R^{34}$ represents hydrogen or $C_1$-$C_8$-alkyl, $R^{21}$ represents the group

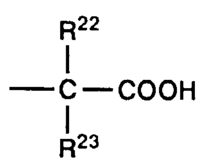

and $R^{22}$ and $R^{23}$ represent hydrogen or $C_1$-$C_8$-alkyl, characterized in that corresponding starting compounds are used.

**35.** A process for the manufacture of compounds according to claim 34 in which $R^{22}$ and $R^{23}$ both represent methyl, characterized in that corresponding starting compounds are used.

**36.** A process for the manufacture of compounds according to claim 35 in which $R^{33}$ and $R^{30}$ represent hydrogen or fluorine, $R^{31}$ represents ethyl or 2-fluoroethyl and $R^{34}$ represents methyl, characterized in that corresponding starting compounds are used.

**37.** A process for the manufacture of compounds according to claim 36 in which $R^{33}$ and $R^{30}$ represent fluorine and $R^{31}$ represents 2-fluoroethyl, characterized in that corresponding starting compounds are used.

**38.** A process for the manufacture of compounds according to claim 35 in which $R^{33}$ represents fluorine, $R^{30}$ represent hydrogen, $R^{31}$ represents 2-fluoroethyl and $R^{34}$ represents methyl, characterized in that corresponding starting compounds are used.

**39.** A process for the manufacture of compounds according to claim 35 in which $R^{33}$ represents fluorine, $R^{30}$ represent hydrogen, $R^{31}$ represents ethyl and $R^{34}$ represents methyl, characterized in that corresponding starting compounds are used.

**40.** A process for the manufacture of compounds according to claim 35 in which $R^{33}$ represents fluorine, $R^{31}$ represent 2-fluoroethyl, $R^{30}$ represents hydrogen and $R^{34}$ represents methyl, characterized in that corresponding starting compounds are used.

**41.** A process for the manufacture of compounds according to claim 1, [6R-(6α,7β)]-3-[[[(1-ethyl-1,4-dihydro-7-methyl-4-oxo-1,8-naphthyridin-3-yl)carbonyl]oxy]methyl]-8-oxo-7-[(phenoxyacetyl)amino]-5-thia-1-azabicyclo[4.2.0]oct-2-ene-2-carboxylic acid as well as salts of this compound and hydrates of this compound and salts, characterized in that correspondingly substituted starting compounds are used.

**42.** A process for the manufacture of compounds according to claim 1, [6R-(6α,7β)]-3-[[[(5-ethyl-5,8-dihydro-8-oxo-1,3-dioxolo[4,5-g]quinolin-7-yl)carbonyl]oxy]methyl]-8-oxo-7-[(phenoxyacetyl]amino]-5-thia-1-azabicyclo[4.2.0]oct-2-ene-2-carboxylic acid as well as salts of this compound and hydrates of this compound and salts, characterized in that correspondingly substituted starting compounds are used.

**43.** A process for the manufacture of compounds according to claim 1, [6R-(6α,7β)(Z)]-7-[[(2-amino-4-thiazolyl)(methoxyimino)acetyl]amino]-3-[[[(1-ethyl-1,4-dihdyro-7-methyl-4-oxo-1,8-naphthyridin-3-yl)-carbonyl]oxy]methy]-8-oxo-5-thia-1-azabicyclo[4.2.0]oct-2-ene-2-carboxylic acid as well as salts of this compound and hydrates of this compound and salts, characterized in that correspondingly substituted starting compounds are used.

**44.** A process for the manufacture of compounds according to claim 1, [6R-(6α,7β)(Z)-7-[[(2-amino-4-thiazolyl)(methoxyimino)acetyl]amino]-3-[[[(5-ethyl-5,8-dihydro-8-oxo-1,3-dioxolo[4.5-g]quinolin-7-yl)-carbonyl]oxy]methyl]-8-oxo-5-thia-1-azabicyclo[4.2.0]oct-2-ene-2-carboxylic acid as well as salts of this compound and hydrates of this compound and salts, characterized in that correspondingly substituted starting compounds are used.

**45.** A process for the manufacture of compounds according to claim 1, [6R-(6α,7β)]-3-[[[(1-ethyl-6-fluoro-1,4-dihydro-7-(4-formyl-1-piperazinyl)-4-oxoquinolin-3-yl]carbonyl]oxy]methyl]-8-oxo-7[(phenoxyacetyl)-amino]-5-thia-1-azabicyclo[4.2.0]oct-2-ene-2-carboxylic acid as well as salts of this compound and hydrates of this compound and salts, characterized in that correspondingly substituted starting compounds are used.

**46.** A process for the manufacture of compounds according to claim 1, [6R-(6α,7β)]-3-[[[(5-ethyl-5,8-dihdyro-8-oxo-1,3-dioxolo[4,5-g]quinolin-7-yl)carbonyl]oxy]methyl]-7-[(2-thienyl acetyl)amino]-8-oxo-5-thia-1-azabicyclo[4.2.0]oct-2-ene-2-carboxylic acid as well as salts of this compound and hydrates of this compound and salts, characterized in that correspondingly substituted starting compounds are used.

**47.** A process for the manufacture of compounds according to claim 1, [6R-(6α,7β)]-3-[[[(1-ethyl-6-fluoro-1,4-dihydro-7-(4-formyl-1-piperazinyl)-4-oxoquinolin-3-yl]carbonyl]oxy]methyl]-7-[(2-thienylacetyl)amino]-8-oxo-5-thia-1-azabicyclo[4.2.0]-oct-1-ene-2-carboxylic acid as well as salts of this compound and hydrates of this compound and salts, characterized in that correspondingly substituted starting compounds are used.

**48.** A process for the manufacture of compounds according to claim 1, [6R-(6α,7β)]-3-[[[(1-ethyl-6-fluoro-1,4-dihydro-7-(1-pyrrolidinyl)-4-oxoquinolin-3-yl]carbonyl]oxy]methyl]-8-oxo-7-[(phenoxyacetyl)amino]-5-thia-1-azabicyclo[4.2.0]oct-2-ene-2-carboxylic acid as well as salts of this compound and hydrates of this compound and salts, characterized in that correspondingly substituted starting compounds are used.

**49.** A process for the manufacture of compounds according to claim 1, [6R-(6α,7β)(Z)-7-[[(2-amino-4-thiazolyl)(methoxyimino)acetyl]amino]-3-[[[[1-ethyl-6-fluoro-1,4-dihydro-7-(1-pyrrolidinyl)-4-oxoquinolin-3-yl]carbonyl]oxy]methyl]-8-oxo-5-thia-1-azabicyclo[4.2.0]oct-2-ene-2-carboxylicacid as well as salts of this compound and hydrates of this compound and salts, characterized in that correspondingly substituted starting compounds are used.

**50.** A process for the manufacture of compounds according to claim 1, [6R-(6α,7β)(Z)]-7-[[(2-amino-4-thiazolyl)(methoxyimino)acetyl]amino]-3-[[[[1-ethyl-6-fluoro-1,4-dihydro-7-(4-formyl-1-piperazinyl)-4-oxoquinolin-3-yl]carbonyl]oxy]methyl]-8-oxo-5-thia-1-azabicyclo[4.2.0]oct-2-ene-carboxylic acid as well as salts of this compound and hydrates of this compound and salts, characterized in that correspondingly substituted starting compounds are used.

**51.** A process for the manufacture of compounds according to claim 1, [6R-(6α,7β)]-3-[[[(1-ethyl-6-fluoro-1,4-dihydro-7-(4-thiomorpholinyl)-4-oxoquinolin-3-yl]carbonyl]oxy]methyl]-8-oxo-7-[(phenoxyacetyl)-amino]-5-thia-1-azabicyclo[4.2.0]oct-2-3n3-2-carboxylic acid as well as salts of this compound and hydrates of this compound and salts, characterized in that correspondingly substituted starting compounds are used.

**52.** A process for the manufacture of compounds according to claim 1, [6R-(6α,7β)]-3-[[[(1-ethyl-6-fluoro-1,4-dihydro-4-oxo-7-(1H-pyrrol-1-yl)quinolin-3-yl]carbonyl]oxy]methyl]-8-oxo-7-[(phenoxyacetyl)amino]-5-thia-1-azabicyclo[4.3.0]oct-2-ene-2-carboxylic acid as well as salts of this compound and hydrates of this compound and salts, characterized in that correspondingly substituted starting compounds are used.

**53.** A process for the manufacture of compounds according to claim 1, [6R-(6α,7β)]-3-[[[(1-(4-fluorophenyl)-5,8-dihydro-8-oxo-1,3-dioxolo[4,5-g]quinolin-7-yl]carbonyl]oxy]methyl]-8-oxo-7-[(phenoxyacetyl)amino]-5-thia-1-azabicyclo[4.2.0]oct-2-ene-2-carbonxylic acid as well as salts of this compound and hydrates of this compound and salts, characterized in that correspondingly substituted starting compounds are used.

**54.** A process for the manufacture of compounds according to claim 1, [6R-(6α,7β)(Z)-7-[[(2-amino-4-thiazolyl)(methoxyimino)acetyl]amino]-3-[[[[1-ethyl-6-fluoro-1,4-dihydro-7-(4-thiomorphonlinyl)-4-oxo-quinolin-3-yl]carbonyl]oxy]methyl]-8-oxo-5-thia-1-azabicyclo[4.2.0]oct-2-ene-2-carboxylic acid as well as salts of this compound and hydrates of this compound and salts, characterized in that correspondingly substituted starting compounds are used.

**55.** A process for the manufacture of compounds according to claim 1, [6R-(6α,7β)(Z)]]-7-[[(2-amino-4-thiazolyl)(methoxyimino)acetyl]amino]-3-[[[[6,8-difluoro-1-(2-fluoroethyl)-1,4-dihydro-7-(4-methyl-1-piperazinyl)-4-oxo-quinolin-3-yl]carbonyl]oxy]methyl]-8-oxo-5-thia-1-azabicyclo[4.2.0]oct-2-ene-2-carboxylic acid as well as salts of this compound and hydrates of this compound and salts, characterized in that correspondingly substituted starting compounds are used.

**56.** A process for the manufacture of compounds according to claim 1, [6R-(6α,7β)(Z)]]-3-[[[[7-(3-amino-1-pyrrolidinyl)-8-chloro-1-cyclopropyl-6-fluoro-1,4-dihydro-4-oxo-3-quinolinyl]carbonyl]oxy]methyl]-7-[[2-amino-4-thiazolyl)[(carboxymethoxy)-imino]acetyl]amino]-8-oxo-5-thia-1-azabicyclo[4.2.0]oct-2-ene-2-carboxylic acid as well as salts of this compound and hydrates of this compound and salts,

characterized in that correspondingly substituted starting compounds are used.

**57.** A process for the manufacture of compounds according to claim 1, [6R-(6α,7β)(Z)]]-3-[[[[7-(3-amino-1-pyrrolidinyl)-8-chloro-1-cyclopropyl-6-fluoro-1,4-dihydro-4-oxo-3-quinolinyl]carbonyl]oxy]methyl]-7-[[(2-amino-4-thiazolyl)[(1-carboxy-1-methylethoxy)imino]acetyl]amino]-8-oxo-5-thia-1-azabicyclo[4.2.0]oct-2-ene-2-carboxyic acid as well as salts of this compound and hydrates of this compound and salts, characterized in that correspondingly substituted starting compounds are used.

**58.** A process for the manufacture of compounds according to claim 1, [6R-(6α,7β)(Z)]]-3-[[[[7-(3-amino-1-pyrrolidinyl)-8-chloro-1-cyclopropyl-6-fluoro-1,4-dihydro-4-oxo-3-quinolinyl]carbonyl]oxy]methyl]-7-[[(2-amino-4-thiazolyl)(methoxyimino)acetyl]amino]-8-oxo-5-thia-1-azabicyclo[4.2.0]oct-2-ene-2-carboxylic acid as well as salts of this compound and hydrates of this compound and salts, characterized in that correspondingly substituted starting compounds are used.

**59.** A process for the manufacture of pharmaceutical preparations, characterized by mixing a acyl derivative of formula I defined in claim 1 or a readily hydrolyzable ester or a salt of this compound or a hydrate of a compound of formula I or of an ester or salt thereof as the active ingredient with non-toxic, inert, solid a liquid carriers and/or excipients which are usual in such preparations and which are suitable for therapeutic administration.

**60.** The use of compounds according to any one of claims 1-58 for the manufacture of medicaments for the treatment or prophylaxis of infectious diseases.

**Revendications**

**Revendications pour les Etats contractants suivants : BE, CH, DE, FR, GB, IT, LI, LU, NL, SE**

**1.** Dérivés acylés de formule générale

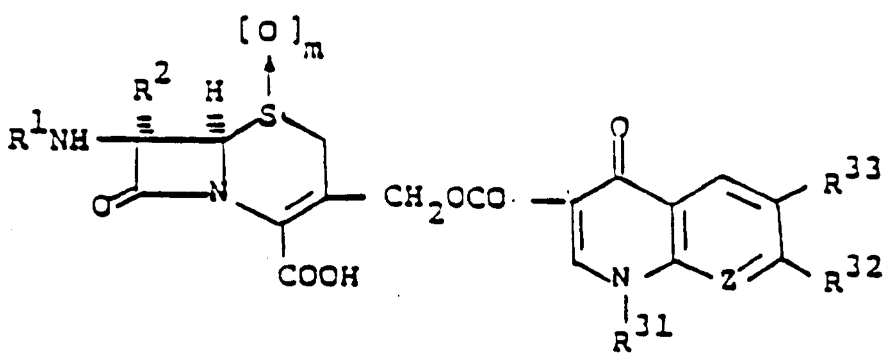

I

dans laquelle m est égal à 0, 1 ou 2, $R^1$ représente l'hydrogène ou un groupe acyle, $R^2$ représente l'hydrogène, un groupe alcoxy en C 1-C 8, amino, alkylthio en C 1-C 8 ou alcanoylamino en C 1-C 7, $R^{31}$ représente l'hydrogène, un groupe alkyle en C 1-C 8, alcényle en C 2-C 6, cycloalkyle en C 3-C 7, halogénoalkyle en C 1-C 8 ou halogénophényle; Z représente $R^{30}$-C ou l'azote, $R^{30}$ représente l'hydrogène ou un halogène ou bien $R^{30}$ et $R^{31}$ forment ensemble un groupe alkylène en C 3-C 5, un groupe (alkyle en C 2-C 4)-monooxy ou (alkylène en C 1-C 2)-dioxy,
$R^{32}$ représente l'hydrogène, un halogène, un groupe alkyle en C 1-C 8 ou un hétérocycle à 5 ou 6 chaînons contenant 1, 2 ou 3 atomes d'oxygène, d'azote et/ou de soufre et éventuellement substitué par des groupes alkyle en C 1-C 8, amino, mercapto, hydroxy, carbamoyle ou carboxy, et $R^{33}$ représente l'hydrogène ou un halogène ou bien $R^{32}$ et $R^{33}$ forment ensemble un groupe (alkylène en C 1-C 4)-dioxy,
leurs esters faciles à hydrolyser et leurs sels, et les hydrates des composés de formule I, de leurs esters et de leurs sels.

**2.** Composés selon revendication 1, caractérisés en ce que m = 0 et $R^2$ représente l'hydrogène.

3. Composés selon revendication 1 ou 2, caractérisés en ce que Z représente $R^{30}$-C dans lequel $R^{30}$ représentel'hydrogène ou un halogène, $R^{31}$ représente un groupe alkyle en C 1-C 8, halogénoalkyle en C 1-C 8 ou cycloalkyle en C 3-C 7, $R^{32}$ représente un groupe alkyle en C 1-C 8, pipérazinyle ou (alkyle en C 1-C 8)-pipérazinyle et $R^{33}$ l'hydrogène ou un halogène.

4. Composés selon revendication 3, caractérisés en ce que $R^{30}$ represente l'hydrogène ou le fluor, $R^{31}$ représente un groupe éthyle, fluoréthyle ou cyclopropyle, $R^{32}$ représente un groupe pipérazinyle ou 4-méthylpipérazinyle et $R^{33}$ l'hydrogène ou le fluor.

5. Composés selon l'une des revendications 1 à 4, caractérisés en ce que $R^1$ représente l'un des groupes acyle suivants :

a) un groupe aliphatique de formule

$R^5$-CO-

dans laquelle $R^5$ représente un groupe alkyle en C 1-C 8, cycloalkyle en C 3-C 7, alcoxy en C 1-C 8, alcényle en C 2-C 6, cycloalcényle en C 3-C 7 ou cyclohexadiényle, ou un groupe alkyle en C 1-C 8 ou alcényle en C 2-C 6 portant un ou plusieurs substituants halogéno, cyano, nitro, amino, mercapto, alkylthio en C 1-C 8 ou cyanométhylthio;

(b) un groupe aromatique carbocyclique répondant à l'une des formules générales

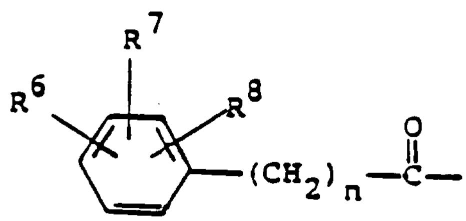

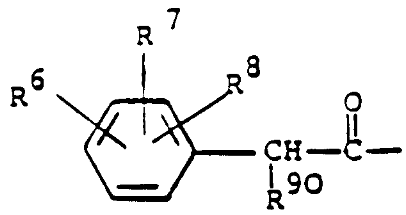

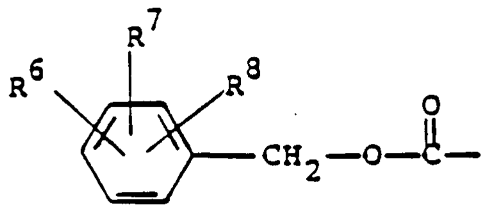

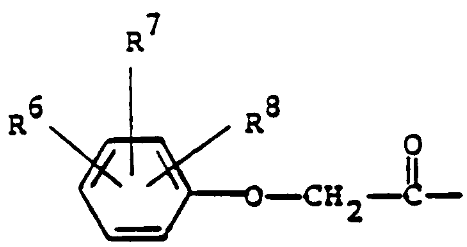

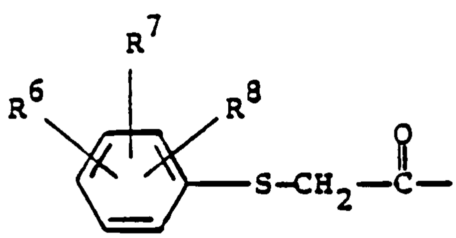

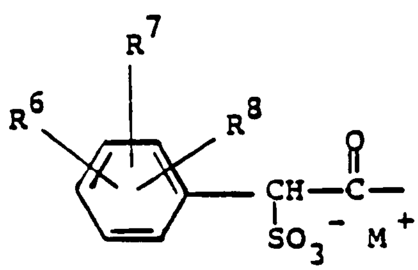

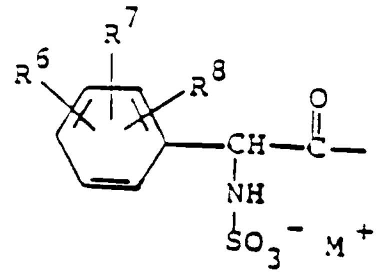

dans lesquelles n est égal à 0, 1, 2 ou 3; $R^6$, $R^7$ et $R^8$ représentent l'hydrogène, des halogènes, des groupes hydroxy, nitro, amino, cyano, trifluorométhyle, alkyle en C 1-C 4, alcoxy en C 1- C 4 ou aminométhyle, $R^{90}$ représente un groupe amino, hydroxy, un groupe carboxy à l'état de sel, un groupe carboxy protégé, un groupe formyloxy ou azido et M est un cation;
(c) un groupe hétéroaromatique répondant à l'une des formules générales

$R^{101}$-$(CH_2)_n$-CO-

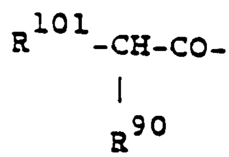

$R^{101}$-O-$CH_2$-CO-
$R^{101}$-S-$CH_2$-CO-

ou

$R^{101}$-CO-CO-

dans lesquelles $R^{90}$ a les significations indiquées ci-dessus, n est égal à 0, 1, 2 ou 3 et $R^{101}$ représente un hétérocycle à 5, 6 ou 7 chaînons contenant 1, 2, 3 ou 4 atomes d'azote, d'oxygène et/ou de soufre et éventuellement substitué par des groupes alkyle en C 1-C 8, alcoxy en C 1-C 8, des halogènes, des groupes halogénoalkyle en C 1-C 8, amino, mercapto, hydroxy, carbamoyle ou carboxy;
(d) un groupe [[4-$R^{120}$-2,3-dioxo-1-pipérazinyl)carbonyl]-amino]-arylacétyle de formule

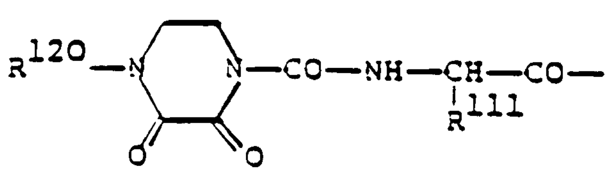

dans laquelle $R^{111}$ représente un groupe alkyle en C 1-C 8, hydroxyalkyle en C 1-C 8, ou un groupe aromatique de formule générale

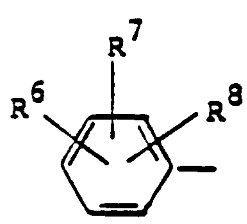

(dans laquelle $R^6$, $R^7$ et $R^8$ ont les significations indiquées ci-dessus), ou un noyau hétérocyclique tel que défini en référence à $R^{101}$, et $R^{120}$ représente un groupe alkyle en C 1-C 8 portant éventuellement un ou plusieurs substituants halogéno, cyano, nitro, amino et/ou mercapto;

(e) un groupe (oxyimino substitué)-arylacétyle de formule générale

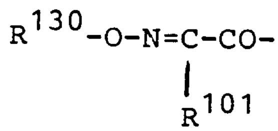

dans laquelle $R^{101}$ a les significations indiquées ci-dessus et $R^{130}$ représente l'hydrogène, un groupe alkyle en C 1-C 8, cycloalkyle en C 3-C 7, carboxy-cycloalkyle en C 3-C 7 ou alkyle en C 1-C 8 substitué, ce groupe alkyle en C 1-C 8 substitué portant un ou plusieurs substituants halogéno, cyano, nitro, amino, mercapto, alkylthio en C 1-C 8, un groupe aromatique tel que défini ci-dessus en référence à $R^{111}$, un groupe carboxy (y compris à l'état de sel), un groupe alcanoylamino en C 1-C 7, alcoxycarbonyle en C 2-C 9, phénylméthoxycarbonyle, diphénylméthoxycarbonyle, hydroxy-(alcoxy en C 1-C 8)-phosphinyle, dihydroxyphosphonyle, hydroxy-(phénylméthoxy)-phosphinyle et/ou di-(alcoxy en C 1-C 8)-phosphinyle;

(f) un groupe (acylamino)-arylacétyle de formule générale

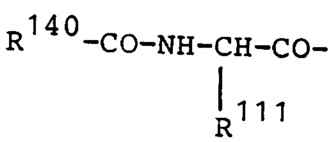

dans laquelle $R^{111}$ a les significations indiquées ci-dessus et $R^{140}$ représente un groupe de formule générale

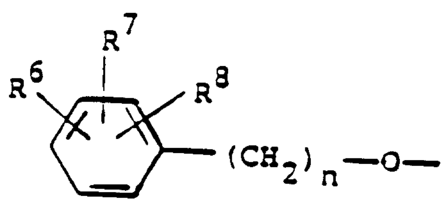

(dans laquelle $R^6$, $R^7$, $R^8$ et n ont les significations indiquées ci-dessus), l'hydrogène, un groupe alkyle en C 1-C 8 substitué, amino, alkylamino en C 1-C 8 ou cyanoalkylamino en C 1-C 8;

(g) un groupe [[[3-$R^{15}$-2-oxo-1-imidazolidinyl]-carbonyl]-amino]-arylacétyle de formule générale

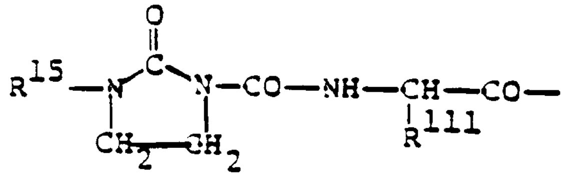

dans laquelle $R^{111}$ a les significations indiquées ci-dessus et $R^{15}$ represente l'hydrogène, un groupe alkylsulfonyle en C 1-C 8, -N=CH-$R^{111}$ (dans lequel $R^{111}$ a les significations indiquées ci-dessus), $R^{16}$-CO (dans lequel $R^{16}$ représente l'hydrogène ou un groupe alkyle en C 1-C 8 éventuellement substitué par des halogènes, un groupe aromatique tel que défini ci-dessus en référence à $R^{111}$ ou un groupe alkyle en C 1-C 8 portant éventuellement un ou plusieurs substituants halogèno, cyano, nitro, amino et/ou mercapto.

6. Composés selon l'une des revendications 1 à 5, caractérisés en ce que $R^1$ représente un groupe aromatique carbocyclique de formule générale

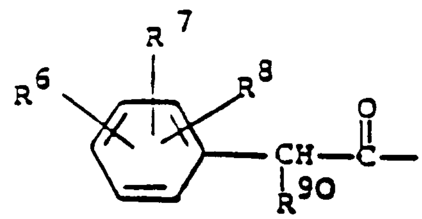

dans laquelle $R^{90}$ représente un groupe amino, hydroxy, un groupe carboxy à l'état de sel, un groupe benzyloxycarbonyle, formyloxy ou azido et $R^6$, $R^7$ et $R^8$ représentent l'hydrogène, un halogène, des groupes hydroxy, nitro, amino, cyano, trifluorométhyle, alkyle en C 1-C 4, alcoxy en C 1-C 4 ou aminométhyle.

7. Composés selon revendication 6, caractérisés en ce que $R^6$, $R^7$ et $R^8$ représentent l'hydrogène et $R^{90}$ l'hydrogène ou un groupe hydroxy.

8. Composés selon l'une des revendications 1 à 5, caractérisés en ce que $R^1$ représente un groupe acyle de formule générale

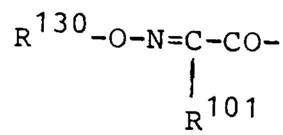

dans laquelle $R^{101}$ représente un noyau hétérocyclique à 5, 6 ou 7 chaînons et 1, 2, 3 ou 4 atomes d'azote, d'oxygène et/ou de soufre, éventuellement substitué par des halogènes, des groupes hydroxy, nitro, amino, cyano, trifluorométhyle, alkyle en C 1-C 4, alcoxy en C 1-C 4, et $R^{130}$ représente l'hydrogène, un groupe alkyle en C 1-C 8, cycloalkyle en C 3-C 7, carboxy-cycloalkyle en C 3-C 7 ou un groupe alkyle en C 1-C 8 portant un ou plusieurs substituants halogéno, cyano, nitro, amino, mercapto, alkylthio en C 1-C 8, un groupe aromatique tel que défini en référence à $R^{111}$ dans la revendication 5, carboxy (y compris à l'état de sel), alcanoylamino en C 1-C 7, alcoxy carbonyle en C 2-C 9, phénylméthoxycarbonyle, diphénylméthoxycarbonyle, hydroxy-(alcoxy en C 1-C 8)-phosphinyle, dihydroxyphosphinyle, hydroxy-(phénylméthoxy)phosphinyle et/ou di-(alcoxy en C 1-C 8)-phosphinyle.

9. Composés selon l'une des revendications 1 à 5 et 8, caractérisés en ce que $R^1$ représente un groupe acyle de formule générale

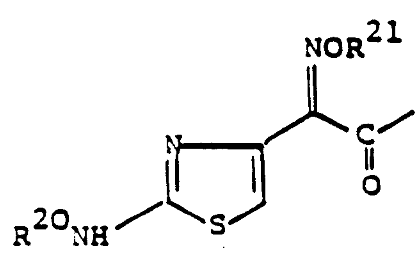

dans laquelle $R^{20}$ représente l'hydrogène ou un groupe protecteur du groupe amino et $R^{21}$ représente l'hydrogène, un groupe alkyle en C 1-C 8 ou un groupe de formule générale

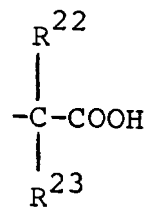

dans laquelle $R^{22}$ et $R^{23}$ représentent l'hydrogène, des groupes alkyle en C 1-C 8 ou bien forment ensemble et avec l'atome de carbone auquel ils sont reliés un noyau carbocyclique de 3 à 7 chaînons.

**10.** Composés selon revendication 9, caractérisés en ce que $R^{20}$ représente l'hydrogène et $R^{21}$ un groupe méthyle ou

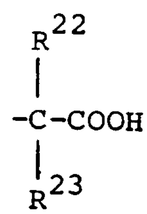

dans laquelle $R^{22}$ et $R^{23}$ représentent l'hydrogène ou un groupe méthyle.

**11.** Composés selon revendication 1, de formule générale

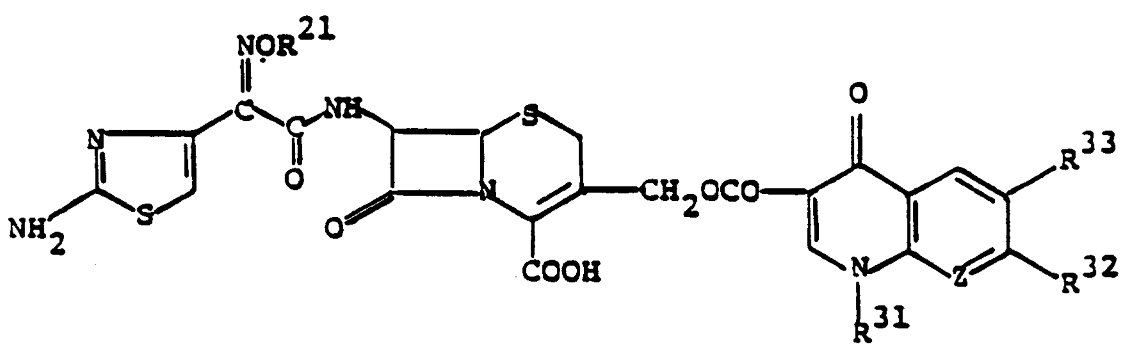

dans laquelle $R^{21}$ a les significations indiquées dans la revendication 9 et Z, $R^{31}$, $R^{32}$ et $R^{33}$ les significations indiquées dans la revendication 1.

**12.** Composés selon l'une des revendications 1 à 11, caractérisés en ce que $R^{21}$ représente un groupe méthyle ou un groupe de formule générale

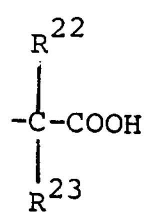

dans laquelle $R^{22}$ et $R^{23}$ représentent l'hydrogène ou un groupe méthyle, Z représente le groupe $R^{30}$-C, $R^{30}$ représente l'hydrogène ou un halogène, $R^{31}$ représente un groupe alkyle en C 1-C 8, halogénoalkyle en C 1-C 8 ou cycloalkyle en C 3-C 7, $R^{32}$ représente un groupe alkyle en C 1-C 8, pipérazinyle ou

(alkyle en C 1-C 8)-pipérazinyle et $R^{33}$ l'hydrogène ou un halogène.

**13.** Composés selon revendication 12, caractérisés en ce que $R^{30}$ représente l'hydrogène ou le fluor, $R^{31}$ un groupe éthyle, fluoréthyle ou cyclopropyle, $R^{32}$ représente un groupe méthyle, 4-méthylpipérazinyle ou pipérazinyle et $R^{33}$ l'hydrogène ou le fluor.

**14.** Composés selon revendication 1, caractérisés en ce qu'ils consistent en acides [6R-[6-alpha,7-bêta(Z)]-]-7-[[(2-amino-4-thiazolyl)($R^{21}$-oxyimino)acétyl]amino]-3-[[[[6-$R^{33}$,8-$R^{30}$, 1-$R^{31}$-1,4-dihydro-7-(4-$R^{34}$-1-pipérazinyl) -4-oxo-3-quinoléinyl]carbonyl]oxy]méthyl]-8-oxo-5-thia-1-azabicyclo[4.2.0]octa-2-ène-2-carboxyliques, leurs sels et les hydrates de ces acides carboxyliques et leurs sels, pour lesquels $R^{33}$ et $R^{30}$ représentent l'hydrogène ou un halogène, $R^{31}$ représente l'hydrogène, un groupe alkyle en C 1-C 8, ou 2-halogéno-alkyle en C 1-C 8 et $R^{34}$ représente l'hydrogène ou un groupe alkyle en C 1-C 8 et $R^{21}$ représente l'hydrogène, un groupe alkyle en C 1-C 8 ou un groupe de formule générale

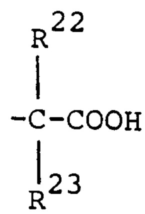

et $R^{22}$ et $R^{23}$ représentent l'hydrogène ou un groupe alkyle en C 1-C 8.

**15.** Composés selon revendication 14, caractérisés en ce que $R^{21}$ représente un groupe méthyle.

**16.** Composés selon revendication 15, caractérisés en ce que $R^{33}$ et $R^{30}$ représentent l'hydrogène ou le fluor, $R^{31}$ représente un groupe éthyle ou 2-fluoréthyle et $R^{34}$ un groupe méthyle.

**17.** Composés selon revendication 16, caractérisés en ce que $R^{33}$ et $R^{30}$ représentent le fluor et $R^{31}$ représente un groupe 2-fluoréthyle.

**18.** Composés selon revendication 15, caractérisés en ce que $R^{33}$ représente le fluor, $R^{30}$ l'hydrogène, $R^{31}$ un groupe 2-fluoréthyle et $R^{34}$ un groupe méthyle.

**19.** Composés selon revendication 15, caractérisés en ce que $R^{33}$ représente le fluor, $R^{30}$ l'hydrogène, $R^{31}$ un groupe éthyle et $R^{34}$ un groupe méthyle.

**20.** Composés selon revendication 15, caractérisés en ce que $R^{33}$ représente le fluor, $R^{31}$ un groupe 2-fluoréthyle, $R^{30}$ l'hydrogène et $R^{34}$ un groupe méthyle.

**21.** Composés selon l'une des revendications 8 à 20, caractérisés en ce que les groupes de formules générales

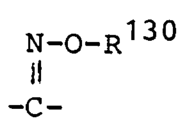

et

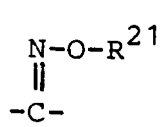

sont sous la forme isomère syn ou à l'état de mélanges dans lesquels cette forme est prédominante.

**22.** Composés selon revendication 1, caractérisés en ce qu'ils consistent en les acides [6R-[6-alpha, 7-bêta]]-3-[[[[6-$R^{33}$,8-$R^{30}$, 1-$R^{31}$-1,4-dihydro-7-(4-$R^{34}$-1-pipérazinyl)-4-oxo-3-quinoléinyl]carbonyl]oxy]-méthyl]-8-oxo-7-[(phénoxyacétyl)amino]-5-thia-1-azabicyclo [4.2.0]octa-2-ène-2-carboxyliques, leurs sels et les hydrates de ces acides carboxyliques et sels, pour lesquels $R^{33}$ et $R^{30}$ représentent l'hydrogene ou des halogènes, $R^{31}$ représente l'hydrogène ou un groupe alkyle en C 1-C 8 ou 2-halogénoalkyle en C 1-C 8 et $R^{34}$ représente l'hydrogène ou un groupe alkyle en C 1-C 8.

**23.** Composés selon revendication 22, caractérisés en ce que $R^{33}$ et $R^{30}$ représentent l'hydrogène ou le fluor, $R^{31}$ un groupe éthyle ou 2-fluoréthyle et $R^{34}$ un groupe méthyle.

**24.** Composés selon revendication 23, caractérisés en ce que $R^{33}$ et $R^{30}$ représentent le fluor et $R^{31}$ un groupe 2-fluoréthyle.

**25.** Composés selon revendication 22, caractérisés en ce que $R^{33}$ représente le fluor, $R^{30}$ l'hydrogène, $R^{31}$ un groupe 2-fluoréthyle et $R^{34}$ un groupe méthyle.

**26.** Composés selon revendication 22, caractérisés en ce que $R^{33}$ représente le fluor, $R^{30}$ l'hydrogène, $R^{31}$ un groupe éthyle et $R^{34}$ un groupe méthyle.

**27.** Composés selon revendication 22, caractérisés en ce que $R^{33}$ représente le fluor, $R^{31}$ un groupe 2-fluoréthyle, $R^{30}$ l'hydrogène et $R^{34}$ un groupe méthyle.

**28.** Composés selon revendication 1, caractérisés en ce qu'ils consistent en les acides [6R-[6-alpha, 7-bêta]]-7-(formylamino)-3-[[[[6-$R^{33}$,8-$R^{30}$, 1-$R^{31}$-1,4-dihydro-7-(4-$R^{34}$-1-pipérazinyl)-4-oxo-3-quinoléinyl] carbonyl]oxy]méthyl]-8-oxo-5-thia-1-aza-bicyclo[4.2.0] octa-2-ène-2-carboxyliques, leurs sels, les hydrates de ces acides carboxyliques et sels, pour lesquels $R^{33}$ et $R^{30}$ représentent l'hydrogène ou des halogènes, $R^{31}$ représente l'hydrogène, un groupe alkyle en C 1-C 8 ou 2-halogénoalkyle en C 1-C 8 et $R^{34}$ représente l'hydrogène ou un groupe alkyle en C 1-C 8.

**29.** Composés selon revendication 28, caractérisés en ce que $R^{33}$ et $R^{30}$ représentent l'hydrogène ou le fluor, $R^{31}$ un groupe éthyle ou 2-fluoréthyle et $R^{34}$ un groupe méthyle.

**30.** Composés selon revendication 29, caractérisés en ce que $R^{33}$ et $R^{30}$ représentent le fluor et $R^{31}$ un groupe 2-fluoréthyle.

**31.** Composés selon revendication 28, caractérisés en ce que $R^{33}$ représente le fluor, $R^{30}$ l'hydrogène, $R^{31}$ un groupe 2-fluoréthyle et $R^{34}$ un groupe méthyle.

**32.** Composés selon revendication 28, caractérisés en ce que $R^{33}$ représente le fluor, $R^{30}$ l'hydrogène, $R^{31}$ un groupe éthyle et $R^{34}$ un groupe méthyle.

**33.** Composés selon revendication 28, caractérisés en ce que $R^{33}$ représente le fluor, $R^{31}$ un groupe 2-fluoréthyle, $R^{30}$ l'hydrogène et $R^{34}$ un groupe méthyle.

**34.** Composés selon revendication 1, caractérisés en ce qu'ils consistent en les acides [6R-[6-alpha, 7-bêta(Z)]]-7-[[(2-amino-4-thiazolyl)($R^{21}$-oxyimino)acétyl] amino]-3-[[[[6-$R^{33}$,8-$R^{30}$, 1-$R^{31}$-1,4-dihydro-7-(4-$R^{34}$-1-pipérazinyl)-4-oxo-3-quinoléinyl]carbonyl]oxy]méthyl]-8-oxo-5-thia-1-azabicyclo[4.2.0]octa-2-ène-carboxyliques, leurs sels et les hydrates de ces acides carboxyliques et sels, pour lesquels $R^{33}$ et $R^{30}$ représentent l'hydrogène ou des halogènes, $R^{31}$ représente l'hydrogène, un groupe alkyle en C 1-C 8 ou 2-halogénoalkyle en C 1-C 8, $R^{34}$ représente l'hydrogène ou un groupe alkyle en C 1-C 8, $R^{21}$ le groupe

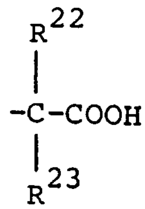

et $R^{22}$ et $R^{23}$ représentent l'hydrogène ou un groupe alkyle en C 1-C 8.

**35.** Composés selon revendication 34, caractérisés en ce que $R^{22}$ et $R^{23}$ représentent un groupe méthyle.

**36.** Composés selon revendication 35, caractérisés en ce que $R^{33}$ et $R^{30}$ représentent l'hydrogène ou le fluor, $R^{31}$ un groupe éthyle ou 2-fluoréthyle et $R^{34}$ un groupe méthyle.

**37.** Composés selon revendication 36, caractérisés en ce que $R^{33}$ et $R^{30}$ représentent le fluor et $R^{31}$ un groupe 2-fluoréthyle.

**38.** Composés selon revendication 35, caractérisés en ce que $R^{33}$ représente le fluor, $R^{30}$ l'hydrogène, $R^{31}$ un groupe 2-fluoréthyle et $R^{34}$ un groupe méthyle.

**39.** Composés selon revendication 35, caractérisés en ce que $R^{33}$ représente le fluor, $R^{30}$ l'hydrogène, $R^{31}$ un groupe éthyle et $R^{34}$ un groupe méthyle.

**40.** Composés selon revendication 35, caractérisés en ce que $R^{33}$ représente le fluor, $R^{31}$ un groupe 2-fluoréthyle, $R^{30}$ l'hydrogène et $R^{34}$ un groupe méthyle.

**41.** Composés selon revendication 1,
l'acide [6R-(6α,7β)]-3-[[[(1-éthyl-1,4-dihydro-7-méthyl-4-oxo-1,8-naphtiridine-3-yl)carbonyl]oxy]méthyl]-8-oxo-7-[(phénoxyacétyl)amino]-5-thia-1-azabicyclo[4.2.0]octa-2-ène-2-carboxylique, les sels de ce composé et les hydrates de ce composé et de ses sels.

**42.** Composés selon revendication 1,
l'acide [6R-(6α,7β)]-3-[[[(5-éthyl-5,8-dihydro-8-oxo-1,3-dioxolo[4,5-g]quinoléine-7-yl)carbonyl]oxy]-méthyl]-8-oxo-7-[(phénoxyacétyl]amino]-5-thia-1-azabicyclo[4.2.0]octa-ène-2-carboxylique, les sels de ce composé et les hydrates de ce composé et de ses sels.

**43.** Composés selon revendication 1,
l'acide [6R-(6α,7β)(Z)]-7-[[(2-amino-4-thiazolyl)-(méthoxyimino)acétyl]amino]-3-[[[(1-éthyl-1,4-dihydro-7-méthyl-4-oxo-1,8-naphtiridine-3-yl)carbonyl]oxy]méthyl-8-oxo-5-thia-1-azabicyclo[4.2.0]octa-2-ène-2-carboxylique, les sels de ce composé et les hydrates de ce composé et de ses sels.

**44.** Composés selon revendication 1,
l'acide [6R-(6α,7β)(Z)]-7-[[(2-amino-4-thiazolyl)-(méthoxyimino)acétyl]amino]-3-[[[(5-éthyl-5,8-dihydro-8-oxo-1,3-dioxolo[4,5-g/quinoléine-7-yl)carbonyl]oxy] méthyl]-8-oxo-5-thia-1-azabicyclo[4.2.0]octa-2-ène-2-carboxylique, les sels de ce composé et les hydrates de ce composé et de ses sels.

**45.** Composés selon revendication 1,
l'acide [6R-(6α,7β)]-3-[[[[1-éthyl-6-fluoro-1,4-dihydro-7-(4-formyl-1-pipérazinyl)-4-oxoquinoléine-3-yl]-carbonyl]oxy]méthyl-8-oxo-7-[(phénoxyacétyl)amino]-5-thia-1-aza-bicyclo[4.2.0]octa-2-ène-2-carboxylique, les sels de ce composé et les hydrates de ce composé et de ses sels.

**46.** Composés selon revendication 1,
l'acide [6R-(6α,7β)]-3-[[[(5-éthyl-5,8-dihydro-8-oxo-1,3-dioxolo[4,5-g]quinoléine-7-yl)carbonyl]oxy]-méthyl]-7-[(2-thiénylacétyl)amino]-8-oxo-5-thia-1-azabicyclo[4.2.0]octa-2-ène-2-carboxylique, les sels de ce composé et les hydrates de ce composé et de ses sels.

**47.** Composés selon revendication 1, l'acide [6R-(6α,7β)]-3-[[[[1-éthyl-6-fluoro-1,4-dihydro-7-(4-formyl-1-pipérazinyl)-4-oxoquinoléine-3-yl]-carbonyl] oxy]méthyl]-7-[(2-thiénylacétyl)amino]-8-oxo-5-thia-1-azabicyclo[4.2.0]octa-2-ène-2-carboxylique, les sels de ce composé et les hydrates de ce composé et de ses sels.

**48.** Composés selon revendication 1, l'acide [6R-(6α,7β)]-3-[[[[1-éthyl-6-fluoro-1,4-dihydro-7-(1-pyrrolidinyl)-4-oxoquinoléine-3-yl]carbonyl]-oxy] méthyl]-8-oxo-7-[(phénoxyacétyl)amino]-5-thia-1-azabicyclo[4.2.0]octa-2-ène-2-carboxylique, les sels de ce composé et les hydrates de ce composé et de ses sels.

**49.** Composés selon revendication 1, l'acide [6R-(6α,7β)(Z)]-7-[[(2-amino-4-thiazolyl)-(méthoxyimino)acétyl]amino]-3-[[[[1-éthyl-6-fluoro-1,4-dihydro-7-(1-pyrrolidinyl)-4-oxoquinoléine-3-yl]carbonyl] oxy]méthyl]-8-oxo-5-thia-1-azabicyclo[4.2.0]-octa-2-ène-2-carboxylique, les sels de ce composé et les hydrates de ce composé et de ses sels.

**50.** Composés selon revendication 1, l'acide [6R-(6α,7β)(Z)]-7-[[(2-amino-4-thiazolyl)-(méthoxyimino)acétyl]amino]-3-[[[[1-éthyl-6-fluoro-1,4-dihydro-7-(4-formyl-1-pipérazinyl)-4-oxoquinoléine-3-yl] carbonyl]oxy]méthyl]-8-oxo-5-thia-1-azabicyclo-[4.2.0] octa-2-ène-2-carboxylique, les sels de ce composé et les hydrates de ce composé et de ses sels.

**51.** Composés selon revendication 1, l'acide [6R-(6α,7β)]-3-[[[[1-éthyl-6-fluoro-1,4-dihydro-7-(4-thiomorpholinyl)-4-oxoquinoléine-3-yl]-carbonyl]oxy] méthyl]-8-oxo-7-[(phénoxyacétyl)amino]-5-thia-1-azabicyclo[4.2.0]octa-2-ène-2-carboxylique, les sels de ce composé et les hydrates de ce composé et de ses sels.

**52.** Composés selon revendication 1, l'acide [6R-(6α,7β)]-3-[[[(1-éthyl-6-fluoro-1,4-dihydro-4-oxo-7-(1H-pyrrol-1-yl)quinoléine-3-yl]carbonyl]-oxy] méthyl]-8-oxo-7-[(phénoxyacétyl)amino]-5-thia-1-azabicyclo[4.2.0]octa-2-ène-2-carboxylique, les sels de ce composé et les hydrates de ce composé et de ses sels.

**53.** Composés selon revendication 1, l'acide [6R-(6α,7β)]-3-[[[[5-(4-fluorophényl)-5,8-dihydro-8-oxo-1,3-dioxolo[4.5-g]quinoléine-7-yl]carbonyl] oxy]méthyl]-8-oxo-7-[(phénoxyacétyl)amino]-5-thia-1-azabicyclo[4.2.0]octa-2-ène-2-carboxylique, les sels de ce composé et les hydrates de ce composé et de ses sels.

**54.** Composés selon revendication 1, l'acide [6R-(6α,7β)(Z)]-7-[[(2-amino-4-thiazolyl)-(méthoxyimino)acétyl]amino]-3-[[[[1-éthyl-6-fluoro-1,4-dihydro-7-(4-thiomorpholinyl)-4-oxo-quinoléine-3-yl]carbonyl]oxy]méthyl]-8-oxo-5-thia-1-azabicyclo-[4.2.0] octa-2-ène-2-carboxylique, les sels de ce composé et les hydrates de ce composé et de ses sels.

**55.** Composés selon revendication 1, l'acide [6R-(6α,7β(Z)]-7-[[(2-amino-4-thiazolyl-(méthoxyimino)acétyl]amino]-3-[[[[6,8-difluoro-1-(2-fluoroéthyl)-1,4-dihydro-7-(4-méthyl-1-pipérazinyl)-4-oxo-3-quinoléinyl]carbonyl]oxy]méthyl]-8-oxo-5-thia-1-azabicyclo[4.2.0]octa-2-ène-2-carboxylique, les sels de ce composé et les hydrates de ce composé et de ses sels.

**56.** Composés selon revendication 1, l'acide [6R-[6α,7β(Z)]]-3-[[[[7-(3-amino-1-pyrrolidinyl)-8-chloro-1-cyclopropyl-6-fluoro-1,4-dihydro-4-oxoquinoléine-3-yl]carbonyl]oxy]méthyl]-7-[[(2-amino-4-thiazolyl)[(carboxyméthoxy)-imino]acétyl]amino]-8-oxo-5-thia-1-azabicyclo[4.2.0]octa-2-ène-2-carboxylique, les sels de ce composé et les hydrates de ce composé et de ses sels.

**57.** Composés selon revendication 1, l'acide [6R-[6α,7β(Z)]]-3-[[[[7-(3-amino-1-pyrrolidinyl)-8-chloro-1-cyclopropyl-6-fluoro-1,4-dihydro-4-oxoquinoléine-3-yl]carbonyl]oxy]méthyl]-7-[[(2-amino-4-thiazolyl)[(1-carboxy-1-méthyléthoxy)imino]-acétyl]amino]-8-oxo-5-thia-1-azabicyclo-[4.2.0]octa-2-ène-2-carboxylique, les sels de ce composé et les

hydrates de ce composé et de ses sels.

**58.** Composés selon revendication 1, l'acide [6R-[6α,7β(Z)]]-3-[[[[7-(3-amino-1-pyrrolidinyl) 8-chloro-1-cyclopropyl-6-fluoro-1,4-dihydro-4-oxoquinoléine-3-yl]carbonyl]oxy]méthyl]-7-[[(2-amino-4-thiazolyl)-méthoxyimino)-acétyl]amino]-8-oxo-5-thia-1-azabicyclo[4.2.0]octa-2-ène-2-carboxylique, les sels de ce composé et les hydrates de ce composé et de ses sels.

**59.** Composés selon l'une des revendications 1 à 58, en tant que substances actives pharmaceutiques.

**60.** Composés selon l'une des revendications 1 à 58, en tant que substances actives pharmaceutiques pour le traitement et la prophylaxie de maladies infectieuses.

**61.** Compositions pharmaceutiques, caractérisées en ce qu'elles contiennent un composé selon l'une des revendications 1 à 58.

**62.** Compositions pharmaceutiques pour le traitement et la prophylaxie de maladies infectieuses, caractérisées en ce qu'elles contiennent un composé selon l'une des revendications 1 à 58.

**63.** Procédé de préparation des composés selon l'une des revendications 1 à 58, caractérisé en ce que :

a) pour la préparation d'un ester facile à hydrolyser d'un acide carboxylique de formule I, on fait réagir un composé de formule générale

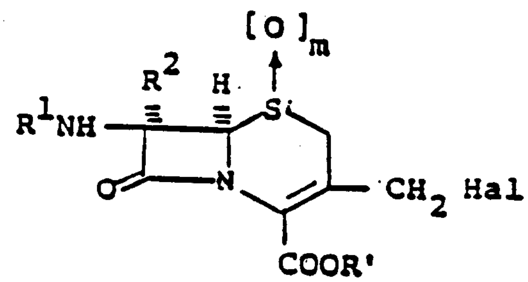

II

dans laquelle m, $R^1$ et $R^2$ ont les significations indiquées dans la revendication 1, Hal représente un halogène et R' le radical d'un ester facile à hydrolyser,
avec un sel d'un acide carboxylique de formule générale

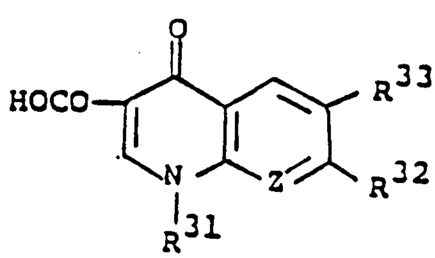

III

dans laquelle $R^{31}$, $R^{32}$ et $R^{33}$ ont les significations indiquées dans la revendication 1, ou bien

b) pour préparer un acide carboxylique de formule I, on convertit un ester de formule générale

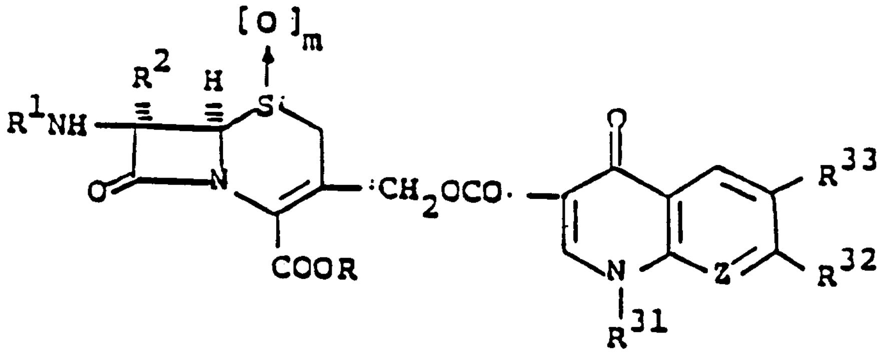

IV

dans laquelle m, $R^1$, $R^2$, $R^{31}$, $R^{32}$ et $R^{33}$ ont les significations indiquées dans la revendication 1, et R représente un groupe protecteur du groupe ester,
en l'acide carboxylique de formule I, ou bien
c) pour préparer un composé de formule I dans laquelle m = 0, on réduit un composé de formule générale

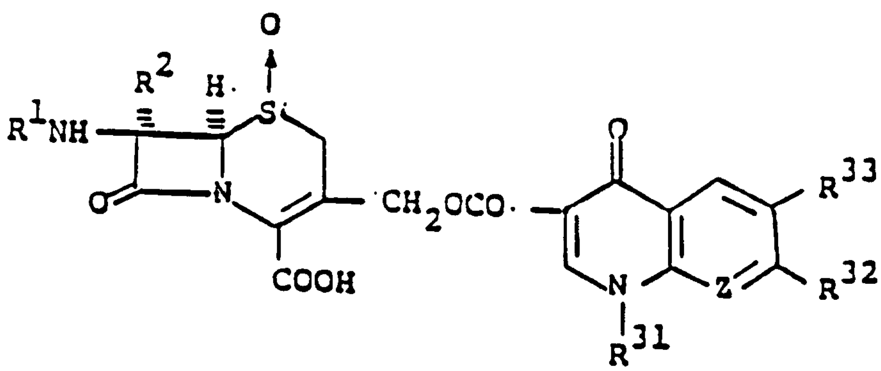

V

dans laquelle $R^1$, $R^2$, $R^{31}$, $R^{32}$ et $R^{33}$ ont les significations indiquées dans la revendication 1, ou bien
d) pour préparer un composé de formule I dans laquelle m = 1 ou 2, ou l'un de ses esters ou sels, on oxyde un composé de formule générale

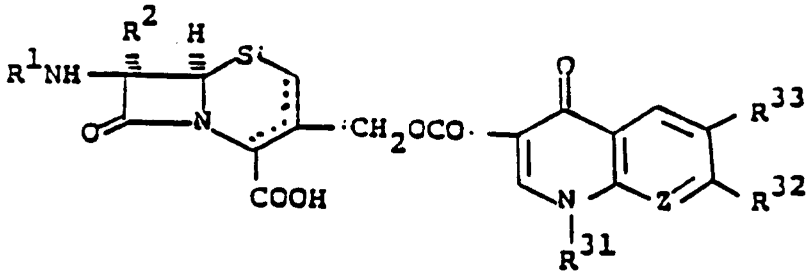

VI

dans laquelle $R^1$, $R^2$, $R^{31}$, $R^{32}$ et $R^{33}$ ont les significations indiquées dans la revendication 1, et le trait interrompu indique la présence d'une double liaison en Δ2 ou Δ3,
ou l'un de ses esters ou sels, ou bien

e) pour préparer un composé de formule I dans laquelle $R^1$ représente le groupe

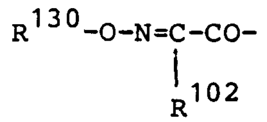

et $R^{102}$ un hétérocycle à 5, 6 ou 7 chaînons et 1, 2, 3 ou 4 atomes d'azote, d'oxygène et/ou de soufre, portant un substituant amino, et $R^{130}$ a les significations indiquées dans la revendication 8, ou l'un de ses esters ou sels, on élimine le groupe protecteur du groupe amino dans le substituant $R^{103}$ d'un composé de formule générale

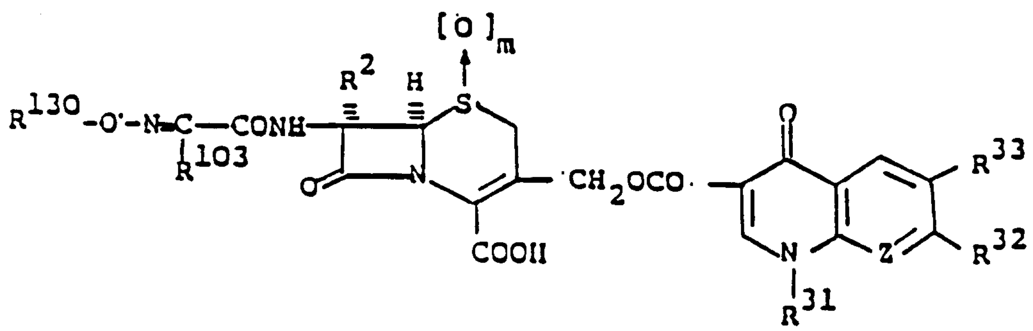

VII

dans laquelle m, $R^2$, $R^{31}$, $R^{32}$ et $R^{33}$ ont les significations indiquées dans la revendication 1, $R^{130}$ les significations indiquées dans la revendication 8 et $R^{103}$ les significations indiquées pour $R^{102}$, mais avec le substituant amino protégé, ou d'un de ses esters ou sels, ou bien

f) pour préparer un ester facile à hydrolyser d'un composé de formule I, on soumet un acide carboxylique de formule I à estérification, ou bien

g) pour préparer les sels ou hydrates d'un composé de formule I ou les hydrates des sels d'un composé de formule I, on convertit un composé de formule I en un sel ou hydrate ou en un hydrate du sel en question.

**64.** Utilisation des composés selon l'une des revendications 1 à 58 pour la préparation de médicaments pour le traitement ou la prophylaxie de maladies infectieuses.

**Revendications pour les Etats contractants suivants : AT, ES, GR**

**1.** Procédé de préparation de dérivés acylés de formule générale

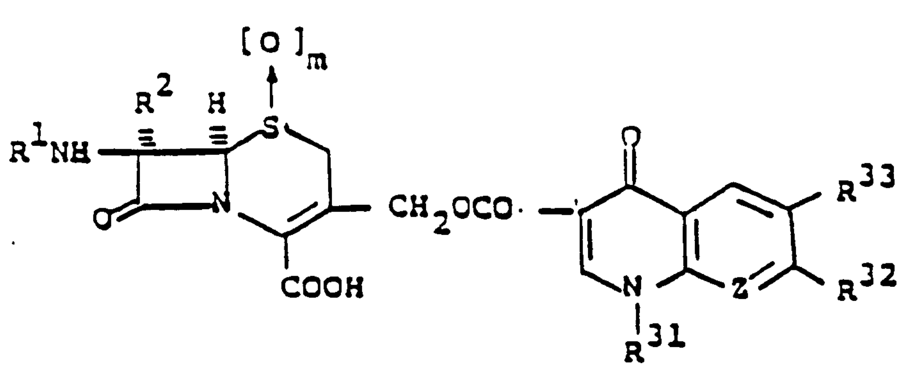

dans laquelle m est égal à 0, 1 ou 2, $R^1$ représente l'hydrogène ou un groupe acyle, $R^2$ représente l'hydrogène, un groupe alcoxy en C 1-C 8, amino, alkylthio en C 1-C 8 ou alcanoylamino en C 1-C 7, $R^{31}$ représente l'hydrogène, un groupe alkyle en C 1-C 8, alcényle en C 2-C 6, cycloalkyle en C 3-C 7, halogénoalkyle en C 1-C 8 ou halogénophényle; Z représente $R^{30}$-C ou l'azote, $R^{30}$ représente l'hydrogène ou un halogène ou bien $R^{30}$ et $R^{31}$ forment ensemble un groupe alkylène en C 3-C 5, un

groupe (alkyle en C 2-C 4)-monooxy ou (alkylène en C 1-C 2)-dioxy,
$R^{32}$ représente l'hydrogène, un halogène, un groupe alkyle en C 1-C 8 ou un hétérocycle à 5 ou 6 chaînons contenant 1, 2 ou 3 atomes d'oxygène, d'azote et/ou de soufre et éventuellement substitué par des groupes alkyle en C 1-C 8, amino, mercapto, hydroxy, carbamoyle ou carboxy, et $R^{33}$ représente l'hydrogène ou un halogène ou bien $R^{32}$ et $R^{33}$ forment ensemble un groupe (alkylène en C 1-C 4)-dioxy,
de leurs esters faciles à hydrolyser, des sels de ces composés et des hydrates des composés de formule I, de leurs esters et de leurs sels, caractérisé en ce que :

a) pour préparer un ester facile à hydrolyser d'un acide carboxylique de formule I, on fait réagir un composé de formule générale

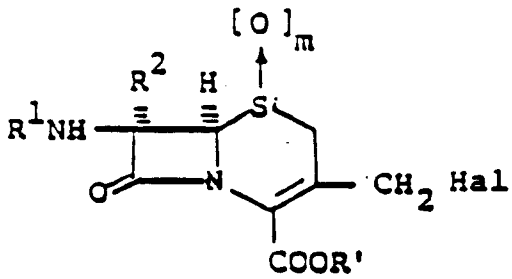

II

dans laquelle m, $R^1$ et $R^2$ ont les significations indiquées ci-dessus, Hal représente un halogène et R' le radical d'un ester facile à hydrolyser,
avec un sel d'un acide carboxylique de formule générale

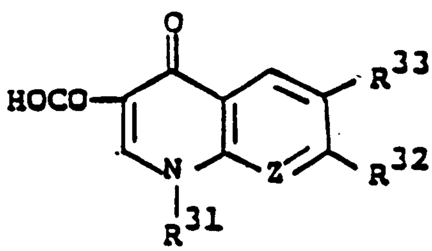

III

dans laquelle $R^{31}$, $R^{32}$ et $R^{33}$ ont les significations indiquées ci-dessus, ou bien

b) pour préparer un acide carboxylique de formule I, on convertit un ester de formule générale

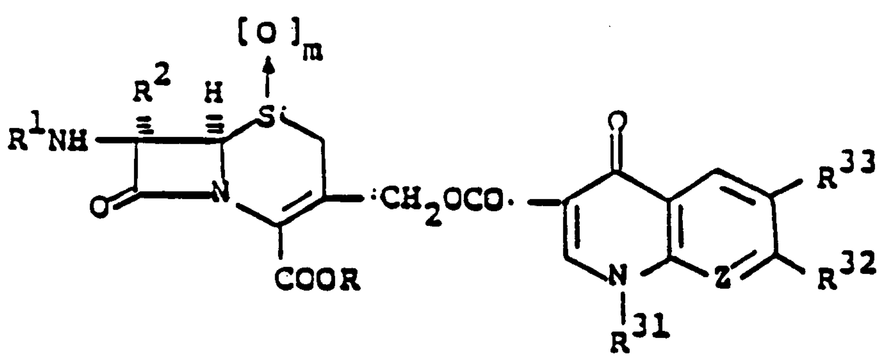

IV

dans laquelle m, $R^1$, $R^2$, $R^{31}$, $R^{32}$ et $R^{33}$ ont les significlations indiquées ci-dessus, et R représente un groupe protecteur du groupe ester,
en l'acide carboxylique de formule I, ou bien

c) pour préparer un composé de formule I dans laquelle m = 0, on réduit un composé de formule générale

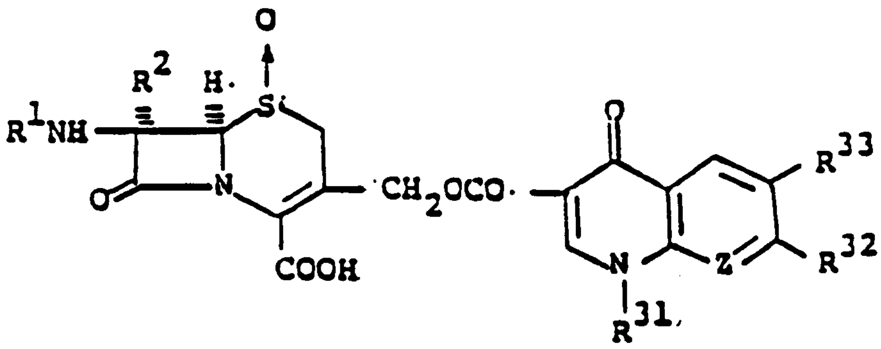

V

dans laquelle $R^1$, $R^2$, $R^{31}$, $R^{32}$ et $R^{33}$ ont les significations indiquées ci-dessus, ou bien

d) pour préparer un composé de formule I dans laquelle m est égal à 1 ou 2, ou un de ses esters ou sels, on oxyde un composé de formule générale

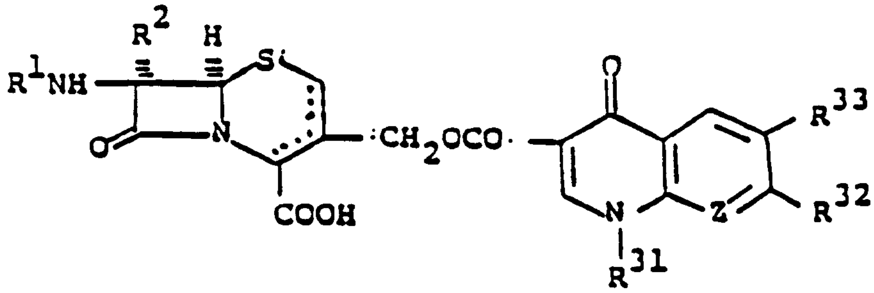

VI

dans laquelle $R^1$, $R^2$, $R^{31}$ $R^{32}$ et $R^{33}$ ont les significations indiquées ci-dessus, et le trait interrompu indique la présence d'une double liaison en Δ2 ou Δ3, un de ses esters ou sels, ou bien

e) pour préparer un composé de formule I dans laquelle $R^1$ représente le groupe

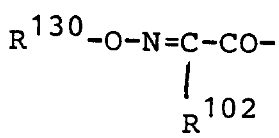

et $R^{102}$ un un noyau hétérocyclique à 5, 6 et 7 chaînons et 1, 2, 3 ou 4 atomes d'azote, d'oxygène et/ou de soufre, à substituant amino, et $R^{130}$ a les significations indiquées dans la revendication 8, ou l'un de ses esters ou sels, on élimine le groupe protecteur du groupe amino dans le substituant $R^{103}$ d'un composé de formule générale

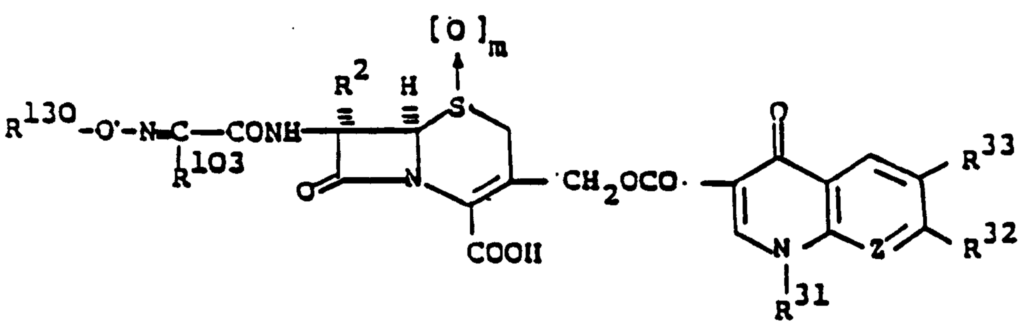

VII

dans laquelle m, $R^2$, $R^{31}$, $R^{32}$ et $R^{33}$ ont les significations indiquées ci-dessus, $R^{130}$ a les significations indiquées dans la revendication 8 et $R^{103}$ les significations indiquées pour $R^{102}$, mais avec le substituant amino protégé, ou d'un de ses esters ou sels, ou bien

f) pour préparer un ester facile à hydrolyser d'un composé de formule I, on soumet un acide carboxylique de formule I à l'estérification voulue, ou bien
g) pour préparer les sels ou hydrates d'un composé de formule I ou les hydrates de ses sels, on convertit un composé de formule I en un sel ou hydrate ou en un hydrate du sel en question.

**2.** Procédé de préparation des composés de formule I dans laquelle m = 0 et $R^2$ représente l'hydrogène, caractérisé en ce que l'on utilise des composés de départ portant les substituants correspondants.

**3.** Procédé de préparation des composés selon revendication 1 ou 2, pour lesquels Z représente le groupe Z $R^{30}$-C, $R^{30}$ représente l'hydrogène ou un halogène, $R^{31}$ un groupe alkyle en C 1-C 8, halogénoalkyle en C 1-C 8, ou cycloalkyle en C 3-C 7, $R^{32}$ représente un groupe alkyle en C 1-C 8, pipérazinyle ou (alkyle en C 1-C 8)-pipérazinyle et $R^{33}$ l'hydrogène ou un halogène, caractérisé en ce que l'on utilise des composés de départ portant les substituants correspondants.

**4.** Procédé de préparation des composés selon revendication 3 pour lesquels $R^{30}$ représente l'hydrogène ou le fluor, $R^{31}$ un groupe éthyle, fluoréthyle ou cyclopropyle, $R^{32}$ un groupe pipérazinyle ou 4-méthylpipérazinyle et $R^{33}$ l'hydrogène ou le fluor, caractérisé en ce que l'on utilise des composés de départ portant les substituants correspondants.

**5.** Procédé de préparation des composés selon une des revendications 1 à 4, pour lesquels $R^1$ représente l'un des groupes acyle suivants :
(a) un groupe aliphatique de formule

$R^5$-CO-

dans laquelle $R^5$ représente un groupe alkyle en C 1-C 8, cycloalkyle en C 3-C 7, alcoxy en C 1-C 8, alcényle en C 2-C 6, cycloalcényle en C 3-C 7 ou cyclohexadiényle, ou un groupe alkyle en C 1-C 8 ou alcényle en C 2-C 6 portant un ou plusieurs substituants halogéno, cyano, nitro, amino, mercapto, alkylthio en C 1-C 8 ou cyanométhylthio;
(b) un groupe aromatique carbocyclique répondant à l'une des formules générales

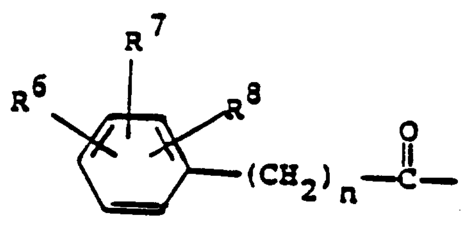

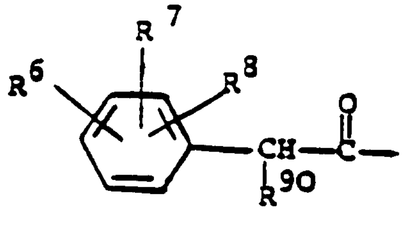

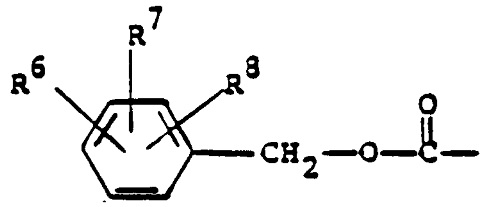

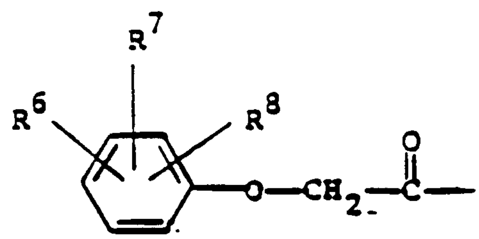

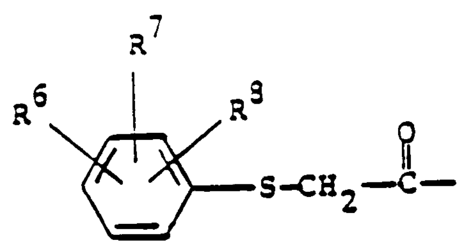

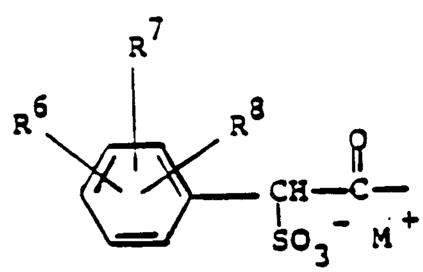

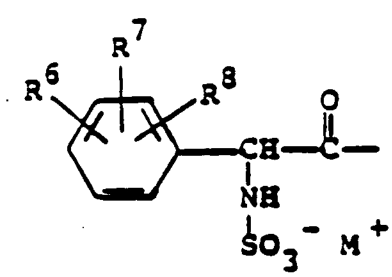

dans lesquelles n est égal à 0, 1, 2 ou 3; $R^6$, $R^7$ et $R^8$ représentent l'hydrogène, des halogènes, des groupes hydroxy, nitro, amino, cyano, trifluorométhyle, alkyle en C 1-C 4, alcoxy en C 1-C 4 ou aminométhyle, $R^{90}$ représente un groupe amino, hydroxy, un groupe carboxy à l'état de sel, un groupe carboxy protégé, un groupe formyloxy ou azido et M est un cation;
(c) un groupe hétéroaromatique répondant à l'une des formules générales

$R^{101}-(CH_2)_n-CO-$

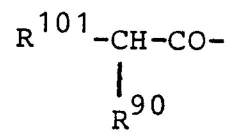

$R^{101}-O-CH_2-CO-$
$R^{101}-S-CH_2-CO-$
$R^{101}-CO-CO-$

dans lesquelles $R^{90}$ a les significations indiquées ci-dessus, n est égal à 0, 1, 2 ou 3 et $R^{101}$ représente un hétérocycle à 5, 6 ou 7 chaînons contenant 1, 2, 3 ou 4 atomes d'azote, d'oxygène et/ou de soufre et éventuellement substitué par des groupes alkyle en C 1-C 8, alcoxy en C 1-C 8, des halogènes, des groupes halogénoalkyle en C 1-C 8, amino, mercapto, hydroxy, carbamoyle ou carboxy;

(d) un groupe [[4-$R^{120}$-2,3-dioxo-1-pipérazinyl)-carbonyl]-amino]-arylacétyle de formule

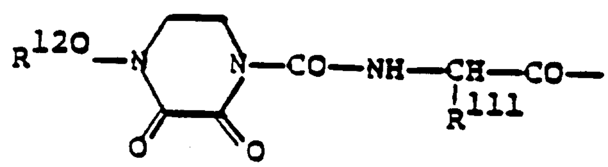

dans laquelle $R^{111}$ représente un groupe alkyle en C 1-C 8, hydroxyalkyle en C 1-C 8, ou un groupe aromatique de formule générale

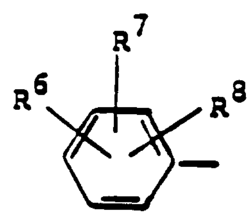

(dans laquelle $R^6$, $R^7$ et $R^8$ ont les significations indiquées ci-dessus), ou un noyau hétérocyclique tel que défini en référence à $R^{101}$, et $R^{120}$ représente un groupe alkyle en C 1-C 8 portant éventuellement un ou plusieurs substituants halogéno, cyano, nitro, amino et/ou mercapto;

(e) un groupe (oxyimino substitué)-arylacétyle de formule générale

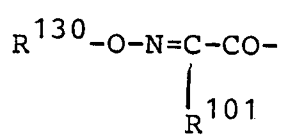

dans laquelle $R^{101}$ a les significations indiquées ci-dessus et $R^{130}$ représente l'hydrogène, un groupe alkyle en C 1-C 8, cycloalkyle en C 3-C 7, carboxy-cycloalkyle en C 3-C 7 ou alkyle en C 1-C 8 substitué, ce groupe alkyle en C 1-C 8 substitué portant un ou plusieurs substituants halogéno, cyano, nitro, amino, mercapto, alkylthio en C 1-C 8, un groupe aromatique tel que défini ci-dessus en référence à $R^{111}$, un groupe carboxy (y compris à l'état de sel), un groupe alcanoylamino en C 1-C 7, alcoxycarbonyle en C 2-C 9, phénylméthoxycarbonyle, diphénylméthoxycarbonyle, hydroxy-(alcoxy en C 1-C 8)-phosphinyle, dihydroxyphosphonyle, hydroxy-(phénylméthoxy)-phosphinyle et/ou di-(alcoxy en C 1-C 8)-phosphinyle;

(f) un groupe (acylamino)-arylacétyle de formule générale

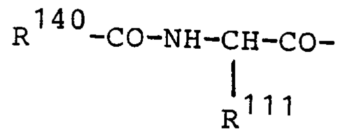

dans laquelle $R^{111}$ a les significations indiquées ci-dessus et $R^{140}$ représente un groupe de formule générale

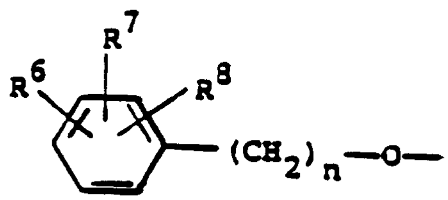

(dans laquelle $R^6$, $R^7$, $R^8$ et n ont les significations indiquées ci-dessus), l'hydrogène, un groupe alkyle en C 1-C 8 substitué, amino, alkylamino en C 1-C 8 ou cyanoalkylamino en C 1-C 8;

(g) un groupe [[[3-$R^{15}$-2-oxo-1-imidazolidinyl]-carbonyl]-amino]-arylacétyle de formule générale

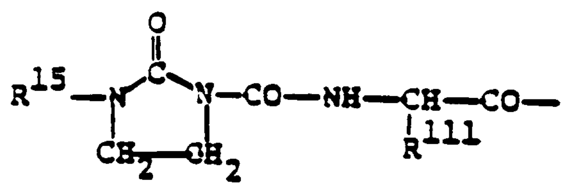

dans laquelle $R^{111}$ a les significations indiquées ci-dessus et $R^{15}$ représente l'hydrogène, un groupe alkylsulfonyle en C 1-C 8, -N=CH-$R^{111}$ (dans lequel $R^{111}$ a les significations indiquées ci-dessus), $R^{16}$-CO (dans lequel $R^{16}$ représente l'hydrogène ou un groupe alkyle en C 1-C 8 éventuellement substitué par des halogènes, un groupe aromatique tel que défini ci-dessus en référence à $R^{111}$ ou un groupe alkyle en C 1-C 8 portant éventuellement un ou plusieurs substituants halogéno, cyano, nitro, amino et/ou mercapto,

caractérisé en ce que l'on utilise des composés de départ portant les substituants correspondants.

6. Procédé de préparation des composés selon l'une des revendications 1 à 5 pour lesquels $R^1$ représente un groupe aromatique carbocyclique de formule générale

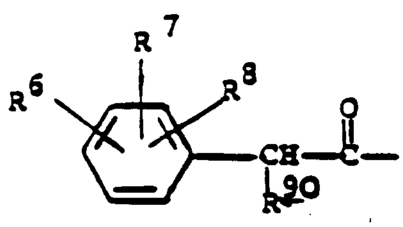

dans laquelle $R^{90}$ représente un groupe amino, hydroxy, carboxy à l'état de sel, benzyloxycarbonyle, formyloxy ou azido, et $R^6$, $R^7$ et $R^8$ représentent l'hydrogène, des halogènes, des groupes hydroxy, nitro, amino, cyano, trifluorométhyle, alkyle en C 1-C 4, alcoxy en C 1-C 4 ou aminométhyle, caractérisé en ce que l'on utilise les composés de départ portant les substituants correspondants.

7. Procédé de préparation des composés selon la revendication 6 pour lesquels $R^6$, $R^7$ et $R^8$ représentent l'hydrogène et $R^{90}$ l'hydrogène ou un groupe hydroxy, caractérisé en ce que l'on utilise des composés de départ portant les substituants correspondants.

**8.** Procédé de préparation des composés selon l'une des revendications 1 à 5 pour lesquels $R^1$ représente un groupe acyle de formule générale

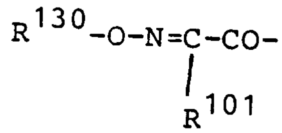

dans laquelle $R^{101}$ représente un noyau hétérocyclique à 5, 6 ou 7 chaînons et 1, 2, 3 ou 4 atomes d'azote, d'oxygène et/ou de soufre, éventuellement substitué par des halogènes, des groupes hydroxy, nitro, amino, cyano, trifluorométhyle, alkyle en C 1-C 4, alcoxy en C 1-C 4, et $R^{130}$ représente l'hydrogène, un groupe alkyle en C 1-C 8, cycloalkyle en C 3-C 7, carboxy-cycloalkyle en C 3-C 7 ou un groupe alkyle en C 1-C 8 portant un ou plusieurs substituants halogéno, cyano, nitro, amino, mercapto, alkylthio en C 1-C 8, un groupe aromatique tel que défini en référence à $R^{111}$ dans la revendication 5, carboxy (y compris à l'état de sel), alcanoylamino en C 1-C 7, alcoxy carbonyle en C 2-C 9, phénylméthoxycarbonyle, diphénylméthoxycarbonyle, hydroxy-(alcoxy en C 1-C 8)-phosphinyle, dihydroxyphosphinyle, hydroxy-(phénylméthoxy)-phosphinyle et/ou di-(alcoxy en C 1-C 8)-phosphinyle, caractérisé en ce que l'on utilise des composés portant les substituants correspondants .

**9.** Procédé de préparation des composés selon l'une des revendications 1 à 5 et 8, pour lesquels $R^1$ représente un groupe acyle de formule générale

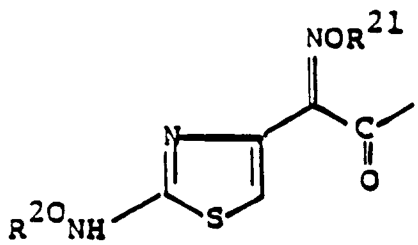

dans laquelle $R^{20}$ représente l'hydrogène ou un groupe protecteur du groupe amino et $R^{21}$ l'hydrogène, un groupe alkyle en C 1-C 8 ou un groupe de formule générale

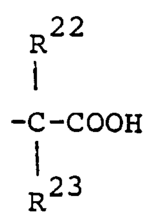

dans laquelle $R^{22}$ et $R^{23}$ représentent l'hydrogène, des groupes alkyle en C 1-C 8 ou forment ensemble et avec l'atome de carbone auquel ils sont reliés, un noyau carbocyclique de 3 à 7 chaînons,
caractérisé en ce que l'on utilise des composés de départ portant les substituants correspondants.

**10.** Procédé de préparation des composés selon la revendication 9 pour lesquels $R^{20}$ représente l'hydrogène et $R^{21}$ un groupe méthyle ou

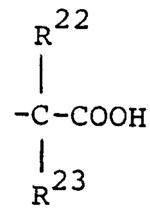

dans lequel $R^{22}$ et $R^{23}$ représentent l'hydrogène ou un groupe méthyle,
caractérisé en ce que l'on utilise des composés de départ portant les substituants correspondants.

**11.** Procédé de préparation des composés selon revendication 1, de formule générale

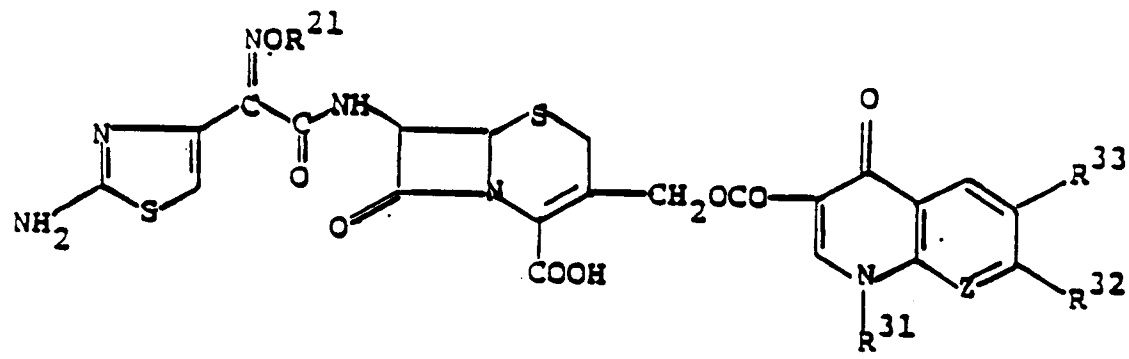

dans laquelle $R^{21}$ a les significations indiquées dans la revendication 9, et Z, $R^{31}$, $R^{32}$ et $R^{33}$ les significations indiquées dans la revendication 1,
caractérisé en ce que l'on utilise des composés de départ portant les substituants correspondants.

**12.** Procédé de préparation des composés selon l'une des revendications 1 à 11 pour lesquels $R^{21}$ représente un groupe méthyle ou un groupe de formule générale

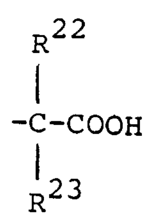

$R^{22}$ et $R^{23}$ représentent l'hydrogène ou des groupes méthyle, Z représente le groupe $R^{30}$-C, $R^{30}$ représente l'hydrogène ou un halogène, $R^{31}$ un groupe alkyle en C 1-C 8, halogénoalkyle en C 1-C 8 ou cycloalkyle en C 3-C 7, $R^{32}$ un groupe alkyle en C 1-C 8, pipérazinyle ou (alkyle en C 1-C 8)-pipérazinyle et $R^{33}$ l'hydrogène ou un halogène, caractérisé en ce que l'on utilise des composés de départ portant les substituants correspondants.

**13.** Procédé de préparation des composés selon la revendication 12 pour lesquels $R^{30}$ représente l'hydrogène ou le fluor, $R^{31}$ un groupe éthyle, fluoréthyle ou cyclopropyle, $R^{32}$ un groupe méthyle, 4-méthylpipérazinyle ou pipérazinyle et $R^{33}$ l'hydrogène ou le fluor, caractérisé en ce que l'on utilise des composés de départ portant les substituants correspondants.

**14.** Procédé de préparation des composés selon revendication 1, caractérisé en ce qu'ils consistent en les acides [6R-[6-alpha,7-bêta(Z)]]-7-[[(2-amino-4-thiazolyl)($R^{21}$-oxyimino)acétyl]amino]-3-[[[[6-$R^{33}$,8-$R^{30}$,1-$R^{31}$-1,4-dihydro-7-(4-$R^{34}$-1-pipérazinyl)-4-oxo-3-quinoléinyl] carbonyl]oxy]méthyl]-8-oxo-5-thia-1-

azabicyclo[4.2.0] octa-2-ène-2-carboxyliques, leurs sels et les hydrates de ces acides carboxyliques et leurs sels, pour lesquels $R^{33}$ et $R^{30}$ représentent l'hydrogène ou un halogène, $R^{31}$ représente l'hydrogène, un groupe alkyle en C 1-C 8, ou 2-halogéno-alkyle en C 1-C 8 et $R^{34}$ représente l'hydrogène ou un groupe alkyle en C 1-C 8 et $R^{21}$ représente l'hydrogène, un groupe alkyle en C 1-C 8 ou un groupe de formule générale

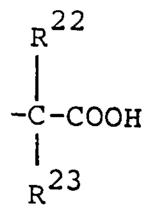

et $R^{22}$ et $R^{23}$ représentent l'hydrogène ou un groupe alkyle en C 1-C 8,
caractérisé en ce que l'on utilise des composés de départ portant les substituants correspondants;

**15.** Procédé de préparation des composés selon revendication 14 pour lesquels $R^{21}$ représente un groupe méthyle, caractérisé en ce que l'on utilise des composés de départ portant les substituants correspondants.

**16.** Procédé de préparation des composés selon revendication 15 pour lesquels $R^{33}$ et $R^{30}$ représentent l'hydrogène ou le fluor, $R^{31}$ un groupe éthyle ou 2-fluoréthyle et $R^{34}$ un groupe méthyle, caractérisé en ce que l'on utilise des composés de départ portant les substituants correspondants.

**17.** Procédé de préparation des composés selon revendication 16 pour lesquels $R^{33}$ et $R^{30}$ représentent le fluor et $R^{31}$ un groupe 2-fluoréthyle, caractérisé en ce que l'on utilise des composés de départ portant les substituants correspondants.

**18.** Procédé de préparation des composés selon revendication 15 pour lesquels $R^{33}$ représente le fluor, $R^{30}$ l'hydrogène, $R^{31}$ un groupe 2-fluoréthyle et $R^{34}$ un groupe méthyle, caractérisé en ce que l'on utilise des composés de départ portant les substituants correspondants.

**19.** Procédé de préparation des composés selon revendication 15 pour lesquels $R^{33}$ représente le fluor, $R^{30}$ l'hydrogène, $R^{31}$ un groupe éthyle et $R^{34}$ un groupe méthyle, caractérisé en ce que l'on utilise des composés de départ portant les substituants correspondants.

**20.** Procédé de préparation des composés selon revendication 15 pour lesquels $R^{33}$ représente le fluor, $R^{31}$ un groupe 2-fluoréthyle, $R^{30}$ l'hydrogène et $R^{34}$ un groupe méthyle, caractérisé en ce que l'on utilise des composés de départ portant les substituants correspondants.

**21.** Procédé de préparation des composés selon l'une des revendications 8 à 20 pour lesquels les groupes de formules générales

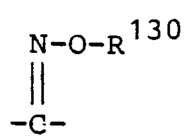

et

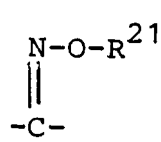

sont sous la forme isomère syn ou à l'état de mélanges dans lesquels cette forme est prépondérante, caractérisé en ce que l'on utilise des composés de départ portant les substituants correspondants.

**22.** Procédé de préparation des composés selon revendication 1, caractérisé en ce qu'ils consistent en les acides [6R-[6-alpha-7-bêta]]-3-[[[[6-$R^{33}$,8-$R^{30}$,1-$R^{31}$-1,4-dihydro-7-(4-$R^{34}$-1-pipérazinyl)-4-oxo-3-quinoléinyl]carbonyl]oxy]méthyl]-8-oxo-7-[(phénoxyacétyl) amino]-5-thia-1-azabicyclo[4.2.0]octa-2-ène-2-carboxyliques, leurs sels et les hydrates de ces acides carboxyliques et sels, pour lesquels $R^{33}$ et $R^{30}$ représentent l'hydrogène ou des halogènes, $R^{31}$ représente l'hydrogène ou un groupe alkyle en C 1-C 8 ou 2-halogénoalkyle en C 1-C 8 et $R^{34}$ représente l'hydrogène ou un groupe alkyle en C 1-C 8, caractérisé en ce que l'on utilise des composés de départ portant les substituants correspondants.

**23.** Procédé de préparation des composés selon revendication 22 pour lesquels $R^{33}$ et $R^{30}$ représentent l'hydrogène ou le fluor, $R^{31}$ un groupe éthyle ou 2-fluoréthyle et $R^{34}$ un groupe méthyle, caractérisé en ce que l'on utilise des composés de départ portant les substituants correspondants.

**24.** Procédé de préparation des composés selon revendication 23, pour lesquels $R^{33}$ et $R^{30}$ représentent le fluor et $R^{31}$ un groupe 2-fluoréthyle, caractérisé en ce que l'on utilise des composés de départ portant les substituants correspondants.

**25.** Procédé de préparation des composés selon revendication 22 pour lesquels $R^{33}$ représente le fluor, $R^{30}$ l'hydrogène, $R^{31}$ un groupe 2-fluoréthyle et $R^{34}$ un groupe méthyle, caractérisé en ce que l'on utilise des composés de départ portant les substituants correspondants.

**26.** Procédé de préparation des composés selon revendication 22 pour lesquels $R^{33}$ représente le fluor, $R^{30}$ l'hydrogène, $R^{31}$ un groupe éthyle et $R^{34}$ un groupe méthyle, caractérisé en ce que l'on utilise des composés de départ portant les substituants correspondants.

**27.** Procédé de préparation des composés selon revendication 22 pour lesquels $R^{33}$ représente le fluor, $R^{31}$ un groupe 2-fluoréthyle, $R^{30}$ l'hydrogène et $R^{34}$ un groupe méthyle, caractérisé en ce que l'on utilise des composés de départ portant les substituants correspondants.

**28.** Procédé de préparation des composés selon revendication 1 caractérisés en ce qu'ils consistent en les acides [6R-[6-alpha,7-bêta]]-7-(formylamino)-3-[[[[6-$R^{33}$,8-$R^{30}$, 1-$R^{31}$-1,4-dihydro-7-(4-$R^{34}$-1-pipérazinyl)-4-oxo-3-quinoléinyl]carbonyl]oxy]méthyl]-8-oxo-5-thia-1-aza-bicyclo[4.2.0]octa-2-ène-2-carboxyliques, leurs sels, les hydrates de ces acides carboxyliques et sels, pour lesquels $R^{33}$ et $R^{30}$ représentent l'hydrogène ou des halogènes, $R^{31}$ représente l'hydrogène, un groupe alkyle en C 1-C 8 ou 2-halogénoalkyle en C 1-C 8 et $R^{34}$ représente l'hydrogène ou un groupe alkyle en C 1-C 8, caractérisé en ce que l'on utilise des composés de départ portant les substituants correspondants.

**29.** Procédé de préparation des composés selon revendication 28 pour lesquels $R^{33}$ et $R^{30}$ représentent l'hydrogène ou le fluor, $R^{31}$ un groupe éthyle ou 2-fluoréthyle et $R^{34}$ un groupe méthyle, caractérisé en ce que l'on utilise des composés de départ portant les substituants correspondants.

**30.** Procédé de préparation des composés selon revendication 29 pour lesquels $R^{33}$ et $R^{30}$ représentent le fluor et $R^{31}$ un groupe 2-fluoréthyle, caractérisé en ce que l'on utilise des composés de départ portant les substituants correspondants.

**31.** Procédé de préparation des composés selon revendication 28 pour lesquels $R^{33}$ représente le fluor, $R^{30}$ l'hydrogène, $R^{31}$ un groupe 2-fluoréthyle et $R^{34}$ un groupe méthyle, caractérisé en ce que l'on utilise des composés de départ portant les substituants correspondants.

**32.** Procédé de préparation des composés selon revendication 28 pour lesquels $R^{33}$ représente le fluor, $R^{30}$ l'hydrogène, $R^{31}$ un groupe éthyle et $R^{34}$ un groupe méthyle, caractérisé en ce que l'on utilise des composés de départ portant les substituants correspondants .

**33.** Procédé de préparation des composés selon revendication 28 pour lesquels $R^{33}$ représente le fluor, $R^{31}$ un groupe 2-fluoréthyle, $R^{30}$ l'hydrogène et $R^{34}$ un groupe méthyle, caractérisé en ce que l'on utilise des composés de départ portant les substituants correspondants.

**34.** Procédé de préparation des composés selon revendication 1 caractérisés en ce qu'ils consistent en les acides [6R-[6-alpha,7-bêta(Z)]]-7-[[(2-amino-4-thiazolyl)($R^{21}$-oxyimino)acétyl]amino]-3-[[[[6-$R^{33}$,8-$R^{30}$,1-$R^{31}$-1,4-dihydro-7-(4-$R^{34}$-1-pipérazinyl)-4-oxo-3-quinoléinyl]carbonyl]oxy]méthyl]-8-oxo-5-thia-1-azabicyclo[4.2.0]octa-2-ène-carboxyliques, leurs sels et les hydrates de ces acides carboxyliques et sels, pour lesquels $R^{33}$ et $R^{30}$ représentent l'hydrogène ou des halogènes, $R^{31}$ représente l'hydrogène, un groupe alkyle en C 1-C 8 ou 2-halogénoalkyle en C 1-C 8, $R^{34}$ représente l'hydrogène ou -un groupe alkyle en C 1-C 8, $R^{21}$ le groupe

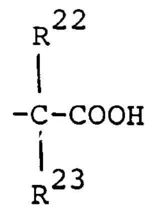

et $R^{22}$ et $R^{23}$ représentent l'hydrogène ou un groupe alkyle en C 1-C 8, caractérisé en ce que l'on utilise des composés de départ portant les substituants correspondants.

**35.** Procédé de préparation des composés selon revendication 34 pour lesquels $R^{22}$ et $R^{23}$ représentent des groupes méthyle, caractérisé en ce que l'on utilise des composés de départ portant les substituants correspondants.

**36.** Procédé de préparation des composés selon revendication 35 pour lesquels $R^{33}$ et $R^{30}$ représentent l'hydrogène ou le fluor, $R^{31}$ un groupe éthyle ou 2-fluoréthyle et $R^{34}$ un groupe méthyle, caractérisé en ce que l'on utilise des composés de départ portant les substituants correspondants.

**37.** Procédé de préparation des composés selon revendication 36 pour lesquels $R^{33}$ et $R^{30}$ représentent le fluor et $R^{31}$ un groupe 2-fluoréthyle, caractérisé en ce que l'on utilise des composés de départ portant les substituants correspondants.

**38.** Procédé de préparation des composés selon revendication 35 pour lesquels $R^{33}$ représente le fluor, $R^{30}$ l'hydrogène, $R^{31}$ un groupe 2-fluoréthyle et $R^{34}$ un groupe méthyle, caractérisé en ce que l'on utilise des composés de départ portant les substituants correspondants.

**39.** Procédé de préparation des composés selon revendication 35 pour lesquels $R^{33}$ représente le fluor, $R^{30}$ l'hydrogène, $R^{31}$ un groupe éthyle et $R^{34}$ un groupe méthyle, caractérisé en ce que l'on utilise des composés de départ portant les substituants correspondants.

**40.** Procédé de préparation des composés selon revendication 35 pour lesquels $R^{33}$ représente le fluor, $R^{31}$ un groupe 2-fluoréthyle, $R^{30}$ l'hydrogène et $R^{34}$ un groupe méthyle, caractérisé en ce que l'on utilise des composés de départ portant les substituants correspondants.

**41.** Procédé de préparation de composés selon revendication 1, l'acide [6R-(6α,7β)]-3-[[[(1-éthyl-1,4-dihydro-7-méthyl-4-oxo-1,8-naphtiridine-3-yl)carbonyl] oxy]méthyl]-8-oxo-7-[(phénoxyacétyl)amino]-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**42.** Procédé de préparation de composés selon revendication 1, l'acide [6R-(6α,7β)]-3-[[[(5-éthyl-5,8-dihydro-8-oxo-1,3-dioxolo[4,5-g]quinoléine-7-yl)carbonyl] oxy]méthyl]-8-oxo-7-[(phénoxyacétyl]amino]-5-thia-1-azabicyclo[4.2.0]octa-ène-2-carboxylique, les sels de ce composé et les hydrates de ce composé et de ses sels, caractérisé en ce que l'on utilise des composés de départ portant les substituants correspondants.

**43.** Procédé de préparation de composés selon revendication 1, l'acide [6R-(6α,7β)(Z)]-7-[[(2-amino-4-thiazolyl)-(méthoxyimino)acétyl]amino]-3-[[[(1-éthyl-1,4-dihydro-7-méthyl-4-oxo-1,8-naphtiridine-3-yl)-

carbonyl]oxy] méthyl-8-oxo-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**44.** Procédé de préparation de composés selon revendication 1, l'acide [6R-(6α,7β)(Z)]-7-[[(2-amino-4-thiazolyl)-(méthoxyimino)acétyl]amino]-3-[[[(5-éthyl-5,8-dihydro-8-oxo-1,3-dioxolo[4,5-g]quinoléine-7-yl)-carbonyl] oxy]méthyl]-8-oxo-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**45.** Procédé de préparation de composés selon revendication 1, l'acide [6R-(6α,7β)]-3-[[[[1-éthyl-6-fluoro-1,4-dihydro-7-(4-formyl-1-pipérazinyl)-4-oxoquinoléine-3-yl]-carbonyl]oxy]méthyl-8-oxo-7-[-(phénoxyacétyl) amino]-5-thia-1-aza-bicyclo[4.2.0]octa-2-ène-2-carboxylique, les sels de ce composé et les hydrates de ce composé et de ses sels, caractérisé en ce que l'on utilise des composés de départ portant les substituants correspondants.

**46.** Procédé de préparation de composés selon revendication 1, l'acide [6R-(6α,7β)]-3-[[[(5-éthyl-5,8-dihydro-8-oxo-1,3-dioxolo[4,5-g]quinoléine-7-yl)carbonyl] oxy]méthyl]-7-[(2-thiénylacétyl)amino]-8-oxo-5-thia-1-azabicyclo-[4.2.0]octa-2-ène-2-carboxylique, les sels de ce composé et les hydrates de ce composé et de ses sels, caractérisé en ce que l'on utilise des composés de départ portant les substituants correspondants.

**47.** Procédé de préparation de composés selon revendication 1, l'acide [6R-(6α,7β)]-3-[[[[1-éthyl-6-fluoro-1,4-dihydro-7-(4-formyl-1-pipérazinyl)-4-oxoquinoléine-3-yl]carbonyl]oxy]méthyl]-7-[(2-thiénylacétyl) amino]-8-oxo-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**48.** Procédé de préparation de composés selon revendication 1, l'acide [6R-(6α,7β)]-3-[[[[1-éthyl-6-fluoro-1,4-dihydro-7-(1-pyrrolidinyl)-4-oxoquinoléine-3-yl]carbonyl]oxy]méthyl]-8-oxo-7-[(phénoxyacétyl)amino]-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**49.** Procédé de préparation de composés selon revendication 1, l'acide [6R-(6α,7β)(Z)]-7-[[(2-amino-4-thiazolyl)-(méthoxyimino)acétyl]amino]-3-[[[[1-éthyl-6-fluoro-1,4-dihydro-7-(1-pyrrolidinyl)-4-oxoquinoléine-3-yl]carbonyl]oxy]méthyl]-8-oxo-5-thia-1-azabicyclo[4.2.0] octa-2-ène-2-carboxylique, les sels de ce composé et les hydrates de ce composé et de ses sels, caractérisé en ce que l'on utilise des composés de départ portant les substituants correspondants.

**50.** Procédé de préparation de composés selon revendication 1, l'acide [6R-(6α,7β)(Z)]-7-[[(2-amino-4-thiazolyl)-(méthoxyimino)acétyl]amino]-3-[[[[1-éthyl-6-fluoro-1,4-dihydro-7-(4-formyl-1-pipérazinyl)-4-oxoquinoléine-3-yl]carbonyl]oxy]méthyl]-8-oxo-5-thia-1-azabicyclo [4.2.0]octa-2-ène-2-carboxylique, les sels de ce composé et les hydrates de ce composé et de ses sels, caractérisé en ce que l'on utilise des composés de départ portant les substituants correspondants.

**51.** Procédé de préparation de composés selon revendication 1, l'acide [6R-(6α,7β)]-3-[[[[1-éthyl-6-fluoro-1,4-dihydro-7-(4-thiomorpholinyl)-4-oxoquinoléine-3-yl]carbonyl]oxy]méthyl]-8-oxo-7-[(phénoxyacétyl)-amino]-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**52.** Procédé de préparation de composés selon revendication 1, l'acide [6R-(6α,7β)]-3-[[[(1-éthyl-6-fluoro-1,4-dihydro-4-oxo-7-(1H-pyrrol-1-yl)quinoléine-3-yl]carbonyl]oxy]méthyl]-8-oxo-7-[(phénoxyacétyl)-amino]-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**53.** Procédé de préparation de composés selon revendication 1, l'acide [6R-(6α,7β)]-3-[[[[5-(4-fluorophényl)-5,8-dihydro-8-oxo-1,3-dioxolo[4,5-g]quinoléine-7-yl]carbonyl]oxy]méthyl]-8-oxo-7-[(phénoxyacétyl)-amino]-5-thia-1-aza-bicyclo[4.2.0]octa-2-ène-2-carboxylique, les sels de ce composé et les hydrates de ce composé et de ses sels, caractérisé en ce que l'on utilise des composés de départ portant les substituants correspondants.

**54.** Procédé de préparation de composés selon revendication 1, l'acide [6R-(6α,7β)(Z)]-7-[[(2-amino-4-thiazolyl)-(méthoxyimino)acétyl]amino]-3-[[[[1-éthyl-6-fluoro-1,4-dihydro-7-(4-thiomorpholinyl)-4-oxo-quinoléine-3-yl]carbonyl]oxy]méthyl]-8-oxo-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**55.** Procédé de préparation de composés selon revendication 1, l'acide [6R-(6α,7β)(Z)]]-7-[[(2-amino-4-thiazolyl-(méthoxyimino)acétyl]amino]-3-[[[[6,8-difluoro-1-(2-fluoro-éthyl)-1,4-dihydro-7-(4-méthyl-1-pipérazinyl)-4-oxo-3-quinoléinyl]carbonyl]oxy]méthyl]-8-oxo-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**56.** Procédé de préparation de composés selon revendication 1, l'acide [6R-[6α,7β(Z)]]-3-[[[[7-(3-amino-1-pyrrolidinyl)-8-chloro-1-cyclopropyl-6-fluoro-1,4-dihydro-4-oxoquinoléine-3-yl]carbonyl]oxy]méthyl]-7-[[-(2-amino-4-thiazolyl)[(carboxyméthoxy)-imino]acétyl] amino]-8-oxo-5-thia-1-azabicyclo[4.2.0]octa-2-ène-2-carboxylique, les sels de ce composé et les hydrates de cd composé et de ses sels, caractérisé en ce que l'on utilise des composés de départ portant les substituants correspondants.

**57.** Procédé de préparation de composés selon revendication 1, l'acide [6R-[6α,7β(Z)]]-3-[[[[7-(3-amino-1-pyrrolidinyl)-8-chloro-1-cyclopropyl-6-fluoro-1,4-dihydro-4-oxoquinoléine-3-yl]carbonyl]oxy]méthyl]-7-[[-(2-amino-4-thiazolyl)[(1-carboxy-1-méthyléthoxy)imino] acétyl]amino]-8-oxo-5-thia-1-azaoicyclo[4.2.0]-octa-2-ène-2-carboxylique, les sels de ce composé et les hydrates de ce composé et de ses sels, caractérisé en ce que l'on utilise des composés de départ portant les substituants correspondants.

**58.** Procédé de préparation de composés selon revendication 1, l'acide [6R-[6α,7β(Z)]]-3-[[[[7-(3-amino-1-pyrrolidinyl)-8-chloro-1-cyclopropyl-6-fluoro-1,4-dihydro-4-oxoquinoléine-3-yl]carbonyl]oxy]méthyl]-7-[[-(2-amino-4-thiazolyl)-méthoxyimino)-acétyl]amino]-8-oxo-5-thia-1-azabicyclo[4.2.0]octa-2-ène-2-carboxylique, les sels de ce composé et les hydrates de ce composé et de ses sels, caractérisé en ce que l'on utilise des composés de départ portant les substituants correspondants.

**59.** Procédé de préparation de compositions pharmaceutiques, caractérisé en ce que l'on mélange un dérivé acylé de formule I défini dans la revendication 1, ou un ester facile à hydrolyser ou un sel de ce composé ou un hydrate d'un composé de formule I ou d'un ester ou sel d'un tel composé, servant de composant actif, avec des véhicules ou excipients solides et liquides usuels, non toxiques, inertes, et convenant pour l'administration thérapeutique.

**60.** Utilisation des composés selon l'une des revendications 1 à 58 pour la préparation de médicaments pour le traitement et la prophylaxie de maladies infectieuses.